# EUROPEAN PATENT APPLICATION

(11) **EP 4 813 992 A1**
(43) Date of publication of application: **30.09.2026**
(21) Application number: 24896622.8
(22) Date of filing: 28.11.2024
(51) Int. Cl.: C07D 487/04, A61K 31/4985, A61P 35/00

(54) **POLYARYL DERIVATIVE AND USE THEREOF**

(30) Priority: 28.11.2023 CN 202311598628; 05.07.2024 CN 202410901880
(71) Applicant: Hangzhou HealZen Therapeutics Co., Ltd., Hangzhou, Zhejiang 310018 (CN)
(72) Inventor: ZHOU, Xinglu, Hangzhou, Zhejiang 310018 (CN); WU, Yizhe, Hangzhou, Zhejiang 310018 (CN); XIE, Jiangfeng, Hangzhou, Zhejiang 310018 (CN); HUANG, Jinglai, Hangzhou, Zhejiang 310018 (CN); HU, Miao, Hangzhou, Zhejiang 310018 (CN)
(74) Representative: Gong, Jinping
(86) International application number: PCT/CN2024/135116
(87) International publication number: WO 2025/113536

(57) **Abstract**

Disclosed is a polyaryl HPK1 degrader, a preparation method therefor, a pharmaceutical composition containing the same, and an application thereof in drugs. The polyaryl HPK1 degrader has a polyaryl derivative of general formula (I), or stereoisomers, tautomers, or pharmaceutically acceptable salts thereof, and a use as an HPK1 degrader. The compound of the invention has the ability to strongly inhibit the activity of HPK1 kinase. The compound of the invention has the ability to strongly degrade HPK1 protein. The compound of the invention exhibits excellent degradation selectivity. The compound of the invention has good exposure levels when administered orally.

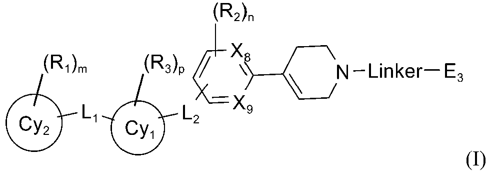

## Description

### FIELD OF THE INVENTION

The invention belongs to the technical field of drug synthesis and design, in particular, to a polyaryl HPK1 degrader, a composition containing derivatives thereof, and a preparation and a use thereof as an HPK1 degrader.

### BACKGROUND OF THE INVENTION

In recent years, the immune escape strategies utilizing own immune system of a patient to overcome tumor cells and enhancing the anti-tumor immunity of the body have become a novel cancer treatment strategy. One strategy is to recognize tumor antigens as non-self antigens by inhibiting negative regulators of the immune response that normally play a role in maintaining peripheral tolerance for thereby overcoming the immune escape of tumor cells. Hematopoietic progenitor cell kinase 1 (HPK1), also known as MAP4K1 (a member of the MAP4K family), is a negative regulator of the activation response of dendritic cells (DCs), T cells, and B cells. Inhibiting its activity can specifically enhance the anti-tumor immunity of the body. HPK1 is primarily expressed by hematopoietic cells, including early hematopoietic progenitor cells. In T cells, HPK1 is thought to negatively regulate the activation of T cells by phosphorylating the Ser376 site of the downstream SLP76 protein and the Thr254 site of the Gads protein and recruiting 14-3-3 protein to degrade these proteins for thereby reducing the persistence of signaling microclusters. HPK1 can also be activated in response to prostaglandins (PGE2) typically secreted by tumors, thereby helping tumor cells escape from the immune system. Targeted disruption of the allele of the HPK1 kinase can increase the production of Th1 cytokines (IL-2, IFNγ, etc.) by T cells in the TCR response. HPK1 plays multiple roles in the immune system and is associated with the pathogenesis of autoimmune diseases, cancer, and inflammatory responses. Proliferation of HPK1 kinase-/- T cells much faster than wild-type monocytogens, and mice transfected with HPK1 kinase-/- T cells are able to resist tumor growth. Furthermore, dendritic cells (DCs) that lack HPK1 kinase have better antigen presentation capabilities than wild-type cells and can better express anti-tumor immune responses. In addition, animal studies have shown that HPK1 inhibition and PD-1/PD-L1 antibody drugs have significant synergistic anti-tumor activity. Therefore, HPK1 kinase plays a crucial role in disease treatment, especially cancer treatment.

Significant progress has been made in proteolysis targeting chimeras (PROTAC) in recent years. This technology achieves the degradation of target proteins by inducing ubiquitination of target proteins. Drugs employing this technology do not need to continuously occupy the target protein; the catalytic concentration is sufficient to achieve target degradation, exhibiting high selectivity, high activity, and low toxicity.

Currently, there are no marketed inhibitors targeting HPK1 targets. In addition, HPK1 inhibitors have drawbacks such as weak immune activation effect, narrow effective window, and cytotoxicity at high concentrations. Therefore, further modification and optimization are necessary. To meet the enormous clinical needs of the future, HPK1 degraders with selectivity and high activity are designed and developed to provide novel drugs for the treatment of immune-related diseases, especially cancer, and to provide single or combined medications for immune-related diseases, especially cancer treatment, including chemotherapy, radiotherapy, targeted cancer drugs, other cancer immunotherapies (small molecule compounds and antibodies), cancer vaccines, and CAR-T immunotherapy.

### BRIEF SUMMARY OF THE DISCLOSURE

An objective of the invention is to provide a novel HPK1 degrader with selectivity and high activity that has not been reported in the literature, or stereoisomers, tautomers, or pharmaceutically acceptable salts thereof.

The invention relates to a degrader capable of degrading HPK1 protein, or stereoisomers, tautomers, or pharmaceutically acceptable salts thereof, and an application thereof in preparing drugs for preventing and/or treating diseases related to activities or expression levels of HPK1 protein, wherein the diseases include solid tumors, hematological disorders, or autoimmune diseases.

The invention provides a compound of general formula (I) or stereoisomers, tautomers or pharmaceutically acceptable salts thereof, wherein 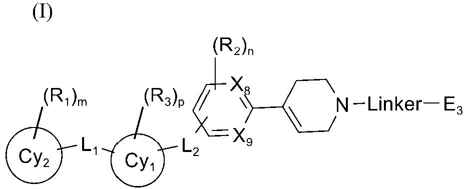
Cy₁ is selected from 5-12 membered heteroaryl or 8-10 membered fused bicyclic;
Cy₂ is selected from C₃-C₁₂ cycloalkyl, 3-12 membered heterocyclic, C₆-C₁₀ aryl, 5-12 membered heteroaryl or 8-10 fused bicyclic;
X₈ and X₉ are each independently selected from CRₐ or N;
L₁ and L₂ are each independently selected from chemical bonds, C₁-C₈ alkylene, C₃-C₁₂ cycloalkylene, 3-12 membered heterocyclic, -O- C₁-C₈ alkylene, - C₁-C₈ alkylene-O-, -NR_{b}- C₁-C₈ alkylene-, - C₁-C₈ alkylene-NR_{b}-, -O-, -NR_{b}C(O)-, - C(O)-NR_{b}-, -CO-, -SO-, -SO₂-, C₂-C₈ alkenyl or C₂-C₈ ynylene;
R₁ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, -POR_{b}R_{c} or -Linker-E3; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, C₁-C₈ haloalkyl, -C₁-C₈ alkyl-CONR_{b}R_{c}, -C₁-C₈ alkyl-NR_{b}COR_{c}, -C₁-C₈ alkyl-OR_{b}, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
or two R₁ and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
R₂ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
or two R₂ and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
R₃ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d} -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d} -NR_{b}SO₂NR_{c}R_{d}, -NR_{b}SO₂R_{c}, -SO(=NR_{b})R_{c} or -POR_{b}R_{c};
Linker is selected from -(CH₂)ᵣ-; one or more CH₂ be optionally substituted by one or more selected from -COO-, -CONR_{b}-, -OCONR_{b}-, -NR_{b}CONR_{b}-, -O-, -NR_{b}-, -S-, -CO-, -CR_{b}=CR_{b}-, -C≡C-, -CR_{b}CR_{c}-, -CR_{b}=N-, -SO-, - SO₂-, -POR_{b}-, C₃-C₁₀ cycloalkylene, 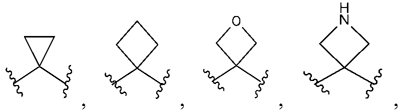 3-10 membered heterocycloalkylene, phenylene, 5-6 membered heteroarylene or -CR_{b}R_{c}-; the heterocycloalkylene group be further substituted by halogen or C₁-C₃ alkyl;
E₃ is selected from ligands for E₃ ligase;
Rₐ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂R_{b}, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂R_{b}, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
R_{b}, R_{c} and R_{d} are each independently selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, C₁-C₄ haloalkyl, -C₁-C₄ alkyl-CONRₑR_{f}, -C₁-C₃ alkyl-ORₑ, and -ORₑ or -NRₑR_{f}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, C₁-C₈ haloalkyl, -C₁-C₈ alkyl-CONRₑR_{f}, -C₁-C₈ alkyl-ORₑ, oxo, -CN, -NO₂, -ORₑ, -SO₂Rₑ, -SO₂NRₑR_{f}, -CORₑ, -COORₑ, -CONRₑR_{f}, -C(=NRₑ)NR_{f}R_{g}, -NRₑR_{f}, -NRₑCOR_{f}, -NRₑCONR_{f}R_{g}, -NRₑCO₂R_{f}, -NRₑSONR_{f}R_{g}, -NRₑSO₂NR_{f}R_{g}, -NRₑSO₂R_{f}, -SO(=NRₑ)R_{f} or -PORₑR_{f};
R_{c} and R_{b}, or R_{d} and R_{c}, substituted on the same atom, be linked together to form a ring; the ring be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, --CN, -NO₂, -ORₑ, -SO₂Rₑ, -SO₂NRₑR_{f}, -CORₑ, -COORₑ, -CONRₑR_{f}, -C(=NRₑ)NR_{f}R_{g}, -NRₑR_{f}, -NRₑCOR_{f}, -NRₑCONR_{f}R_{g}, -NRₑCO₂Rₑ, -NRₑSONR_{f}R_{g}, -NRₑSO₂NR_{f}R_{g}, -NRₑSO₂R_{f}, -SO(=NRₑ)R_{f} or -PORₑR_{f};
Rₑ, R_{f} and R_{g} are each independently selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, or 5-12 membered heteroaryl; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₃-C₁₂ cycloalkyl, 3-12 membered heterocyclic, C₆-C₁₀ aryl, 5-12 membered heteroaryl, cyano, hydroxyl, amino, or nitro;
m, n, o, and p are each independently selected from 0, 1, 2, 3, 4, 5 and 6;
r is selected from an integer from 0 to 20.
r is preferably selected from 0, 1, 2, 3, 4, 5, 6, 7, 8, 9, 10, 11, 12, 13, 14, 15, 16, 17, 18, 19 and 20.

In a preferred solution of the invention, a compound of general formula (I) or stereoisomers, tautomers or pharmaceutically acceptable salts thereof is provided,
wherein Cy₁ is selected from; 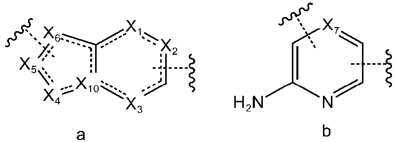 wherein
------- is a single bond or a double bond;
X₁, X₂, X₃, X₄, X₅, X₆ and X₁₀ are independently selected from O, S, C, CH, CH₂, N, NH, or CO, provided that the valence bond theory is satisfied;
X₇ is selected from CRₐ' or N;
Rₐ^{'} is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OH, -NH₂, or -O- C₁-C₈ alkyl.

In a preferred solution of the invention, a compound of general formula (IIa) or (IIb) or stereoisomers, tautomers or pharmaceutically acceptable salts thereof is provided,

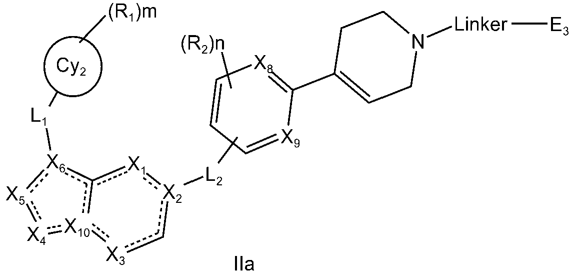

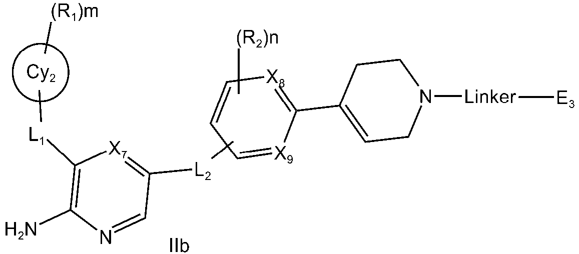

In a preferred solution of the invention, a compound of general formula (IIIa) or (IIIb) or stereoisomers, tautomers or pharmaceutically acceptable salts thereof is provided, 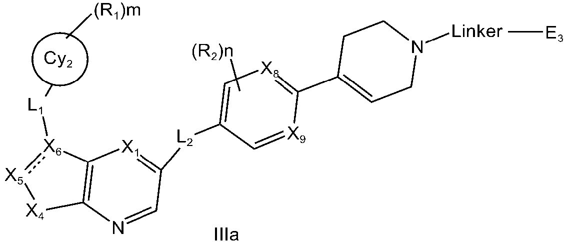 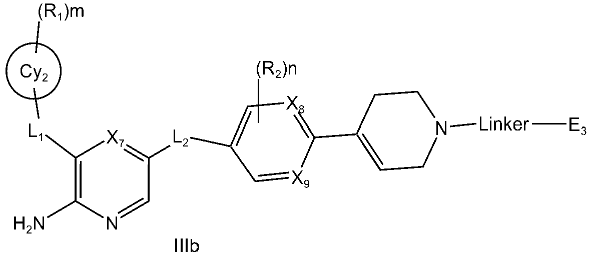 wherein
X₁ is selected from CH or N;
X₄ is selected from NH or S;
X₈ and X₉ are independently selected from CH, N or C-Me;
when -------- is selected from the double bond, X₆ is C, and X₅ be CH or N; when -------- is selected from the single bond, X₆ is N or CH, and X₅ is CO or CH₂;
L₁ and L₂ are each independently selected from chemical bonds, -O-, -O-C₁-C₃ alkylene, or -C₁-C₃ alkylene-O-;
Cy₂ is selected from 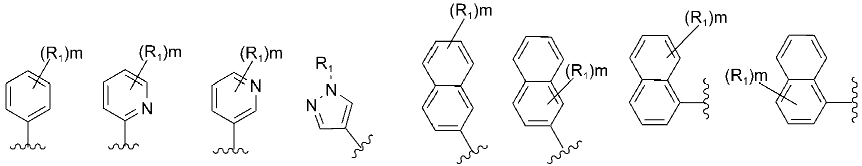 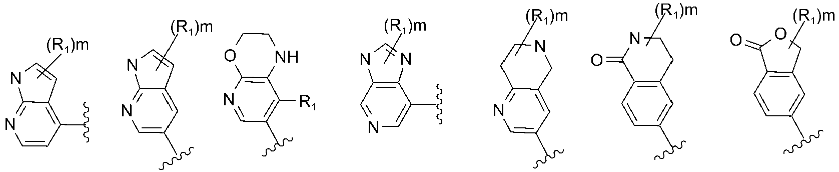
R₂ is selected from H, halogen, C₁-C₈ alkyl, C₂-C₈ alkenyl, C₂-C₈ alkynyl, C₃-C₁₂ cycloalkyl, 3-12 membered heterocyclic, -CN, -NO₂, -OR_{b}, -SO₂Rₐ, -SO₂NR_{b}R_{c}, -COR_{b}, -COOR_{b}, -CONR_{b}R_{c}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, or -POR_{b}R_{c}; the alkyl, the alkenyl, the alkynyl, the cycloalkyl, and the heterocyclic be further substituted by one or more substituents; the substituents are selected from halogen, oxo, -CN, -OR_{b} or -NR_{b}R_{c};
m is selected from 0, 1, 2, 3, 4, and 5;
n is selected from 0, 1, 2, and 3.

In a preferred solution of the invention, a compound of general formula (IV) or stereoisomers, tautomers or pharmaceutically acceptable salts thereof is provided, 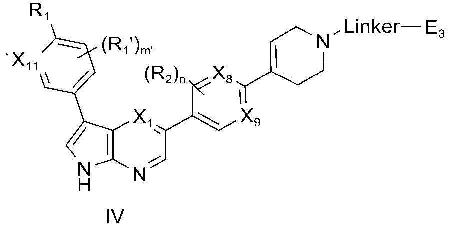 wherein
X₁ is selected from N or CH;
X₁₁ is selected from N or CH;
R₁ is selected from H, halogen, C₁-C₄ alkyl, C₃-C₈ cycloalkyl, 3-10 membered heterocyclic, C₆-C₁₀ aryl, 5-10 membered heteroaryl, -OR_{b}, -COR_{b}, -CONR_{b}R_{c}, -NR_{b}R_{c}, -NR_{b}COR_{c} or SO(=NR_{b})R_{c}, SO₂NR_{b}R_{c}, cyano or oxo; the alkyl, the cycloalkyl, the heterocyclic, the aryl, and the heteroaryl be further substituted by halogen, C₁-C₃ alkyl, C₃-C₅ cycloalkyl, C₁-C₃ haloalkyl, -C₁-C₃ alkyl-NR_{b}COR_{c}, -C₁-C₃ alkyl-CONR_{b}R_{c}, -C₁-C₃ alkyl-OR_{b}, hydroxyl, cyano, amino or nitro;
R₁' is selected from H, halogen, C₁-C₄ alkyl, hydroxyl, cyano, amino or nitro;
or R₁ and R₁', one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
or R₁' and R₁', and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
R_{b} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, C₁-C₃ alkoxy, hydroxyl, cyano, amino, or nitro;
R_{c} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, C₁-C₃ alkoxy, hydroxyl, cyano, amino, or nitro;
R₂ is selected from H or 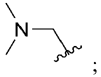
n or m' is selected from 0, 1, 2, or 3.

In a preferred solution of the invention, a compound of general formula (IV) or stereoisomers, tautomers or pharmaceutically acceptable salts thereof is provided, 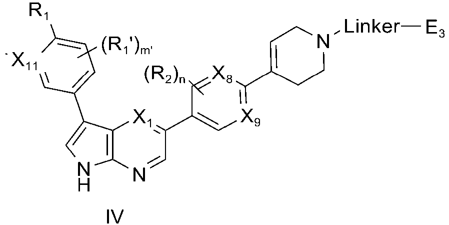 wherein
R₁ is selected from H, C₁-C₃ alkyl, halogen, C₁-C₃ haloalkyl, cyano, oxo, hydroxymethyl, hydroxyethyl, 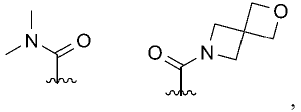 cyclopropyl, cyclobutyl, cyclopentyl, 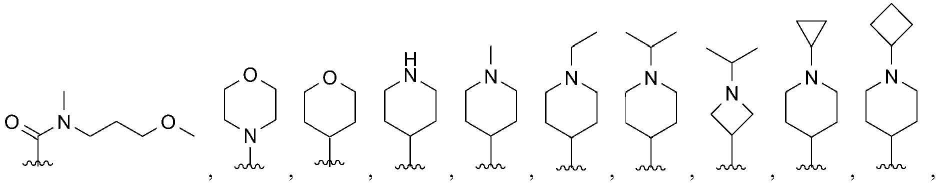 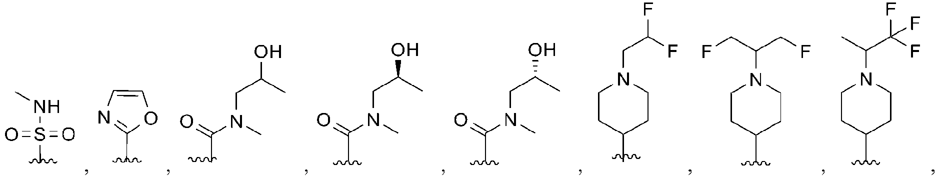 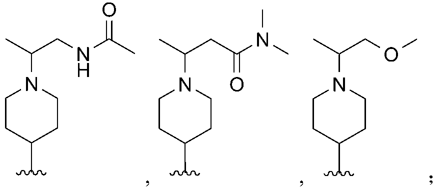
R₁' is selected from H, Cl, F, methyl, ethyl or cyano;
X₈ and X₉ are each independently selected from CRₐ or N;
Rₐ is selected from H, methyl, ethyl, isopropyl, F, methylamino, ethylamino, methoxy, ethoxy, 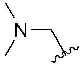 or 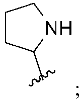
n or m' is selected from 0, 1, 2, or 3.

In a preferred solution of the invention, a compound of general formula (I) or stereoisomers, tautomers or pharmaceutically acceptable salts thereof is provided,
wherein 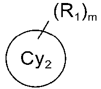 is selected from 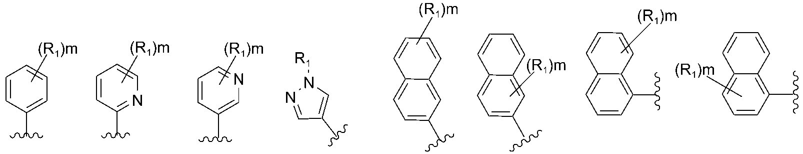 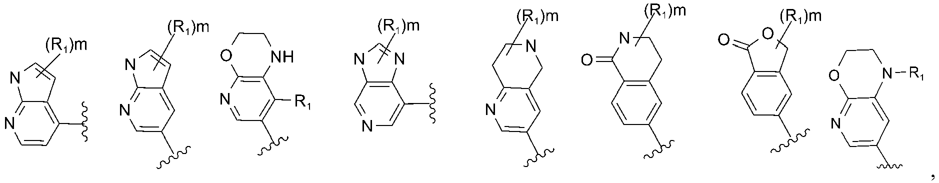 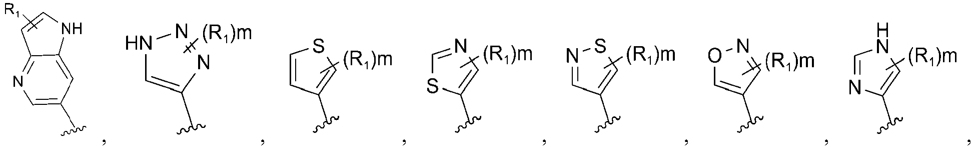 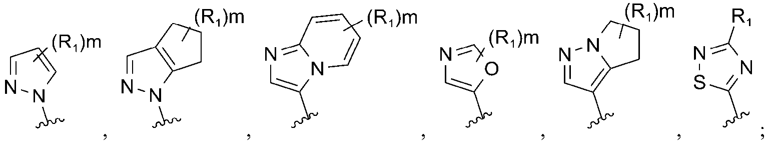
R₁ is selected from H, halogen, C₁-C₃ alkyl, C₃-C₆ cycloalkyl, 4-6 membered heterocyclic, C₆-C₁₀ aryl, 5-6 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, -POR_{b}R_{c} or -Linker-E3; the alkyl, the cycloalkyl, the heterocyclic, the aryl, and the heteroaryl optionally be further substituted by one or more substituents; the substituents are selected from halogen, C₁-C₃ alkyl, C₃-C₅ cycloalkyl, 3-6 membered heterocyclic, C₆-C₁₀ aryl, 5-7 membered heteroaryl, C₁-C₃ haloalkyl, -C₁-C₃ alkyl-CONR_{b}R_{c}, -C₁-C₃ alkyl-NR_{b}COR_{c}, -C₁-C₃ alkyl-OR_{b}, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
or two R₁ and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
m is selected from 0, 1, 2, 3, 4, and 5;
R_{b}, R_{c} and R_{d} are each independently selected from H, halogen, methyl, ethyl, propyl, isopropyl, C₃-C₆ cycloalkyl, 5-7 membered heterocyclic, C₆-C₁₀ aryl, 5-6 membered heteroaryl, C₁-C₄ haloalkyl, -C₁-C₄ alkyl-CON(CH₃)₂, -C₁-C₃ alkyl-OCH₃, -ORₑ or -NRₑR_{f}; the methyl, the ethyl, the propyl, the isopropyl, the cycloalkyl, the heterocyclic, the aryl, and the heteroaryl be optionally further substituted by one or more substituents; the substituents are selected from halogen, C1-C3 alkyl, C₃-C₆ cycloalkyl, 3-8 membered heterocyclic, C₆-C₁₀ aryl, 5-6 membered heteroaryl, C₁-C₄ haloalkyl, -C₁-C₄ alkyl-CON(CH₃)₂, -C₁-C₃ alkyl-OCH₃, oxo, -CN, -NO₂, -OH or -NH₂.

In a preferred solution of the invention, a compound of general formula (I) or stereoisomers, tautomers or pharmaceutically acceptable salts thereof is provided,
wherein 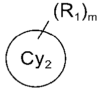 is selected from 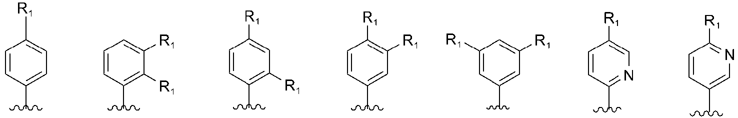 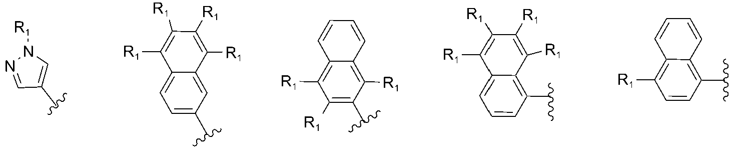 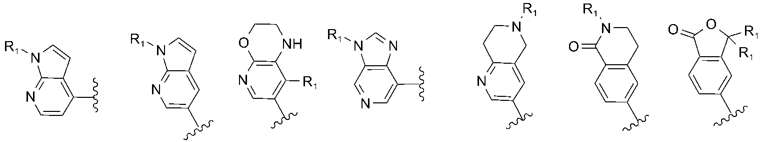 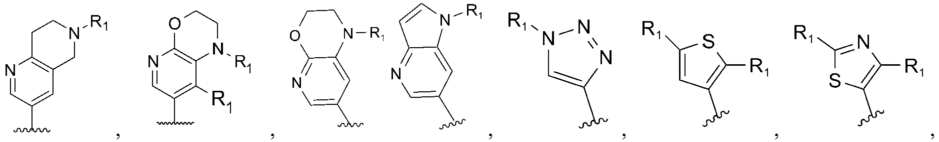 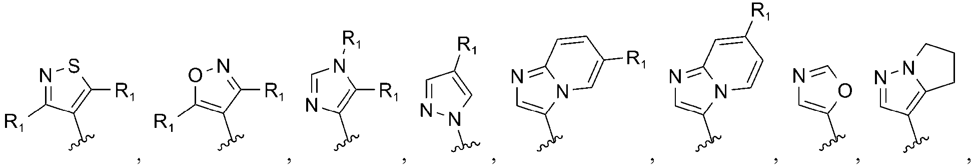 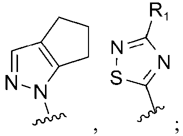 R₁ is selected from H, C₁-C₃ alkyl, halogen, C₁-C₃ haloalkyl, cyano, oxo, hydroxymethyl, hydroxyethyl, 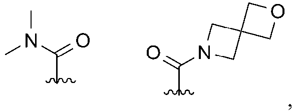 cyclopropyl, cyclobutyl, cyclopentyl,

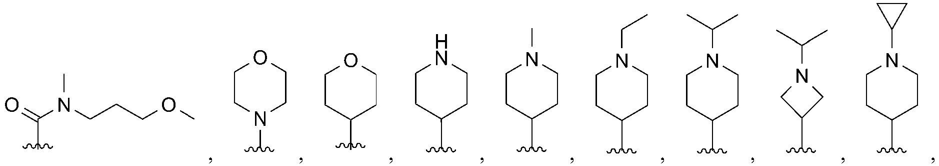

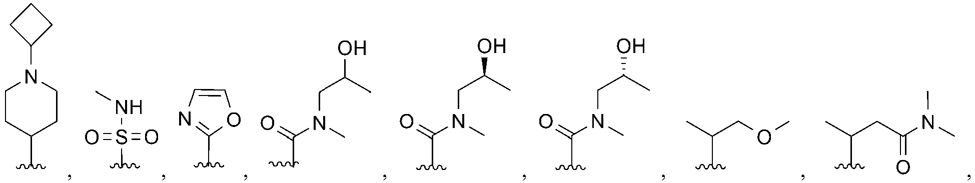

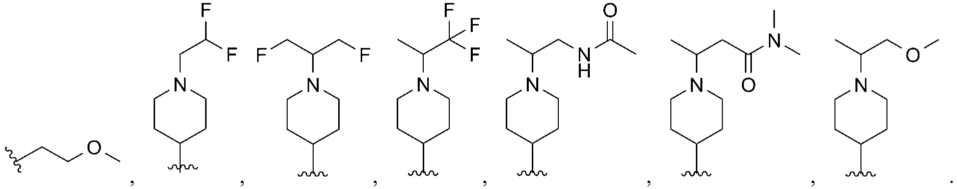

In a preferred solution of the invention, a compound of general formula (I) or stereoisomers, tautomers or pharmaceutically acceptable salts thereof is provided,
wherein 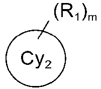 is selected from

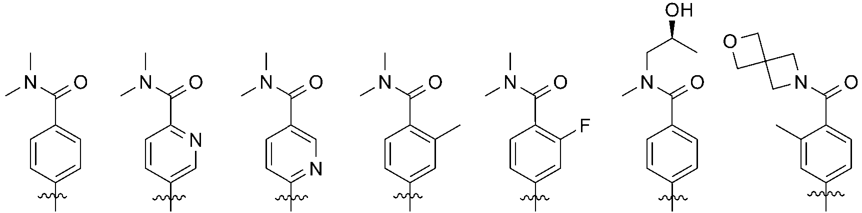

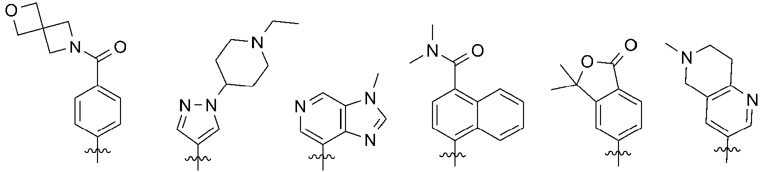

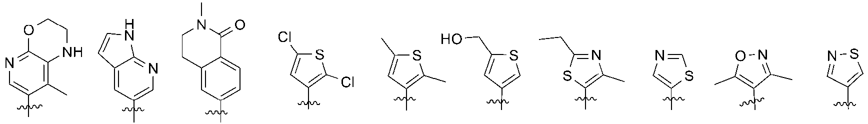

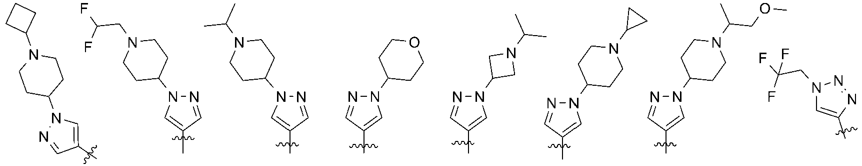

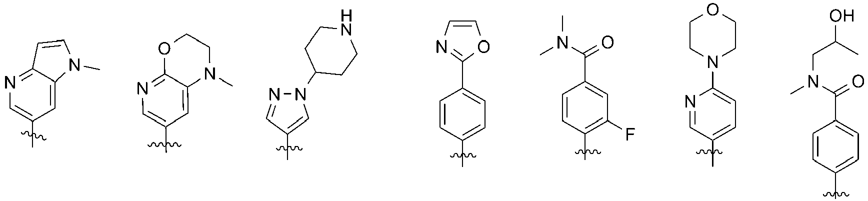

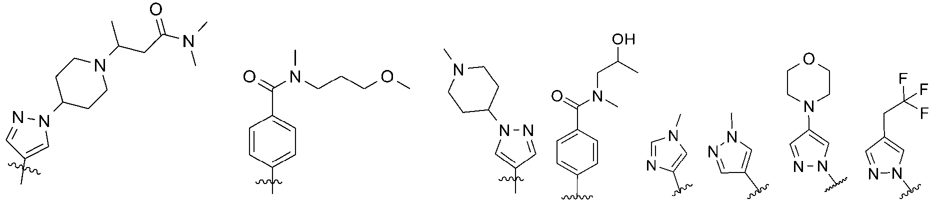

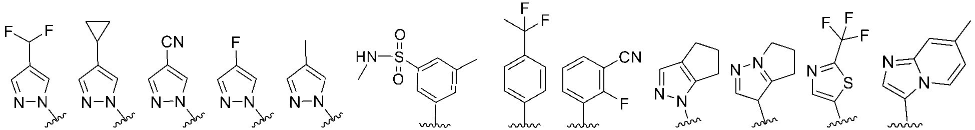

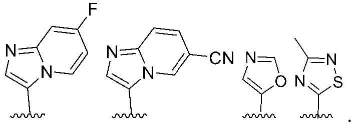

In a preferred solution of the invention, a compound of general formula (VI) or stereoisomers, tautomers or pharmaceutically acceptable salts thereof is provided, 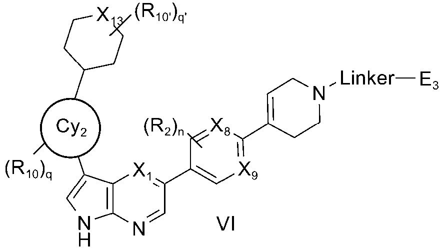
X₁₃ is selected from NH or O;
X₁ is selected from CH or N;
R₁₀ is selected from H, halogen, C₁-C₄ alkyl, C₁-C₄ haloalkyl, C₁-C₄ alkoxy, hydroxyl, cyano, amino or nitro;
R_{10'} is selected from H, halogen, C₁-C₄ alkyl, C₃-C₅ cycloalkyl or 3-8 membered heterocyclic, C₁-C₄ haloalkyl, -C₁-C₄ alkyl-CONRbRc, -C₁-C₈ alkyl-OR_{b}, oxo or -CN; the alkyl, the cycloalkyl, or the heterocyclic optionally be further substituted by one or more substituents selected from halogen, C₁-C₈ alkyl, -CN, -CONR_{b}R_{c}, -NR_{b}COR_{c} or -OR_{b}.

Preferably, R_{10'} is selected from H, halogen, cyano, oxo, methyl, ethyl, propyl, isopropyl, isobutyl, -CH₂F, -CHF₂, -CF₃, -CH₂CH₂F, -CH₂CHF₂, -CH₂CF₃, cyclopropyl, cyclobutyl, cyclopentyl, 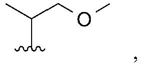 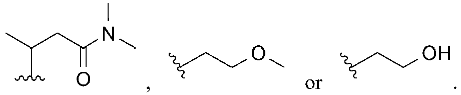
R₂ is selected from H, halogen, C₁-C₄ alkyl, C₃-C₆ cycloalkyl, 4-6 membered heterocyclic, -CN, -NO₂, -OR_{b} or -NR_{b}R_{c}, and the alkyl, the cycloalkyl, and the heterocyclic be further substituted by one or more substituents selected from halogen, oxo, -CN, -OR_{b}, and -NR_{b}R_{c};
R_{b} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by halogen, C₁-C₃ alkyl, hydroxyl, cyano, amino, or nitro;
R_{c} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by halogen, C₁-C₃ alkyl, hydroxyl, cyano, amino, or nitro;
q or q' is selected from 0, 1, 2, or 3;
Cy₂ is selected from a 5 membered heteroaromatic ring containing 1-3 heteroatoms.

In a preferred solution of the invention, a compound or stereoisomers, tautomers or pharmaceutically acceptable salts thereof is provided, the 5 membered heteroaromatic ring from which Cy₂ is selected is preferably selected from

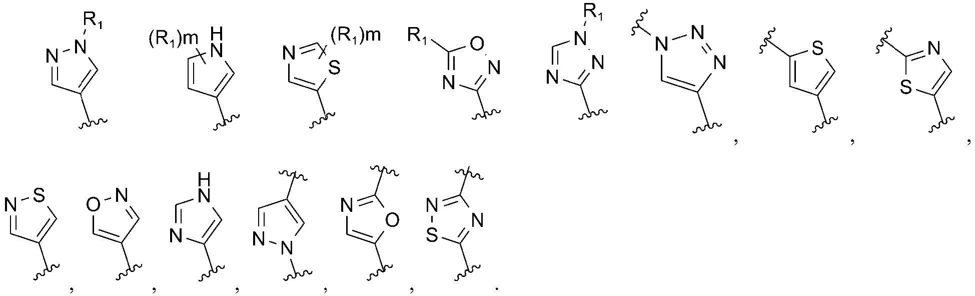

Preferably, Cy₂ is selected from

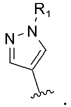

In a preferred solution of the invention, a compound of general formula (VII) or stereoisomers, tautomers or pharmaceutically acceptable salts thereof is provided, 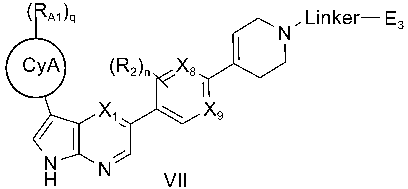
CyA is selected from phenyl, 5-10 membered heteroaryl or 8-10 membered fused bicyclic;
the heteroaryl from which CyA is selected are preferably selected from 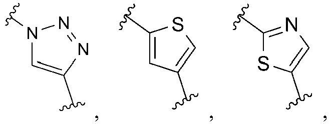 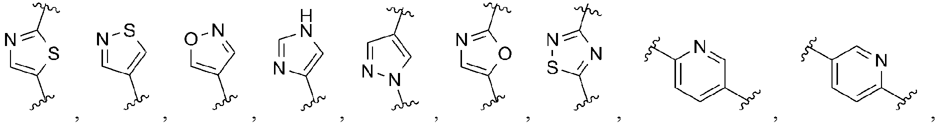 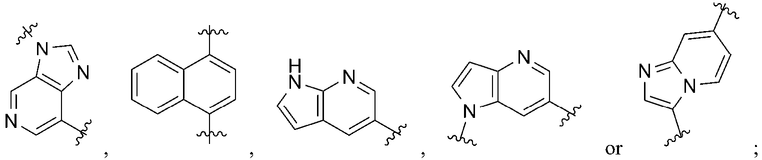 the fused bicyclic are selected from 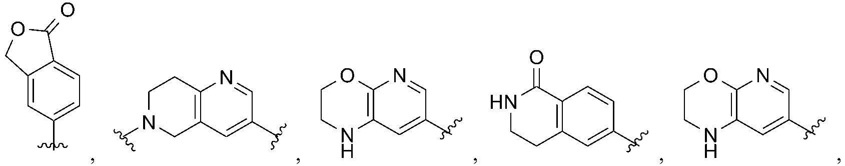 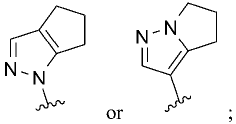
R_{A1} is selected from H, halogen, C₁-C₄ alkyl, C₃-C₈ cycloalkyl, 3-10 membered heterocyclic, C₆-C₁₀ aryl, 5-10 membered heteroaryl, oxo, -CN, -OR_{b} , -COR_{B}, -CONR_{B}R_{C}, -NR_{B}R_{C}, -NR_{B}COR_{C} or SO(=NR_{B})R_{C}; the alkyl, the cycloalkyl, the heterocyclic, the aryl, or the heteroaryl be further substituted by halogen, C₁-C₃ alkyl, hydroxyl, cyano, amino, or nitro;
R_{B} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, hydroxyl, cyano, amino, or nitro;
R_{C} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, hydroxyl, cyano, amino, or nitro;
q is selected from 0, 1, 2, or 3;
the definitions of X₁, X₈, X₉, R₂, n, Linker and E3 are as described in formula (I).

In a preferred solution of the invention, X₈ and X₉ are selected from CRa or N;
Ra is selected from H, halogen, methyl, ethyl, isopropyl, 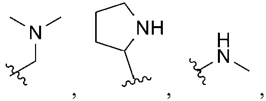 methoxy, ethoxy.
L₁ is selected from chemical bonds, -O- or -CH₂-O-.

In a preferred solution of the invention, the compound or stereoisomers, tautomers or pharmaceutically acceptable salts thereof is provided, wherein Linker is selected from

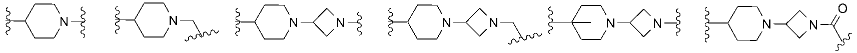

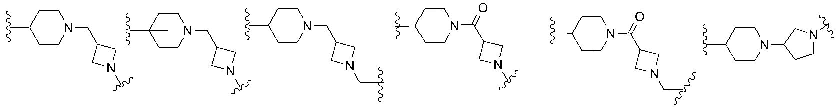

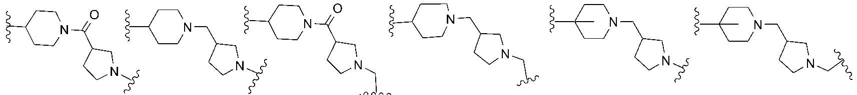

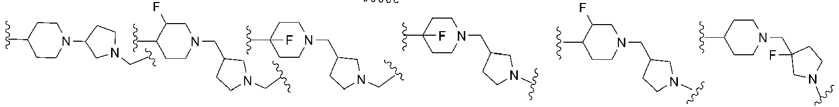

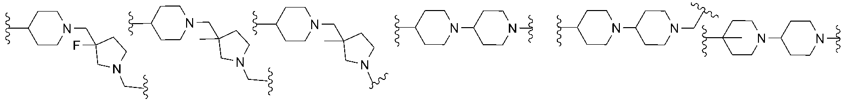

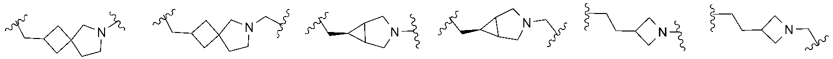

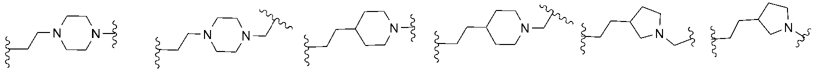

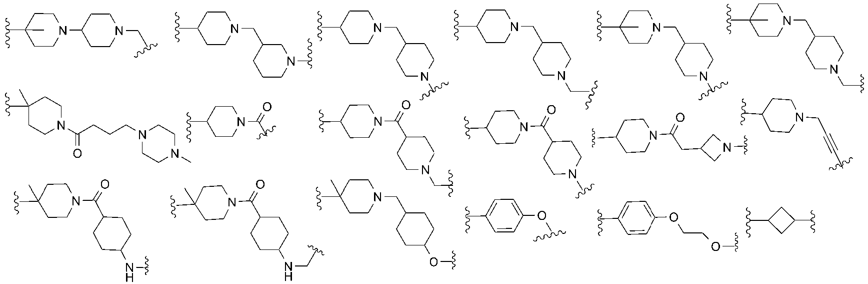

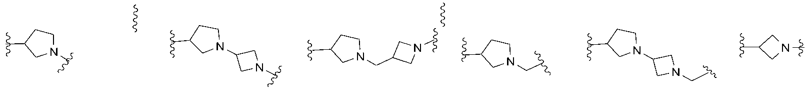

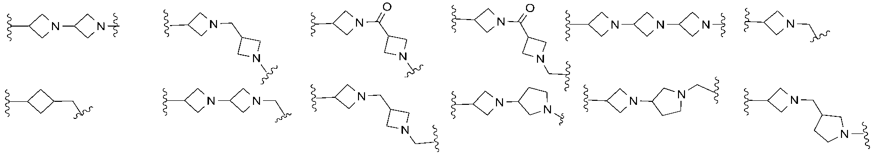

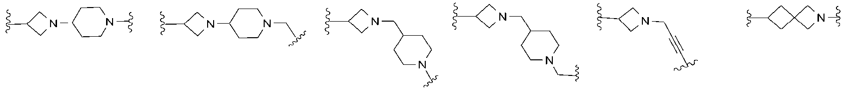

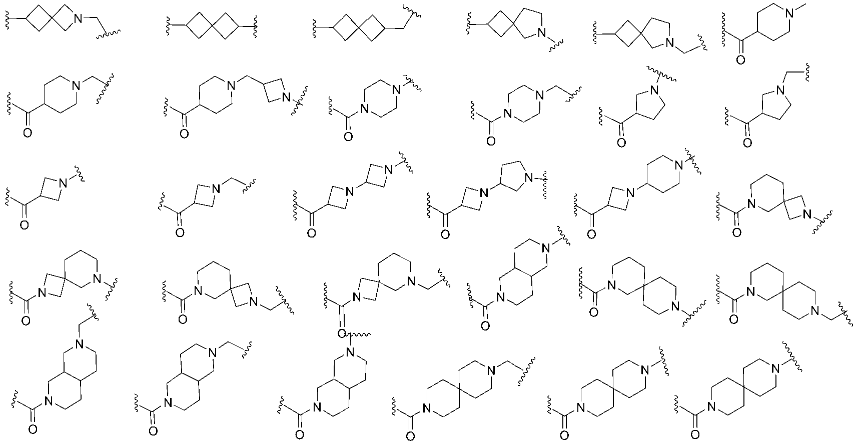

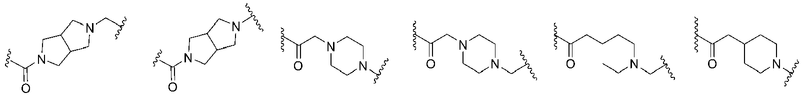

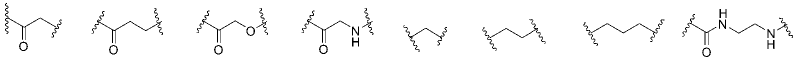

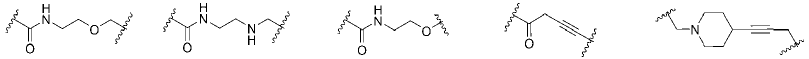

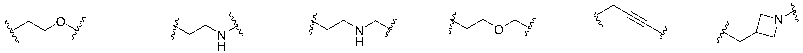

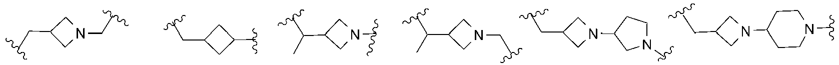

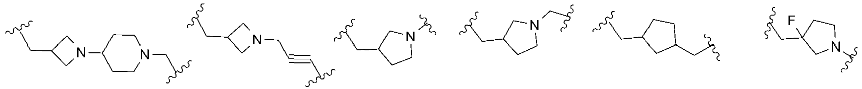

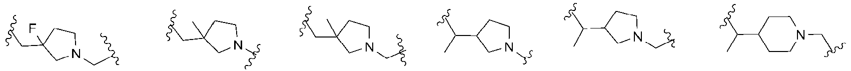

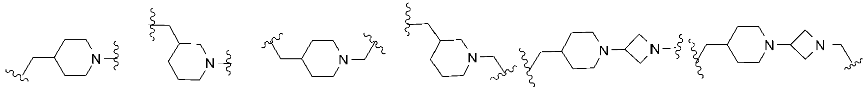

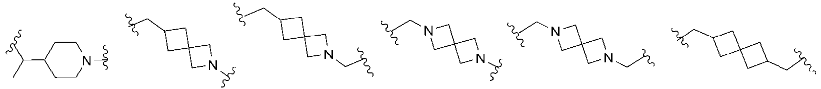

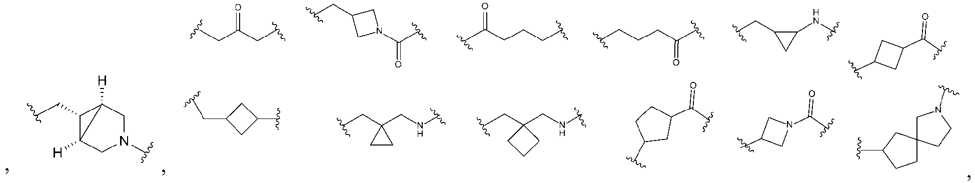

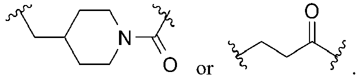

In a preferred solution of the invention, Linker is preferably selected from

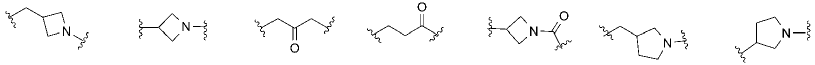

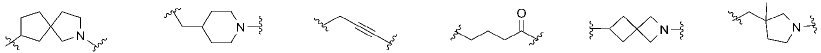

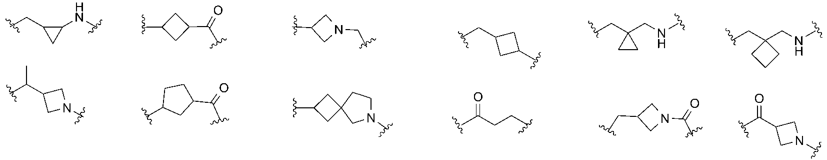

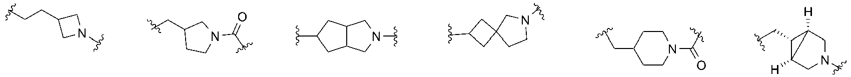

In a preferred solution of the invention, the compound or stereoisomers, tautomers or pharmaceutically acceptable salts thereof is provided, wherein E₃ is selected from 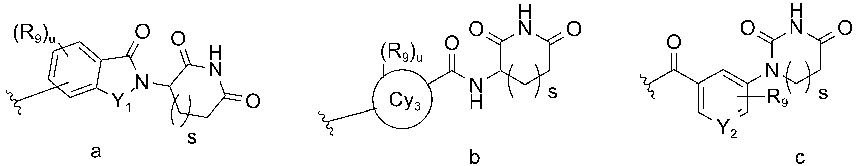 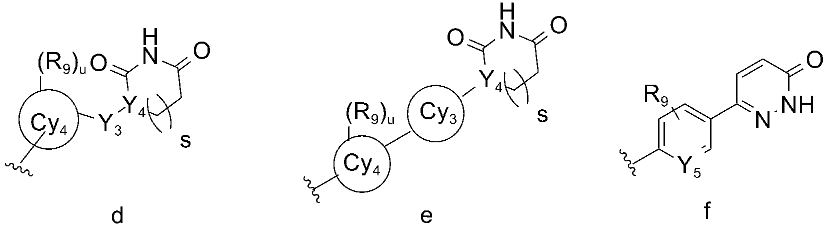 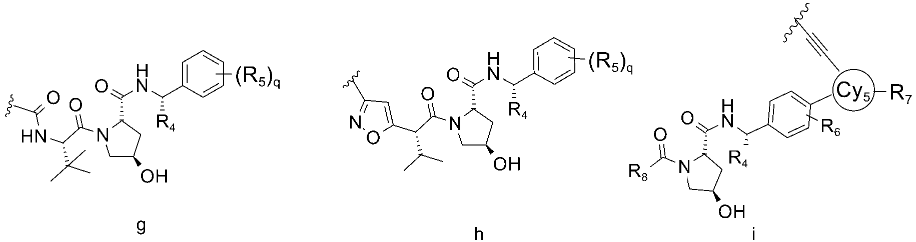 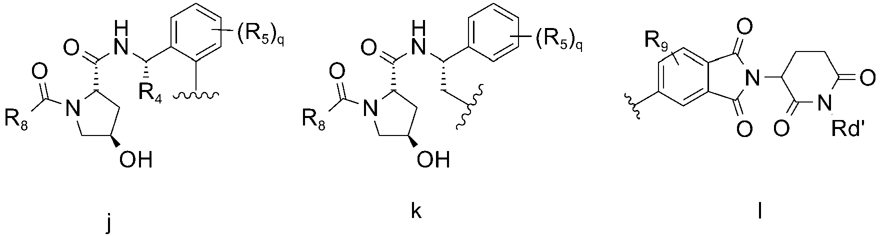 wherein
q, u, and s are each independently selected from 0, 1, and 2;
Y₁ is independently selected from CO, methylene, vinylene, or ethyl;
Y₂ is independently selected from CH or N;
Y₃ is independently selected from absent, CH₂, NH, NMe, or O;
Y₄ is independently selected from CH or N;
Y₅ is independently selected from CH or N;
R₄, R₅, R₆, R₇, R₈ and R₉ are each independently selected from H, halogen, C₁-C₈ alkyl, C₂-C₈ alkenyl, C₂-C₈ alkynyl, C₃-C₈ cycloalkyl, 5-12 membered heterocyclic, 6-10 membered aryl, 5-10 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c}; the alkyl, the alkenyl, the alkynyl, the cycloalkyl, the heterocyclic, the aryl, and the heteroaryl be further substituted by one or more substituents; the substituents are selected from halogen, C₁-C₈ alkyl, C₂-C₈ alkenyl, C₂-C₈ alkynyl, C₃-C₈ cycloalkyl, 5-12 membered heterocyclic, 6-10 membered aryl, 6-10 membered heteroaryl, oxo, -CN, -NO₂, -OR_{b}, -SO₂R_{b}, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
Rd' is selected from H, -R_{f} OCOR_{g}, -R_{f}OCOOR_{g}, -R_{f}OCONR_{g}Rₕ, -COOR_{f} or -CONR_{f}R_{g};
R_{f}, R_{g} and Rₕ are each independently selected from H, C₁-C₈ alkyl, 3-8 membered cycloalkyl, 3-8 membered heterocyclic, C₁-C₆ alkyl 3-8 membered cycloalkyl or C₁-C₆ alkyl 3-8 membered heterocyclic;
Cy₃ is selected from 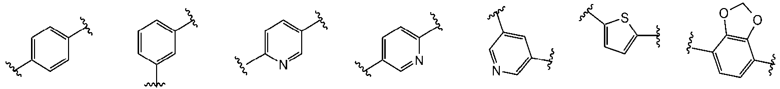 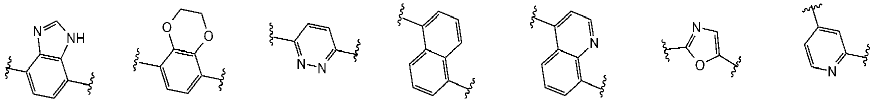 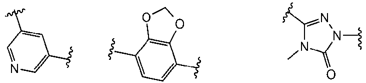
Cy₄ is selected from 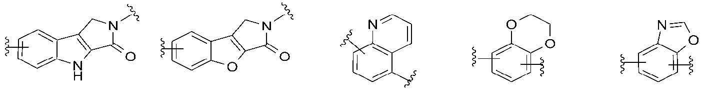 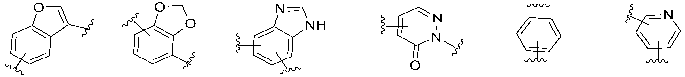 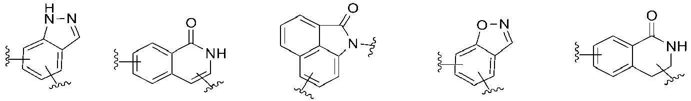 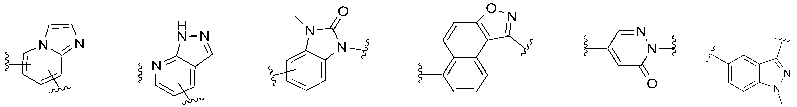
Cy₅ is selected from

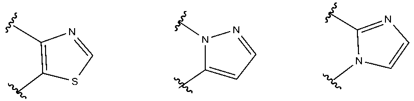

In a preferred solution of the invention, the compound or stereoisomers, tautomers or pharmaceutically acceptable salts thereof is provided, wherein E3 is selected from

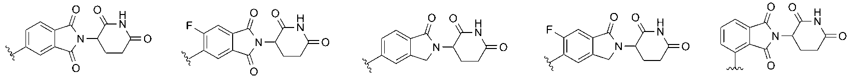

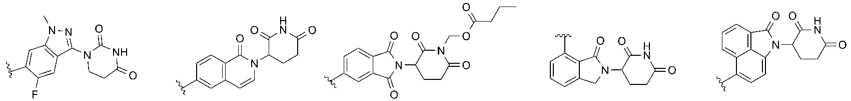

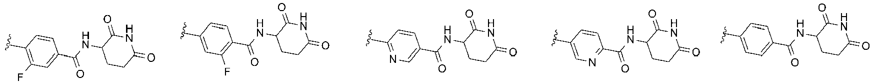

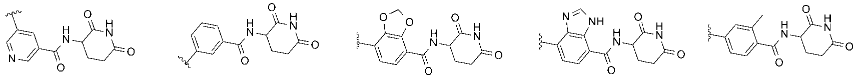

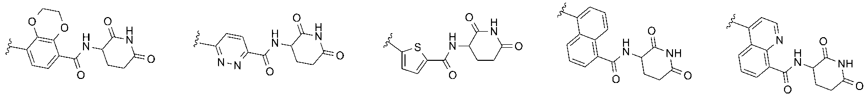

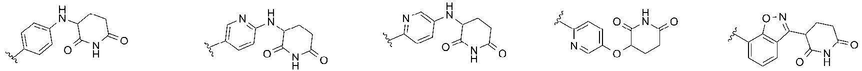

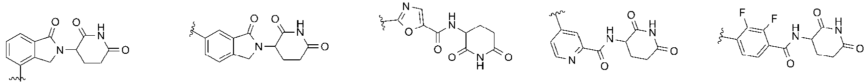

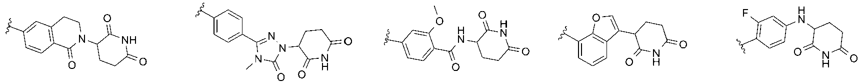

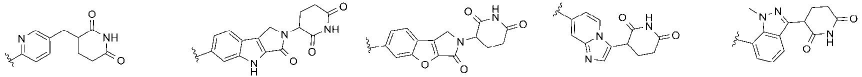

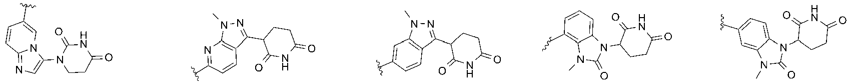

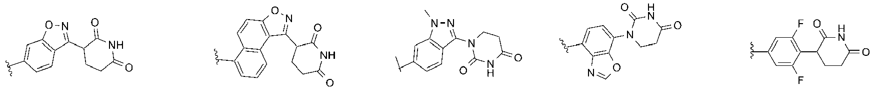

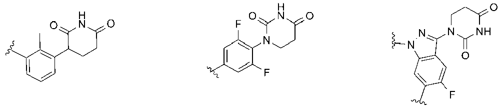

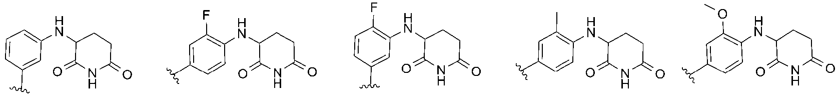

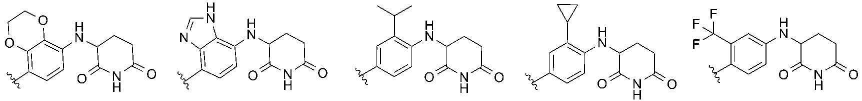

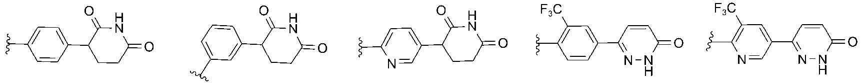

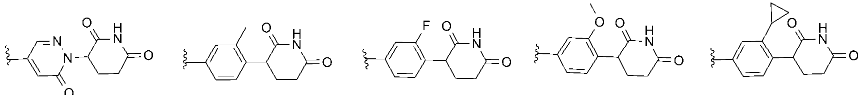

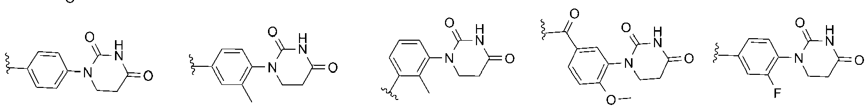

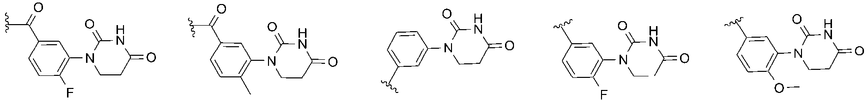

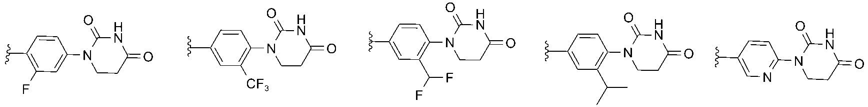

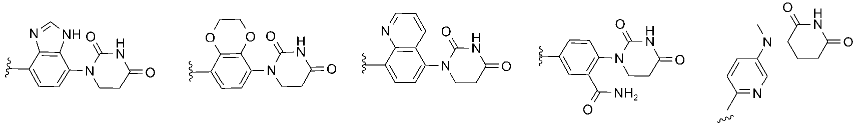

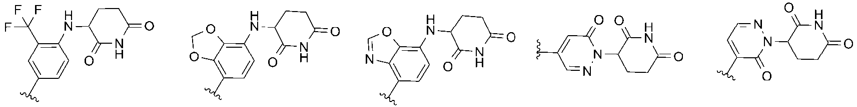

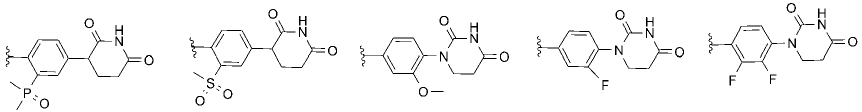

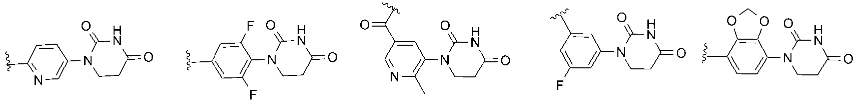

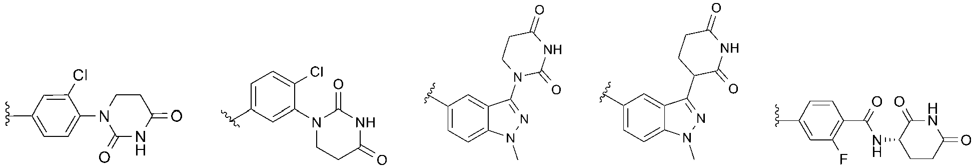

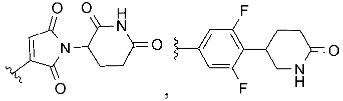

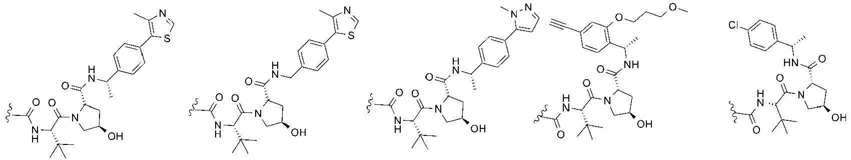

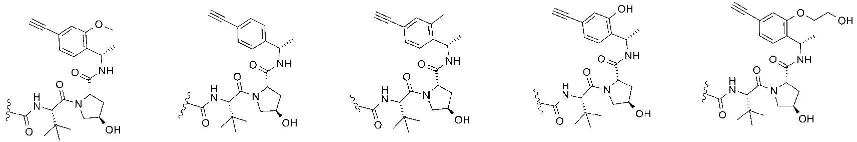

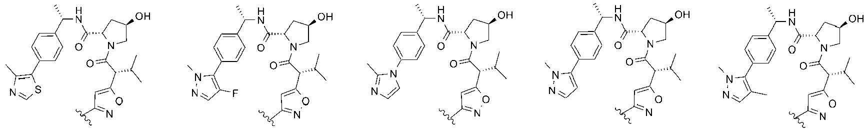

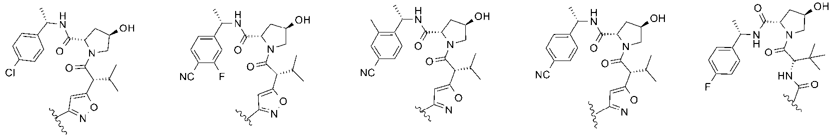

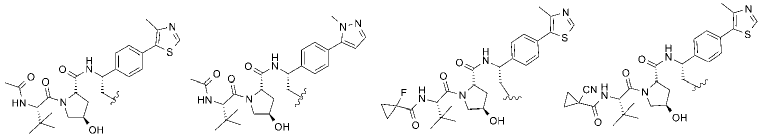

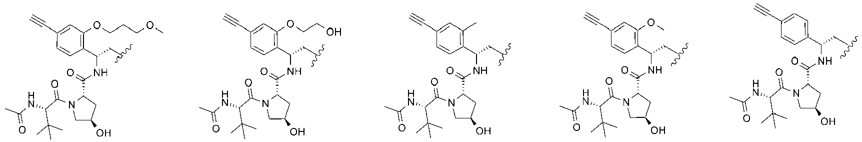

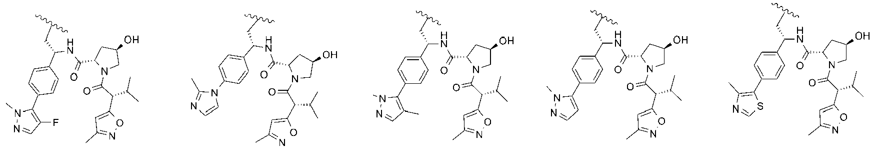

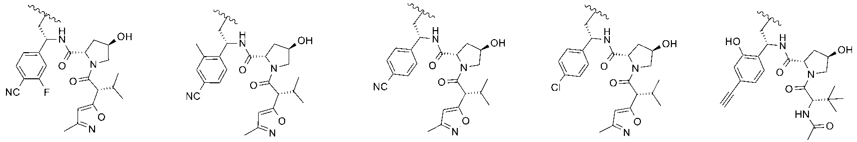

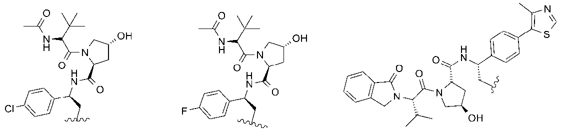

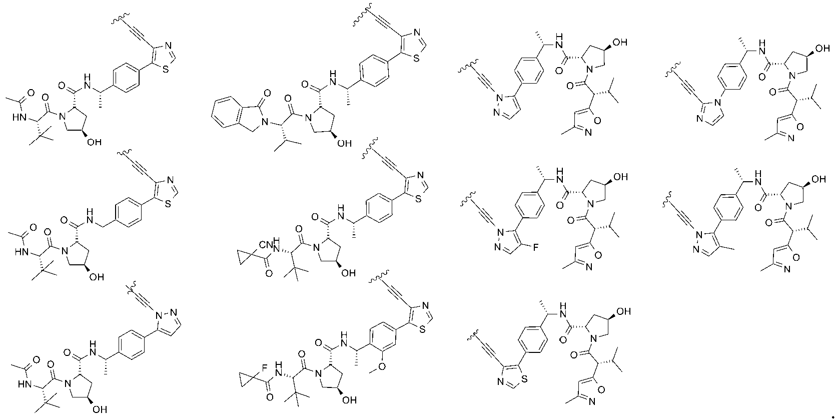

In a preferred solution of the invention, E3 is preferably selected from

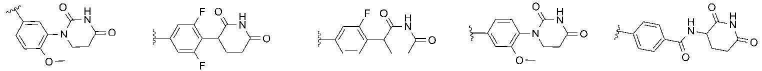

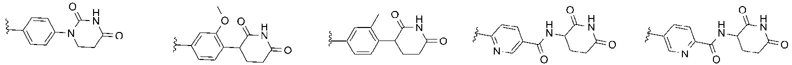

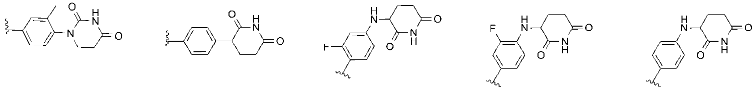

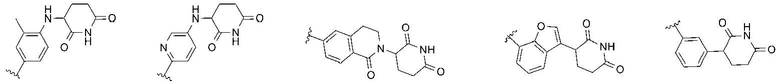

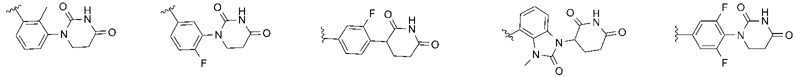

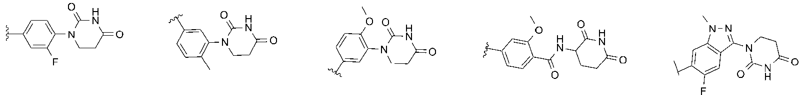

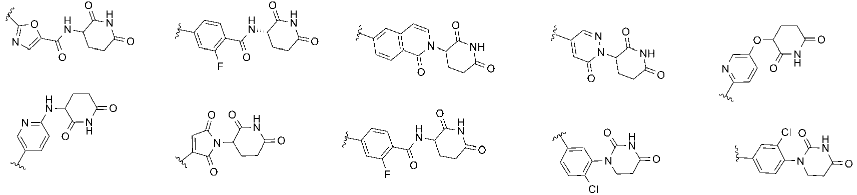

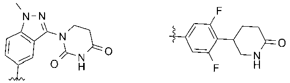

As a more specific option, X₈ and X₉ are each independently selected from CRₐ or N; Ra is selected from H, C₁-C₃ alkyl, -CH₂N (CH₃) ₂, halogen, 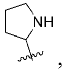 -NHCH₃, -O C₁-C₃ alkyl; 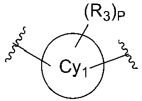 is selected from 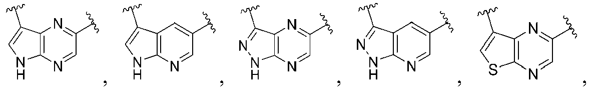 or 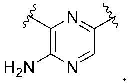 L₁ and L₂ are each independently selected from chemical bonds, O or -OCH₂-.

In a preferred solution of the invention, the compound of general formula (I) is selected from

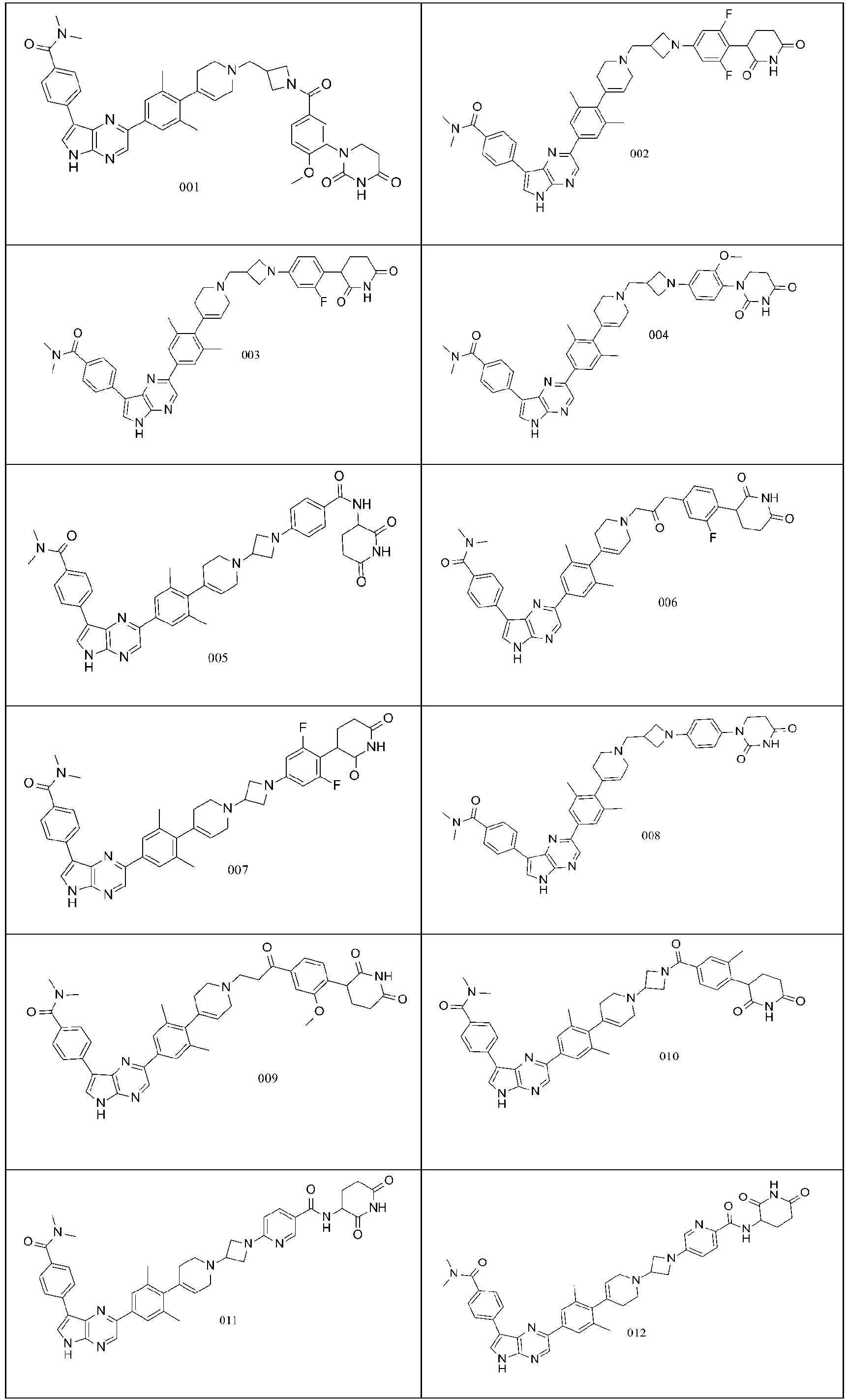

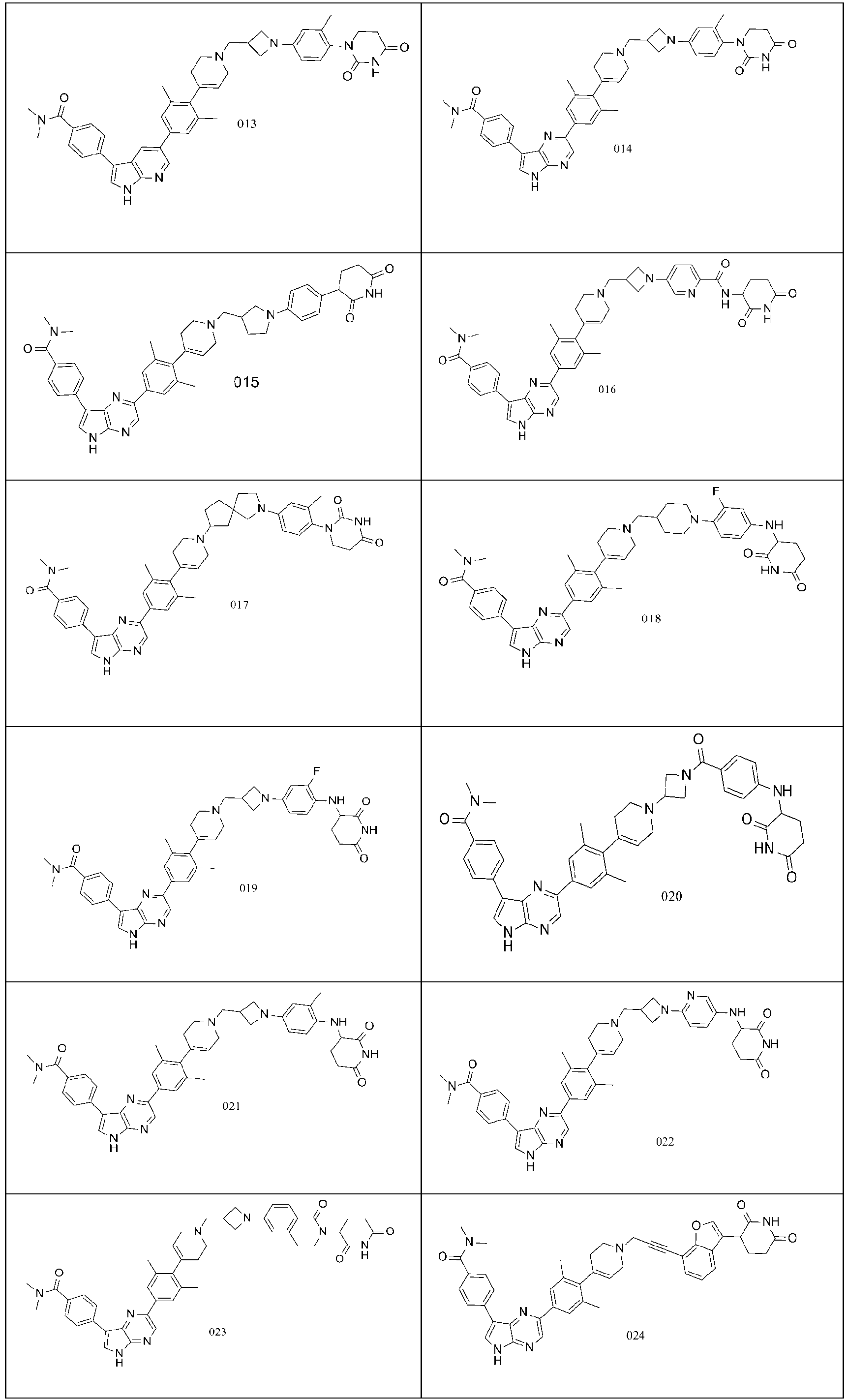

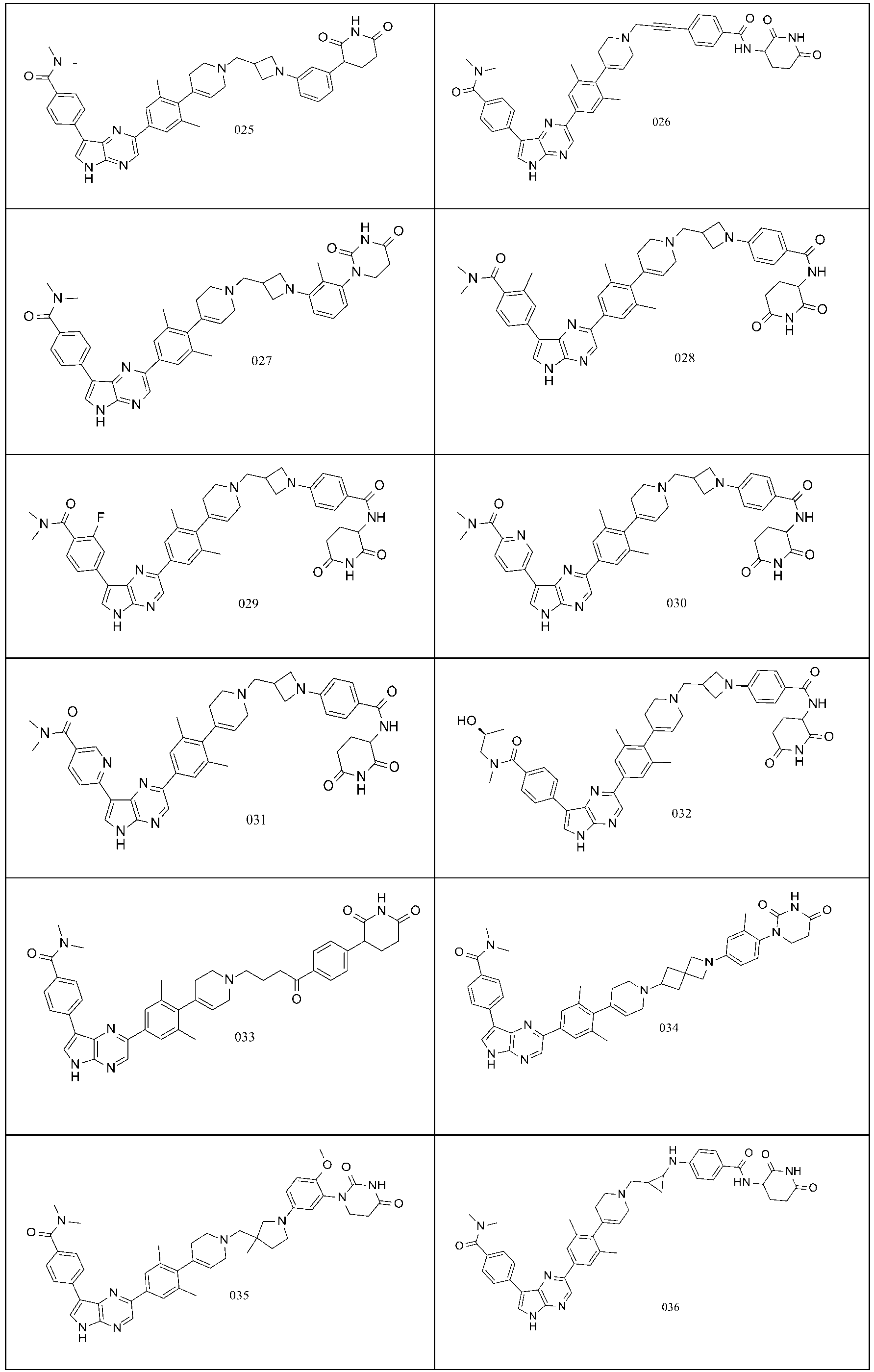

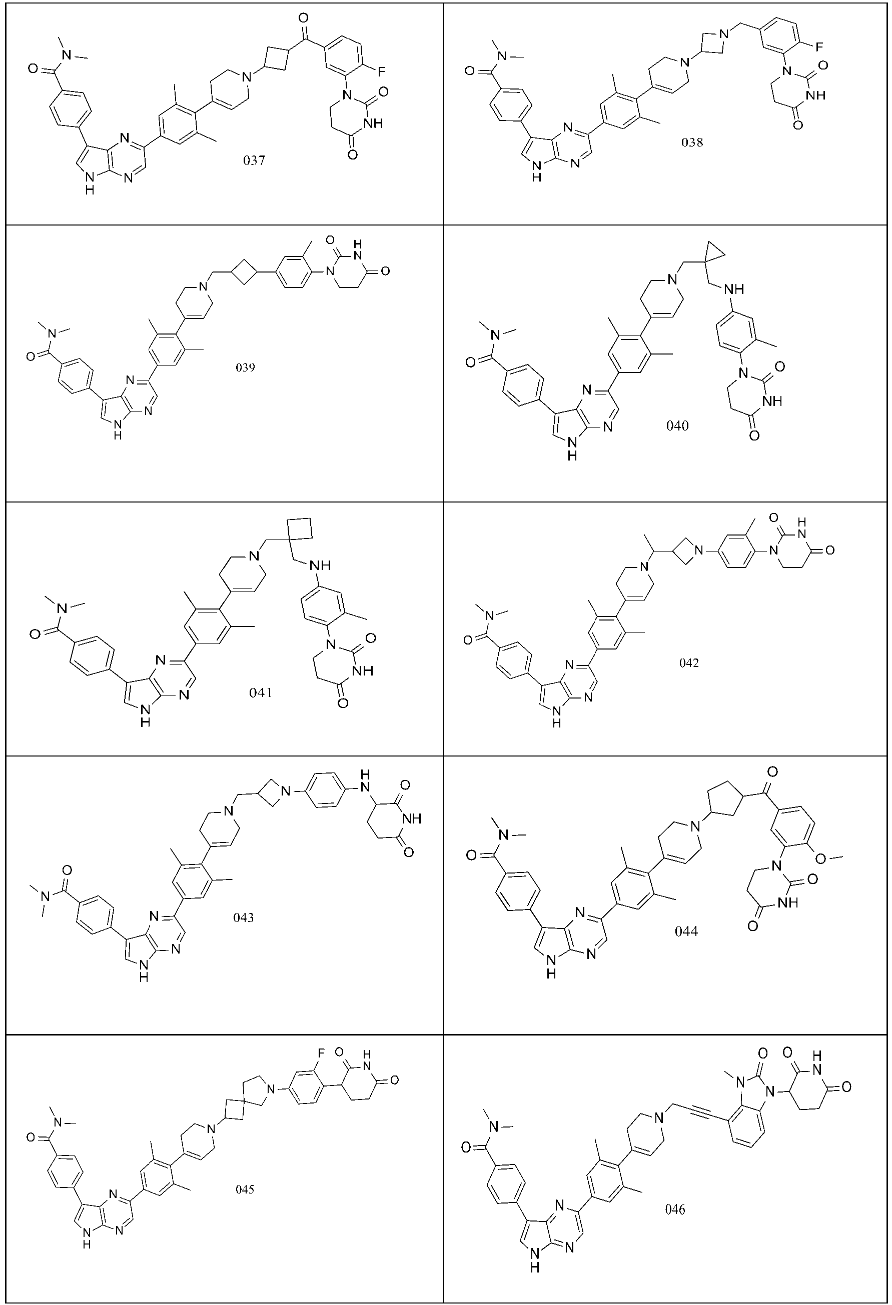

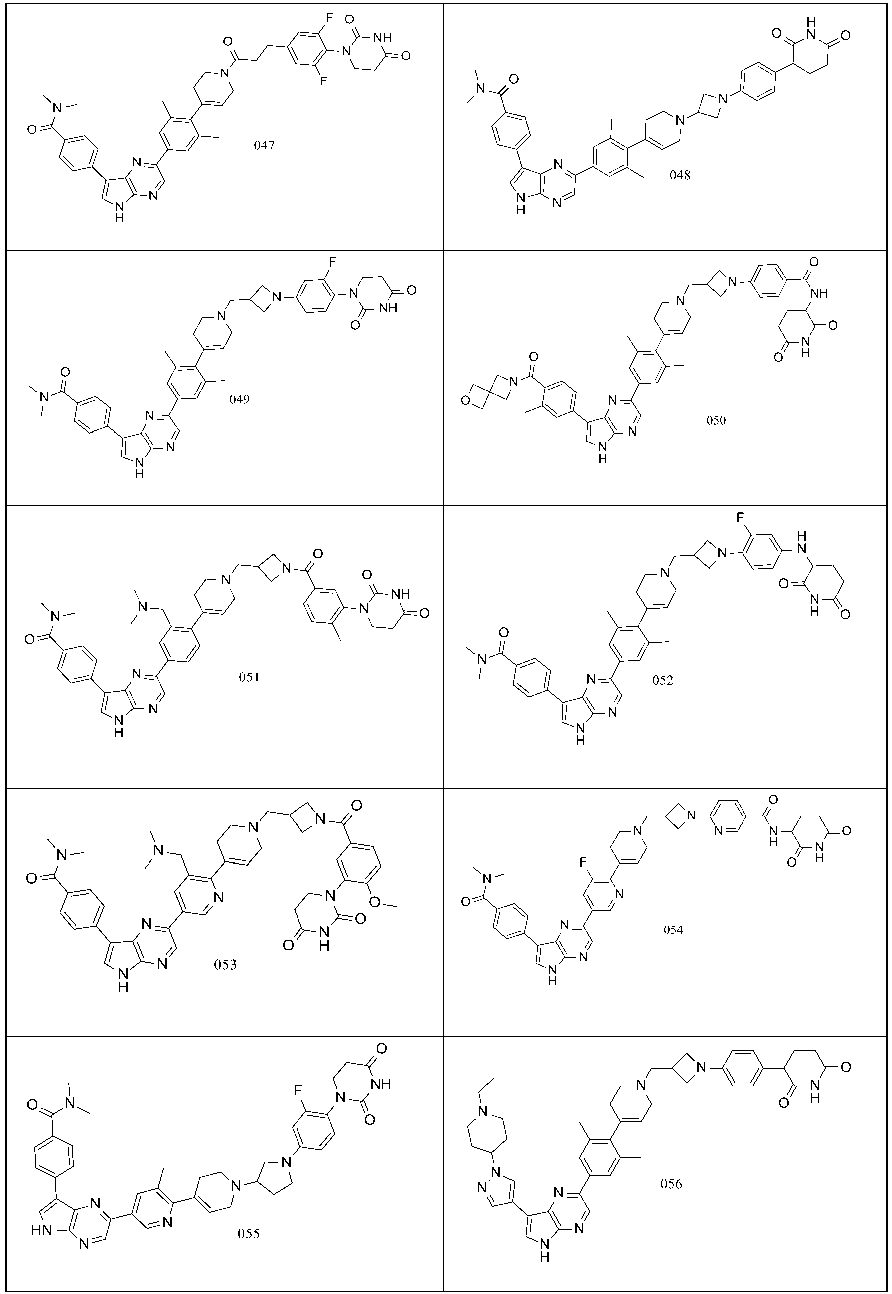

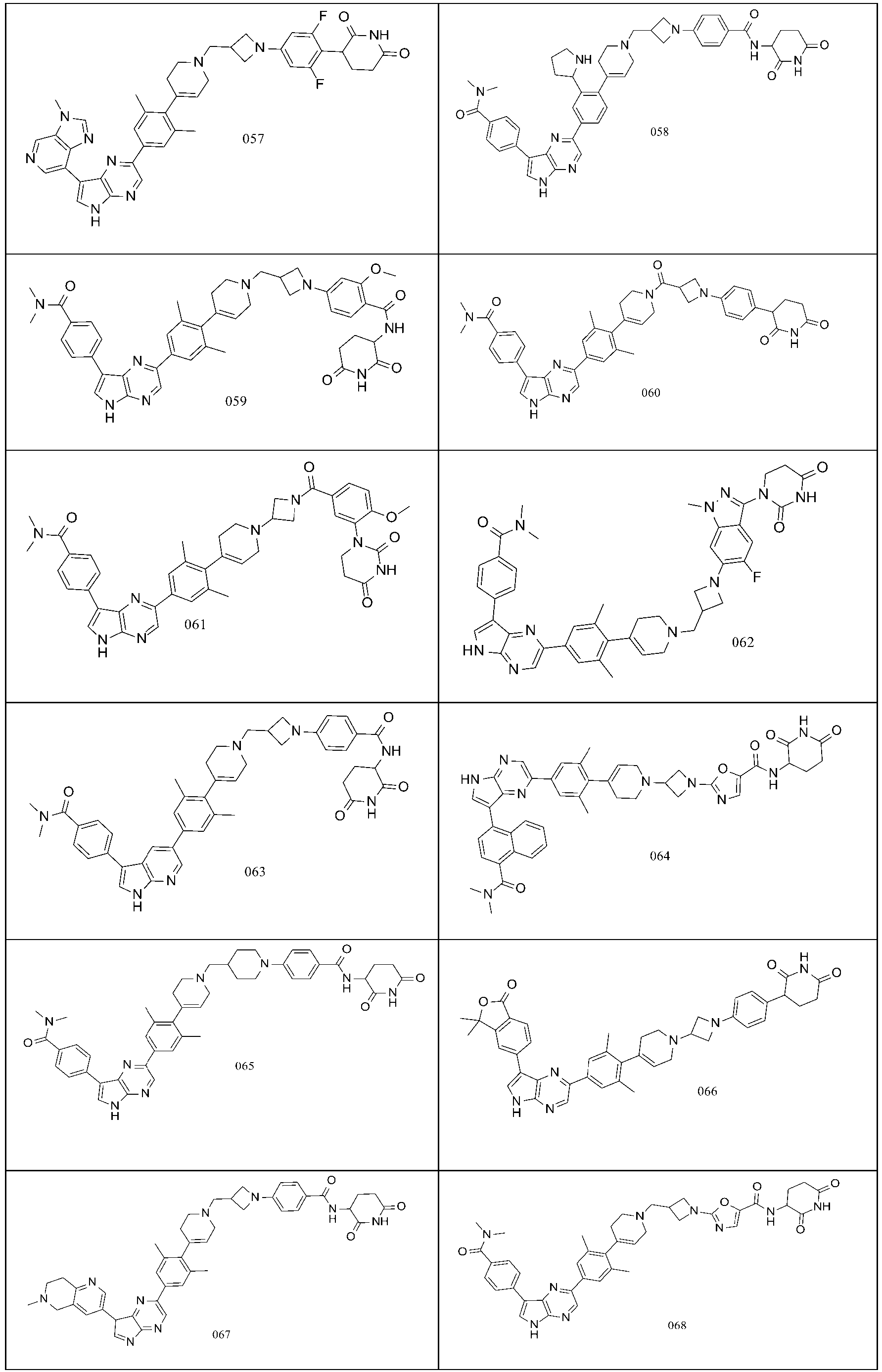

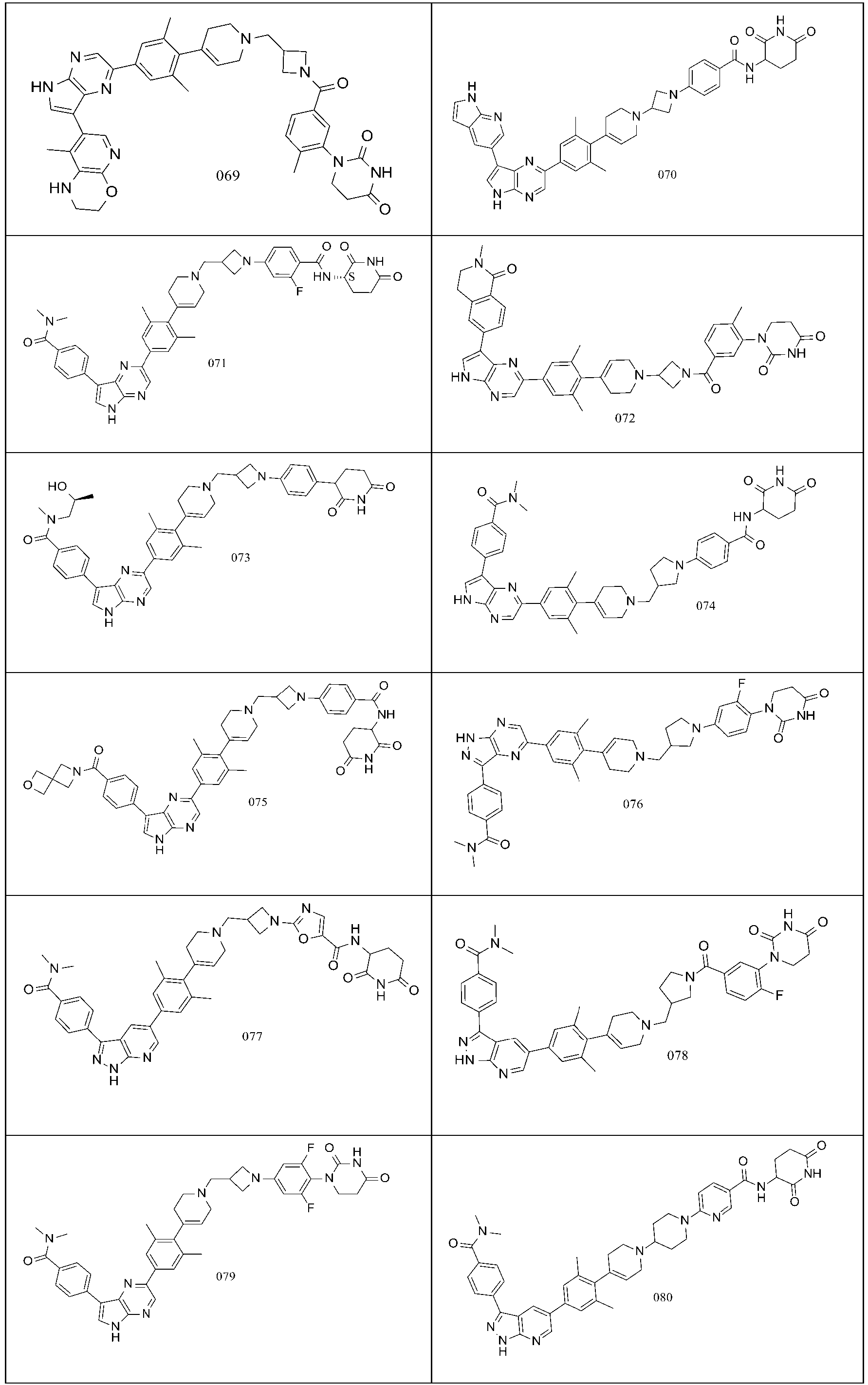

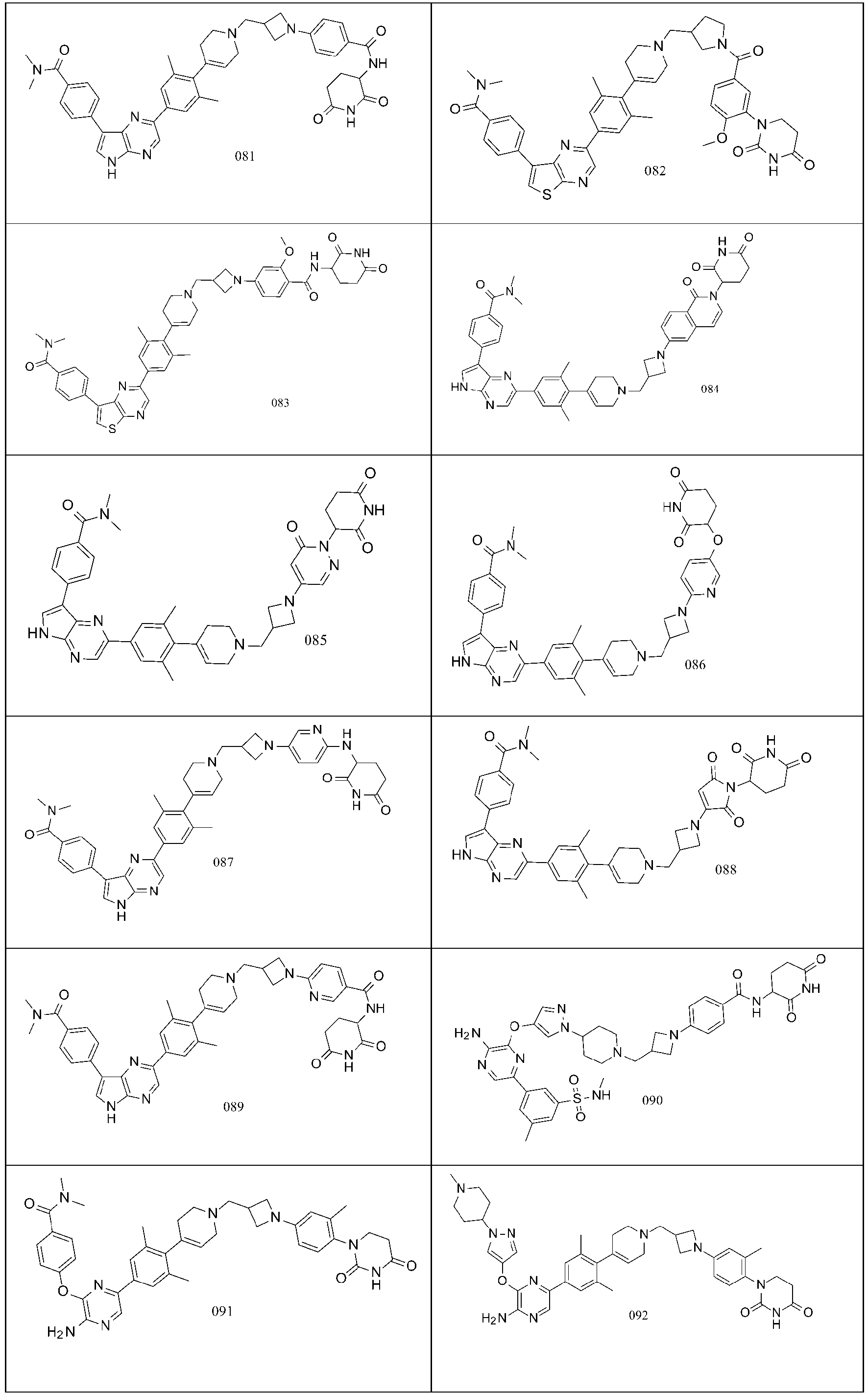

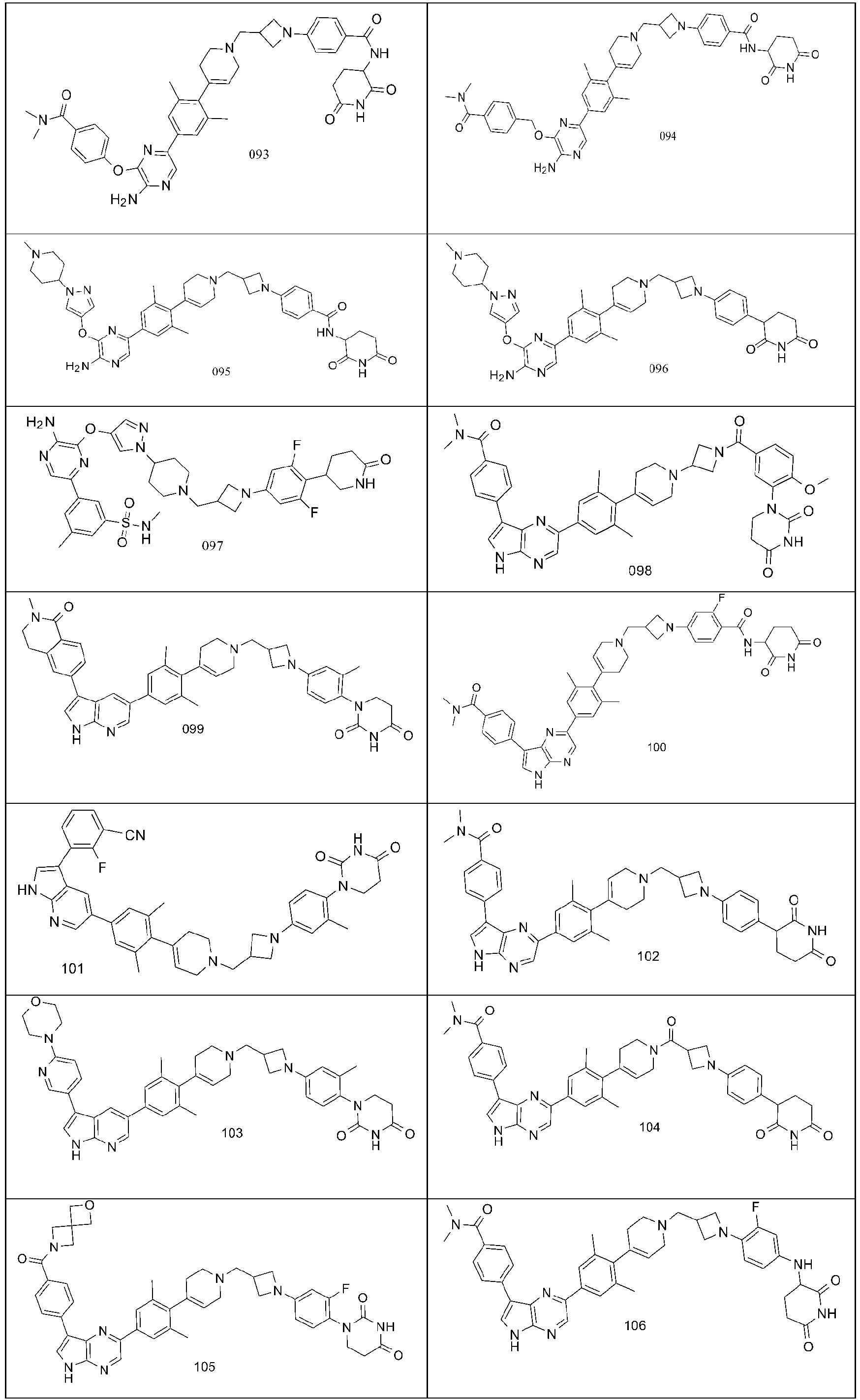

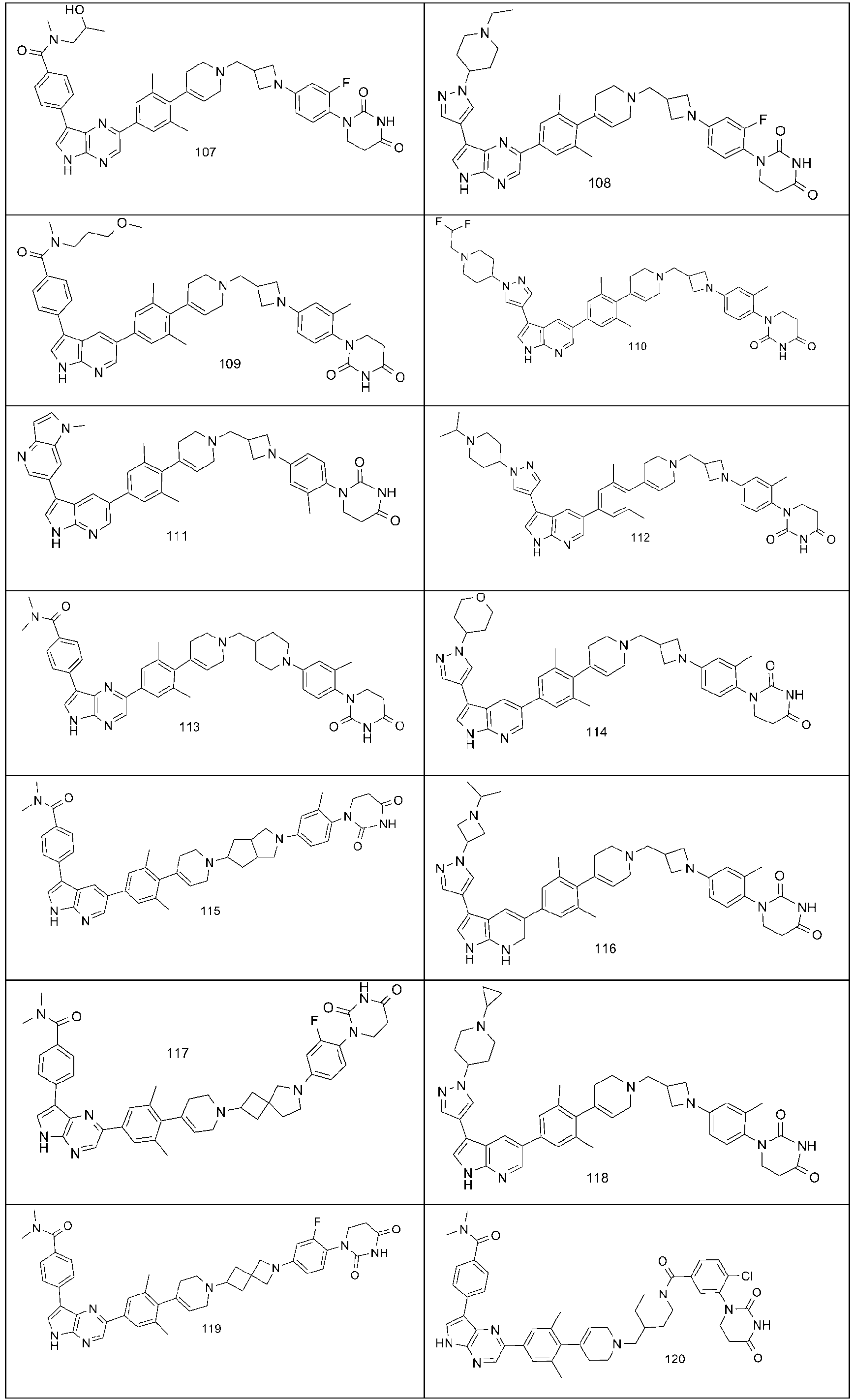

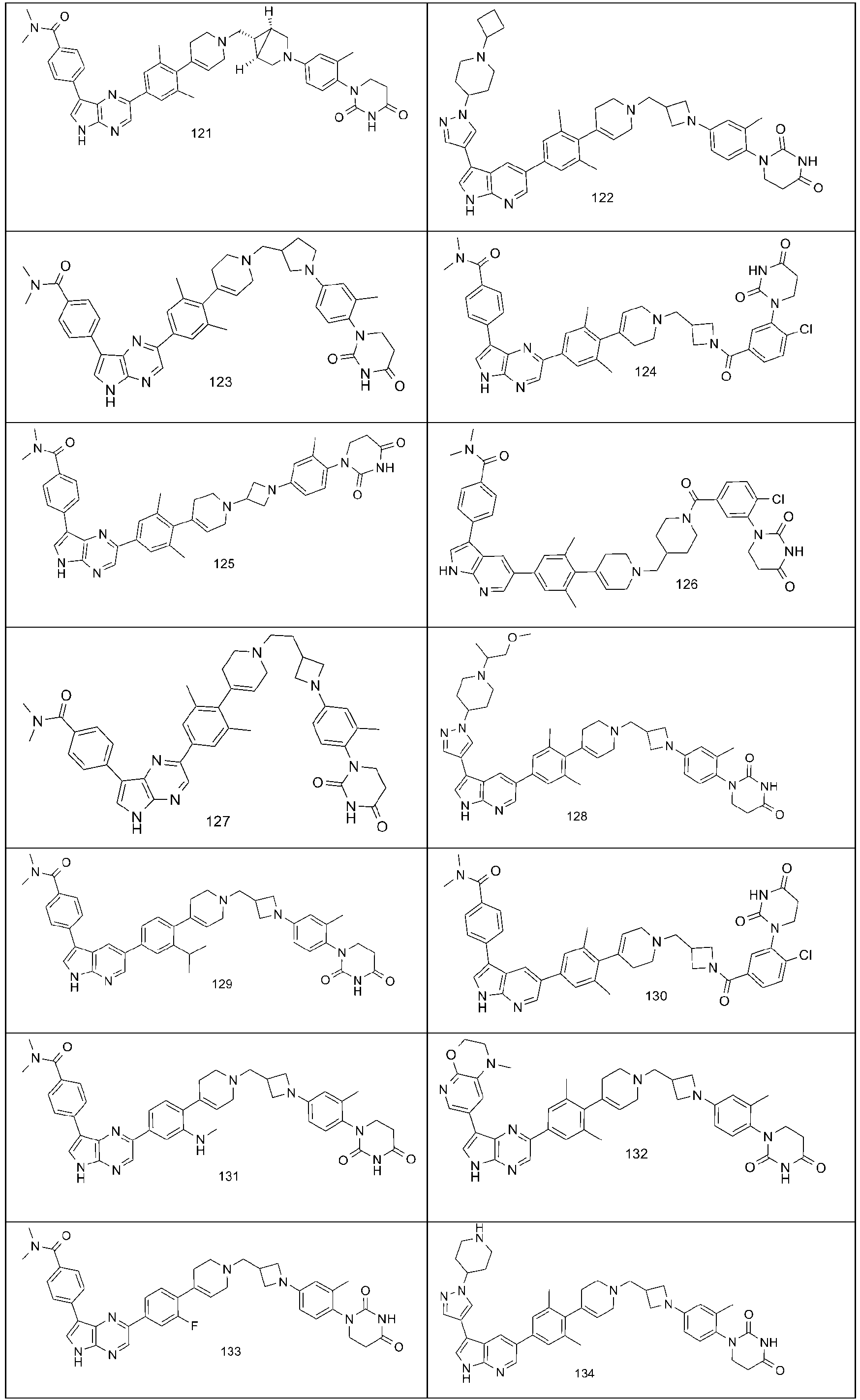

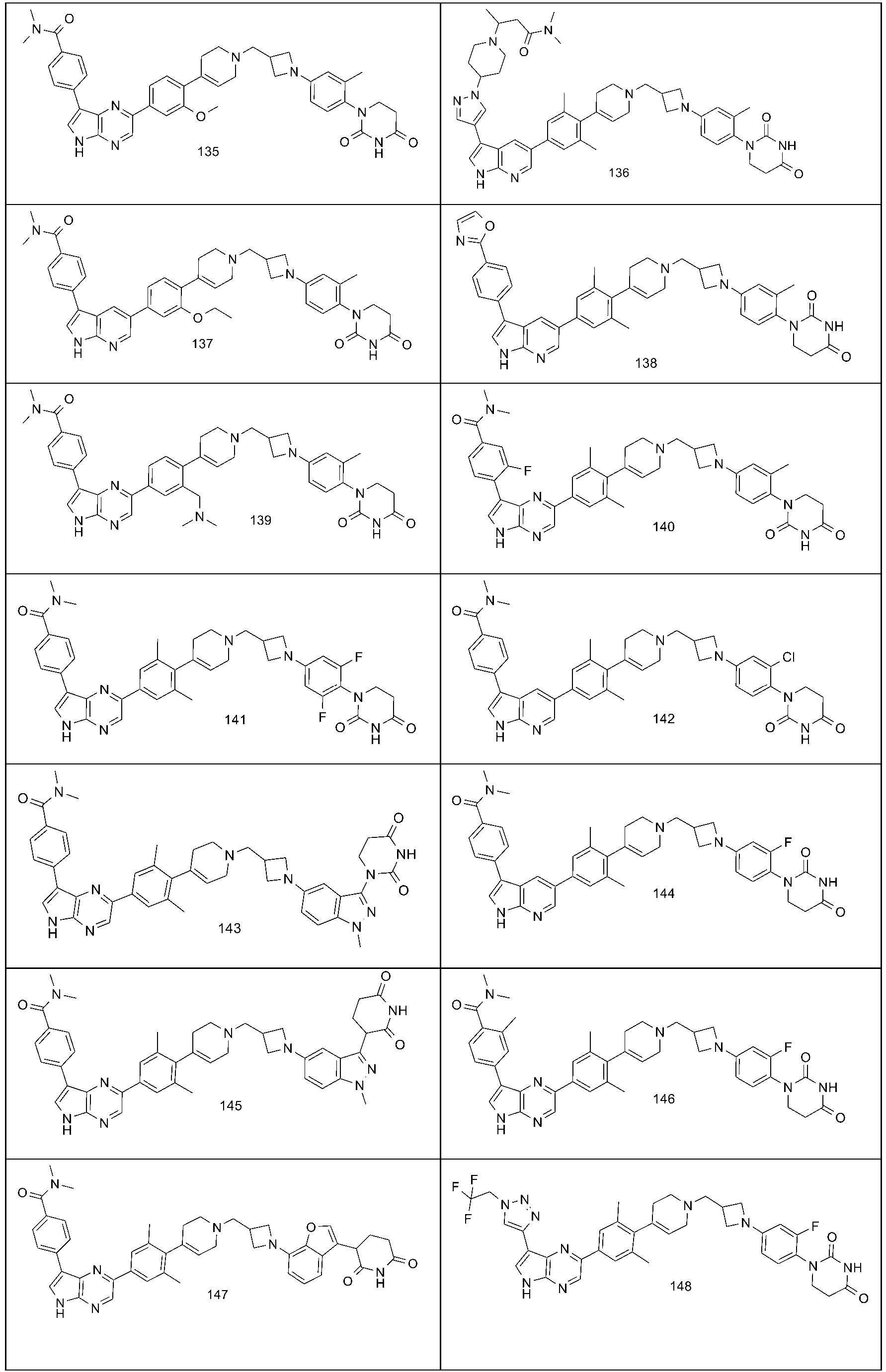

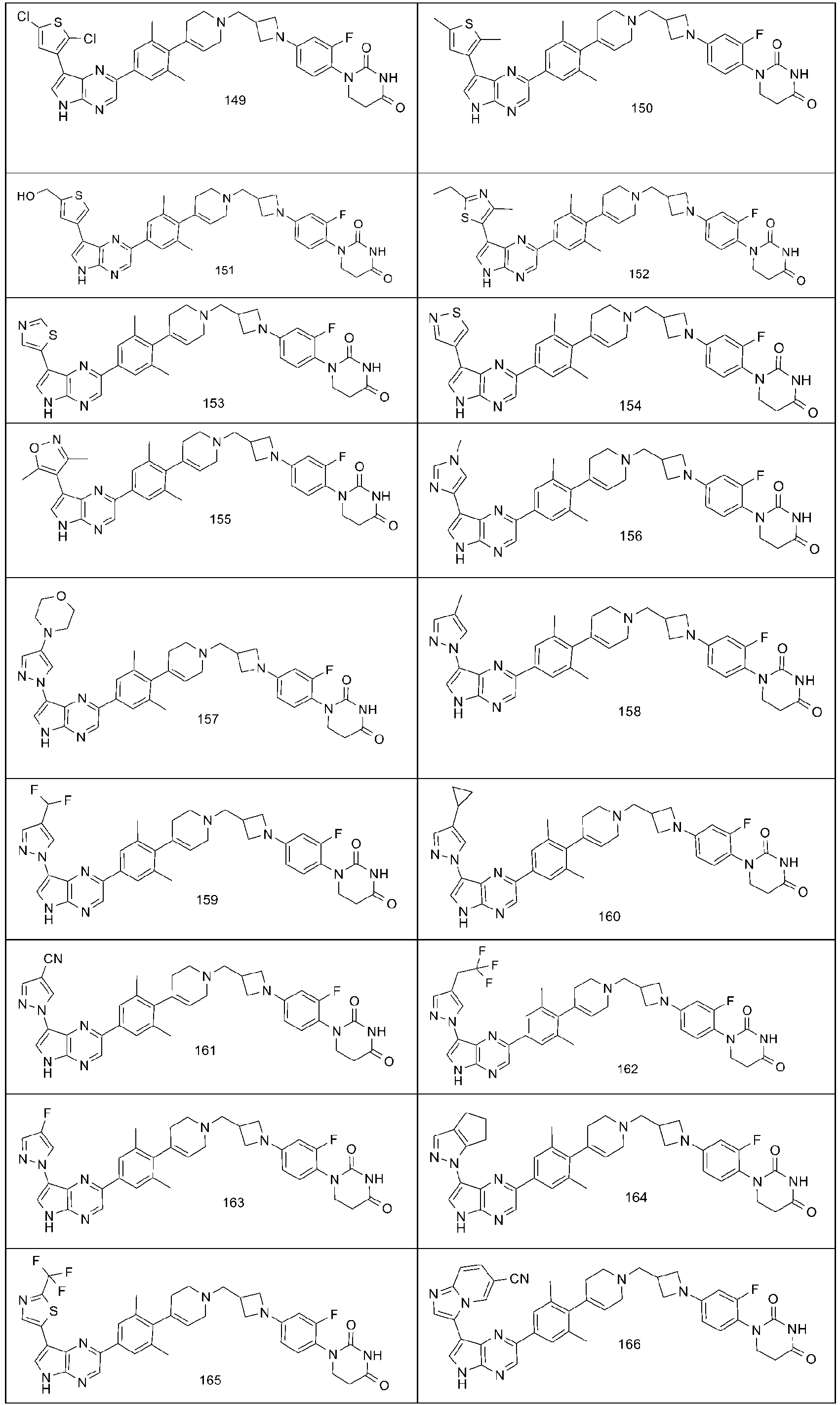

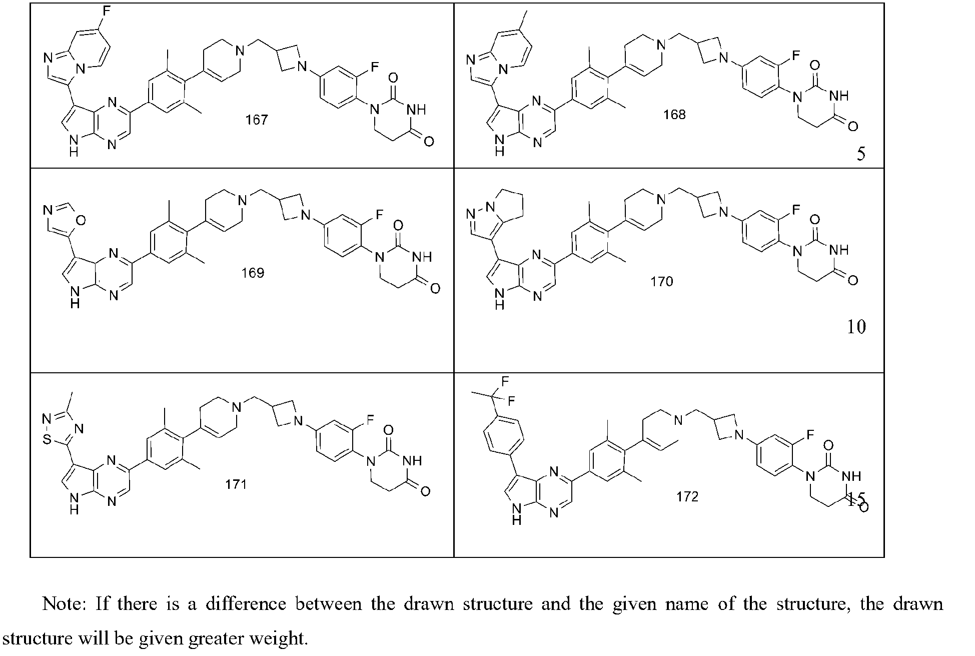

It should be noted that when specific compounds are mentioned in the invention, the corresponding number below the compound (such as 115 in the table above) corresponds to the corresponding compound; the number of the compound refers to the compound when being mentioned in other parts of the description, such as "compound 115" which corresponds to the compound structure of number 115.

Furthermore, in the same group, when substituents appear at different positions with the same letter, the substituents at these different positions are independent of each other and can be the same or different. For example, the R_{b} on the two N atoms before and after "-NR_{b}CONR_{b}-" can be the same or different. The above explanation also applies to other cases where they are the same.

When substituents with the same letter appear in different groups, the same letter acts independently and without restriction on each other in different substituents; they can be the same or different. For example, Rb is defined in R₁, L₁, L₂, R₂, Linker, R₃, etc., and R_{b} in R₁, L₁, L₂, R₂, Linker and R₃ are independent of each other and can be the same or different.

The invention provides a pharmaceutical composition, which includes a therapeutically effective amount of the compound or stereoisomers, tautomers or pharmaceutically acceptable salts thereof according to any one of general formulas (I), (VI), (IIa), (IIb), (IIIa), (IIIb), (IV), (V) or (VII), and a pharmaceutically acceptable carrier, an excipient or a combination thereof.

The pharmaceutically acceptable carrier provided by the invention be one or more solid or liquid fillers or gel substances suitable for human use. The pharmaceutically acceptable carrier be any conventional carrier and/or diluent in the field of pharmaceutical preparations, preferably having sufficient purity and sufficiently low toxicity, and being compatible with the active ingredient of the invention without significantly reducing the efficacy of the active ingredient. For example, the pharmaceutically acceptable carrier be fillers, adhesives, disintegrants, lubricants, aqueous solvents, or non-aqueous solvents.

The pharmaceutical preparations provided by the invention be formulated into any pharmaceutically acceptable dosage form and administered to patients or subjects requiring such treatment via any suitable route of administration, such as oral, parenteral, rectal, or pulmonary administration. When used for oral administration, the preparations be formulated into solid dosage forms, such as capsules, tablets, pills, lozenges, sugar-coated tablets, granules, powders, ointments, creams, drops, etc.; the preparations also be formulated into liquid dosage forms, such as elixirs, syrups, emulsions, dispersants, suspensions, solutions, sprays, etc. When used for parenteral administration, the preparations be formulated into injection solutions, sterile powders for injection, etc.

The invention provides a compound of general formulas (I), (IIa), (IIb), (IIIa), (IIIb), (IV), (V) or (VII), or stereoisomers, tautomers or pharmaceutically acceptable salts thereof, or an application of the pharmaceutical composition thereof in preparing a drug that degrades HPK1 protein.

The invention provides a compound of general formulas (I), (IIa), (IIb), (IIIa), (IIIb), (IV), (V) or (VII), or stereoisomers, tautomers or pharmaceutically acceptable salts thereof, and an application of the pharmaceutical composition thereof in preparing drugs for preventing and/or treating diseases related to activities or expression levels of HPK1 protein, wherein the diseases include solid tumors, hematological disorders, or autoimmune diseases.

The invention provides a compound of general formulas (I), (IIa), (IIb), (IIIa), (IIIb), (IV), (V) or (VII), or stereoisomers, tautomers or pharmaceutically acceptable salts thereof, and an application of the pharmaceutical, composition thereof in preparing drugs for preventing and/or treating diseases related to activities or expression levels of HPK1 protein, wherein the solid tumor diseases mentioned include, but are not limited to, one or more of lung cancer, squamous cell carcinoma, bladder cancer, stomach cancer, ovarian cancer, peritoneal cancer, breast cancer, mammary ductal carcinoma, head and neck cancer, endometrial cancer, uterine cancer, rectal cancer, liver cancer, kidney cancer, renal pelvis cancer, esophageal cancer, esophageal adenocarcinoma, glioma, prostate cancer, thyroid cancer, female reproductive system cancers, in situ pain, lymphoma, neurofibromatosis, bone cancer, skin cancer, brain cancer, colon cancer, testis, gastrointestinal stromal tumors, oral cancer, pharyngeal cancer, colonic villonoma, melanoma, cell tumors and sarcomas; the autoimmune diseases include one or more of inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Odd's thyroiditis, Graves' disease, rheumatoid arthritis syndrome, and multiple sclerosis, infectious neuritis, acute disseminated encephalomyelitis, Addison's disease, oculoclonus-myoclonus syndrome, ankylosing spondylitis, antiphospholipid antibody syndrome, aplastic anemia, autoimmune hepatitis, celiac disease, Goodpasseur syndrome, immune thrombocytopenic purpura, optic neuritis, scleroderma, and primary biliary cirrhosis, Leter syndrome, high-stress arteritis, temporal arteritis, warm autoimmune hemolytic anemia, Wegener's granulomatosis, psoriasis, alopecia areata, Behget's disease, chronic fatigue, familial autonomic dysfunction, endometriosis, interstitial cystitis, neuromuscular rigidity, scleroderma or vulvar pain, and chronic graft-versus-host disease. The hematologic diseases include, but are not limited to, one or more of the following: acute myeloid leukemia, acute lymphoblastic leukemia, chronic myeloid leukemia, myelofibrosis, myelodysplastic syndrome, diffuse large B-cell lymphoma, follicular lymphoma, chronic lymphocytic leukemia, small lymphocytic lymphoma, mantle cell lymphoma, Waldenström macroglobulinemia, multiple myeloma, and T-cell lymphoma.

### Explanation of Terminology

Unless otherwise stated, some terms used in the specification and claims are defined as follows.

A "bond" indicates that the substituent is absent, and the two ends of the substituent are directly connected to form a bond.

When "alkyl group" is used as a group or part of a group, it refers to an aliphatic hydrocarbon group that includes C₁-C₂₀ straight chains or branches. Preferably, the "alkyl group" is C₁-C₁₀ alkyl group, and more preferably, the "alkyl group" is C₁-C₈ alkyl group. Embodiments of the alkyl include, but are not limited to, methyl group, ethyl group, n-propyl group, isopropyl group, n-butyl group, isobutyl group, tert-butyl group, sec-butyl group, n-pentyl group, 1,1-dimethylpropyl group, 1,2-dimethylpropyl group, 2,2-dimethylpropyl group, 1-ethylpropyl group, 2-methylbutyl group, 3-methylbutyl group, n-hexyl group, 1-ethyl-2-methylpropyl group, 1,1,2-trimethylpropyl group, 1,1-dimethylbutyl group, 1,2-dimethylbutyl group, 2,2-dimethylbutyl group, 1,3-dimethylbutyl group, 2-ethylbutyl group, 2-methylpentyl group, 3-methylpentyl group, 4-methylpentyl group, 2,3-dimethylbutyl group, etc. The alkylmay be substituted or unsubstituted.

The "alkylene group" refers to a divalent alkyl group, wherein the alkyl group is as defined above. The alkylene group is preferably an alkylene group having 1 to 12 carbon atoms (i.e., C₁-₁₂ alkylene group), more preferably an alkylene group containing 1 to 6 carbon atoms (i.e., C₁-₆ alkylene group), and even more preferably an alkylene group containing 1 to 4 carbon atoms (i.e., C₁-₄ alkylene group). Non-limiting examples of the alkylene include, but are not limited to, methylene group (-CH₂-), 1,1-ethylene group (-CH(CH₃)-), 1,2-ethylene group (-CH₂CH₂)-, 1,1-propylene group (-CH(CH₂CH₃)-), 1,2-propylene group (-CH₂CH(CH₃)-), 1,3-propylene group (-CH₂CH₂CH₂-)), and 1,4-butylene group (-CH₂CH₂CH₂CH₂-). The alkylene group may be substituted or unsubstituted; when substituted, the alkylene group be substituted at any usable junction; the substituents be selected from one or more of alkyl, alkenyl, alkynyl, alkoxy, haloalkoxy, alkylthio, alkylamino, halogen, mercapto, hydroxyl, nitro, cyano, cycloalkyl, heterocyclic, aryl, heteroaryl, cycloalkoxy, heterocyclic alkoxy, cycloalkylthio, heterocyclic alkylthio, and oxo.

The "alkenyl group" refers to an aliphatic hydrocarbon group containing a carbon-carbon double bond, which may be straight-chain or branched. Preferably, the "alkenyl group" is C₂-C₁₀ alkenyl group, and more preferably, the "alkenyl group" is C₂-C₈ alkenyl group. Representative examples include, but are not limited to, vinyl, 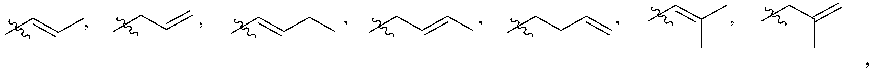 etc. The alkenylmay be substituted or unsubstituted.

The "alkyne group" refers to an aliphatic hydrocarbon group containing a carbon-carbon triple bond, which be straight-chain or branched. Preferably, the "alkyne group" is C₂-C₁₀ alkyne group, more preferably C₂-C₈ alkenyl group, and most preferably C₂-C₄ alkyne group. Examples of the alkyne include, but are not limited to, acetylene, 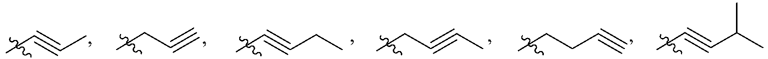 etc. The alkynemay be substituted or unsubstituted.

The "alkenyl group" refers to a divalent straight-chain or branched aliphatic hydrocarbon group containing one or more carbon-carbon double bonds, having a specified number of carbon atoms, such as 2 to 8 carbon atoms, for example -CH=CH-, -CH2CH=CH-, -C(CH3)=CH-, etc., and the alkenyl group optionally be substituted by one or more (such as 1 to 3) identical or different substituents.

The "ynylene group" refers to a divalent straight-chain or branched hydrocarbon group having one or more carbon-carbon triple bonds, containing a specified number of carbon atoms, such as 2 to 8 carbon atoms, including but not limited to, etc., and the ynylene group optionally be substituted by one or more (such as 1 to 3) identical or different substituents.

The "cycloalkyl group" refers to a monocyclic, fused, bridged and spirocyclic carbon ring that is saturated or partially saturated Preferably, the "cycloalkyl group" is C₃-C₁₂ cycloalkyl group, more preferably C₃-C₈ cycloalkyl group, and most preferably C₃-C₆ cycloalkyl group. Examples of monocyclic cycloalkyl include, but are not limited to, cyclopropyl, cyclobutyl, cyclopentyl, cyclopentenyl, cyclohexyl, cyclohexenyl, cyclohexadienyl cycloheptyl, cycloheptanetrienyl, cyclooctyl, etc., with cyclopropyl and cyclohexenyl being preferred. The cycloalkylmay be substituted or unsubstituted.

The "cycloalkylene group" refers to a divalent monocyclic, fused, bridged and spirocyclic carbon ring that is saturated or partially saturated, is connected to a group by one single bond and to other by another single bond. For example, C₃-₁₀ cycloalkylene group contains 3-10 carbon atoms, and C₃₋₆ cycloalkylene group contains 3-6 carbon atoms. Common cycloalkylene include (but are not limited to) cyclopropane-1,1-yl group, cyclopropane-1,2-yl group, cyclobutane-1,1-yl group, cyclobutane-1,2-yl group, cyclobutane-1,3-yl group, etc.

The "spirocycloalkyl group" refers to a polycyclic group with 5 to 18 members, two or more cyclic structures, and the monocyclic rings share a carbon atom (called a spiro atom) with each other. The rings contain one or more double bonds, but none of the rings are aromatic. The group preferably has 6 to 14 members, and more preferably 7 to 10 members. The spirocycloalkyl are classified into monospirocycloalkyl, bispirocycloalkyl, or multispirocycloalkyl based on the number of shared spiro atoms between rings. Monospirocycloalkyl and bispirocycloalkyl are preferred, and 4-membered/4-membered, 4-membered/5-membered, 4-membered/6-membered, 5-membered/5-membered, or 5-membered/6-membered are preferred. Examples of the "spirocycloalkyl" include, but are not limited to: spiro[4.5]decyl group, spiro[4.4]nonyl group, spiro[3.5]nonyl group, and spiro[2.4]heptyl group.

The "fused cycloalkylene group" refers to a 5-18 membered polycyclic aromatic hydrocarbon group containing two or more cyclic structures that share a pair of carbon atoms. One or more rings contain one or more double bonds, but none of the rings are aromatic. 6-12 membered are preferred, and 7-10 membered are more preferred. Based on the number of rings, the group be classified as bicyclic, tricyclic, tetracyclic or polycyclic fused cycloalkylene, preferably bicyclic or tricyclic, and more preferably 5-membered/5-membered or 5-membered/6-membered bicyclic cycloalkylene group. Examples of the "fused cycloalkylene group" include, but are not limited to bicyclo[3.1.0]hexyl group, bicyclo[3.2.0]hept-1-enyl group, bicyclo[3.2.0]heptyl group, decahydronaphthyl group or tetradecahydrophenanthryl group.

The "bridged cycloalkylene group" refers to a 5-18 membered polycyclic aromatic hydrocarbon group containing two or more cyclic structures that share two non-directly connected carbon atoms. One or more rings contain one or more double bonds, but none of the rings are aromatic. 6-14 membered are preferred, and 7-10 membered are more preferred. Based on the number of rings, the group be classified as bicyclic, tricyclic, tetracyclic or polycyclic bridged cycloalkylene, preferably bicyclic, tricyclic or tetracyclic, and more preferably bicyclic or tricyclic. Examples of the "bridged cycloalkylene group" include, but are not limited to (1s,4s)-bicyclo[2.2.1]heptyl group, bicyclo[3.2.1]octyl group, (1s,5s)-bicyclo[3.3.1]nonyl group, bicyclo[2.2.2]octyl group, (1r,5r)-bicyclo[3.3.2]decyl group, and bicyclo[1.1.1]pentyl group.

The terms "heterocyclic group," "heterocyclic ring," or "heterocyclic" are used interchangeably in the application and all refer to a non-aromatic heterocyclic group in which one or more cyclic atoms are heteroatoms, such as N, O, S, P, or Se, including monocyclic, fused, bridged, and spirocyclic rings, and contain 1 or more double bonds. Preferably, 3 to 12 ring atoms are included, more preferably 4-7 membered monocyclic or 7-10 membered bis- or tricyclic ring, which contain 1, 2 or 3 atoms selected from N, O, S(O)ₙ (where n is selected from 0, 1 or 2), P(O)ₘ (where m is selected from 0 or 1), and Se. Examples of the "heterocyclic" include, but are not limited to, morpholino group, oxetane group, thiomorpholino group, tetrahydropyrano group, 1,1-dioxo-thiomorpholino group, piperidino group, 2-oxo-piperidino group, pyrrolyl group, 2-oxo-pyrrolyl group, piperazine-2-ketone group, 8-oxa-3-aza-bicyclo[3.2.1]octyl group, piperazine group, 1,2,3,6-tetrahydropyridino group or 3,6-dihydro-2H-pyrano group. The heterocyclicmay be substituted or unsubstituted.

The "heterocycloalkylene group" refers to a divalent non-aromatic heterocyclic group in which one or more cyclic atoms are heteroatoms, such as N, O, S, P, Se, including monocyclic, fused, bridged, and spirocyclic; the ring contain 1 or more double bonds, which are connected to a group through one single bond and to other (or ring systems) through another single bond; examples include 3-10 membered heterocycloalkylene, 3-7 membered heterocycloalkylene, or 4-10 membered heterocycloalkylene; common heterocycloalkylene include (but are not limited to) oxepane-2,2-ylene group, oxepane-2,3-ylene group, aziridine-2,2-ylene group, aziridine-2,3-ylene group, aziridine-2,4-ylene group, tetrahydrofuran-2,5-ylene group, tetrahydro-2H-pyran-2,3-ylidene group, tetrahydro-2H-pyran-2,4-ylidene group, tetrahydro-2H-pyran-2,5-ylidene group, tetrahydro-2H-pyran-2,6-ylidene group, pyrrolidine-1,2-ylidene group, pyrrolidine-1,3-ylidene group, pyrrolidine-2,3-ylidene group, pyrrolidine-2,4-ylidene group, pyrrolidine-2,5-ylidene group, piperidine-1,2-ylidene group, piperidine-1,3-ylidene group, piperidine-1,4-ylidene group, piperidine-2,3-ylidene group, piperidine-2,4-ylidene group, piperidine-2,5-ylidene group, piperidine-2,6-ylidene group, etc.

The "spiro-heterocyclic group" refers to a polycyclic group with 5 to 18 members, consisting of two or more cyclic structures, where the monocyclic rings share an atom with each other. The rings contain 1 or more double bonds, but none of the rings are aromatic. One or more ring atoms are selected from heteroatoms of N, O, S(O)ₙ (where n is selected from 0, 1 or 2), P(O)ₘ (where m is selected from 0 or 1), and Se, and the remaining ring atoms are carbon. The group preferably has 6 to 14 members, and more preferably 7 to 10 members. The spiro-heterocyclic are classified into mono-spiro-heterocyclic group, bi-spiro-heterocyclic group, or multi-spiro-heterocyclic group based on the number of shared spiro atoms between rings, with mono-spiro-heterocyclic group and bi-spiro-heterocyclic group being preferred. 4 membered/4 membered, 4 membered/5 membered, 4 membered/6 membered, 5 membered/5 membered or 5 membered/6 membered mono-spiro-heterocyclic are more preferred. Examples of the "spiro-heterocyclic" include, but are not limited to 1,7-dioxaspiro[4.5]decyl group, 2-oxa-7-azaspiro[4.4]nonyl group, 7-oxaspiro[3.5]nonyl group and 5-oxaspiro[2.4]heptyl group.

The "fused heterocyclic group" refers to a fully carbon polycyclic group containing two or more cyclic structures that share a pair of atoms. One or more rings contain one or more double bonds, but none of the rings are aromatic, wherein one or more ring atoms are selected from heteroatoms of N, O, S(O)n (where n is selected from 0, 1 or 2), P(O)ₘ (where m is selected from 0 or 1), and Se, and the remaining ring atoms are carbon. The group preferably has 6 to 14 members, and more preferably 7 to 10 members. Based on the number of rings, the group be classified as bicyclic, tricyclic, tetracyclic or polycyclic fused heterocyclic, preferably bicyclic or tricyclic, and more preferably 5-membered/5-membered or 5-membered/6-membered bicyclic fused heterocyclic group. Non-limiting examples of the "fused heterocyclic" include, but are not limited to octahydropyrrolo[3,4-c]pyrrole group, octahydro-1H-isoindolyl group, 3-azabicyclo[3.1.0]hexyl group, and octahydrobenzo[b][1,4]dioxine.

The "bridged heterocyclic" refer to polycyclic with 5 to 18 members, containing two or more cyclic structures that share two atoms that are not directly connected with each other. One or more rings contain one or more double bonds, but none of the rings are aromatic, wherein one or more ring atoms are selected from heteroatoms of N, O, S(O)ₙ (where n is selected from 0, 1 or 2), P(O)ₘ (where m is selected from 0 or 1), and Se, and the remaining ring atoms are carbon. The group preferably has 6 to 14 members, and more preferably 7 to 10 members. Based on the number of rings, the group be classified as bicyclic, tricyclic, tetracyclic or polycyclic bridged heterocyclic, preferably bicyclic, tricyclic or tetracyclic, and more preferably bicyclic or tricyclic. Examples of the "bridged heterocyclic group" include, but are not limited to 2-azabicyclo[2.2.1]heptyl group, 2-azabicyclo[2.2.2]octyl group and 2-azabicyclo[3.3.2]decyl group.

The "aryl group" refers to a carbocyclic aromatic system containing one or two rings, wherein the rings be connected together in a fused manner. The "aryl group" includes monocyclic or bicyclic aryl, such as aromatic of phenyl group, naphthyl group, and tetrahydronaphthyl group. Preferably, the aryl group is C₆-C₁₀ aryl group, more preferably phenyl group and naphthyl group, and most preferably phenyl group. The aryl be substituted or unsubstituted.

The "arylene group" refers to two monovalent group centers obtained by removing two hydrogen atoms from the same carbon atom or two different carbon atoms of the parent aryl group of the aryl group defined herein. Typical arylene include, but are not limited to, phenylene group and naphthylene group.

The terms "heteroaryl group" and "heteroary ring" are used interchangeably in the application, both referring to a monocyclic or polycyclic aromatic cyclic group containing 5 to 14 cyclic atoms, which contain 1 to 4 atoms selected from N, O, S, and Se. Preferably, 5 to 12 ring atoms are included, and 5-6 membered monocyclic heteroaryl group or 8-10 membered bicyclic heteroaryl group is more preferred. Examples of the "heteroaryl group" include, but are not limited to, furanyl group, pyridyl group, 2-oxo-1,2-dihydropyridyl group, pyridinyl group, pyrimidinyl group, pyrazinyl group, thiophenyl group, isoxazolyl group, oxazolyl group, oxadiazolyl group, imidazole group, pyrrole group, pyrazol group, triazol group, tetrazol group, thiazolyl group, isothiazol group, 1,2,3-thiadiazol group, benzo[m]dioxacyclopentenyl group, benzothiophene group, benzimidazolyl group, indolyl group, isoyindolyl group, 1,3-dioxo-isoindolyl group, quinolinyl group, indazole group, benzisothiazolyl group, benzoxazolyl group, benzoxazolyl group,

The heteroaryl may be substituted or unsubstituted.

The "heteroarylene group" refers to two monovalent group centers obtained by removing two hydrogen atoms from the same carbon atom or two different carbon atoms of the parent heteroaryl group of the heteroaryl group described herein, or by removing one hydrogen atom from a carbon atom and one hydrogen atom from a nitrogen atom.

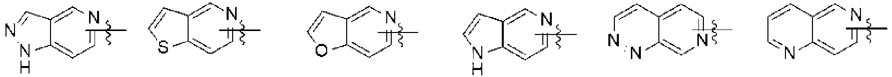

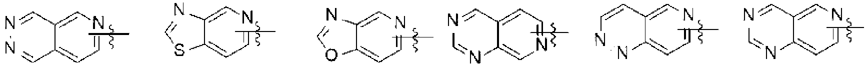

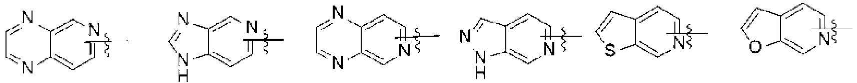

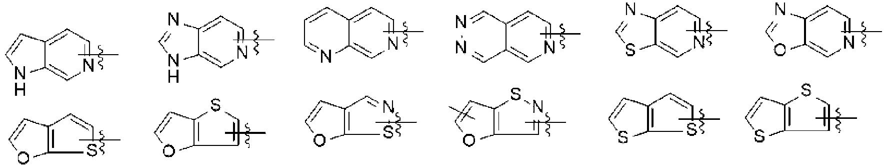

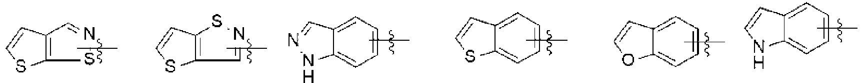

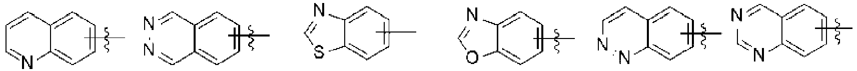

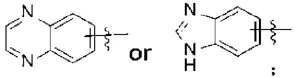

A "fused ring" refers to a polycyclic group in which two or more cyclic structures share a pair of atoms. One or more rings contain one or more double bonds, but at least one ring is not aromatic, and at least one ring is aromatic, wherein 0, 1 or more ring atoms are selected from heteroatoms of N, O, S(O)ₙ (where n is selected from 0, 1 or 2), P(O)ₘ (where m is selected from 0 or 1), and Se, and the remaining ring atoms are carbon. The fused ring preferably includes a bicyclic or tricyclic fused ring, wherein the bicyclic fused ring is preferably a fused ring of an aryl group or a heteroaryl group with a monocyclic heterocyclic group or a monocyclic cycloalkyl group. The group preferably has 7 to 14 members, and more preferably 9 to 10 members. Examples of the "fused ring" include, but are not limited to

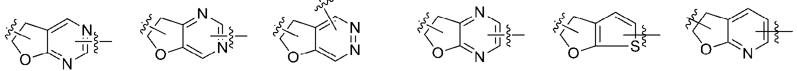

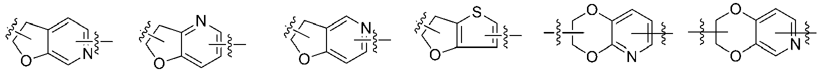

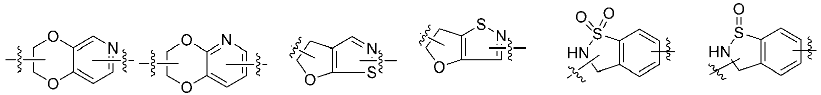

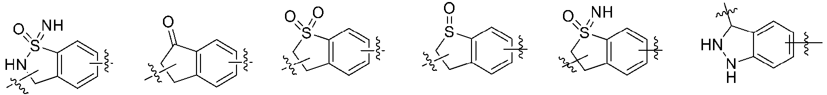

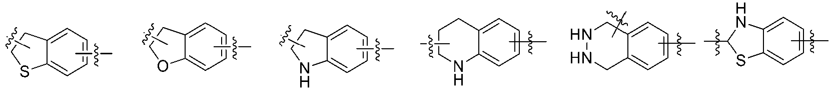

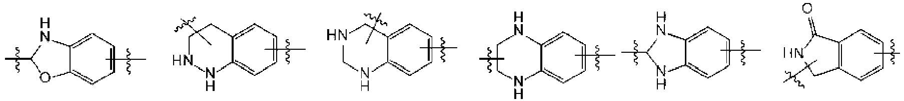

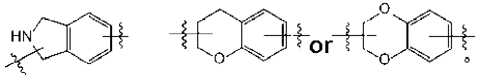

The fused ring may be substituted or unsubstituted.

The "alkoxy group" refers to a (alkyl group-O-) group. The alkyl are defined in the relevant section of the description. The alkoxy group of C₁-C₈ are preferred. Examples include, but are not limited to methoxy group, ethoxy group, n-propoxy group, isopropoxy group, n-butoxy group, isobutoxy group, tert-butoxy group, etc. The alkoxy be substituted or unsubstituted.

The "alkenyloxy group" refers to a (alkenyl group-O-) group. The alkenyl are defined in the relevant section of the description. The alkenyloxy group of C₂-C₈ are preferred. The alkenyloxy be substituted or unsubstituted.

The "hydroxyalkyl group" is a (-alkyl group-OH) group. The alkyl are defined in the relevant section of the description. The hydroxyalkyl group of C₁-C₈ are preferred. Examples include, but are not limited to hydroxymethyl group, hydroxyethyl group, hydroxypropyl group, hydroxyisopropyl group, hydroxybutyl group, etc. The hydroxyalkylmay be substituted or unsubstituted.

The "alkylamino group" refers to a (alkyl group-NH-) group. The alkyl are defined in the relevant section of the description. The alkylamino group of C₁-C₈ are preferred. Examples include, but are not limited to, methylamino group, ethylamino group, n-propylamino group, isopropylamino group, n-butylamino group, isobutoxy group, tert-butoxy group, etc. The alkylamino be substituted or unsubstituted, and the substituents be on the alkyl group or on the N group, such as dimethylamino group and diethylamino group.

The "aminoalkyl group" refers to a (-alkyl-NH₂) group. The alkyl are defined in the relevant section of the description. Examples include, but are not limited to aminomethyl group, aminoethyl group, aminopropyl group, aminoisopropyl group, aminobutyl group, aminopentyl group, etc. The aminoalkyl be substituted or unsubstituted, and the substituents be on the alkyl group or on the N group, such as dimethylaminoalkyl group.

The "alkyl carbonyl group" refers to a (alkyl-C(O)-) group. The alkyl are defined in the relevant section of the description. Examples include, but are not limited to methyl carbonyl group, ethyl carbonyl group, n-propyl carbonyl group, isopropyl carbonyl group, n-butyl carbonyl group, isobutyl carbonyl group, etc. The alkyl carbonylmay be substituted or unsubstituted.

The "alkoxycarbonyl group" refers to a (alkyl-O-C(O)-) group. The alkyl are defined in the relevant section of the description. Examples include, but are not limited to methoxycarbonyl group, ethoxycarbonyl group, n-propoxycarbonyl group, isopropoxycarbonyl group, etc. The alkoxycarbonyl may be substituted or unsubstituted.

The "haloalkyl group" refers to alkyl that have been substituted by halogen. The halogen and the alkyl are defined in the relevant section of the description.

The "haloalkenyloxy" refers to alkenyloxy that have been substituted by halogen. The halogen and the alkenyloxy are defined in the relevant section of the description.

The "halohydroxyalkyl" refers to hydroxyalkyl that have been substituted by halogen. The halogen and the hydroxyalkyl are defined in the relevant section of the description.

The "haloalkylamino group" refers to alkylamino that have been substituted by halogen. The halogen and the alkylamino are defined in the relevant section of the description.

The "cycloalkoxy group" refers to a (cycloalkyl-O-) group. The cycloalkyl are defined in the relevant section of the description.

The "heteroepoxy group" refers to a (heterocyclic-O-) group. The heterocyclic are defined in the relevant section of the description.

The "hydroxy group" refers to the -OH group.

The "halogen" refers to fluorine, chlorine, bromine, and iodine.

The "amino group" refers to -NH₂.

The "cyano group" refers to -CN.

The "nitro group" refers to -NO₂.

The "carboxyl group" refers to -C(O)OH.

The "amide group" refers to -C(O)NH₂.

The "substituted" refers to one or more hydrogen atoms in a group, preferably 1 to 5, more preferably 1 to 3 hydrogen atoms that are independently substituted by the corresponding number of substituents. It goes without saying that substituents are only in their possible chemical positions, and those skilled in the art determine (by experiment or theory) possible or impossible substitutions without much effort. For example, the amino or hydroxyl with free hydrogen be unstable when combined with carbon atoms with unsaturated (such as olefins) bonds.

Unless otherwise specified, the terms "substitution" or "substituted" in the specification mean that a group be substituted by one or more selected from H, deuterium, halogen, C₁-C₈ alkyl, C₁-C₈ alkoxy, C₂-C₈ alkenyl, C₂-C₈ alkynyl, C₂-C₈ alkenyloxy, C₃-C₁₂ cycloalkyl, C₃-C₈ cycloalkoxy, 3-12 membered heterocyclic, 3-12 membered heteroepoxy, aminosulfonyl, C₆-C₁₀ aryl, 5-12 membered heteroaryl, cyano, amino, nitro, hydroxy, oxo, carboxyl, amide, hydroxyalkyl, aminoalkyl, alkyl carbonyl, alkoxycarbonyl, C₁-C₈ alkylamino, C₁-C₈ haloalkylamino, -OR_{g}, -SR_{g}, -C₁-C₈ alkylene-R_{g}, -OC(O)R_{g}, -C(O)R_{g}, -C(O)OR_{g}, -C(O)N(Rₓ)R_{y}, -NRₓR_{y}, -N(CH₃)R_{g}, -N(Rₓ)C(O)R_{y}, -N(Rₓ)C(O)NRₓR_{y}, -N(Rₓ)C(O)OR_{g}, -N(Rₓ)S(O)NRₓR_{y}, -N(Rₓ)S(O)₂NRₓR_{y}, -N(Rₓ)S(O)₂R_{g}, -S(O)R_{g}, -S(O)₂R_{g}, -S(O)₂NRₓR_{y} or -P(O)RₓR_{y}; the alkyl, the alkylene, the alkoxy, the alkenyl, the alkynyl, the alkenyloxy, the cycloalkyl, the cycloalkoxy, the heterocyclic, the heteroepoxy, the aryl, the heteroaryl, 3-12 membered ring, the amino, the hydroxyl or the amide optionally be further substituted with one or more Rₒ;
when two Rₒ are substituted on the same atom, the two Rₒ together with the atoms attached thereto form a 3-6 membered ring; or when two Rₒ are substituted on adjacent atoms, the two Rₒ together with the atoms attached thereto form a 3-12 membered ring;
R_{g}, Rₓ, R_{y} and Rₒ are each independently selected from H, deuterium, halogen, C₁-C₈ alkyl, C₁-C₈ alkoxy, C₂-C₈ alkenyl, C₂-C₈ alkynyl, C₂-C₈ alkenyloxy, C₃-C₁₂ cycloalkyl, C₃-C₈ cycloalkoxy, 3-12 membered heterocyclic, 3-12 membered heteroepoxy, aminosulfonyl, C₆-C₁₀ aryl, 5-12 membered heteroaryl, cyano, amino, nitro, hydroxy, oxo, carboxyl, amide, hydroxyalkyl, aminoalkyl, alkyl carbonyl, alkoxycarbonyl, C₁-C₈ alkylamino, C₁-C₈ haloalkylamino, -ORₛ, -SRₛ, -C₁-C₈ alkylene-Rₛ, -OC(O)Rₛ, -C(O)Rₛ, -C(O)ORₛ, -C(O)N(Rₛ)Rₜ, -NRₛRₜ, -N(CH₃)Rₛ, -N(Rₛ)C(O)Rₜ, -N(Rₛ)C(O)NRₛRₜ, -N(Rₛ)C(O)ORₜ, -N(Rₛ)S(O)NRₛRₜ, -N(Rₛ)S(O)₂NRₛRₜ, -N(Rₛ)S(O)₂Rₜ, -S(O)Rₛ, -S(O)₂Rₛ, -S(O)₂NRₛRₜ or -P(O)RₛRₜ; the alkyl, the alkylene, the cycloalkyl, the heterocyclic, the aryl or the heteroaryl optionally be further substituted with one or more Rᵣ;
when two Rᵣ are substituted on the same atom, the two Rᵣ together with the atoms attached thereto form a 3-6 membered ring; or when two Rᵣ are substituted on adjacent atoms, the two Rᵣ together with the atoms attached thereto form a 3-12 membered ring;
Rᵣ, Rₛ and Rₜ are each independently selected from H, deuterium, C1-C8 alkyl, C₂-C₈ alkenyl, C2-C8 alkynyl, halogen, cyano, amino, nitro, hydroxyl, oxo, C₁-C₈ alkoxy, C₁-C₈ haloalkyl, hydroxyalkyl, aminoalkyl, C₁-C₈ alkylamino, alkyl carbonyl, alkoxycarbonyl, halohydroxyalkyl, C₁-C₈ haloalkylamino, C₃-C₁₂ cycloalkyl, 3-12 membered heterocyclic, carboxyl, amide, C₆-C₁₀ aryl or 5-12 membered heteroaryl.

The compound of the invention contain asymmetric centers or chiral centers, and thus exist in different stereoisomer forms. It is contemplated that all stereoisomers of the compound of the invention, including but not limited to diastereomers, enantiomers and atropisomers and geometric (conformational) isomers and mixtures thereof, such as racemic mixtures, are within the scope of the invention.

Unless otherwise indicated, the structure described in the invention also includes all isomers of the structure (e.g., diastereomers, enantiomers, and shunting isomers and geometric (conformal) isomers; for example, R and S configurations of each asymmetric center, (Z) and (E) double bond isomers, and (Z) and (E) conformational isomers). Therefore, individual stereoisomers of the compound of the invention, as well as enantiomer mixtures, diastereomer mixtures, and geometric (conformational) isomer mixtures, are all within the scope of the invention.

The C, H, O, S, N, F, Cl, Br, I, etc. involved in the and compound described in the invention include their isotopic forms. The C, H, O, S, N, F, Cl, Br, and I involved in the and compounds described in the invention optionally be substituted by one or more of their corresponding isotopes, including but not limited to: carbon isotopes ¹²C, ¹³C, and ¹⁴C; hydrogen isotopes protium (H), deuterium (D), and tritium (T); oxygen isotopes ¹⁶O, ¹⁷O, and ¹⁸O; sulfur isotopes ³²S, ³³S, ³⁴S, and ³⁶S; nitrogen isotopes ¹⁴N and ¹⁵N; fluorine isotopes ¹⁷F and ¹⁹F; chlorine isotopes ³⁵Cl and ³⁷Cl; and bromine isotopes ⁷⁹Br and ⁸¹Br, etc.

It should be understood that the general description above and the detailed description below are merely exemplary and explanatory, and are not restrictive on any of the claims. It should be noted that, unless otherwise stated in the specification and appended claims, singular forms such as "a," "an," and "the" include plural forms. It should also be noted that, unless otherwise stated, "or" means "and/or". Furthermore, terms such as "comprises" and "includes" are not restrictive.

"Pharmaceutically acceptable salts" refers to certain salts of the above compounds that can retain their original biological activity and are suitable for pharmaceutical use. The pharmaceutically acceptable salts of the compound represented by formula (I) be a metal salt, a salt formed with a suitable acid, or a salt formed with a suitable base. A preferred type of salt is the salt formed by the compound of the invention and an acid; the acids suitable for forming the salt include, but are not limited to inorganic acids such as hydrochloric acid, hydrobromic acid, hydrofluoric acid, hydroiodic acid, sulfuric acid, nitric acid, phosphoric acid, and carbonic acid; and organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, p-toluenesulfonic acid, ethanesulfonic acid, benzenesulfonic acid, camphorsulfonic acid, citric acid, isonicotinic acid, salicylic acid, ascorbic acid, gentian acid, gluconic acid, pyruvic acid, naphthalenesulfonic acid, stearic acid, phenylacetic acid, p-aminobenzenesulfonic acid, hydroxyethanesulfonic acid, dihydroxynaphthalic acid, and tannic acid, as well as acidic amino acids such as aspartic acid and glutamic acid. One preferred type of salt is the salt formed by the compound of the invention and a base. Suitable bases for forming salts include, but are not limited to, inorganic bases such as sodium hydroxide, potassium hydroxide, sodium carbonate, sodium bicarbonate, and sodium phosphate, and organic bases such as ammonia, triethylamine, diethylamine, piperazine, guanidine, and diethanolamine.

When applied to animals, humans, experimental subjects, cells, tissues, organs, or biological fluids, "administration," "administering," "leating," and "treatment" mean contact between an exogenous drug, therapeutic agent, diagnostic agent, or composition and the animal, human, subject, cell, tissue, organ, or biological fluid. Cell processing encompasses contact between reagents and cells, as well as contact between reagents and fluids, wherein the fluids are in contact with the cells. The terms "administration," "administering," "leating," and "treatment" also refer to in vitro and ex vivo treatment by means of reagents, diagnostic agents, conjugated compounds, or by means of another cell, such as cells. The term "subject" used herein includes any living organism, preferably an animal, more preferably a mammal (e.g., rat, mouse, dog, cat, and rabbit), and most preferably a human.

"Effective amount" or "therapeutically effective amount" means an amount of an active ingredient (such as a compound) that is sufficient to affect such treatment of a disease, disorder, or symptom when the compound is administered to a subject to treat a disease or at least one clinical symptom of a disease or disorder. The "therapeutically effective amount" vary depending on the following: compound, disease, disorder, and/or symptoms of disease or disorder, severity of disease, disorder, and/or symptoms of disease or disorder, age of the subject to be treated, and/or weight of the subject to be treated. In any given example, the suitable/appropriate amount will be clear to those skilled in the art, or can be determined by routine experiments. In some implementations, the "therapeutically effective amount" is the amount of at least one compound disclosed herein and/or at least one stereoisomer thereof and/or at least one pharmaceutically acceptable salt thereof that is effective in "treating" (as defined above) a disease or disorder of a subject. In the case of combination therapy, the "therapeutically effective amount" refers to the total amount of a combination of substances used to effectively treat a disease, disorder, or symptom.

"Pharmaceutically acceptable carrier" refers to one or more solid or liquid fillers or gel substances suitable for human use. The pharmaceutically acceptable carrier be any conventional carrier and/or diluent in the field of pharmaceutical preparations, preferably having sufficient purity and sufficiently low toxicity, and being compatible with the active ingredient of the invention without significantly reducing the efficacy of the active ingredient. For example, the pharmaceutically acceptable carrier be fillers, adhesives, disintegrants, lubricants, aqueous solvents, or non-aqueous solvents. The amount of active ingredient that can be combined with a carrier substance to produce a single dosage form usually refers to the amount of compound that can produce a therapeutic effect.

The "pharmaceutical preparations" mean any pharmaceutically acceptable dosage form prepared for administration to a patient or subject in need of such treatment by any suitable route of administration, such as local, oral, transdermal, rectal, vaginal, non-intestinal, intranasal, intrapulmonary, intraocular, intravenous, intramuscular, intraarterial, intrathecal, intradermal, intraperitoneal, subcutaneous, subcutaneous, or inhalation. The pharmaceutical compositions containing active ingredients be in forms suitable for oral administration, such as tablets, sugar tablets, lozenges, liquid formulations such as water or oil suspensions, dispersible powders or granules, emulsions, hard or soft capsules, or syrups, elixirs, solutions or suspensions. The tablets contain the active ingredient and a non-toxic, pharmaceutically acceptable carrier suitable for tablet preparation, used for mixing. For parenteral administration, the pharmaceutical composition be a solution, aqueous solution, oil suspension concentrate, lyophilized powder, etc. Preferably, the formulation of the pharmaceutical composition is selected from tablets, coated tablets, capsules, suppositories, nasal sprays, or injections, more preferably tablets or capsules. The pharmaceutical composition be administered as a single unit with an accurate dosage. In addition, the pharmaceutical composition contain other active ingredients. The dosage forms for local or transdermal administration include powders, sprays, ointments, pastes, creams, lotions, gels, solutions, patches, and inhalers. The active compound be mixed with a pharmaceutically acceptable carrier under sterile conditions, and be mixed with any preservatives, buffers or propellants that be required.

The term "disease" refers to any illness, discomfort, ailment, symptom, or indication, and is interchangeable with the terms "symptom" or "disorder".

Experimental results show that the compound of the invention has good inhibitory activity against HPK1 kinase.

Experimental results show that the compound of the invention has a strong ability to degrade HPK1 protein.

Experimental results show that the compound of the invention has high selectivity for the degradation of HPK1 protein.

Experimental results show that oral administration of the compound of the invention provides good exposure levels.

### Detailed Description of the Invention

The chemical substances represented by some of the abbreviations in the invention are as follows: DCM: dichloromethane; DMF: N,N-dimethylformamide; HATU: 2-(7-azabenzotriazole)-N,N,N',N'-tetramethylurea hexafluorophosphate; H₂SO₄: sulfuric acid; STAB: sodium triacetylborohydride; Dioxane: dioxane; Pd(dppf)Cl₂: [1,1'-bis(diphenylphosphino)ferrocene]palladium dichloride; Pd/C: palladium on carbon.

### Embodiment 1: Synthesis of intermediate A1

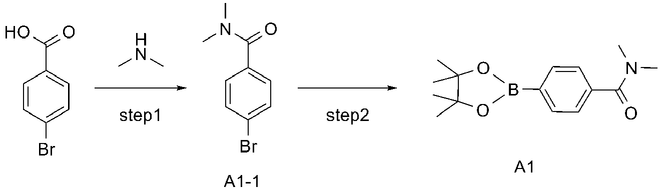

### Synthesis Step 1: Synthesis of A1-1

4-Bromobenzoic acid (3g, 15.00mmol) is dissolved in DCM (30mL), and dimethylamine (810mg, 18.00mmol), HATU (8.55g, 22.50mmol), and triethylamine (3.04g, 30mmol) are added to the system in sequence, to perform the reaction at room temperature for 5h. After the reaction is completed, quenching is performed with water, and extraction is performed with dichloromethane; the organic phases are combined, drying is performed over anhydrous sodium sulfate, and concentration is performed under reduced pressure. 2.2 g of A1-1 is obtained by column chromatography, with a yield of 65%. ESI-MS(M+H)⁺ =228.0.

### Synthesis Step 2: Synthesis of A1

A1-1 (2.00 g, 8.81 mmol) is dissolved in dioxane (20 mL), and bis(pinacolato)diboron (3.34 g, 13.21 mmol), [1,1'-bis(diphenylphosphino)ferrocene]palladium dichloride (644 mg, 0.88 mmol), and potassium acetate (2.60 g, 26.43 mmol) are added to the system in sequence, to perform the reaction at 80 °C under nitrogen protection for 4 h. After the reaction is completed, quenching is performed with water, and extraction is performed with dichloromethane; the organic phases are combined, drying is performed over anhydrous sodium sulfate, and concentration is performed under reduced pressure. 1.5 g of A1 is obtained by column chromatography, with a yield of 67%. ESI-MS(M+H)⁺=276.2.

Following the synthesis route and method for the intermediate A1, the following intermediate compounds are obtained by synthesis:

| No. of intermediate | Structure of intermediate and ESI-MS(M+H)⁺ | Intermediate No. | Structure of intermediate and ESI-MS(M+H)⁺ |
|---|---|---|---|
| A2 | 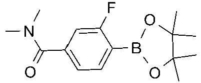 ESI-MS(M+H)⁺=294.2 | A3 | 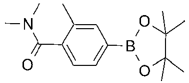 ESI-MS(M+H)⁺=290.2 |
| A4 | 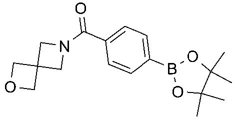 ESI-MS(M+H)⁺=330.2 | A5 | 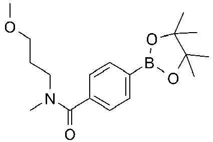 ESI-MS(M+H)⁺=334.2 |
| A6 | 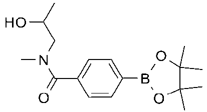 ESI-MS(M+H)⁺=320.2 | | |

Following the synthesis method in Synthesis Step 2 for the intermediate A1, the following intermediate compounds are obtained by synthesis:

| No. of intermediate | Structure of intermediate and ESI-MS(M+H)⁺ | Intermediate No. | Structure of intermediate and ESI-MS(M+H)⁺ |
|---|---|---|---|
| A7 | 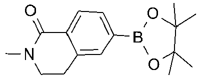 ESI-MS(M+H)⁺=288.2 | A8 | 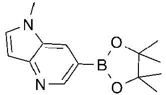 ESI-MS(M+H)⁺=259.2 |
| A9 | 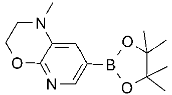 ESI-MS(M+H)⁺=277.2 | | |

### Embodiment 2: Synthesis of intermediate A10

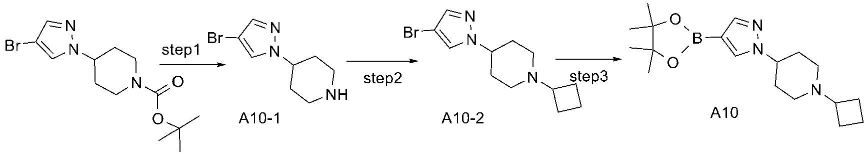

### Synthesis Step 1: Synthesis of A10-1

Tert-Butyl 4-(4-bromopyrazole-1-yl)piperidine-1-carboxylic acid tert-butyl ester (3g, 9.09 mmol) is dissolved in dioxane (30 mL), and 1,4-dioxane containing HCl is added to the system, to perform reaction at room temperature for 5 h. After the reaction is completed, concentration is performed under reduced pressure, to obtain 2.3g of a crude product A10-1, with a yield of 95%. ESI-MS(M+H)⁺=230.0.

### Synthesis Step 2: Synthesis of A10-2

A10-1 (2g, 8.70mmol) is dissolved in DCM (20mL), and cyclobutanone (1mL, 13.05mmol) is added to the system, to perform stirring at room temperature for 0.5h, following by adding STAB (5.5g, 26.10mmol) and performing reaction at room temperature for 3h. After the reaction is completed, quenching is performed with water, and extraction is performed with dichloromethane; the organic phases are combined, drying is performed over anhydrous sodium sulfate, and concentration is performed under reduced pressure. 1.8 g of A10-2 is obtained by column chromatography, with a yield of 82%. ESI-MS(M+H)⁺ =284.1.

### Synthesis Step 3: Synthesis of A10

The synthesis of A10 follows the same method as Synthesis Step 2 in the synthesis of the intermediate A1.

Following the synthesis method in Synthesis Step 2 for the intermediate A10, the following intermediate compounds are obtained by synthesis:

| No. of intermediate | Structure of intermediate and ESI-MS(M+H)⁺ | Intermediate No. | Structure of intermediate and ESI-MS(M+H)⁺ |
|---|---|---|---|
| A11 | 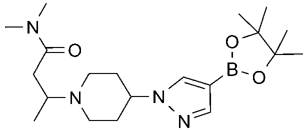 ESI-MS(M+H)⁺=391.3 | A12 | 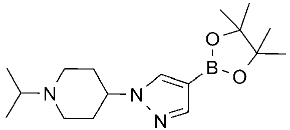 ESI-MS(M+H)⁺=320.2 |
| A13 | 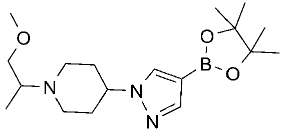 ESI-MS(M+H)⁺=350.3 | A14 | 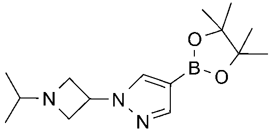 ESI-MS(M+H)⁺=292.2 |

### Embodiment 3: Synthesis of intermediate A15

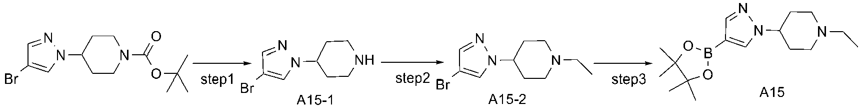

### Synthesis Step 1: Synthesis of A15-1

The synthesis of A15-1 follows the same method as Synthesis Step 1 in the synthesis of the intermediate A10.

### Synthesis Step 2: Synthesis of A15-2

A15-1 (2g, 8.70mmol) is dissolved in DMF (20mL), then cesium carbonate (5.7g, 17.4mmol) and iodoethane (1mL, 13.05mmol) are added to the system in sequence, to perform stirring and reaction at room temperature for 2h.

After the reaction is completed, quenching is performed with water, and extraction is performed with ethyl acetate; the organic phases are combined, drying is performed over anhydrous sodium sulfate, and concentration is performed under reduced pressure. 1.6 g of A15-2 is obtained by column chromatography, with a yield of 71%. ESI-MS(M+H)⁺ =258.1.

### Synthesis Step 3: Synthesis of A15

The synthesis of A15 follows the same method as Synthesis Step 2 in the synthesis of the intermediate A1.

Following the synthesis method for the intermediate A15, the following intermediate compounds are obtained by synthesis:

| No. of intermediate | Structure of intermediate and ESI-MS(M+H)⁺ | Intermediate No. | Structure of intermediate and ESI-MS(M+H)⁺ |
|---|---|---|---|
| A16 | 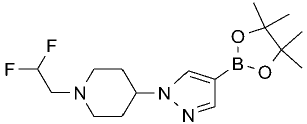 ESI-MS(M+H)⁺=342.2 | A17 | 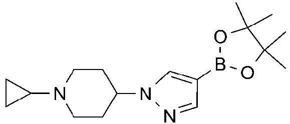 ESI-MS(M+H)⁺=318.2 |
| A18 | 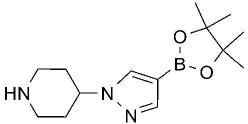 ESI-MS(M+H)⁺=278.2 | | |

### Embodiment 4: Synthesis of intermediate B1

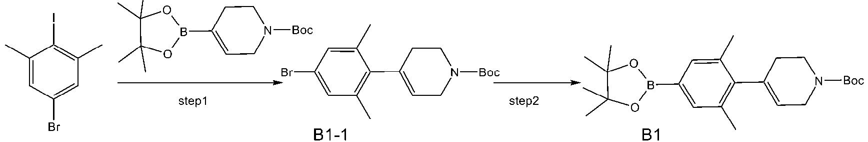

### Synthesis Step 1: Synthesis of B1-1

5-Bromo-2-iodo-1,3-dimethylbenzene (3 g, 9.71 mmol) is dissolved in dioxane (30 mL) and water (6 mL), and N-Boc-1,2,5,6-tetrahydropyridine-4-boronic acid pinacol ester (3.6 g, 11.65 mmol), [1,1'-bis(diphenylphosphine)ferrocene]palladium dichloride (710 mg, 0.97 mmol) and sodium carbonate (3.1 g, 29.13 mmol) are added to the system in sequence, to perform the reaction at room temperature for 5 h. After the reaction is completed, quenching is performed with water, and extraction is performed with dichloromethane; the organic phases are combined, drying is performed over anhydrous sodium sulfate, and concentration is performed under reduced pressure. 2.1 g of B1-1 is obtained by column chromatography, with a yield of 59%. ESI-MS(M+H)⁺ =366.1.

### Synthesis Step 2: Synthesis of B1

B1-1 (2.00 g, 5.48 mmol) is dissolved in dioxane (20 mL), and bis(pinacolato)diboron (2.1 g, 8.22 mmol), [1,1'-bis(diphenylphosphino)ferrocene]palladium dichloride (401 mg, 0.55 mmol), and potassium acetate (1.6 g, 16.44 mmol) are added to the system in sequence, to perform the reaction at 80°C under nitrogen protection for 4 h. After the reaction is completed, quenching is performed with water, and extraction is performed with dichloromethane; the organic phases are combined, drying is performed over anhydrous sodium sulfate, and concentration is performed under reduced pressure. 1.5 g of B1 is obtained by column chromatography, with a yield of 66%. ESI-MS(M+H)⁺=414.3.

Following the synthesis route and method for the intermediate B1, the following intermediate compounds are obtained by synthesis:

| No. of intermediate | Structure of intermediate and ESI-MS(M+H)⁺ | Intermediate No. | Structure of intermediate and ESI-MS(M+H)⁺ |
|---|---|---|---|
| B2 | 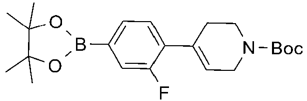 ESI-MS(M+H)⁺=404.2 | B3 | 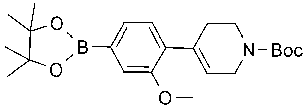 ESI-MS(M+H)⁺=416.3 |
| B4 | 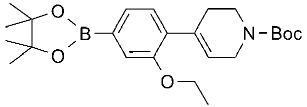 ESI-MS(M+H)⁺=430.3 | B5 | 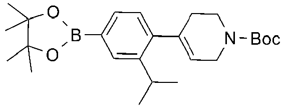 ESI-MS(M+H)⁺=428.3 |

### Embodiment 5: Synthesis of intermediate B6

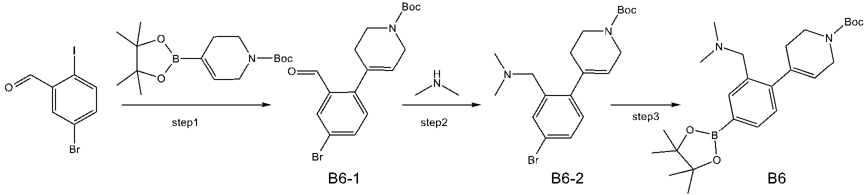

### Synthesis Step 1: Synthesis of B6-1

The synthesis of B6-1 follows the same method as Synthesis Step 1 in the synthesis of the intermediate B1.

### Synthesis Step 2: Synthesis of B6-2

B6-1 (2.00 g, 5.48 mmol) is dissolved in dichloromethane (20 mL), and dimethylamine (370 mg, 8.22 mmol) and STAB (3.5 g, 16.44 mmol) are added to the system in sequence, to perform the reaction at room temperature for 5 h. After the reaction is completed, quenching is performed with water, and extraction is performed with dichloromethane; the organic phases are combined, drying is performed over anhydrous sodium sulfate, and concentration is performed under reduced pressure. 1.5 g of B6-2 is obtained by column chromatography, with a yield of 70%. ESI-MS(M+H)⁺=395.1.

### Synthesis Step 3: Synthesis of B6

The synthesis of B6 follows the same method as Synthesis Step 2 in the synthesis of the intermediate B1.

### Embodiment 6: Synthesis of intermediate B7

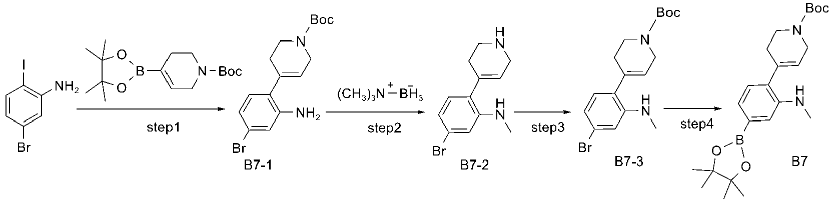

### Synthesis Step 1: Synthesis of B7-1

The synthesis of B7-1 follows the same method as Synthesis Step 1 in the synthesis of the intermediate B1.

### Synthesis Step 2: Synthesis of B7-2

B7-1 (5.00 g, 14.15 mmol) is dissolved in DMF (20 mL), sodium hydride (407 mg, 16.98 mmol) is slowly added to the system at 0 °C for stirring at 0 °C for 10 min, and then borane trimethylamine complex (1.5 mL, 16.98 mmol) is added to the system at 0 °C, to perform the reaction at 80 °C for 2 h. After the reaction is completed, quenching is performed with a saturated aqueous solution of ammonium chloride, and extraction is performed with ethyl acetate; the organic phases are combined, drying is performed over anhydrous sodium sulfate, and concentration is performed under reduced pressure. 3.1 g of B7-2 is obtained by column chromatography, with a yield of 81%. ESI-MS(M+H)⁺=267.0.

### Synthesis Step 3: Synthesis of B7-3

B7-2 (3.00 g, 11.23 mmol) is dissolved in dichloromethane (30 mL), and triethylamine (4.7 mL, 33.68 mmol) and di-tert-butyl dicarbonate (3.8 mL, 16.84 mmol) are added to the system in sequence at 0 °C, to perform stirring and reaction at room temperature for 2 h. After the reaction is completed, quenching is performed with water, and extraction is performed with dichloromethane; the organic phases are combined, drying is performed over anhydrous sodium sulfate, and concentration is performed under reduced pressure. 3.3 g of B7-3 is obtained by column chromatography, with a yield of 80%. ESI-MS(M+H)⁺=367.1.

### Synthesis Step 3: Synthesis of B7

The synthesis of B7 follows the same method as Synthesis Step 2 in the synthesis of the intermediate B1.

### Embodiment 7: Synthesis of intermediate M1

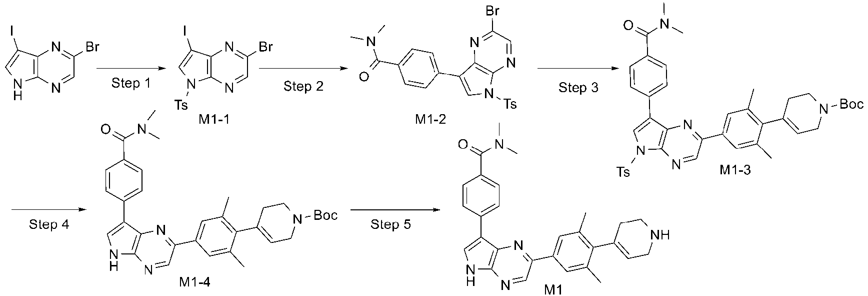

### Synthesis Step 1: Synthesis of M1-1

2-Bromo-7-iodo-5H-pyrrolo[2,3-b]pyrazine (3.20 g, 9.93 mmol) is dissolved in DMF (20 mL), and p-toluenesulfonyl chloride (2.40 g, 12.90 mmol) and sodium hydride (500 mg, 12.90 mmol) are added to the system in sequence, to perform reaction at room temperature for 3 h. After the reaction is completed, quenching is performed with water, and extraction is performed with dichloromethane; the organic phases are combined, drying is performed over anhydrous sodium sulfate, and concentration is performed under reduced pressure. M1-1 is obtained by column chromatography. ESI-MS(M+H)⁺=477.9.

### Synthesis Step 2: Synthesis of M1-2

M1-1 (3.00 g, 6.29 mmol) is dissolved in dioxane of 30 mL, and *N*,N-dimethyl-4-(4,4,5,5-tetramethyl-1,3,2-dioxaborhexacyclopentan-2-yl)benzamide (2.0 g, 7.54 mmol), [1,1'-bis(diphenylphosphino)ferrocene]palladium dichloride (460 mg, 0.63 mmol), potassium carbonate (1.7 g, 12.58 mmol), and water (10 mL) are added to the system in sequence, to perform reaction at 50 °C under nitrogen protection for 4 h. After the reaction is completed, quenching is performed with water, and extraction is performed with dichloromethane; the organic phases are combined, drying is performed over anhydrous sodium sulfate, and concentration is performed under reduced pressure. M1-2 is obtained by column chromatography. ESI-MS(M+H)⁺ =499.0.

### Synthesis Step 3: Synthesis of M1-3

M1-2 (2.0 g, 4.01 mmol) is dissolved in dioxane of 15 mL, and 4-[2,6-dimethyl-4-(4,4,5,5-tetramethyl-1,3,2-dioxaborhecyclopentan-2-yl)phenyl]-1,2,3,6-tetrahydropyridine-1-carb oxylic acid-2-methylpropyl-2-yl ester (1.9 g, 4.81 mmol), Pd(dppf)Cl₂ (290 mg, 0.40 mmol), potassium carbonate (1.6 g, 12.03 mmol), and water (5 mL) are added to the system in sequence, to perform reaction at 100°C under nitrogen protection for 4 h. After the reaction is completed, quenching is performed with water, and extraction is performed with dichloromethane; the organic phases are combined, drying is performed over anhydrous sodium sulfate, and concentration is performed under reduced pressure. M1-3 is obtained by column chromatography. ESI-MS(M+H)⁺=706.3.

### Synthesis Step 4: Synthesis of M1-4

M1-3 (2.3 g, 3.26 mmol) is dissolved in tetrahydrofuran (15 mL), and a saturated aqueous solution of sodium hydroxide (15 mL) is added to the system, to perform reaction at 70 °C for 4 h. After the reaction is completed, quenching is performed with water, and extraction is performed with dichloromethane; the organic phases are combined, drying is performed over anhydrous sodium sulfate, and concentration is performed under reduced pressure. M1-4 is obtained by column chromatography (elution with dichloromethane/methanol). ESI-MS(M+H)⁺ =552.3.

### Synthesis Step 5: Synthesis of intermediate M1

M1-4 (1.4 g, 2.53 mmol) is dissolved in dichloromethane (10 mL), and a solution of 1,4-dioxane in hydrogen chloride (14 mL, 6 N) is added to the system, to perform reaction at room temperature for 4 h. After the reaction is completed, dilution is performed with dichloromethane, and washing is performed with a saturated aqueous solution of sodium carbonate; the organic phases are combined, drying is performed , and concentration is performed under reduced pressure, to obtain the intermediate M1. ESI-MS(M+H)⁺=452.2.

Following the synthesis route and method for the intermediate M1, the following intermediate compounds are obtained by synthesis:

| No. of intermediate | Structure of intermediate and ESI-MS(M+H)⁺ | Intermediate No. | Structure of intermediate and ESI-MS(M+H)⁺ |
|---|---|---|---|
| M2 | 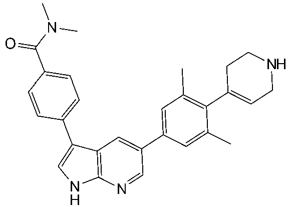 ESI-MS(M+H)⁺=451.2 | M3 | 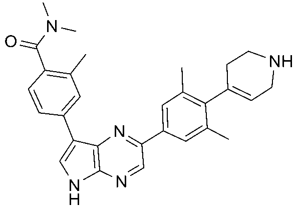 ESI-MS(M+H)⁺=466.2 |
| M4 | 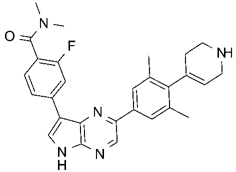 ESI-MS(M+H)⁺=470.2 | M5 | 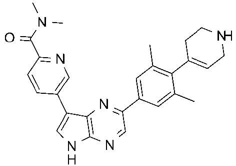 ESI-MS(M+H)⁺=453.2 |
| M6 | 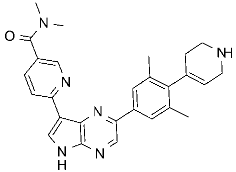 ESI-MS(M+H)⁺=453.2 | M7 | 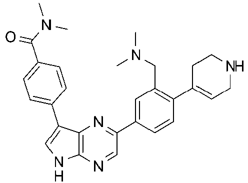 ESI-MS(M+H)⁺=481.2 |
| M8 | 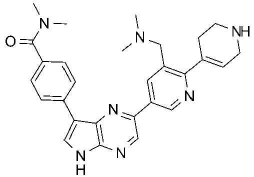 ESI-MS(M+H)⁺=482.2 | M9 | 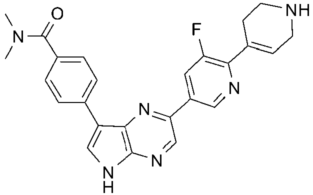 ESI-MS(M+H)⁺=443.1 |
| M10 | 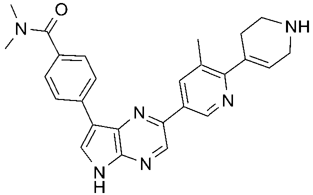 ESI-MS(M+H)⁺=439.2 | M11 | 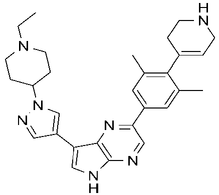 ESI-MS(M+H)⁺=482.2 |
| M12 | 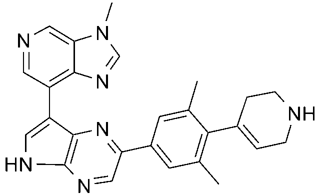 ESI-MS(M+H)⁺=436.2 | M13 | 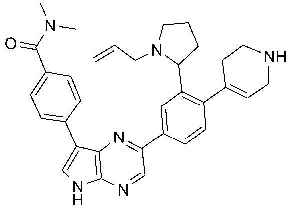 ESI-MS(M+H)⁺=533.2 |
| M14 | 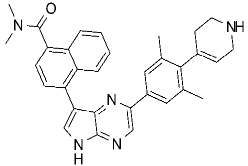 ESI-MS(M+H)⁺=502.2 | M15 | 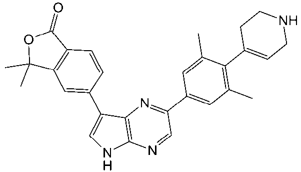 ESI-MS(M+H)⁺=465.2 |
| M16 | 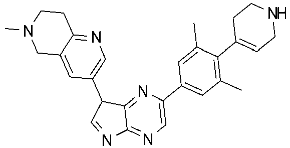 ESI-MS(M+H)⁺=451.2 | M17 | 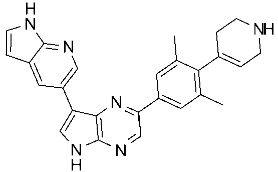 ESI-MS(M+H)⁺=421.2 |
| M18 | 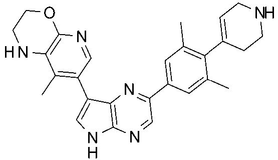 ESI-MS(M+H)⁺=453.2 | M19 | 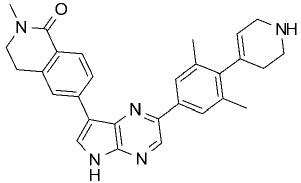 ESI-MS(M+H)⁺=464.2 |
| M20 | 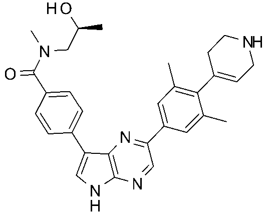 ESI-MS(M+H)⁺=496.2 | M21 | 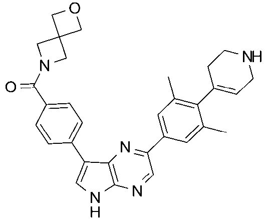 ESI-MS(M+H)⁺=506.2 |
| M22 | 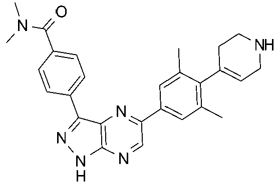 ESI-MS(M+H)⁺=453.2 | M23 | 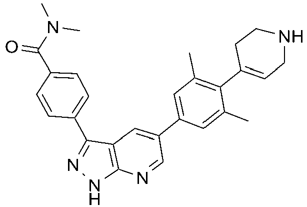 ESI-MS(M+H)⁺=452.2 |
| M24 | 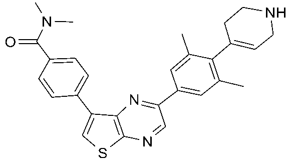 ESI-MS(M+H)⁺=469.1 | M25 | 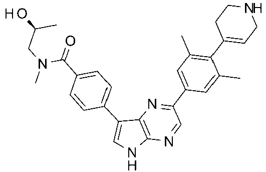 ESI-MS(M+H)⁺=496.2 |
| M26 | 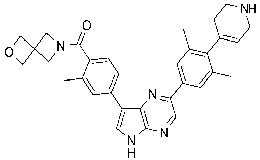 ESI-MS(M+H)⁺=520.2 | C4 | 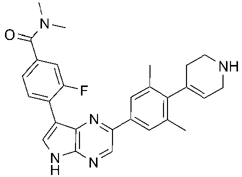 ESI-MS(M+H)⁺=470.2 |
| C7 | 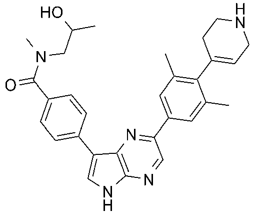 ESI-MS(M+H)⁺=496.3 | C8 | 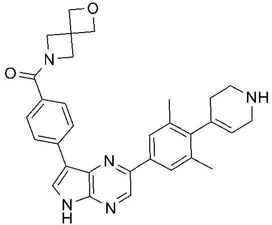 ESI-MS(M+H)⁺=506.2 |
| C9 | 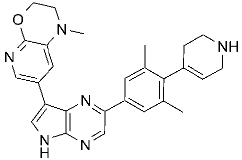 ESI-MS(M+H)⁺=453.2 | C10 | 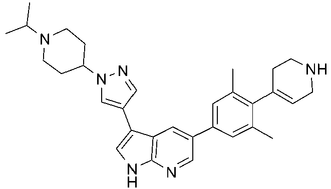 ESI-MS(M+H)⁺=495.3 |
| C11 | 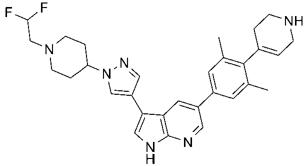 ESI-MS(M+H)⁺=517.3 | C12 | 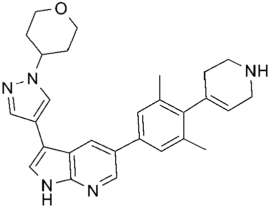 ESI-MS(M+H)⁺=470.3 |
| C13 | 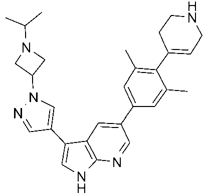 ESI-MS(M+H)⁺=467.3 | C14 | 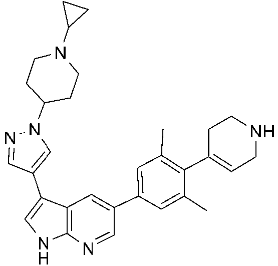 ESI-MS(M+H)⁺=493.3 |
| C15 | 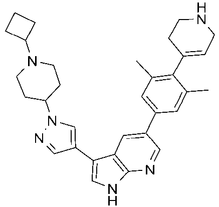 ESI-MS(M+H)⁺=507.3 | C16 | 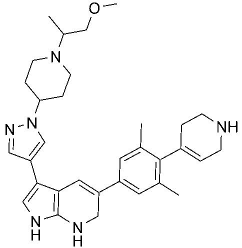 ESI-MS(M+H)⁺=527.3 |
| C17 | 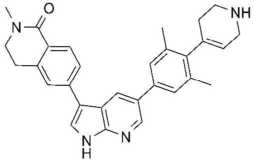 ESI-MS(M+H)⁺=463.2 | C18 | 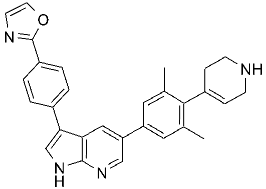 ESI-MS(M+H)⁺=447.2 |
| C19 | 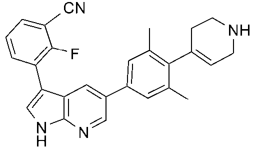 ESI-MS(M+H)⁺=423.2 | C20 | 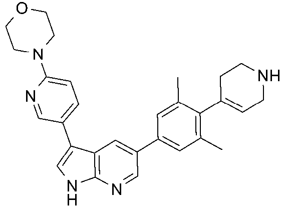 ESI-MS(M+H)⁺=466.3 |
| C21 | 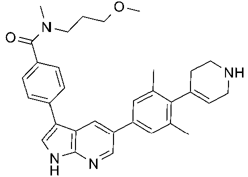 ESI-MS(M+H)⁺=509.3 | C22 | 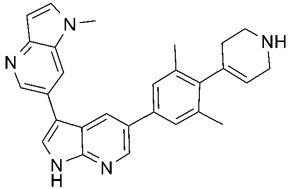 ESI-MS(M+H)⁺=434.2 |
| C23 | 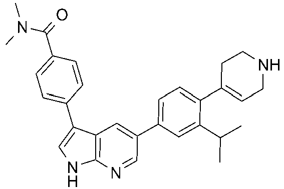 ESI-MS(M+H)⁺=465.3 | C24 | 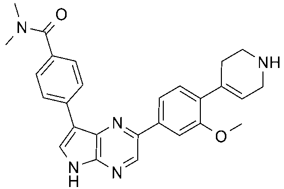 ESI-MS(M+H)⁺=454.2 |
| C25 | 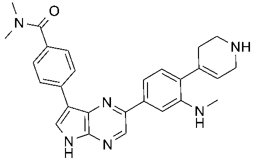 ESI-MS(M+H)⁺=453.2 | C26 | 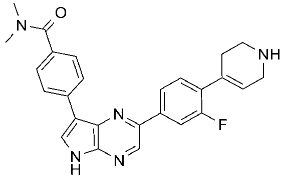 ESI-MS(M+H)⁺=442.2 |
| C27 | 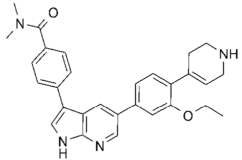 ESI-MS(M+H)⁺=467.2 | C28 | 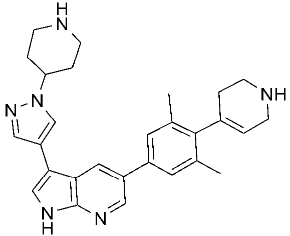 ESI-MS(M+H)⁺=453.3 |
| C29 | 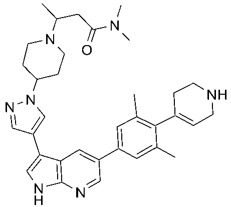 ESI-MS(M+H)⁺=566.4 | | |

### Embodiment 8: Synthesis of intermediate M27

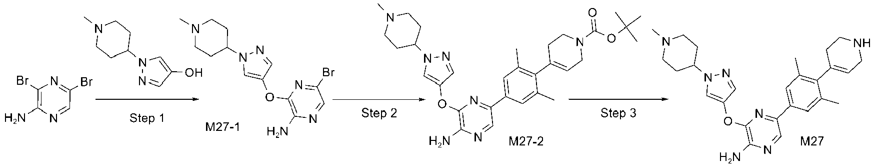

### Synthesis Step 1: Synthesis of M27-1

3,5-Dibromopyrazin-2-amine (2.0 g, 7.91 mmol) is dissolved in N,N-dimethylformamide (40 mL), and 1-(1-methylpiperidin-4-yl)-1H-pyrazole-4-ol (1.4 g, 7.91 mmol) and cesium carbonate (5.2 g, 15.82 mmol) are added to the system in sequence, to perform reaction at 90 °C for 2 h. After the reaction is completed, cooling and quenching is performed with water, and extraction is performed with ethyl acetate; the organic phases are combined, concentration is performed under reduced pressure, and purification is performed by column chromatography, to obtain M27-1. ESI-MS(M+H)⁺=353.1.

### Step 2: Synthesis of intermediate M27

The intermediate M27 is obtained by referring to Synthesis Steps 3 and 5 of the intermediate M1, with ESI-MS(M+H)+=460.2.

Following the synthesis route and method for the intermediate M27, the following intermediate compounds are obtained by synthesis:

| No. of intermediate | Structure of intermediate and ESI-MS(M+H)⁺ | Intermediate No. | Structure of intermediate and ESI-MS(M+H)⁺ |
|---|---|---|---|
| M28 | 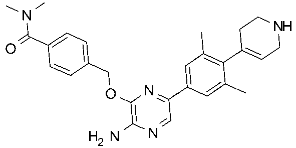 ESI-MS(M+H)⁺=458.2 | M29 | 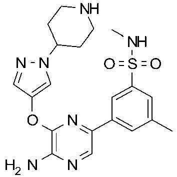 ESI-MS(M+H) ⁺=444.2 |
| M30 | 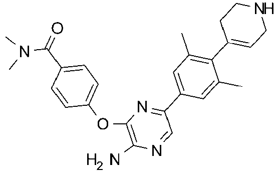 ESI-MS(M+H)⁺=444.2 | | |

### Embodiment 9: Synthesis of intermediate M31

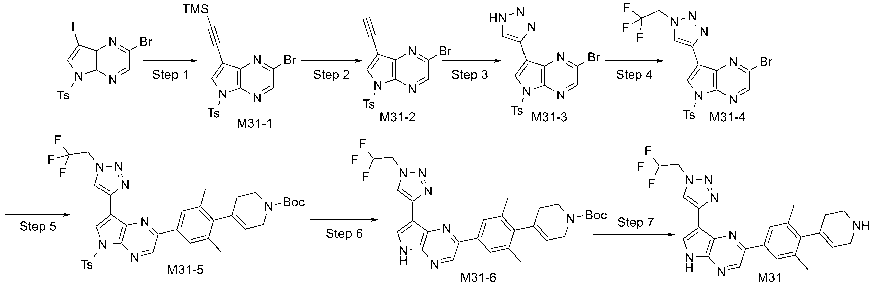

### Synthesis Step 1: Synthesis of M31-1

M1-1 (5.0 g, 10.49 mmol) is dissolved in DMSO (50 mL), and cesium carbonate (10.2 g, 31.47 mmol), cuprous iodide (200 mg, 1.05 mmol), and [1,1'-bis(diphenylphosphine)ferrocene]palladium dichloride (768 mg, 1.05 mmol) are added to the system in sequence, while purging the atmosphere with nitrogen for protection, heating the temperature to 80°C and adding a DMSO solution of trimethylsilylacetylene (3.7 g, 52.45 mmol) dropwise, to perform stirring and reaction at 80°C for 2 h. After the reaction is completed, quenching is performed with water, and extraction is performed with EA; the organic phases are combined, drying is performed over anhydrous sodium sulfate, and concentration is performed under reduced pressure. M31-1 is obtained by column chromatography. ESI-MS(M+H)⁺=448.0.

### Step 2: Synthesis of M31-2

M31-1 (3.9 g, 8.72 mmol) is dissolved in MeOH (40 mL), and potassium carbonate (414 mg, 26.16 mmol) is added to the system, to perform stirring and reaction overnight at room temperature. After the reaction is completed, quenching is performed with water, concentration is performed under reduced pressure until a fixed volume is achieved, and extraction is performed with EA; the organic phases are combined, drying is performed over anhydrous sodium sulfate, and concentration is performed under reduced pressure. M31-2 is obtained by column chromatography. ESI-MS(M+H)⁺=376.0.

### Step 3: Synthesis of M31-3

M31-2 (3.0 g, 7.92 mmol) is dissolved in DMF (30 mL), then methanol (3 mL), trimethylsilyl azidosilane (1.4 g, 11.88 mmol) and cuprous iodide (75 mg, 0.40 mmol) are added to the system in sequence. while purging the atmosphere with nitrogen for protection and heating to 100 °C for reaction for 2 h. After the reaction is completed, quenching is performed with water, and extraction is performed with EA; the organic phases are combined, drying is performed over anhydrous sodium sulfate, and concentration is performed under reduced pressure. M31-3 is obtained by column chromatography. ESI-MS(M+H)⁺=419.0.

### Step 4: Synthesis of M31-4

M31-3 (2.7 g, 6.53 mmol) is dissolved in DCM (30 mL), and 2,2,2-trifluoroacetaldehyde (961 mg, 9.80 mmol) and STAB (4.2 g, 19.59 mmol) are added to the system in sequence, to perform reaction at room temperature for 2 h. After the reaction is completed, quenching is performed with water, and extraction is performed with EA; the organic phases are combined, drying is performed over anhydrous sodium sulfate, and concentration is performed under reduced pressure. M31-4 is obtained by column chromatography. ESI-MS(M+H)⁺ =501.0. The product structure is confirmed by H-H COSY.

### Step 5: Synthesis of M31

The synthesis methods for M31-5, M31-6, and M31 refer to Synthesis Steps 3, 4, and 5 for the intermediate M1 to obtain the intermediate M31, with ESI-MS(M+H)⁺=454.2.

### Embodiment 10: Synthesis of intermediate M32

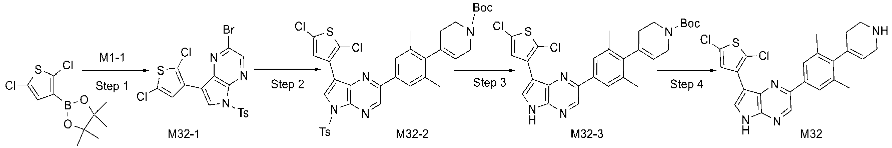

### Synthesis Step 1: Synthesis of M32-1

M1-1 (5.0 g, 10.49 mmol) is dissolved in dioxane (50 mL), and 2-(2,5-dichlorothiophen-3-yl)-4,4,5,5-tetramethyl-1,3,2-dioxaborane (3.51 g, 12.59 mmol), potassium carbonate (4.2 g, 31.47 mmol), [1,1'-bis(diphenylphosphino)ferrocene]palladium dichloride (768 mg, 1.05 mmol) and water (16 mL) are added to the system in sequence, perform reaction at 100 °C under nitrogen protection for 4 h. After the reaction is completed, quenching is performed with water, and extraction is performed with dichloromethane; the organic phases are combined, drying is performed over anhydrous sodium sulfate, and concentration is performed under reduced pressure. M32-1 is obtained by column chromatography. ESI-MS(M+H)⁺=501.9.

### Step 2: Synthesis of M32

The synthesis methods for M32-2, M32-3, and M32 refer to Synthesis Steps 3, 4, and 5 for the intermediate M1 to obtain the intermediate M31, with ESI-MS(M+H)⁺=455.1.

Following the synthesis route and method for the intermediate M32, the following intermediate compounds are obtained by synthesis:

| | | | |
|---|---|---|---|
| M35 | 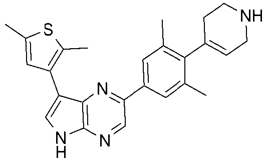 ESI-MS(M+H)⁺=415.2 | M36 | 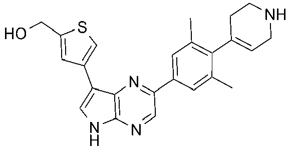 ESI-MS(M+H)⁺=417.2 |
| M37 | 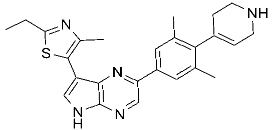 ESI-MS(M+H)⁺=430.2 | M38 | 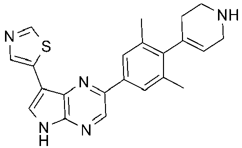 ESI-MS(M+H)⁺=388.2 |
| M39 | 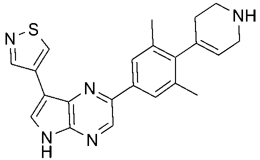 ESI-MS(M+H)⁺=388.2 | M40 | 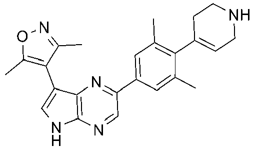 ESI-MS(M+H)⁺=400.2 |
| M41 | 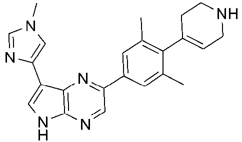 ESI-MS(M+H)⁺=385.2 | M42 | 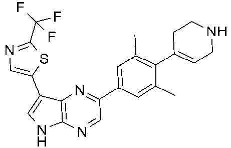 ESI-MS(M+H)⁺=456.1 |
| M43 | 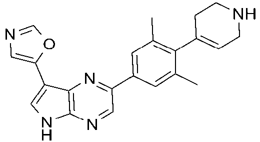 ESI-MS(M+H)⁺=372.1 | M44 | 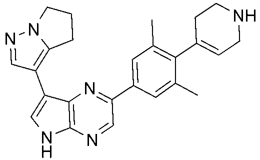 ESI-MS(M+H)⁺=411.2 |
| M45 | 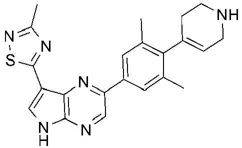 ESI-MS(M+H)⁺=403.2 | M46 | 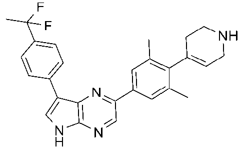 ESI-MS(M+H)⁺=445.2 |

### Embodiment 11: Synthesis of intermediate M33

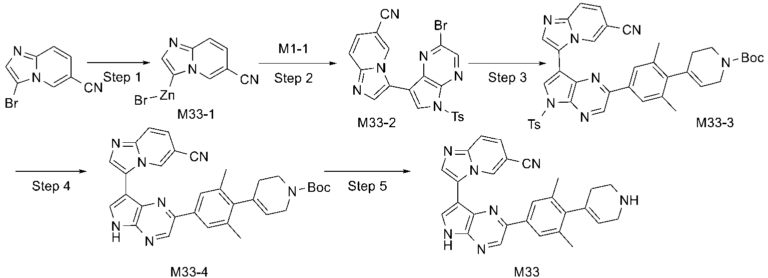

### Synthesis Step 1: Synthesis of M33-1

3-Bromo-6-cyanomimidazolo[1,2-A]pyridine (5.0 g, 22.52 mmol) is dissolved in tetrahydrofuran (50 mL), a solution of n-butyllithium in n-hexane (11.7 mL, 29.28 mmol) is added dropwise to the system at -70 °C and stirred for 1 h, and then, a solution of zinc chloride in tetrahydrofuran (29 mL, 29.28 mmol) is added at -50 °C, to perform reaction at 0 °C for 1 h. For direct use in the next step.

### Step 2: Synthesis of M33-2

M1-1 (5.0 g, 10.49 mmol) is dissolved in tetrahydrofuran (50 mL), and chloro(2-dicyclohexylphosphino-2',4',6'-triisopropyl-1,1'-biphenyl)[2-(2'-amino-1,1'-biphenyl)]palladium (412 mg, 0.52 mmol) and M33-1 (11.54 mmol) are added to the system, to perform reaction at 40 °C for 1 h. After the reaction is completed, quenching is performed with water, and extraction is performed with dichloromethane; the organic phases are combined, drying is performed over anhydrous sodium sulfate, and concentration is performed under reduced pressure. M33-2 is obtained by column chromatography. ESI-MS(M+H)⁺=493.0.

### Step 3: Synthesis of M33

The synthesis methods for M33-3, M33-4, and M33 refer to Synthesis Steps 3, 4, and 5 for the intermediate M1 to obtain the intermediate M33, with ESI-MS(M+H)⁺=446.2.

Following the synthesis route and method for the intermediate M33, the following intermediate compounds are obtained by synthesis:

| | | | |
|---|---|---|---|
| M47 | 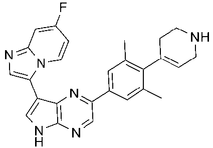 ESI-MS(M+H)⁺=439.2 | M48 | 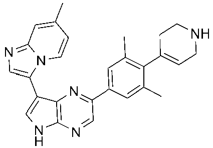 ESI-MS(M+H)⁺=435.2 |

### Embodiment 12: Synthesis of intermediate M34

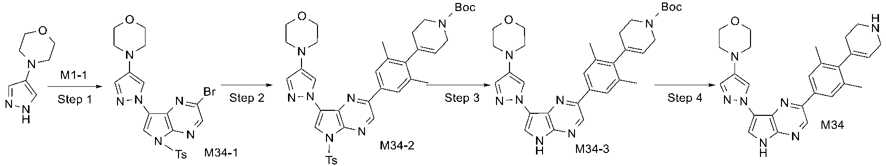

### Synthesis

### Step 1: Synthesis of M34-1

M1-1 (5.0 g, 10.49 mmol) is dissolved in DMSO (50 mL), and cesium carbonate (6.8 g, 20.98 mmol), 4-(1H-pyrazol-4-yl)morpholine (2.4 g, 15.74 mmol) and cuprous oxide (150 mg, 1.05 mmol) are added to the system in sequence, to perform reaction at 100 °C under nitrogen protection for 4 h. After the reaction is completed, quenching is performed with water, and extraction is performed with dichloromethane; the organic phases are combined, drying is performed over anhydrous sodium sulfate, and concentration is performed under reduced pressure. M34-1 is obtained by column chromatography. ESI-MS(M+H)⁺=503.0.

### Step 2: Synthesis of M34

The synthesis methods for M34-2, M34-3, and M34 refer to Synthesis Steps 3, 4, and 5 for the intermediate M1 to obtain the intermediate M34, with ESI-MS(M+H)⁺=456.2.

Following the synthesis route and method for the intermediate M34, the following intermediate compounds are obtained by synthesis:

| | | | |
|---|---|---|---|
| M49 | 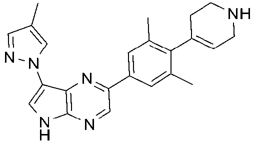 ESI-MS(M+H)⁺=385.2 | M50 | 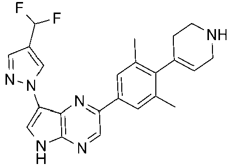 ESI-MS(M+H)⁺=421.2 |
| M51 | 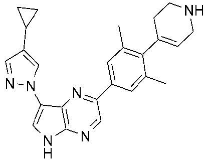 ESI-MS(M+H)⁺=411.2 | M52 | 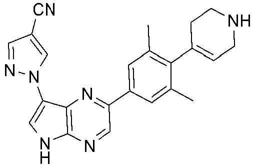 ESI-MS(M+H)⁺=396.2 |
| M53 | 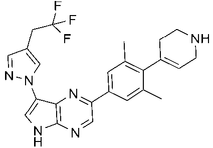 ESI-MS(M+H)⁺=453.2 | M54 | 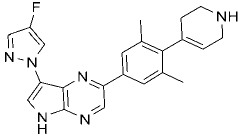 ESI-MS(M+H)⁺=389.1 |
| M55 | 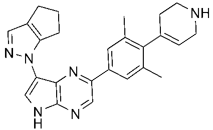 ESI-MS(M+H)⁺=411.2 | | |

### Embodiment 13: Synthesis of intermediate E71

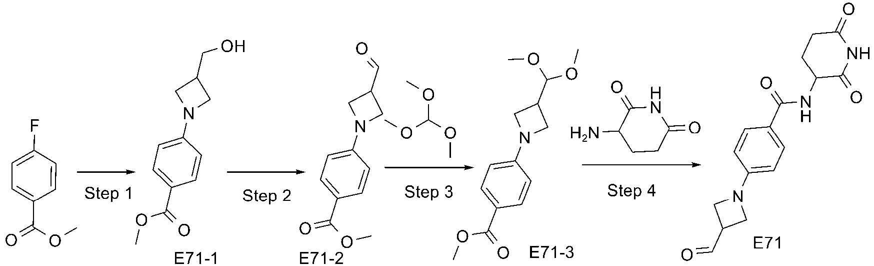

### Synthesis Step 1: Synthesis of M71-1

Methyl 6-fluorobenzoate (5.0 g, 32.22 mmol) is dissolved in 30 mL of N,N-dimethylformamide, and 3-hydroxymethylazine hydrochloride (4.8 g, 38.67 mmol) and potassium carbonate (13.3 g, 96.66 mmol) are added to the system in sequence, to perform reaction at 90 °C for 16 h. After the reaction is completed, quenching is performed with water, and extraction is performed with ethyl acetate; the organic phases are combined, and concentration is performed under reduced pressure, to obtain E71-1 by column chromatography, with a yield of 98%. ESI-MS(M+H)⁺=222.1.

### Synthesis Step 2: Synthesis of M71-2

E71-1 (3g, 13.5mmol) is dissolved in dichloromethane (50mL) and stirred at room temperature until dissolved, and desmartin reagent (8.6g, 20.25mmol) is added in 10 portions, to perform reaction at room temperature for 1h. After the reaction is completed, an appropriate amount of an aqueous solution of saturated sodium bicarbonate is added, and extraction is performed with dichloromethane; the organic phases are combined, and concentration is performed under reduced pressure, to obtain E71-2 by column chromatography, with a yield of 67%. ESI-MS(M+H)⁺=220.1.

### Synthesis Step 3: Synthesis of M71-3

E71-2 (1.9 g, 8.63 mol) is dissolved in methanol (20 mL), trimethoxymethane (1.3 g, 12.94 mmol) is added to the system in sequence for stirring at room temperature for 15 minutes, and then p-toluenesulfonic acid (150 mg, 0.86 mmol) is added, to perform reaction at 60 °C for 4 h. After the reaction is completed, the pH is adjusted to 8 using an aqueous solution of sodium bicarbonate, the methanol is removed by concentration, and extraction is performed with dichloromethane; the organic phases are combined, and drying and concentration is performed under reduced pressure, to obtain 1.5g of E71-3 by column chromatography, with a yield of 65%. ESI-MS(M+H)⁺=266.1.

### Synthesis Step 4: Synthesis of E71

E71-3 (800 mg, 3.17 mmol) is dissolved in N,N-dimethylformamide (10 mL) for stirring at room temperature until being dissolved completely, 3-aminopiperidine-2,6-dione (800 mg, 6.34 mmol), 1-hydroxybenzotriazole (510 mg, 3.80 mmol) and triethylamine (1.6 mL, 12.68 mmol) are added for stirring at room temperature for 30 min, and then carbodiimide hydrochloride (1.8 g, 9.51 mmol) is added, to perform reaction at room temperature for 3 h. After the reaction is completed, an aqueous solution of ammonium chloride is added for quenching, and extraction is performed with ethyl acetate; the organic phases are combined, and drying and concentration is performed, to obtain E71 by column chromatography, with a yield of 49%. ESI-MS(M-H)⁻=316.1.

Following the synthesis route and method for the intermediate E71, the following intermediate compounds are obtained by synthesis:

| No. of intermediate | Structure of intermediate and ESI-MS(M+H)⁺ | No. of intermediate | Structure of intermediate and ESI-MS(M+H)⁺ |
|---|---|---|---|
| E1 | 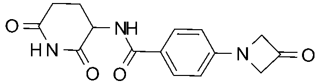 ESI-MS(M+H)⁺=302.1 | E2 | 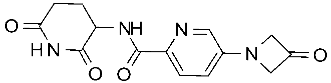 ESI-MS(M+H)⁺=303.1 |
| E3 | 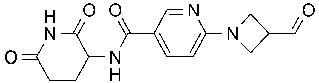 ESI-MS(M+H)⁺=317.1 | E4 | 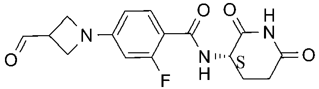 ESI-MS(M+H) ⁺=334.1 |
| E5 | 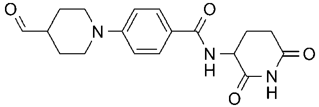 ESI-MS(M+H)⁺=344.2 | E6 | 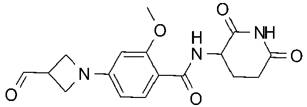 ESI-MS(M+H)⁺=346.1 |
| E7 | 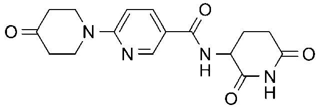 ESI-MS(M+H)⁺=331.1 | E8 | 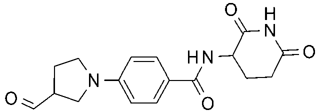 ESI-MS(M+H)⁺=330.1 |
| E9 | 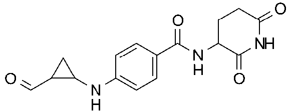 ESI-MS(M+H)⁺=316.1 | E10 | 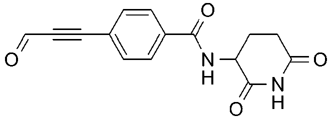 ESI-MS(M+H)⁺=285.1 |
| E44 | 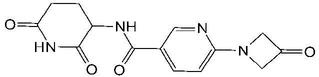 ESI-MS(M+H)⁺=303.1 | E47 | 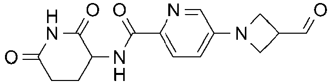 ESI-MS(M+H)⁺=317.1 |

### Embodiment 14: Synthesis of intermediate E11

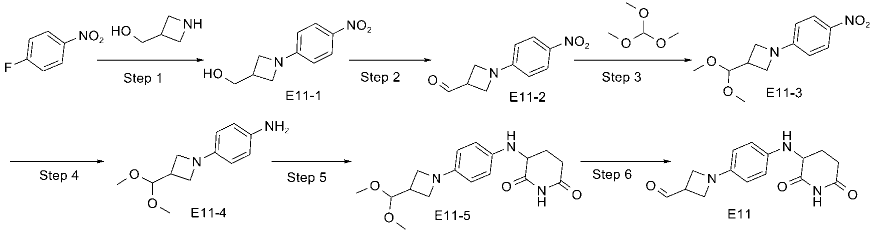

### Synthesis Step 1: Synthesis of E11-1

1-Fluoro-4-nitrobenzene (2.2 g, 15.60 mmol) is dissolved in N,N-dimethylformamide (10 mL), and 3-hydroxymethylazine hydrochloride (1.7 g, 15.60 mmol) and potassium carbonate (6.5 g, 46.8 mmol) are added to the system in sequence, to perform reaction at room temperature for 16 h. After the reaction is completed, quenching is performed with water, and extraction is performed with ethyl acetate; the organic phases are combined, and drying and concentration is performed under reduced pressure, to obtain 3.1g of E11-1 by column chromatography, with ESI-MS(M+H)⁺=209.1.

### Synthesis Step 2: Synthesis of E11-2

E11-1 (2.5 g, 12.01 mmol) is dissolved in dichloromethane (50 mL), and desmartin reagent (7.2 g, 16.93 mmol) is added to the system in 10 portions, to perform reaction at room temperature for 1 h. After the reaction is completed, a saturated sodium bicarbonate solution is added for quenching, and extraction is performed with dichloromethane; the organic phases are combined, and drying and concentration is performed under reduced pressure, to obtain E11-2 by column chromatography, with a yield of 81%. ESI-MS(M+H)⁺=207.1.

### Synthesis Step 3: Synthesis of E11-3

E11-2 (1.8 g, 8.73 mmol) is dissolved in methanol (20 mL), and trimethyl orthoformate (1.4 g, 13.09 mmol) and p-toluenesulfonic acid (150 mg, 0.87 mmol) are added to the system in sequence, to perfrom reaction at 50 °C for 16 h. After the reaction is completed, water is added for quenching, the organic solvent is concentrated, and extraction is performed with dichloromethane; the organic phases are combined, and drying and concentration is performed under reduced pressure, to obtain E11-3 by column chromatography, with a yield of 78%. ESI-MS(M+H)⁺=253.1.

### Synthesis Step 4: Synthesis of E11-4

E11-3 (1.6 g, 6.35 mmol) is dissolved in tetrahydrofuran (20 mL), and palladium on carbon (160 mg, 10% wt) is added to the system, to perform reaction at 50 °C for 16 h under hydrogen atmosphere. After the reaction is completed, filtering is performed rapidly, and the mother liquor is concentrated, to obtain E11-4 by column chromatography, with a yield of 85%. ESI-MS(M+H)⁺=223.1.

### Synthesis Step 5: Synthesis of E11-5

E11-4 (1 g, 4.50 mmol) is dissolved in N,N-dimethylformamide (10 mL), and 3-bromopiperidine-2,6-dione (1.3 g, 6.75 mmol) and sodium bicarbonate (1.1 g, 13.50 mmol) are added to the system in sequence, to perform reaction at room temperature for 16 h. After the reaction is completed, quenching is performed with water, and extraction is performed with ethyl acetate; the organic phases are combined, drying is performed over anhydrous sodium sulfate, and the organic phases are concentrated under reduced pressure, to obtain E11-5 by purification and column chromatography, with a yield of 87%. ESI-MS(M+H)⁺=334.2.

### Synthesis Step 6: Synthesis of E11

E11-5 (1g, 3.00mmol) is dissolved in tetrahydrofuran (10 mL), and an aqueous solution of sulfuric acid (10 mL, 1mol/L) is added to the system, to perform reaction at 50°C for 16 h. After the reaction is completed, the pH is adjusted to 7 using an aqueous solution of sodium bicarbonate, and extraction is performed with ethyl acetate; the organic phases are combined, and drying and concentration is performed under reduced pressure, to obtain E11 by column chromatography, with a yield of 32%. ESI-MS(M+H)⁺=288.1.

Following the synthesis route and method for the intermediate E11, the following intermediate compounds are obtained by synthesis:

| No. of intermedi ate | Structure of intermediate and ESI-MS(M+H) ⁺ | No. of interme diate | Structure of intermediate and ESI-MS(M+H) ⁺ |
|---|---|---|---|
| E12 | 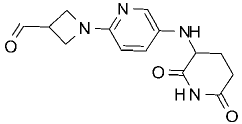 ESI-MS(M+H) ⁺=289.1 | E13 | ESI-MS(M+H)⁺=289.1 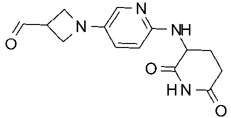 |
| E14 | 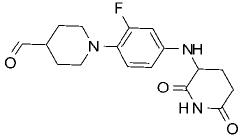 ESI-MS(M+H) ⁺=334.1 | E15 | 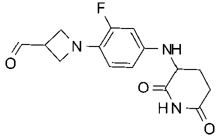 ESI-MS(M+H) ⁺=306.1 |
| E16 | 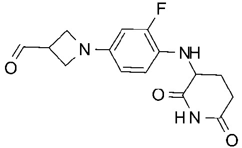 ESI-MS(M+H) ⁺=306.1 | E17 | 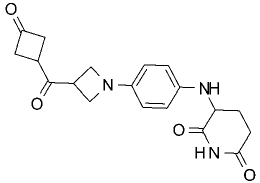 ESI-MS(M+H) ⁺=356.2 |
| E18 | 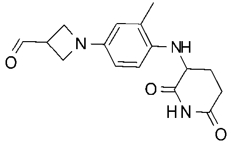 ESI-MS(M+H) ⁺=302.1 | | |

### Embodiment 15: Synthesis of intermediate E23

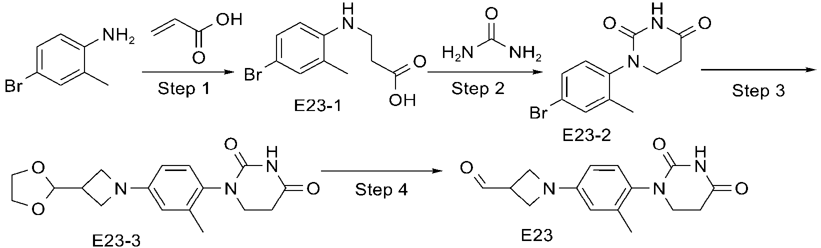

### Synthesis Step 1: Synthesis of M23-1

4-Bromo-2-methylaniline (5.0 g, 27.03 mmol) is dissolved in toluene (50 mL), and acrylic acid (3.9 g, 54.06 mmol) is added to the system, to perform reaction at 100 °C for 16 h. After the reaction is completed, quenching is performed with water, and extraction is performed with ethyl acetate; the organic phases are combined, and drying and concentration is performed under reduced pressure, to obtain 5.6g of E23-1 by column chromatography. ESI-MS(M+H)⁺= 258.0.

### Synthesis Step 2: Synthesis of M23-2

E23-1 (5.6 g, 21.78 mmol) is dissolved in acetic acid (50 mL), and urea (2.6 g, 43.56 mmol) is added to the system, to perform reaction at 110 °C for 16 h. After the reaction is completed, quenching is performed with water, and extraction is performed with ethyl acetate; the organic phases are combined, and drying and concentration is performed under reduced pressure, to obtain E23-2 by column chromatography. ESI-MS(M+H)⁺=283.0.

### Synthesis Step 3: Synthesis of M23-3

E23-2 (3.5 g, 12.41 mmol) is dissolved in dichloromethane (30 mL), and 3-(1,3-dioxolane-2-yl)azacyclobutane (1.9 g, 14.89 mmol) and sodium triacetylborohydride (7.9 g, 37.23 mmol) are added to the system in sequence, to perform reaction at room temperature for 16 h. After the reaction is completed, quenching is performed with water, and extraction is performed with ethyl acetate; the organic phases are combined, and drying and concentration is performed under reduced pressure, to obtain E23-3 by column chromatography, with ESI-MS(M+H)⁺= 332.2.

### Synthesis Step 4: Synthesis of E23

E23-3 (2.8g, 8.45mmol) is dissolved in tetrahydrofuran (20 mL), and an aqueous solution of sulfuric acid (10 mL, 1mol/L) is added to the system, to perform reaction at 50°C for 16 h. After the reaction is completed, the pH is adjusted to 7 using sodium bicarbonate, and extraction is performed with ethyl acetate; the organic phases are combined, and drying and concentration is performed under reduced pressure, to obtain E23 by column chromatography. ESI-MS(M+H)⁺= 288.1.

Following the synthesis route and method for the intermediate E23, the following intermediate compounds are obtained by synthesis:

| No. of intermediate | Structure of intermediate and ESI-MS(M+H)⁺ | No. of intermediate | Structure of intermediate and ESI-MS(M+H)⁺ |
|---|---|---|---|
| E19 | 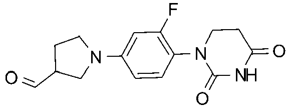 ESI-MS(M+H)⁺= 306.1 | E20 | ESI-MS(M+H)⁺= 304.1 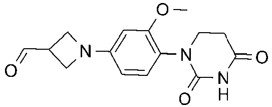 |
| E21 | 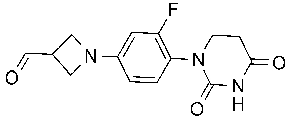 ESI-MS(M+H)⁺= 292.1 | E22 | 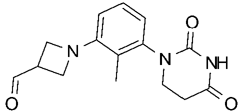 ESI-MS(M+H)⁺= 288.2 |
| E24 | 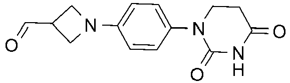 ESI-MS(M+H)⁺= 274.1 | E25 | 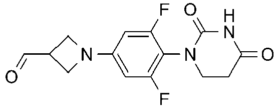 ESI-MS(M+H)⁺= 310.1 |
| E26 | 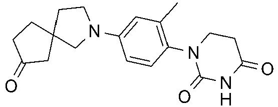 ESI-MS(M+H)⁺= 342.2 | E27 | 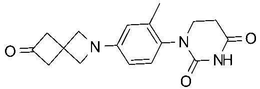 ESI-MS(M+H)⁺= 314.1 |
| E28 | 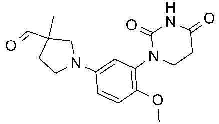 ESI-MS(M+H)⁺= 332.2 | E29 | 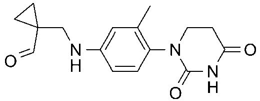 ESI-MS(M+H)⁺= 302.1 |
| E30 | 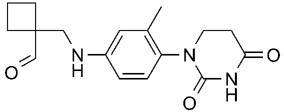 ESI-MS(M+H)⁺= 316.2 | E31 | 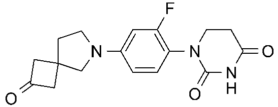 ESI-MS(M+H)⁺= 332.1 |
| E32 | 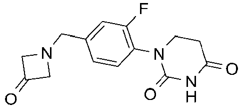 ESI-MS(M+H)⁺= 292.1 | E33 | 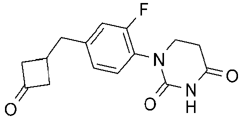 ESI-MS(M+H)⁺= 291.1 |
| E34 | 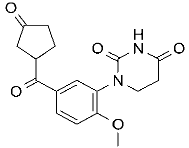 ESI-MS(M+H)⁺= 333.1 | E35 | 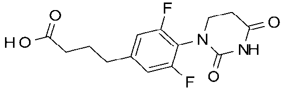 ESI-MS(M+H)⁺=313.1 |
| E67 | 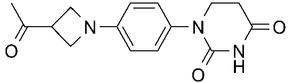 ESI-MS(M+H)⁺= 288.1 | E72 | 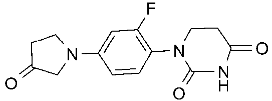 ESI-MS(M+H)⁺= 292.1 |
| D3 | 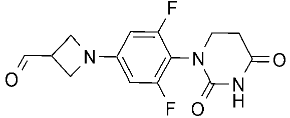 ESI-MS(M+H)⁺= 310.1 | D5 | 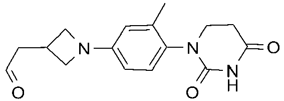 ESI-MS(M+H)⁺= 302.1 |
| D6 | 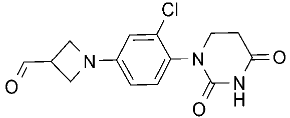 ESI-MS(M+H)⁺= 308.1 | D7 | 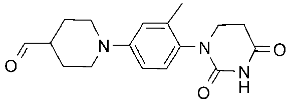 ESI-MS(M+H)⁺= 316.2 |
| D8 | 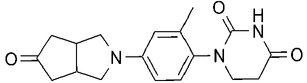 ESI-MS(M+H)⁺= 328.2 | D9 | 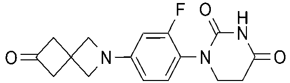 ESI-MS(M+H)⁺= 318.1 |
| D10 | 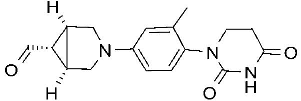 ESI-MS(M+H)⁺= 314.1 | D11 | 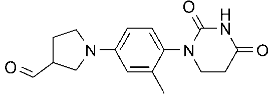 ESI-MS(M+H)⁺= 302.1 |
| D12 | 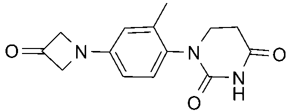 ESI-MS(M+H)⁺= 274.1 | D13 | 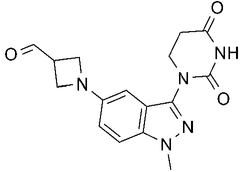 ESI-MS(M+H)⁺= 328.1 |

### Embodiment 16: Synthesis of intermediate E36

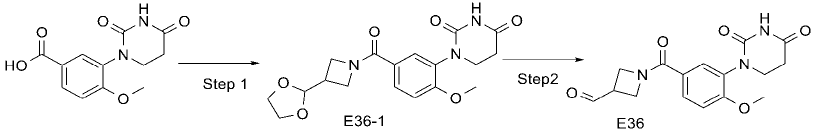

### Synthesis Step 1: Synthesis of E36-1

3-(2,4-dioxotetrahydropyrimidine-1(2H)-yl)-4-methoxybenzoic acid (1.5 g, 5.68 mmol) is dissolved in 15 mL of N,N-dimethylformamide, and 3-(1,3-dioxacyclopentan-2-yl)azacyclobutane (879 mg, 6.81 mmol), 1-hydroxybenzotriazole (996 mg, 7.38 mmol), 1-(3-dimethylaminopropyl)-3-ethylcarbodiimide hydrochloride (3.2 g, 17.04 mmol) and triethylamine (1.8 g, 17.04 mmol) are added to the system in sequence, to perform reaction at room temperature for 1 h. After the reaction is completed, water is added for quenching, and extraction is performed with dichloromethane; the organic phases are combined, and drying and concentration is performed under reduced pressure, to obtain 1.6g of E36-1 by column chromatography, with a yield of 75%. ESI-MS(M+H)⁺=376.1.

### Synthesis Step 2: Synthesis of E36

E36-1 (1.5 g, 4.00 mmol) is dissolved in trifluoroacetic acid (15 mL) to perform reaction at 70 °C for 1 h, and then 1 N sulfuric acid is added to the system to perform reaction at 70 °C for 1 h. After the reaction is completed, the pH is adjusted to slightly alkaline by adding saturated sodium carbonate, and extraction is performed with dichloromethane; the organic phases are combined, and drying and concentration is performed under reduced pressure, to obtain 1.3g of the intermediate E36 by column chromatography, with a yield of 98%. ESI-MS(M+H)⁺=332.1.

Following the synthesis route and method for the intermediate E36, the following intermediate compounds are obtained by synthesis:

| No. of intermediate | Structure of intermediate and ESI-MS(M+H)⁺ | No. of intermediate | Structure of intermediate and ESI-MS(M+H)⁺ |
|---|---|---|---|
| E37 | 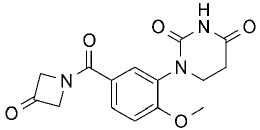 | E38 | 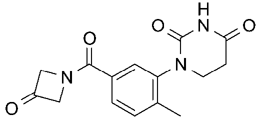 |
| | ESI-MS(M+H)⁺=318.1 | | ESI-MS(M+H)⁺=302.1 |
| E39 | 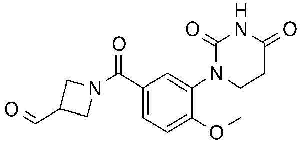 ESI-MS(M+H) ⁺=332.1 | E40 | 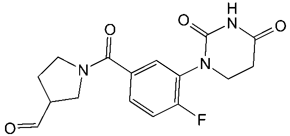 ESI-MS(M+H) ⁺=334.1 |
| E41 | 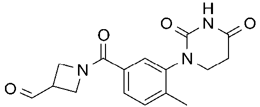 ESI-MS(M+H) ⁺=316.1 | E42 | 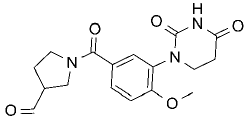 ESI-MS(M+H) ⁺=346.1 |
| E43 | 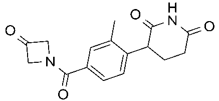 ESI-MS(M+H) ⁺=301.1 | | |

### Embodiment 17: Synthesis of intermediate D21

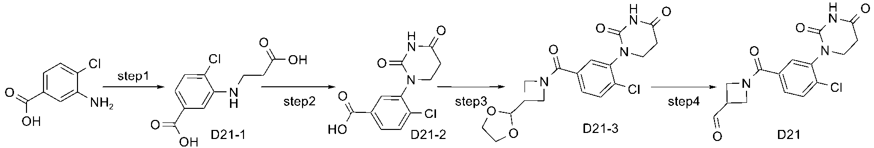

### Synthesis Step 1: Synthesis of D21-1

3-Amino-4-chlorobenzoic acid (5.0 g, 29.24 mmol) is dissolved in toluene (50 ml), and acrylic acid (4.2 g, 58.48 mmol) is added to the system, to perform reaction at 100 °C for 16 h. After the reaction is completed, concentration is performed under reduced pressure for then direct use in the next step. ESI-MS(M+H)⁺= 244.0.

### Synthesis Step 2: Synthesis of D21-2

The above residue is dissolved in acetic acid (50 ml), and urea (2.6 g, 43.56 mmol) is added to the system, to perform reaction at 110 °C for 16 h. After the reaction is completed, quenching is performed with water, and extraction is performed with ethyl acetate; the organic phases are combined, and drying and concentration is performed under reduced pressure, to obtain 2.1g of D21-2 by column chromatography. ESI-MS(M+H)⁺=269.0.

### Synthesis Steps 3--4: Synthesis of D21

The synthesis of D21 follows the same synthetic route and method as Synthesis Steps 1-2 for the intermediate E36.

Following the synthesis route and method for the intermediate D21, the following intermediate compounds are obtained by synthesis:

| No. of intermediate | Structure of intermediate and ESI-MS(M+H)⁺ |
|---|---|
| D22 | 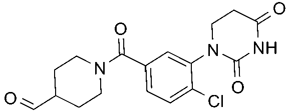 ESI-MS(M+H)⁺=364.1 |

### Embodiment 18: Synthesis of intermediate E45

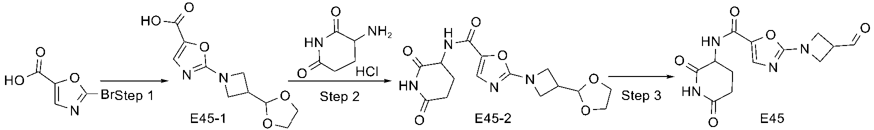

### Synthesis

### Step 1: Synthesis of E45-1

2-Bromoxazole-5-carboxylic acid (1.5 g, 7.85 mmol) is dissolved in tetrahydrofuran (15 mL), and 3-(1,3-dioxacyclopentan-2-yl)azacyclobutane (1.3 g, 10.20 mmol) and cesium carbonate (7.6 g, 23.55 mmol) are added to the system in sequence, to perform reaction at 50 °C for 5 h. After the reaction is completed, extraction is performed with dichloromethane, the organic phases are combined, and drying and concentration is performed under reduced pressure, to obtain E45-1 by column chromatography, with a yield of 69%. ESI-MS(M+H)⁺=241.1.

### Synthesis Step 2: Synthesis of E45-2

E45-1 (1.2 g, 5.00 mmol) is dissolved in N,N-dimethylformamide (15 mL), and 3-aminopiperidine-2,6-dione hydrochloride (1.0 g, 6.50 mmol), 1-hydroxybenzotriazole (877 mg, 6.50 mmol), 1-(3-dimethylaminopropyl)-3-ethylcarbodiimide hydrochloride (2.8 g, 15.00 mmol) and triethylamine (1.6 g, 15.00 mmol) are added to the system in sequence, to perform reaction at room temperature for 1 h. After the reaction is completed, quenching is performed with water, and extraction is performed with dichloromethane; the organic phases are combined, drying is performed over anhydrous sodium sulfate, and concentration is performed under reduced pressure, to obtain E45-2 by column chromatography. ESI-MS(M+H)⁺=351.1.

### Synthesis Step 3: Synthesis of intermediate E45

E45-2 (1.2 g, 3.42 mmol) is dissolved in trifluoroacetic acid (15 mL) to perform reaction at 70 °C for 1 h, and then 1 N sulfuric acid is added to the system to perform reaction at 70 °C for 1 h. After the reaction is completed, the pH is adjusted to slightly alkaline by adding saturated sodium carbonate, and extraction is performed with dichloromethane; the organic phases are combined, drying is performed over anhydrous sodium sulfate, and concentration is performed under reduced pressure, to obtain E45 by column chromatography. ESI-MS(M+H)⁺=307.1.

Following the synthesis route and method for the intermediate E45, the following intermediate compounds are obtained by synthesis:

| No. of intermediate | Structure of intermediate and ESI-MS(M+H)⁺ |
|---|---|
| E46 | 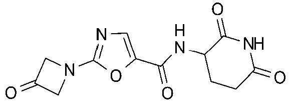 ESI-MS(M+H)⁺=293.2 |

### Embodiment 19: Synthesis of intermediate E48

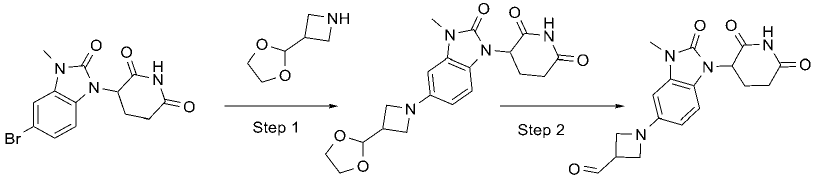

### Synthesis Step 1: Synthesis of E48-1

3-(5-Bromo-3-methyl-2-oxo-2,3-dihydro-1H-benzo[d]imidazol-1-yl)piperidine-2,6-dione (3 g, 8.90 mmol) is dissolved in toluene (30 mL), and 3-(1,3-dioxacyclopentan-2-yl)azacyclobutane (1.7 g, 13.35 mmol), bis(trimethylsilylamino)lithium (22 mL, 22.25 mmol) and chloro(2-dicyclohexylphosphino-2',6'-di-isopropoxy-1,1'-biphenyl)(2-amino-1,1'-biphenyl-2-yl)palladium(II) (689 mg, 0.89 mmol) are added to the system in sequence, to perform reaction at 100 °C for 4 h. After the reaction is completed, quenching is performed with water, and extraction is performed with dichloromethane; the organic phases are combined, and drying and concentration is performed under reduced pressure, to obtain E48-1 by column chromatography, with ESI-MS(M+H)⁺=387.2.

### Synthesis Step 2: Synthesis of intermediate E48

E48-1 (900 mg, 2.33 mmol) is dissolved in trifluoroacetic acid (10 mL) to perform reaction at 70 °C for 1 h, and then 1 N sulfuric acid is added to the system to perform reaction at 70 °C for 1 h. After the reaction is completed, the pH is adjusted to slightly alkaline by adding saturated sodium carbonate, and extraction is performed with dichloromethane; the organic phases are combined, and drying and concentration is performed under reduced pressure, to obtain E48 by column chromatography. ESI-MS(M+H)⁺=343.1.

Following the synthesis route and method for the intermediate E48, the following intermediate compounds are obtained by synthesis:

| No. of intermediate | Structure of intermediate and ESI-MS(M+H)⁺ | No. of intermediate | Structure of intermediate and ESI-MS(M+H)⁺ |
|---|---|---|---|
| E49 | 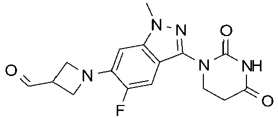 ESI-MS(M+H)⁺=346.1 | E50 | 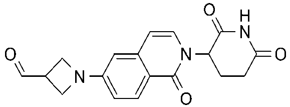 ESI-MS(M+H) ⁺=340.1 |
| E51 | 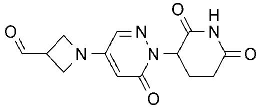 ESI-MS(M+H) ⁺=291.1 | E52 | 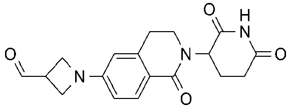 ESI-MS(M+H) ⁺=342.1 |
| E53 | 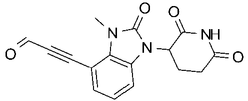 ESI-MS(M+H) ⁺=312.1 | E54 | 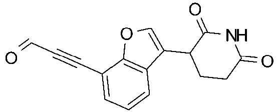 ESI-MS(M+H) ⁺=282.1 |

### Embodiment 20: Synthesis of intermediate E55

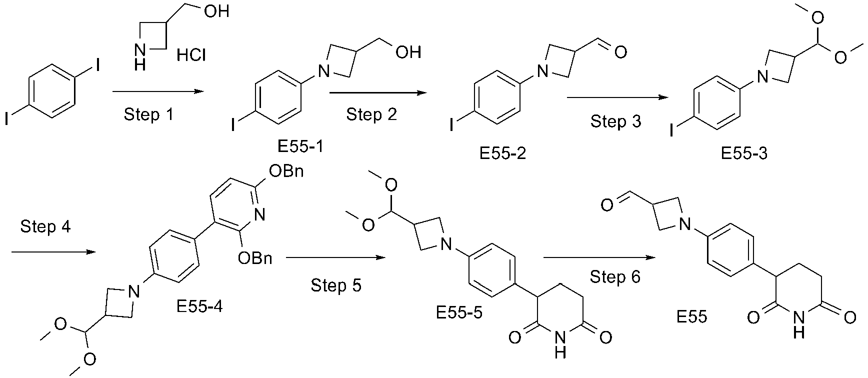

### Synthesis Step 1: Synthesis of E55-1

1,4-Diiodobenzene (5.0 g, 15.16 mmol) is dissolved in dimethyl sulfoxide, and 3-hydroxymethylazine hydrochloride (5.6 g, 45.48 mmol), L-proline (349 mg, 3.03 mmol), potassium carbonate (10.5 g, 75.80 mmol) and cuprous iodide (289 mg, 1.52 mmol) are added to the system in sequence, to perform reaction at 90 °C for 12 h. After the reaction is completed, the system is adjusted to be clear with an aqueous solution of hydrochloric acid (2 mol/L), and extraction is performed with dichloromethane; the organic phases are combined, and drying and concentration is performed under reduced pressure, to obtain E55-1 by column chromatography. ESI-MS(M+H)⁺=290.0.

### Synthesis Step 2: Synthesis of E55-2

E55-1 (2.9 g, 10.12 mmol) is dissolved in dichloromethane (50 mL), and desmartin reagent (6.5 g, 15.18 mmol) is added to the system in 10 portions, to perform reaction at room temperature for 1 h. After the reaction is completed, quenching is performed with a saturated sodium bicarbonate solution, and extraction is performed with dichloromethane; the organic phases are combined, and drying and concentration is performed under reduced pressure, to obtain E55-2 by column chromatography. ESI-MS(M+H)⁺=288.0.

### Synthesis Step 3: Synthesis of E55-3

E55-2 (2.7 g, 9.46 mmol) is dissolved in methanol (30 mL), and trimethyl orthoformate (1.5 g, 14.19 mmol) and p-toluenesulfonic acid (164 mg, 0.95 mmol) are added to the system in sequence, to perform reaction at 50 °C for 16 h. After the reaction is completed, water is added for quenching, concentration is performed to remove methanol, and then extraction is performed with dichloromethane; the organic phases are combined, and drying and concentration is performed under reduced pressure, to obtain E55-3 by column chromatography. ESI-MS(M+H)⁺=334.0.

### Synthesis Step 4: Synthesis of E55-4

E55-3 (1.9 g, 7.57 mmol) is dissolved in dimethyl sulfoxide (20 mL), and 2,6-bis(benzyloxy)-3-(4,4,5,5-tetramethyl-1,3,2-dioxaborane-2-yl)pyridine (3.2 g, 7.57 mmol), sodium tert-butoxide (1.5 g, 15.14 mmol), tris(benzylacetone)palladium (695 mg, 0.76 mmol) and 2,2'-bis(diphenylphosphine)-1,1'-binaphthyl, (±)-BINAP, [1,1'-binaphthyl]-2,2'-bisdiphenylphosphine (473 mg, 0.76 mmol) are added to the system in sequence, to perform reaction at 95 °C for 12 h. After the reaction is completed, water is added for quenching, and extraction is performed with dichloromethane; the organic phases are combined, and drying and concentration is performed under reduced pressure, to obtain E55-4 by column chromatography. ESI-MS(M+H)⁺=497.2.

### Synthesis Step 5: Synthesis of E55-5

E55-4 (1.5 g, 3.06 mmol) is dissolved in tetrahydrofuran (15 mL), and Pd/C (150 mg, 10% wt) is added to the system, to perform reaction at 50 °C for 16 h under H₂ atmosphere. After the reaction is completed, filtering is performed rapidly, and the mother liquor is concentrated, to obtain E55-5 by column chromatography. ESI-MS(M+H)⁺=319.2.

### Synthesis Step 6: Synthesis of E55

E55-5 (1g, 3.12mmol) is dissolved in tetrahydrofuran (10 mL), and an aqueous solution of H₂SO₄ (10 mL, 1mol/L) is added to the system, to perform reaction at 50°C for 16 h. After the reaction is completed, the pH is adjusted to 7 using an aqueous solution of sodium bicarbonate, and extraction is performed with ethyl acetate; the organic phases are combined, and drying and concentration is performed under reduced pressure, to obtain E55 by column chromatography. ESI-MS(M+H)⁺=273.1.

Following the synthesis route and method for the intermediate E55, the following intermediate compounds are obtained by synthesis:

| No. of intermediate | Structure of intermediate and ESI-MS(M+H)⁺ | No. of intermediate | Structure of intermediate and ESI-MS(M+H)⁺ |
|---|---|---|---|
| E56 | 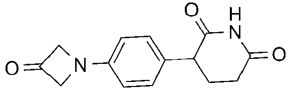 ESI-MS(M+H)⁺=259.1 | E57 | 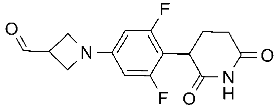 ESI-MS(M+H)⁺= 309.1 |
| E58 | 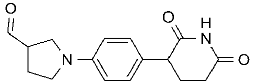 ESI-MS(M+H)⁺= 287.1 | E59 | 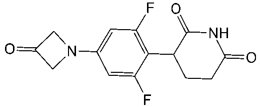 ESI-MS(M+H)⁺=295.1 |
| E60 | 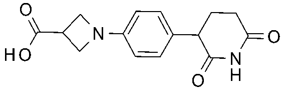 ESI-MS(M+H)⁺= 289.1 | E61 | 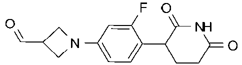 ESI-MS(M+H)⁺= 291.1 |
| E62 | 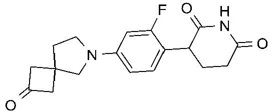 ESI-MS(M+H)⁺= 331.1 | E63 | 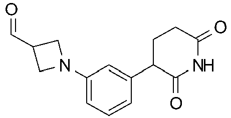 ESI-MS(M+H)⁺= 273.1 |
| E64 | 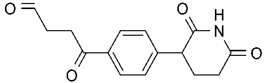 ESI-MS(M+H)⁺= 274.1 | E65 | 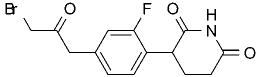 ESI-MS(M+H)⁺= 342.0 |
| E66 | 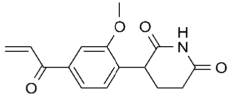 ESI-MS(M+H)⁺= 274.1 | | |

### Embodiment 21: Synthesis of intermediate D16

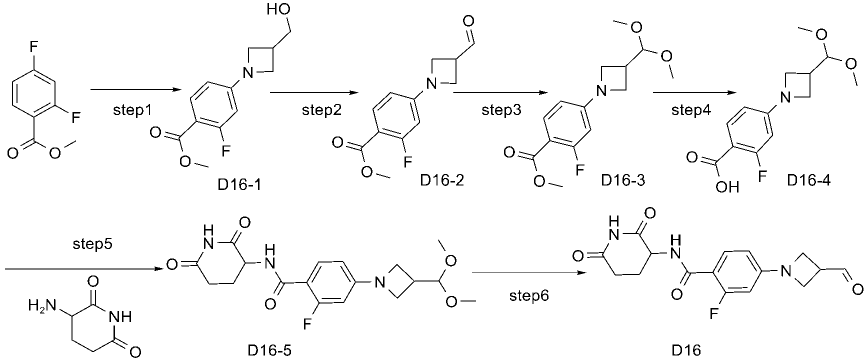

### Synthesis Step 1: Synthesis of D16-1

Methyl 2,4-difluorobenzoate (5.0 g, 32.22 mmol) is dissolved in N,N-dimethylformamide (50mL), and 3-hydroxymethylazine hydrochloride (4.8 g, 38.67 mmol) and potassium carbonate (13.3 g, 96.66 mmol) are added to the system in sequence, to perform reaction at 90 °C for 16 h. After the reaction is completed, quenching is performed with water, and extraction is performed with ethyl acetate; the organic phases are combined, and concentration is performed under reduced pressure, to obtain E16-1 by column chromatography, with a yield of 98%. ESI-MS(M+H)⁺=240.1.

### Synthesis Step 2: Synthesis of D16-2

D16-1 (3g, 13.5mmol) is dissolved in dichloromethane (50mL) and stirred at room temperature until dissolved, and desmartin reagent (8.6g, 20.25mmol) is added in 10 portions, to perform reaction at room temperature for 1h. After the reaction is completed, an appropriate amount of an aqueous solution of saturated sodium bicarbonate is added, and extraction is performed with dichloromethane; the organic phases are combined, and concentration is performed under reduced pressure, to obtain D16-2 by column chromatography, with a yield of 67%. ESI-MS(M+H)⁺=238.1.

### Synthesis Step 3: Synthesis of D16-3

D16-2 (1.9 g, 8.63 mol) is dissolved in methanol (20 mL), trimethoxymethane (1.3 g, 12.94 mmol) is added to the system in sequence for stirring at room temperature for 15 minutes, and then p-toluenesulfonic acid (150 mg, 0.86 mmol) is added, to perform reaction at 60 °C for 4 h. After the reaction is completed, the pH is adjusted to 8 using an aqueous solution of sodium bicarbonate, the methanol is removed by concentration, and extraction is performed with dichloromethane; the organic phases are combined, and drying and concentration is performed under reduced pressure, to obtain 1.5g of D16-3 by column chromatography, with a yield of 65%. ESI-MS(M+H)⁺=284.1.

### Synthesis Step 4: Synthesis of D16-4

D16-3 (1.4 g, 5.26 mmol) is dissolved in a mixed solvent (15 mL, methanol/tetrahydrofuran/water = 1:1:1), and lithium hydroxide (1.2 g, 52.60 mmol) is added to the system, to perform reaction at room temperature for 4 h. After the reaction is completed, the pH is adjusted to 6 using 2N hydrochloric acid, the organic solvents are concentrated, and then extraction is performed with dichloromethane (containing 10% methanol); the organic phases are combined, and drying and concentration is performed under reduced pressure, to obtain D16-4 by column chromatography, with a yield of 68%. ESI-MS(M-H)⁺=270.1.

### Synthesis Step 5: Synthesis of intermediate D16-5

D16-4 (800 mg, 3.17 mmol) is dissolved in N,N-dimethylformamide (10 mL) for stirring at room temperature until being dissolved completely, 3-aminopiperidine-2,6-dione (800 mg, 6.34 mmol), 1-hydroxybenzotriazole (510 mg, 3.80 mmol) and triethylamine (1.6 mL, 12.68 mmol) are added for stirring at room temperature for 30 min, and then carbodiimide hydrochloride (1.8 g, 9.51 mmol) is added, to perform reaction at room temperature for 3 h. After the reaction is completed, an aqueous solution of ammonium chloride is added for quenching, and extraction is performed with ethyl acetate; the organic phases are combined, and drying and concentration is performed, to obtain D16-5 by column chromatography, with a yield of 49%. ESI-MS(M-H)⁺=380.2.

### Synthesis Step 6: Synthesis of intermediate D16

The synthesis of D16 follows the same method as Synthesis Step 6 in the synthesis of the intermediate E55.

### Embodiment 22: Synthesis of intermediate D17

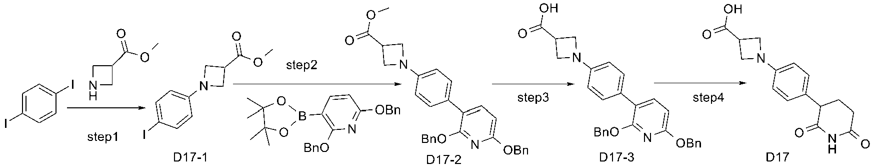

### Synthesis Step 1-2: Synthesis of intermediate D17-2

The synthesis of D17-2 follows the same method as Synthesis Steps 1 and 4 in the synthesis of the intermediate E55.

### Synthesis Step 3: Synthesis of intermediate D17-3

D17-2 (2 g, 4.17 mmol) is dissolved in a mixed solvent (20 mL, methanol/tetrahydrofuran/water = 1:1:1), and lithium hydroxide (500mg, 20.83 mmol) is added to the system, to perform reaction at room temperature for 4 h. After the reaction is completed, the pH is adjusted to 6 using 2N hydrochloric acid, the organic solvents are concentrated, and then extraction is performed with dichloromethane; the organic phases are combined, and drying and concentration is performed under reduced pressure, to obtain D17-3 by column chromatography, with a yield of 61%. ESI-MS(M-H)⁺=467.2.

### Synthesis Step 4: Synthesis of intermediate D17

The synthesis of D17 follows the same method as Synthesis Step 5 in the synthesis of the intermediate E55.

### Embodiment 23: Synthesis of intermediate D19

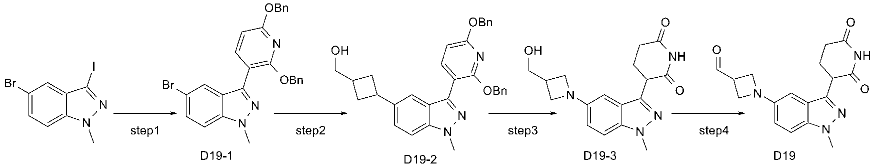

### Synthesis Step 1: Synthesis of D19-1

The synthesis of D19-1 follows the same method as Synthesis Step 4 in the synthesis of the intermediate E55.

### Synthesis Step 2-3: Synthesis of D19-3

The synthesis of D19-3 follows the same method as Synthesis Steps 1 and 5 in the synthesis of the intermediate E55.

### Synthesis Step 4: Synthesis of D19

The synthesis of D19 follows the same method as Synthesis Step 2 in the synthesis of the intermediate E55.

### Embodiment 24: Synthesis of intermediate D20

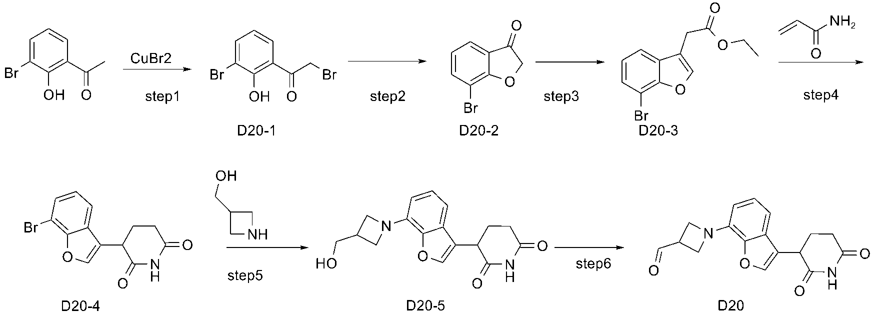

### Synthesis Step 1: Synthesis of D20-1

Under nitrogen protection, 3-bromo-2-hydroxyacetophenone (20g, 93.04 mmol) is dissolved in chloroform (100ml) and ethyl acetate (100ml), followed by adding copper bromide (41.54g, 186 mmol); then, the reaction mixture is heated to 90 °C, and stirred and reacted for 16 hours. After the reaction is completed, cooling is performed to room temperature, and filtering is performed with a filter cake washed with dichloromethane (1.5 L), to obtain a solution of D20-1, which is used directly in the next step. ESI-MS(M+H)⁺=292.9.

### Synthesis Step 2: Synthesis of D20-2

The solution of D20-1 obtained above (350 ml) is cooled to 0 °C, and then triethylamine (14.11 g, 140 mmol) is slowly added dropwise. After the addition is completed, the reaction mixture is slowly heated to room temperature and stirred for 2 hours. After the reaction is completed, water (200 mL) is added to separate the mixture, and the aqueous phase is extracted with dichloromethane (500 mL). The organic phases are combined, washed with saturated brine, dried with anhydrous sodium sulfate and filtered, and the solvent is removed from the filtrate under reduced pressure, to obtain D20-2. ESI-MS(M+H)⁺= 212.9.

### Synthesis Step 3: Synthesis of D20-3

Under nitrogen protection, D20-2 (19.8 g, 93.00 mmol) is dissolved in toluene (150 ml), followed by adding ethoxyformylmethylenetriphenylphosphine (38.88 g, 112 mmol), to heat the reaction mixture to 130 °C, stir and react for 36 hours. After the reaction is completed, cooling is performed to room temperature, concentration is performed under reduced pressure to remove the solvent, and methyl tert-butyl ether (700 mL x 3) is added to the residue, to stir at room temperature for 20 minutes, filtered with the filter cake washed with methyl tert-butyl ether (100 mL) for collecting the filtrate. The solvent is removed from the filtrate under reduced pressure, and the residue is purified by column chromatography to obtain D20-3, with a yield of 32%. ESI-MS(M+H)⁺=283.0.

### Synthesis Step 4: Synthesis of D20-4

D20-3 (3g, 10.60mmol) is added to N,N-dimethylformamide (20mL), followed by adding acrylamide (903.79 mg, 12.72mmol) and potassium tert-butoxide (1.78g, 15.89mmol), to stir the reaction mixture at room temperature for 1 hour. After the reaction is completed, the reaction solution is poured into 1N hydrochloric acid (15mL), water (25mL) is added, and extraction is performed with ethyl acetate (35mL x 3); the organic phases are combined, washing is performed with saturated brine (30mL x 2), drying is performed over anhydrous sodium sulfate, filtering is performed, and concentration is performed under reduced pressure to remove the solvent. The obtained residue is purified by column chromatography to obtain D20-4. The yield is 69%. ESI-MS(M+H)⁺=308.0.

### Synthesis Step 5-6: Synthesis of D20

The synthesis of D20 follows the same routes and operations as Synthesis Step 1-2 in the synthesis of the intermediate E55.

### Embodiment 25: Synthesis of intermediate E68

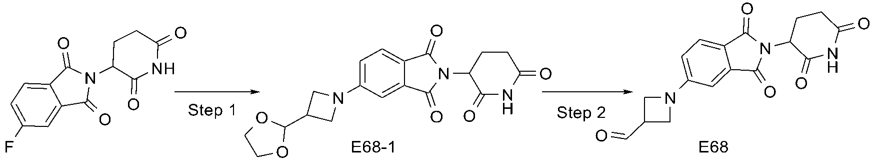

### Synthesis

### Step 1: Synthesis of E68-1

2-(2,6-Dioxo-piperidin-3-yl)-5-fluoro-isoindole-1,3-dione (3.0 g, 10.86 mmol) is dissolved in N,N-dimethylformamide (30 mL), and 3-(1,3-dioxolane-2-yl)azacyclobutane (1.4 g, 10.86 mmol) and N,N-diisopropylethylamine (2.8 g, 21.72 mmol) are added in sequence to the system, to perform reaction at 90 °C for 6 h. After the reaction is completed, water is added for quenching, and extraction is performed with dichloromethane; the organic phases are combined, and drying and concentration is performed under reduced pressure, to obtain E68-1 by column chromatography. ESI-MS(M+H)⁺=386.1.

### Synthesis Step 2: Synthesis of intermediate E68

E68-1 (4.2 g, 8.76 mmol) is dissolved in an aqueous solution of hydrochloric acid (200 mL, 3 mol/L) to perform reaction at 50 °C for 2 h. After the reaction is completed, the pH is adjusted to 7 by sodium bicarbonate, and extraction is performed with dichloromethane; the organic phases are combined, and drying and concentration is performed under reduced pressure, to obtain the intermediate E68 by column chromatography. ESI-MS(M+H)⁺=342.1.

Following the synthesis route and method for the intermediate E68, the following intermediate compounds are obtained by synthesis:

| No. of intermediate | Structure of intermediate and ESI-MS(M+H) ⁺ | No. of intermediate | Structure of intermediate and ESI-MS(M+H) ⁺ |
|---|---|---|---|
| E69 | 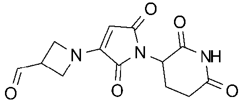 ESI-MS(M+H) ⁺=292.1 | E70 | 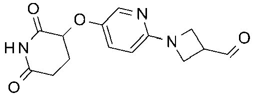 ESI-MS(M+H) ⁺=290.1 |

### Embodiment 26: Synthesis of compound 014

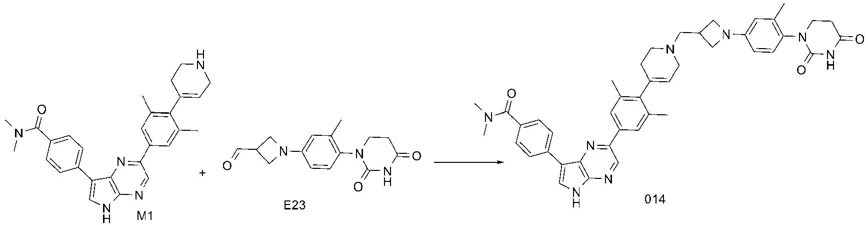

M1 (102 mg, 0.22 mmol) is dissolved in ethanol (5 mL) and stirred at room temperature until being dissolved completely, and E23 (65 mg, 0.222 mmol) is added to the system for reaction at room temperature for 1 h, followed by adding sodium cyanoborohydride (44 mg, 0.66 mmol) to the system for reaction at room temperature for 1 h. After the reaction is completed, quenching is performed with water, ethanol is removed under reduced pressure, and extraction is performed with ethyl acetate; the organic phases are combined, and drying and concentration is performed, to obtain 80mg of 0014 by column chromatography, with a yield of 48%. ESI-MS(M+H)⁺=723.2. 1H NMR (400 MHz, DMSO-d) δ 10.54 (s, 1H), 9.11 - 8.83 (m, 1H), 8.75 (s, 1H), 8.25 (d, J = 8.0 Hz, 2H), 7.78 (d, J = 11.9 Hz, 3H), 7.52 (d, J = 7.9 Hz, 2H), 7.02 (d, J = 8.8 Hz, 1H), 6.27 (d, J = 5.5 Hz, 2H), 5.44 (s, 1H), 3.98 (d, J = 7.5 Hz, 2H), 3.77 (dt, J = 13.8, 7.1 Hz, 1H), 3.63 (dt, J = 12.7, 6.2 Hz, 1H), 3.53 (d, J = 6.6 Hz, 2H), 3.21 - 3.10 (m, 6H), 3.07 (s, 2H), 2.84 (d, J = 6.4 Hz, 2H), 2.80 - 2.71 (m, 4H), 2.35 (m, 6H), 2.27 (m, 3H), 2.23 (m, 3H).

Following the synthetic route and method for 014, the following compounds are synthesized (the compound numbers in the table follow those in the claims and specification):

| Compound No. and Characterization Data | Compound No. and Characterization Data |
|---|---|
| ESI-MS(M+H)⁺=767.4 (Compound 001) | ESI-MS(M+H)⁺=744.3 (Compound 002) |
| ESI-MS(M+H)⁺=726.3 (Compound 003) | ESI-MS(M+H)⁺=739.4 (Compound 004) |
| ESI-MS(M+H)⁺=737.3 (Compound 005) | ESI-MS(M+H)⁺=730.3 (Compound 007) |
| ESI-MS(M+H)⁺=709.4 (Compound 008) | ESI-MS(M+H)⁺=736.4 (Compound 010) |
| ESI-MS(M+H)⁺=738.3 (Compound 011) | ESI-MS(M+H)⁺=722.4 (Compound 013) |
| ¹H NMR (400 MHz, DMSO-*d*₆) δ 12.40 (s 1H), 10.86 (s, 1H), 8.85 (s, 1H), 8.61 - 8.50 (m, 3H), 8.38 (d, *J* = 8.4 Hz, 2H), 7.99 - 7.96 (m, 1H), 7.83 (s, 2H), 7.51 (d, *J* = 8.4 Hz, 2H), 6.43 - 6.42 (m, 1H), 5.50 - 5.48 (m, 1H), 4.77 - 4.72 (m, 1H), 4.16 - 4.10 (m, 2H), 3.97 - 3.94 (m, 2H), 3.10 - 3.09(m, 2H), 2.99 (m, 6H), 2.82 - 2.64 (m, 3H), 2.31 (s, 6H), 2.24 - 2.21 (m, 2H), 2.10 - 1.95 (m, 2H), 1.27 - 1.22 (m, 2H). | ¹H NMR (400 MHz, DMSO-*d*₆) δ 12.07 (d, *J =* 2.7 Hz, 1H), 10.22 (s, 1H), 8.51 (d, *J=* 2.1 Hz, 1H), 8.40 (d, *J =* 2.1 Hz, 1H), 7.97 (d, *J* = 2.7 Hz, 1H), 7.85 - 7.79 (m, 2H), 7.47 - 7.42 (m, 2H), 7.38 (s, 2H), 6.98 (d, *J =* 8.4 Hz, 1H), 6.27 (d, *J =* 2.6 Hz, 1H), 6.23 (dd, *J =* 8.4, 2.7 Hz, 1H), 5.40 (s, 1H), 3.93 (t, *J* = 7.4 Hz, 2H), 3.66 - 3.59 (m, 1H), 3.53 - 3.43 (m, 3H), 3.41 (s, 1H), 2.96 (s, 9H), 2.68 - 2.58 (m, 3H), 2.24 (s, 8H), 2.19 (d, *J =* 6.7 Hz, 2H), 2.06 (s, 3H). |
| ESI-MS(M+H)⁺=738.3 (Compound 012) | ESI-MS(M+H)⁺=694.3 (Compound 048) |
| ESI-MS(M+H)⁺=722.4 (Compound 015) | ESI-MS(M+H)⁺=752.4 (Compound 016) |
| ESI-MS(M+H)⁺=777.4 (Compound 017) | ESI-MS(M+H)⁺=769.4 (Compound 018) |
| ESI-MS(M+H)⁺=741.4 (Compound 019) | ESI-MS(M+H)⁺=737.3 (Compound 020) |
| ESI-MS(M+H)⁺=737.4 (Compound 021) | ESI-MS(M+H)⁺=724.4 (Compound 022) |
| ESI-MS(M+H)⁺=777.4 (Compound 023) | ESI-MS(M+H)⁺=717.3 (Compound 024) |
| ESI-MS(M+H)⁺=708.4 (Compound 025) | ESI-MS(M+H)⁺=720.3 (Compound 026) |
| ESI-MS(M+H)⁺=723.4 (Compound 027) | ESI-MS(M+H)⁺=765.4 (Compound 028) |
| ESI-MS(M+H)⁺=769.4 (Compound 029) | ESI-MS(M+H)⁺=752.3 (Compound 030) |
| ESI-MS(M+H)⁺=752.3 (Compound 031) | ESI-MS(M+H)⁺=795.4 (Compound 032) |
| ESI-MS(M+H)⁺=709.3 (Compound 033) | ESI-MS(M+H)⁺=749.4 (Compound 034) |
| ESI-MS(M+H)⁺=767.4 (Compound 035) | ESI-MS(M+H)⁺=751.4 (Compound 036) |
| ESI-MS(M+H)⁺=740.3 (Compound 037) | ESI-MS(M+H)⁺=727.3 (Compound 038) |
| ESI-MS(M+H)⁺=722.4 (Compound 039) | ESI-MS(M+H)⁺=737.4 (Compound 040) |
| ESI-MS(M+H)⁺=751.4 (Compound 041) | ESI-MS(M+H)⁺=737.4 (Compound 042) |
| ESI-MS(M+H)⁺=723.4 (Compound 043) | ESI-MS(M+H)⁺=766.4 (Compound 044) |
| ESI-MS(M+H)⁺=766.4 (Compound 045) | ESI-MS(M+H)⁺=747.3 (Compound 046) |
| ESI-MS(M+H)⁺=727.3 **(Compound 049)** | ESI-MS(M+H)⁺=741.4 **(Compound 052)** |
| ¹H NMR (400 MHz, DMSO-*d*₆) δ 12.41 (s, 1H), 10.37 (s, 1H), 8.87 (s, 1H), 8.52 (d, *J =* 2.4 Hz, 1H), 8.38 (d, *J* | ¹H NMR (400 MHz, DMSO-*d*₆) δ 12.42 (s 1H), 10.77 (s, 1H), 8.86 (s, 1H), 8.50 - 8.48 (m, 1H), 8.37 (d, *J* = 8.1 |
| = 8.0 Hz, 2H), 7.84 (s, 2H), 7.51 (d, *J =* 7.9 Hz, 2H), 7.17 (t, *J =* 8.6 Hz, 1H), 6.39 - 6.22 (m, 2H), 5.46 (s, 1H), 3.99 (t, *J* = 7.6 Hz, 2H), 3.60 (t, *J* = 6.6 Hz, 2H), 3.54 (t, *J* = 6.5 Hz, 2H), 3.13 - 3.07 (m, 2H), 3.00 (s, 6H), 2.73 (d, *J =* 6.8 Hz, 2H), 2.69 (s, 4H), 2.31 (s, 6H), 2.19 (d, *J =* 7.3 Hz, 2H), 1.91 (s, 1H). | Hz, 2H), 7.82 (s, 2H), 7.51 - 7.49 (m, 2H), 6.51 - 6.36 (m, 3H), 5.56 - 5.54 (m, 1H), 5.44 - 5.43 (m, 1H), 4.21 - 4.16 (m, 1H), 3.92 - 3.88 (m, 2H), 3.18 - 3.16 (m, 2H), 2.99 (m, 6H), 2.78 - 2.70 (m, 4H), 2.59 - 2.55 (m, 1H), 2.29 (s, 6H), 2.22 - 1.78 (m, 6H), 1.29 - 1.19 (m, 2H). |
| ESI-MS(M+H)⁺=780.4 (Compound 051) | ESI-MS(M+H)⁺=819.4 (Compound 050) |
| ESI-MS(M+H)⁺=797.4 (Compound 053) | ESI-MS(M+H)⁺=743.3 (Compound 054) |
| ESI-MS(M+H)⁺=714.3 (Compound 055) | ESI-MS(M+H)⁺=738.4 (Compound 056) |
| ESI-MS(M+H)⁺=728.3 (Compound 057) | ESI-MS(M+H)⁺=792.4 (Compound 058) |
| ESI-MS(M+H)⁺=781.4 (Compound 059) | ESI-MS(M+H)⁺=722.3 (Compound 060) |
| ESI-MS(M+H)⁺=753.3 (Compound 061) | ESI-MS(M+H)⁺=781.4 (Compound 062) |
| ¹H NMR (400 MHz, DMSO-*d*₆) δ 12.42 - 12.40 (m 1H), 10.36 (s, 1H), 8.86 (s, 1H), 8.51 (d, *J* = 3.0 Hz, 1H), 8.38 (d, *J* = 8.4 Hz, 2H), 7.83 (s, 2H), 7.68 - 7.66 (m, 1H), 7.64 - 7.62 (m, 1H), 7.51 - 7.49 (m, 2H), 7.17 (d, *J* = 8.7 Hz, 1H), 5.49 - 5.47 (m, 1H), 4.41 - 4.14 (m, 3H), 3.97 - 3.94 (m, 1H), 3.86 (s, 3H), 3.62 - 3.58 (m, 2H), 3.08 - 3.06 (m, 2H), 3.00 (s, 6H), 2.71 - 2.63 (m, 3H), 2.30 (s, 6H), 2.23 - 2.20 (m, 2H), 1.29 - 1.24 (m, 2H). | |
| ESI-MS(M+H)⁺=750.4 (Compound 063) | ESI-MS(M+H)⁺=778.3 (Compound 064) |
| ESI-MS(M+H)⁺=779.4 (Compound 065) | ESI-MS(M+H)⁺=707.3 (Compound 066) |
| ESI-MS(M+H)⁺=750.4 (Compound 067) | ESI-MS(M+H)⁺=742.3 (Compound 068) |
| ESI-MS(M+H)⁺=752.4 (Compound 069) | ESI-MS(M+H)⁺=706.3 (Compound 070) |
| ESI-MS(M+H)⁺=769.3 **(Compound 071)** | ESI-MS(M+H)⁺=749.3 (Compound 072) |
| ¹H NMR (400 MHz, DMSO-d6) δ 12.38 (d, J = 2.9 Hz, 1H), 10.82 (s, 1H), 8.83 (s, 1H), 8.48 (d, J = 2.6 Hz, 1H), 8.38 - 8.32 (m, 2H), 7.95 (t, J = 7.4 Hz, 1H), 7.80 (s, 2H), 7.59 (t, J = 8.6 Hz, 1H), 7.52 - 7.44 (m, 2H), 6.30 - 6.18 (m, 2H), 5.72 (s, 1H), 5.43 (s, 1H), 4.69 (dt, | |
| J = 12.6, 6.4 Hz, 1H), 4.02 (t, J *=* 7.8 Hz, 2H), 3.59 (dd, J = 7.9, 5.4 Hz, 2H), 3.08 (s, 2H), 2.97 (s, 7H), 2.75 - 2.65 (m, 4H), 2.48 (s, 2H), 2.28 (s, 6H), 2.17 (s, 2H), 2.02 - 1.91 (m, 1H). | |
| ESI-MS(M+H)⁺=752.4 (Compound 073) | ESI-MS(M+H)⁺=765.4 (Compound 074) |
| ESI-MS(M+H)⁺=805.4 (Compound 075) | ESI-MS(M+H)⁺=742.4 (Compound 076) |
| ESI-MS(M+H)⁺=742.3 (Compound 077) | ESI-MS(M+H)⁺=769.4 (Compound 078) |
| ESI-MS(M+H)⁺=745.3 (Compound 079) | ESI-MS(M+H)⁺=766.4 (Compound 080) |
| ESI-MS(M+H)⁺=751.3 **(Compound 081)** | ESI-MS(M+H)⁺=798.3 (Compound 082) |
| ¹H NMR (400 MHz, DMSO-*d*₆) δ 12.38 (d, *J =* 2.9 Hz, 1H), 10.79 (s, 1H), 8.83 (s, 1H), 8.47 (d, *J* = 2.7 Hz, 1H), 8.38 (d, *J =* 8.3 Hz, 1H), 8.36 - 8.32 (m, 2H), 7.80 (s, 2H), 7.74 - 7.68 (m, 2H), 7.54 - 7.44 (m, 2H), 6.43 - 6.38 (m, 2H), 5.46 - 5.39 (m, 1H), 4.70 (ddd, *J =* 12.9, 8.3, 5.3 Hz, 1H), 4.10 (q, *J =* 5.3 Hz, 1H), 4.01 (t, *J =* 7.7 Hz, 2H), 3.57 (dd, *J =* 7.7, 5.4 Hz, 2H), 3.13 (d, *J =* 4.7 Hz, 3H), 3.09 (s, 2H), 2.96 (s, 6H), 2.80 - 2.71 (m, 3H), 2.71 - 2.65 (m, 2H), 2.27 (s, 6H), 2.18 (s, 2H). | |
| ESI-MS(M+H)⁺=798.3 (Compound 083) | ESI-MS(M+H)⁺=775.3 (Compound 084) |
| ESI-MS(M+H)⁺=726.3 (Compound 085) | ESI-MS(M+H)⁺=725.4 (Compound 086) |
| ESI-MS(M+H)⁺=724.4 (Compound 087) | ESI-MS(M+H)⁺=727.3 (Compound 088) |
| ESI-MS(M+H)⁺=752.4 (Compound 089) | ESI-MS(M+H)⁺=743.4 (Compound 090) |
| ESI-MS(M+H)⁺=715.4 (Compound 091) | ESI-MS(M+H)⁺=731.4 (Compound 092) |
| ESI-MS(M+H)⁺=743.4 (Compound 093) | ESI-MS(M+H)⁺=757.3 (Compound 094) |
| ESI-MS(M+H)⁺=759.3 (Compound 095) | ESI-MS(M+H)⁺=716.4 (Compound 096) |
| ESI-MS(M+H)⁺=736.2 (Compound 097) | ESI-MS(M+H)⁺= 753.3 (Compound 098) |
| ESI-MS(M+H)⁺= 734.4 **(Compound 099)** | ESI-MS(M+H)⁺= 769.4 (Compound 100) |
| ¹H NMR (400 MHz, DMSO-*d*₆) δ 12.30 - 11.94 (m, 1H), 10.23 (s, 1H), 8.59 - 8.47 (m, 1H), 8.47 - 8.35 (m, 1H), 7.99 (d, *J =* 2.7 Hz, 1H), 7.91 (d, *J =* 8.1 Hz, 1H), | |
| 7.74 (d, *J=* 8.2 Hz, 1H), 7.65 (s, 1H), 7.36 (s, 2H), 6.96 (d, *J= 8.3* Hz, 1H), 6.25 (d, *J =* 2.5 Hz, 1H), 6.21 (dd, *J* = 8.5, 2.5 Hz, 1H), 5.37 (s, 1H), 3.89 (t, *J =* 7.4 Hz, 2H), 3.52 (t, *J* = 6.6 Hz, 3H), 3.14 (s, 1H), 3.03 (d, *J* = 6.1 Hz, 3H), 3.00 (s, 3H), 2.70 - 2.53 (m, 7H), 2.46 (s, 1H), 2.22 (s, 6H), 2.13 (s, 3H), 2.06 (s, 4H). | |
| ESI-MS(M+H)⁺= 694.3 **(Compound 101)** | ESI-MS(M+H)⁺= 708.4 **(Compound 102)** |
| ¹H NMR (400 MHz, DMSO-d6) δ 10.24 (s, 1H), 9.17 (td, J = 7.8, 1.8 Hz, 1H), 8.91 (s, 1H), 8.37 (d, J = 2.7 Hz, 1H), 7.86 - 7.76 (m, 3H), 7.58 (t, J = 7.8 Hz, 1H), 7.01 (d, J = 8.3 Hz, 1H), 6.32 - 6.24 (m, 2H), 5.45 (s, 1H), 3.96 (t, J = 7.4 Hz, 2H), 3.65 (m, 1H), 3.47 (m, 4H), 3.15 - 3.06 (m, 2H), 2.99 (s, 1H), 2.78 - 2.62 (m, 6H), 2.30 (s, 6H), 2.18 (d, J = 7.4 Hz, 2H), 2.10 (s, 3H). | ¹H NMR (400 MHz, DMSO-*d*₆) δ 12.43 - 12.33 (m, 1H), 10.73 (s, 1H), 8.83 (s, 1H), 8.47 (d, *J* = 2.6 Hz, 1H), 8.35 (d, *J* = 7.9 Hz, 2H), 7.80 (s, 2H), 7.48 (d, *J* = 7.9 Hz, 2H), 6.97 (d, *J* = 8.0 Hz, 2H), 6.36 (d, *J* = 8.0 Hz, 2H), 5.42 (s, 1H), 3.91 (t, *J =* 7.4 Hz, 2H), 3.66 (dd, *J =* 10.9, 5.0 Hz, 1H), 3.44 (t, *J =* 6.4 Hz, 2H), 3.14 (s, 2H), 3.06 (s, 2H), 2.97 (s, 6H), 2.73 - 2.62 (m, 4H), 2.61 - 2.54 (m, 1H), 2.47 (p, *J =* 1.9 Hz, 2H), 2.27 (s, 6H), 2.17 (s, 2H). |
| ESI-MS(M+H)⁺= 737.4 (Compound 103) | ESI-MS(M+H)⁺= 741.4 (Compound 106) |
| ESI-MS(M+H)⁺= 781.4 **(Compound 105)** | ESI-MS(M+H)⁺= 771.4 **(Compound 107)** |
| ¹H NMR (400 MHz, DMSO-*d*₆) δ 12.46 (s, 1H), 10.38 (s, 1H), 8.87 (s, 1H), 8.58 - 8.51 (m, 1H), 8.41 (d, *J* = 8.1 Hz, 2H), 7.84 (s, 2H), 7.72 (d, *J =* 8.0 Hz, 2H), 7.16 (t, *J=* 8.6 Hz, 1H), 6.30 (dd, *J =* 12.5, 2.4 Hz, 1H), 6.24 (dd, *J =* 8.6, 2.4 Hz, 1H), 5.44 (s, 1H), 4.69 (s, 4H), 4.55 (s, 2H), 4.23 (s, 2H), 3.97 (t, *J =* 7.5 Hz, 2H), 3.60 (t, *J* = 6.6 Hz, 2H), 3.52 (t, *J* = 6.5 Hz, 2H), 3.09 (s, 2H), 2.99 (t, *J =* 7.2 Hz, 1H), 2.70 (q, *J =* 7.7, 6.4 Hz, 6H), 2.30 (s, 6H), 2.19 (s, 2H). | ¹H NMR (400 MHz, DMSO-*d*₆) δ 12.40 (s, 1H), 10.37 (s, 1H), 8.87 (s, 1H), 8.51 (s, 1H), 8.37 (s, 2H), 7.84 (s, 2H), 7.51 (d, *J =* 7.8 Hz, 2H), 7.17 (t, *J =* 8.7 Hz, 1H), 6.37 - 6.22 (m, 2H), 5.76 (s, 1H), 5.46(m, 1H),4.85 (s, 1H), 3.99 (t, *J =* 7.6 Hz, 3H), 3.57 (dt, *J =* 25.5, 6.6 Hz, 5H), 3.15 - 2.94 (m, 6H), 2.81 - 2.63 (m, 6H), 2.31 (s, 6H), 2.20 (s, 2H), 1.23 (d, *J =* 6.2 Hz, 3H). |
| ESI-MS(M+H)⁺= 757.4 **(Compound 108)** | ESI-MS(M+H)⁺= 780.4 **(Compound 109)** |
| ¹H NMR (400 MHz, DMSO-*d*₆) δ 11.89 - 11.81 (m, 1H), 10.18 (s, 1H), 8.63 (s, 1H), 8.18 (s, 1H), 8.00 - | ¹H NMR (400 MHz, DMSO-*d*₆) δ 12.06 (s, 1H), 10.22 (s, 1H), 8.52 (s, 1H), 8.40 (s, 1H), 7.96 (s, 1H), 7.81 (d, *J* = 7.9 Hz, 2H), 7.47 - 7.40 (m, 2H), 7.37 (s, 2H), 6.97 (d, |
| 7.84 (m, 2H), 7.66 (s, 2H), 6.97 (t, *J=* 8.6 Hz, 1H), 6.18 - 6.09 (m, 1H), 6.09 - 5.95 (m, 1H), 5.25 (s, 1H), 4.05 (dt, *J=* 11.2, 5.8 Hz, 1H), 3.79 (t, *J=* 7.6 Hz, 2H), 3.42 *(t, J=* 6.7 Hz, 3H), 3.34 (t, *J =* 6.6 Hz, 4H), 2.90 (s, 2H), 2.83 (d, *J =* 11.6 Hz, 3H), 2.32 (s, 2H), 2.25 (q, *J =* 7.4 Hz, 2H), 2.11 (s, 6H), 2.00 (s, 3H), 1.86 (d, *J=* 15.7 Hz, 4H), 1.72 (s, 1H), 1.03 (s, 1H), 0.85 (t, *J=* 7.2 Hz, 3H). | *J =* 8.3 Hz, 1H), 6.26 (s, 1H), 6.22 (d, *J =* 8.4 Hz, 1H), 5.38 (s, 1H), 3.91 (t, *J* = 7.5 Hz, 2H), 3.64 - 3.56 (m, 2H), 3.20 (d, *J =* 14.6 Hz, 4H), 3.06 (d, *J =* 19.0 Hz, 4H), 2.93 (s, 6H), 2.72 - 2.58 (m, 7H), 2.23 (s, 7H), 2.14 (s, 2H), 2.06 (s, 4H). |
| ESI-MS(M+H)⁺= 788.4 **(Compound 110)** | ESI-MS(M+H)⁺= 705.4 (Compound 111) |
| ¹H NMR (400 MHz, DMSO-*d*₆) δ 11.68 (s, 1H), 10.21 (s, 1H), 8.44 (s, 1H), 8.32 (s, 1H), 8.26 (s, 1H), 7.88 (s, 1H), 7.68 (s, 1H), 7.37 (s, 2H), 6.99 (d, *J=* 8.3 Hz, 1H), 6.27 (d, *J* = 8.0 Hz, 2H), 5.42 (s, 1H), 4.15 (t, *J* = 7.9 Hz, 1H), 3.95 (t, *J=* 7.4 Hz, 2H), 3.49 (s, 3H), 3.00 (t, *J* = 9.3 Hz, 6H), 2.87 - 2.69 (m, 6H), 2.65 (d, *J =* 6.3 Hz, 2H), 2.47 (s, 3H), 2.25 (s, 7H), 2.07 (s, 4H), 1.99 (d, *J =* 7.4 Hz, 4H). | |
| ESI-MS(M+H)⁺= 766.5 **(Compound 112)** | ESI-MS(M+H)⁺= 751.4 **(Compound 113)** |
| ¹H NMR (400 MHz, DMSO-*d*₆) δ 11.67 (d, *J =* 2.6 Hz, 1H), 10.20 (s, 1H), 8.43 (d, *J* = 2.1 Hz, 1H), 8.31 (d, *J =* 2.1 Hz, 1H), 8.24 (s, 1H), 7.87 (s, 1H), 7.67 (d, *J =* 2.5 Hz, 1H), 7.36 (s, 2H), 6.98 (d, *J=* 8.3 Hz, 1H), 6.28 (d, *J* = 2.6 Hz, 1H), 6.24 (dd, *J =* 8.3, 2.7 Hz, 1H), 5.46 - 5.36 (m, 1H), 3.94 (t, *J =* 7.4 Hz, 2H), 3.61 (ddd, *J =* 12.4, 8.8, 5.7 Hz, 2H), 3.07 (s, 3H), 2.96 (dd, *J =* 13.9, 7.4 Hz, 4H), 2.71 - 2.63 (m, 6H), 2.24 (s, 7H), 2.18 - 2.13 (m, 2H), 2.06 (s, 4H), 2.05 - 2.00 (m, 3H), 1.87 (s, 2H), 1.00 (d, *J =* 6.5 Hz, 7H). | ¹H NMR (400 MHz, DMSO-*d*₆) δ 12.38 (d, *J =* 2.7 Hz, 1H), 10.23 (s, 1H), 8.83 (s, 1H), 8.47 (d, *J =* 2.4 Hz, 1H), 8.35 (d, *J* = 8.1 Hz, 2H), 7.80 (s, 2H), 7.47 (d, *J* = 8.1 Hz, 2H), 7.00 (d, *J* = 8.6 Hz, 1H), 6.77 (d, *J* = 2.8 Hz, 1H), 6.72 (dd, *J =* 8.8, 2.8 Hz, 1H), 5.40 (s, 1H), 3.70 - 3.60 (m, 4H), 3.03 (s, 3H), 2.96 (s, 6H), 2.68 - 2.63 (m, 3H), 2.63 - 2.56 (m, 4H), 2.46 (p, *J =* 1.8 Hz, 2H), 2.27 (s, 9H), 2.20 - 2.13 (m, 3H), 1.82 - 1.74 (m, 2H). |
| ESI-MS(M+H)⁺= 741.4 (Compound 114) | ESI-MS(M+H)⁺= 762.4 (Compound 115) |
| ESI-MS(M+H)⁺= 738.4 (Compound 116) | ESI-MS(M+H)⁺= 767.4 (Compound 117) |
| ESI-MS(M+H)⁺= 764.4 (Compound 118) | ESI-MS(M+H)⁺= 799.3 (Compound 120) |
| ESI-MS(M+H)⁺= 753.4 **(Compound 119)** | ESI-MS(M+H)⁺= 749.4 **(Compound 121)** |
| ¹H NMR (400 MHz, DMSO-*d*₆) δ 12.38 (d, *J =* 2.9 Hz, 1H), 10.34 (s, 1H), 8.83 (s, 1H), 8.48 (d, *J* = 2.8 Hz, 1H), 8.36 - 8.32 (m, 2H), 7.81 (s, 2H), 7.50 - 7.45 (m, 2H), 7.13 (s, 1H), 6.26 (dd, *J =* 12.4, 2.4 Hz, 1H), 6.20 (dd, *J =* 8.6, 2.4 Hz, 1H), 5.44 (s, 1H), 3.86 (s, 2H), 3.75 (s, 2H), 3.56 (t, *J =* 6.7 Hz, 2H), 3.30 (s, 2H), 3.13 (d, *J* = 5.1 Hz, 2H), 2.97 (s, 9H), 2.65 (t, *J* = 6.7 Hz, 3H), 2.28 (s, 8H), 1.87 (s, 1H). | ¹H NMR (400 MHz, DMSO-d6) δ 12.49 - 12.37 (m, 1H), 10.23 (s, 1H), 8.87 (s, 1H), 8.51 (d, J = 2.4 Hz, 1H), 8.38 (d, J *=* 8.0 Hz, 2H), 7.84 (s, 2H), 7.51 (d, J = 8.0 Hz, 2H), 6.98 (d, J = 8.5 Hz, 1H), 6.38 (s, 2H), 5.47 (s, 1H), 3.58 (dd, J = 34.9, 7.6 Hz, 4H), 3.48 - 3.37 (m, 2H), 3.26 - 3.12 (m, 5H), 2.84 - 2.59 (m, 5H), 2.51 - 2.47 (m,3H) 2.31 (s, 6H), 2.23 (s, 2H), 2.09 (s, 3H), 1.91 (s, 1H), 1.60 (s, 2H). |
| ESI-MS(M+H)⁺= 778.5 (Compound 122) | ESI-MS(M+H)⁺= 737.4 (Compound 123) |
| ESI-MS(M+H)⁺= 771.3 (Compound 124) | ESI-MS(M+H)⁺= 709.4 (Compound 125) |
| ESI-MS(M+H)⁺=798.4 (Compound 126) | ESI-MS(M+H)⁺= 737.4 (Compound 127) |
| ESI-MS(M+H)⁺= 796.5 (Compound 128) | ESI-MS(M+H)⁺= 736.4 (Compound 129) |
| | ¹H NMR (400 MHz, DMSO-*d*₆) δ 12.08 (s, 1H), 10.21 (s, 1H), 8.53 (s, 1H), 8.40 (s, 1H), 7.97 (d, *J* = 2.6 Hz, 1H), 7.82 (d, *J =* 8.0 Hz, 2H), 7.60 (s, 1H), 7.46 (t, *J =* 5.9 Hz, 3H), 7.10 (d, *J* = 7.8 Hz, 1H), 6.98 (d, *J* = 8.3 Hz, 1H), 6.27 (s, 1H), 6.25 - 6.20 (m, 1H), 5.49 (s, 1H), 3.92 (t, *J* = 7.4 Hz, 2H), 3.60 (td, *J* = 8.8, 8.1, 4.3 Hz, 1H), 3.43 (d, *J =* 6.0 Hz, 2H), 3.14 - 3.07 (m, 1H), 3.03 (s, 2H), 2.96 (s, 6H), 2.68 - 2.58 (m, 6H), 2.27 (s, 2H), 2.06 (s, 4H), 1.22 (d, *J =* 6.9 Hz, 6H), 1.18 (s, 1H). |
| ESI-MS(M+H)⁺= 770.3 (Compound 130) | ESI-MS(M+H)⁺= 724.4 (Compound 131) |
| ESI-MS(M+H)⁺= 724.4 (Compound 132) | ESI-MS(M+H)⁺= 713.3 (Compound 133) |
| ¹H NMR (400 MHz, DMSO-*d*₆) δ 12.27 (d, *J =* 2.9 Hz, 1H), 10.24 (s, 1H), 8.88 (s, 1H), 8.42 (d, *J* = 2.9 Hz, 1H), 8.33 (d, *J =* 2.0 Hz, 1H), 8.12 (d, *J =* 2.0 Hz, 1H), 7.88 (s, 2H), 7.02 (d, *J* = 8.4 Hz, 1H), 6.31 (d, *J* = 2.6 Hz, 1H), 6.28 (dd, *J =* 8.4, 2.7 Hz, 1H), 5.49 - 5.43 (m, | ¹H NMR (400 MHz, DMSO-d6) δ 12.44 - 12.23 (m, 1H), 10.20 (s, 1H), 8.84 (s, 1H), 8.47 (d, J = 2.9 Hz, 1H), 8.38 (d, J = 7.9 Hz, 2H), 7.46 (d, J = 8.0 Hz, 2H), 7.31 (d, J = 7.0 Hz, 2H), 7.05 (d, J = 7.6 Hz, 1H), 6.97 (d, J = 8.3 Hz, 1H), 6.31 - 6.13 (m, 2H), 4.26 (s, 1H), 3.86 (t, J |
| 1H), 4.39 (dd, *J =* 5.5, 3.3 Hz, 2H), 3.97 (t, *J =* 7.4 Hz, 2H), 3.65 (ddd, *J =* 12.4, 8.8, 5.7 Hz, 1H), 3.47 (dq, *J =* 18.6, 6.3 Hz, 4H), 3.30 (d, *J* = 4.8 Hz, 2H), 3.12 (s, 2H), 3.02 (s, 3H), 2.81 - 2.61 (m, 6H), 2.29 (s, 6H), 2.21 (s, 2H), 2.10 (s, 3H). | = 7.4 Hz, 2H), 3.65 - 3.56 (m, 1H), 3.43 (m, 3H), 3.18 (s, 3H), 3.02 (s, 2H), 2.96 (s, 6H), 2.64 (m,3H), 2.06 (s, 3H), 1.95 - 1.88 (m, 1H),1.63 (d, J = 11.4 Hz, 1H), 1.49 (d, J = 11.6 Hz, 1H). |
| ESI-MS(M+H)⁺= 724.4 (Compound 134) | ESI-MS(M+H)⁺= 725.4 **(Compound 135)** |
| | ¹H NMR (400 MHz, Chloroform-*d*) δ 9.81 (s, 1H), 8.75 (s, 1H), 8.26 (dd, *J =* 8.3, 1.3 Hz, 2H), 8.07 (s, 1H), 7.86 (s, 1H), 7.70 (d, *J* = 1.5 Hz, 1H), 7.66 - 7.60 (m, 1H), 7.52 (dd, *J =* 8.3, 1.3 Hz, 2H), 7.30 (dd, *J =* 7.8, 1.2 Hz, 1H), 7.05 - 6.95 (m, 1H), 6.26 (s, 2H), 5.84 (s, 1H), 4.01 (t, *J=* 7.4 Hz, 2H), 3.94 (d, *J=* 1.3 Hz, 3H), 3.76 (dd, *J=* 13.0, 6.8 Hz, 1H), 3.70 (d, *J =* 1.2 Hz, 3H), 3.61 (dd, *J =* 12.7, 6.3 Hz, 1H), 3.51 (t, *J =* 6.5 Hz, 2H), 3.13 (s, 3H), 3.11 - 3.02 (m, 6H), 2.84 - 2.78 (m, 5H), 2.65 (s, 3H). |
| ESI-MS(M+H)⁺= 837.5 (Compound 136) | ESI-MS(M+H)⁺= 718.3 (Compound 138) |
| ESI-MS(M+H)⁺= 738.4 **(Compound 137)** | ESI-MS(M+H)⁺= 752.4 **(Compound 139)** |
| ¹H NMR (400 MHz, DMSO-*d*₆) δ 12.10 (s, 1H), 10.21 *(d, J=* 7.4 Hz, 1H), 8.57 (dd, *J =* 6.5, 2.5 Hz, 1H), 8.44 (d, *J =* 2.1 Hz, 1H), 7.98 (s, 1H), 7.83 (d, *J =* 7.8 Hz, 2H), 7.45 (d, *J =* 7.7 Hz, 2H), 7.22 (dd, *J =* 20.4, 8.2 Hz, 3H), 6.97 (d, *J=* 8.3 Hz, 1H), 6.39 - 6.17 (m, 2H), 5.79 (s, 1H), 4.13 (q, *J* = 6.9 Hz, 2H), 3.91 (t, *J* = 7.5 Hz, 2H), 3.69 - 3.54 (m, 2H), 3.04 (s, 2H), 2.96 (s, 9H), 2.72 - 2.60 (m, 4H), 2.58 (t, *J* = 5.6 Hz, 2H), 2.06 (s, 3H), 1.32 (t, *J =* 7.0 Hz, 3H), 1.20 (d, *J =* 12.5 Hz, 2H). | ¹H NMR (400 MHz, Chloroform-d) δ 10.98 (s, 1H), 8.80 (s, 2H), 8.19 (d, *J* = 8.0 Hz, 3H), 8.03 (d, *J* = 8.0 Hz, 1H), 7.77 (s, 1H), 7.47 (d, *J =* 7.9 Hz, 2H), 7.26 (d, *J =* 4.1 Hz, 1H), 6.98 (d, *J* = 9.0 Hz, 1H), 6.25 - 6.15 (m, 2H), 5.55 (s, 1H), 3.94 (t, *J =* 7.4 Hz, 2H), 3.73 (d, *J =* 6.6 Hz, 1H), 3.69 (d, *J =* 6.3 Hz, 2H), 3.59 (dt, *J =* 12.7, 6.3 Hz, 2H), 3.45 (s, 1H), 3.43 (d, *J =* 6.2 Hz, 1H), 3.14 (s, 2H), 3.11 (s, 2H), 3.05 (d, *J* = 6.4 Hz, 2H), 3.03 (s, 2H), 2.83 - 2.79 (m, 2H), 2.75 (s, 1H), 2.74 - 2.71 (m, 2H), 2.37 (s, 6H), 2.19 (s, 4H), 1.34 (t, *J =* 7.3 Hz, 1H), 1.23 (q, *J =* 7.8, 7.0 Hz, 1H). |
| ESI-MS(M+H)⁺= 741.4 **(Compound 140)** | ESI-MS(M+H)⁺= 742.3 **(Compound 142)** |
| ¹H NMR (400 MHz, DMSO-*d*₆) δ 12.55 (s, 1H), 10.25 (s, 1H), 8.99 - 8.85 (m, 2H), 8.34 - 8.28 (m, 1H), 7.82 | ¹H NMR (400 MHz, Chloroform-d) δ 10.24 (s, 1H), 8.71 (s, 1H), 8.59 (d, *J* = 2.0 Hz, 1H), 8.37 (d, *J* = 2.0 Hz, |
| (s, 2H), 7.45 - 7.37 (m, 2H), 7.01 (d, *J =* 8.2 Hz, 1H), 6.34 - 6.23 (m, 2H), 5.43 (s, 1H), 3.95 (t, *J* = 7.4 Hz, 2H), 3.68 - 3.60 (m, 1H), 3.46 (dt, *J =* 9.8, 6.2 Hz, 4H), 3.08 (s, 2H), 3.00 (s, 6H), 2.73 - 2.65 (m, 6H), 2.29 (s, 6H), 2.18 (s, 2H), 2.10 (s, 3H). | 1H), 7.73 - 7.66 (m, 2H), 7.52 (d, *J =* 1.8 Hz, 1H), 7.30 (s, 2H), 7.25 (s, 2H), 7.14 (d, *J =* 8.5 Hz, 1H), 6.50 (d, *J* = 2.5 Hz, 1H), 6.34 (dd, *J =* 8.6, 2.6 Hz, 1H), 5.48 (d, *J =* 3.8 Hz, 1H), 4.08 (t, *J* = 7.6 Hz, 2H), 3.83 - 3.67 (m, 2H), 3.63 (q, *J* = 6.6, 5.9 Hz, 2H), 3.13 (s, 4H), 3.11 - 3.03 (m, 4H), 2.98 - 2.90 (m, 1H), 2.78 (ddd, *J =* 16.7, 6.7, 5.4 Hz, 5H), 2.33 (s, 8H), 2.11 (s, 1H). |
| ESI-MS(M+H)⁺= 745.3 (Compound 141) | ESI-MS(M+H)⁺= 763.4 (Compound 143) |
| ESI-MS(M+H)⁺=762.4 (Compound 145) | ESI-MS(M+H)⁺= 748.4 (Compound 147) |
| ESI-MS(M+H)⁺= 741.4 **(Compound 146)** | ESI-MS(M+H)⁺=726.4. **(Compound 144)** |
| ¹H NMR (400 MHz, DMSO-*d*₆) δ 12.40 - 12.29 (m, 1H), 10.38 (s, 1H), 8.87 (s, 1H), 8.46 (d, *J* = 2.3 Hz, 1H), 8.26 (d, *J* = 1.7 Hz, 1H), 8.20 (dd, *J=* 7.8, 1.7 Hz, 1H), 7.85 (s, 2H), 7.23 (d, *J =* 8.0 Hz, 1H), 7.15 (t, *J =* 8.7 Hz, 1H), 6.29 (dd, *J =* 12.5, 2.4 Hz, 1H), 6.22 (dd, *J* = 8.6, 2.5 Hz, 1H), 5.47 - 5.38 (m, 1H), 3.96 (t, *J =* 7.6 Hz, 2H), 3.60 (t, *J =* 6.7 Hz, 2H), 3.51 (dd, *J=* 7.5, 5.5 Hz, 2H), 3.11 - 3.06 (m, 2H), 3.02 (s, 3H), 3.00 - 2.93 (m, 1H), 2.82 (s, 3H), 2.68 (td, *J =* 8.4, 7.5, 4.1 Hz, 6H), 2.29 (s, 9H), 2.18 (d, *J =* 5.2 Hz, 2H). | (¹H NMR (400 MHz, DMSO-*d*₆) δ 12.06 (d, *J =* 2.8 Hz, 1H), 10.35 (s, 1H), 8.51 (d, *J* = 2.1 Hz, 1H), 8.40 (d, *J =* 2.1 Hz, 1H), 7.97 (d, *J* = 2.7 Hz, 1H), 7.87 - 7.77 (m, 2H), 7.45 (d, *J* = 8.1 Hz, 2H), 7.37 (s, 2H), 7.12 (t, *J* = 8.7 Hz, 1H), 6.27 (dd, *J =* 12.5, 2.5 Hz, 1H), 6.20 (dd, *J* = 8.7, 2.4 Hz, 1H), 5.44 - 5.37 (m, 1H), 3.94 (t, *J=* 7.6 Hz, 2H), 3.56 (t, *J* = 6.7 Hz, 2H), 3.49 (dd, *J* = 7.5, 5.5 Hz, 2H), 3.13 (s, 1H), 3.07 - 3.01 (m, 2H), 2.96 (s, 6H), 2.66 (dd, *J* = 13.8, 7.0 Hz, 6H), 2.23 (s, 6H), 2.14 (s, 2H).) |
| ESI-MS(M+H)⁺=729.3 (Compound 148) | ESI-MS(M+H)⁺=730.2 (Compound 149) |
| ESI-MS(M+H)⁺=690.3 (Compound 150) | ESI-MS(M+H)⁺=692.3 (Compound 151) |
| ESI-MS(M+H)⁺=705.3 (Compound 152) | ESI-MS(M+H)⁺=663.3 (Compound 153) |
| ESI-MS(M+H)⁺=663.3 (Compound 154) | ESI-MS(M+H)⁺=675.3 (Compound 155) |
| ESI-MS(M+H)⁺=660.3 (Compound 156) | ESI-MS(M+H)⁺=731.4 (Compound 157) |
| ESI-MS(M+H)⁺=656.3 (Compound 158) | ESI-MS(M+H)⁺=696.3 (Compound 159) |
| ESI-MS(M+H)⁺=686.3 (Compound 160) | ESI-MS(M+H)⁺=671.3 (Compound 161) |
| ESI-MS(M+H)⁺=728.3 (Compound 162) | ESI-MS(M+H)⁺=664.3 (Compound 163) |
| ESI-MS(M+H)⁺=686.3 (Compound 164) | ESI-MS(M+H)⁺=731.3 (Compound 165) |
| ESI-MS(M+H)⁺=721.3 (Compound 166) | ESI-MS(M+H)⁺=714.3 (Compound 167) |
| ESI-MS(M+H)⁺=710.3 (Compound 168) | ESI-MS(M+H)⁺=647.3 (Compound 169) |
| ESI-MS(M+H)⁺=686.3 (Compound 170) | ESI-MS(M+H)⁺=678.3 (Compound 171) |
| ESI-MS(M+H)⁺=720.3 (Compound 172) | |

### Embodiment 27: Synthesis of compound 006

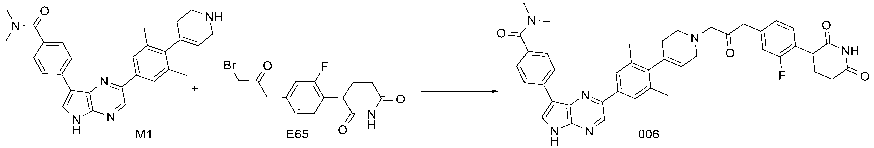

M1 (102 mg, 0.22 mmol) is dissolved in acetonitrile (5 mL) and stirred at room temperature until being dissolved completely, and E65 (66 mg, 0.19 mmol) and cesium carbonate (110 mg, 0.34 mmol) are added to the system in sequence, to perform reaction at 70 °C for 1 h. After the reaction is completed, extraction is performed with dichloromethane, the organic phases are combined, washing is performed with an aqueous solution of saturated ammonium chloride, and drying and concentration is performed under reduced pressure, to obtain 006 by column chromatography. ESI-MS(M+H)⁺=713.2.

### Embodiment 28: Synthesis of compound 009

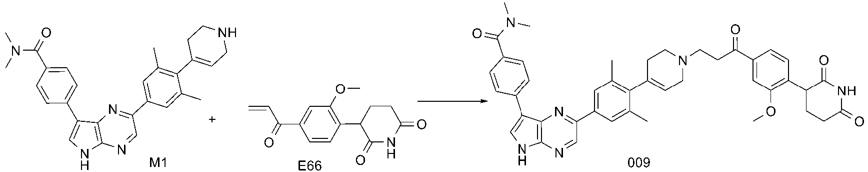

M1 (102 mg, 0.22 mmol) is dissolved in ethanol (5 mL) and stirred at room temperature until being dissolved completely, and E66 (58 mg, 0.21 mmol) is added to the system, to perform reaction at 70 °C for 1 h. After the reaction is completed, extraction is performed with dichloromethane, the organic phases are combined, washing is performed with an aqueous solution of saturated ammonium chloride, and drying and concentration is performed under reduced pressure, to obtain 009 by column chromatography. ESI-MS(M+H)+=725.3.

### Embodiment 29: Synthesis of compound 047

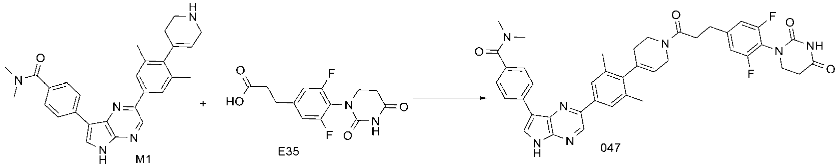

E35 (75 mg, 0.25 mmol) is dissolved in N,N-dimethylformamide (5 mL), and M1 (102 mg, 0.22 mmol), 2-(7-azobenzotriazole)-N,N,N',N'-tetramethylurea hexafluorophosphate (105 mg, 0.27 mmol) and diisopropylethylamine (81 mg, 0.63 mmol) are added to the system in sequence, to perform reaction at room temperature for 1 h. After the reaction is completed, water is added for quenching, and extraction is performed with dichloromethane; the organic phases are combined, washing is performed with an aqueous solution of saturated ammonium chloride, and drying and concentration is performed under reduced pressure, to obtain 047 by column chromatography. ESI-MS(M+H)⁺=732.3.

Following the synthesis route and method for 047, Compound 104 is synthesized, with ESI-MS(M+H)⁺=722.3.

### Biological Evaluation

### Test Example 1. Activity of HPK1 kinase

The Lantha screen assay is used to evaluate the inhibitory effect of the compounds on HPK1 kinase.
**a) Kinase buffer:** 50 mM HEPES (pH 7.5), 10 mM MgCl₂, 4 mM DTT, 0.01% Tween-20, 0.01% BSA.
**b) Compound Preparation:** The compound is diluted with DMSO, and the initial detection concentration of the compound is 1 µM and is diluted to the corresponding concentration.
**c) Kinase response and termination:** A kinase solution is prepared by adding kinase buffer, the kinase solution and the compound are added to the test plate to be incubated at room temperature for 10 minutes. Fluorescein-PKC and ATP are added to the kinase buffer to form a substrate solution, and 5 µl of the substrate solution is added to the test plate to start the reaction to be incubated at room temperature for 90 minutes; then, 10 µl of a mixture of antibody and EDTA is added to terminate the reaction to be incubated at room temperature for 60 minutes.
**d)** The date is read by Envision instrument to plot a curve with the Log concentration of the inhibitor as the X-axis and the inhibition rate as the Y-axis, and then the formula Y=Bottom + (Top-Bottom)/(1+(IC₅₀/X)^HillSlope) is used to obtain IC₅₀.

**Table 1 Inhibitory activity the compounds of the invention on HPK1 kinase**

| Compound No. | HPK1 IC₅₀ (nM) | Compound No. | HPK1 IC₅₀ (nM) | Compound No. | HPK1 IC₅₀ (nM) |
|---|---|---|---|---|---|
| 001 | + | 059 | + | 117 | + |
| 002 | + | 060 | + | 118 | + |
| 003 | + | 061 | + | 119 | + |
| 004 | + | 062 | + | 120 | + |
| 005 | + | 063 | + | 121 | + |
| 006 | + | 064 | + | 122 | + |
| 007 | + | 065 | + | 123 | + |
| 008 | + | 066 | + | 124 | + |
| 009 | + | 067 | + | 125 | + |
| 010 | + | 068 | + | 126 | + |
| 011 | + | 069 | + | 127 | + |
| 012 | + | 070 | + | 128 | + |
| 013 | + | 071 | + | 129 | + |
| 014 | + | 072 | + | 130 | + |
| 015 | + | 073 | + | 131 | + |
| 016 | + | 074 | + | 132 | + |
| 017 | + | 075 | + | 133 | + |
| 018 | + | 076 | + | 134 | + |
| 019 | + | 077 | + | 135 | + |
| 020 | + | 078 | + | 136 | + |
| 021 | + | 079 | + | 137 | + |
| 022 | + | 080 | + | 138 | + |
| 023 | + | 081 | + | 139 | + |
| 024 | + | 082 | + | 140 | + |
| 025 | + | 083 | + | 141 | + |
| 026 | + | 084 | + | 142 | + |
| 027 | + | 085 | + | 143 | + |
| 028 | + | 086 | + | 144 | + |
| 029 | + | 087 | + | 145 | + |
| 030 | + | 088 | + | 146 | + |
| 031 | + | 089 | + | 147 | + |
| 032 | + | 090 | + | 148 | + |
| 033 | + | 091 | + | 149 | + |
| 034 | + | 092 | + | 150 | + |
| 035 | + | 093 | + | 151 | + |
| 036 | + | 094 | + | 152 | + |
| 037 | + | 095 | + | 153 | + |
| 038 | + | 096 | + | 154 | + |
| 039 | + | 097 | + | 155 | + |
| 040 | + | 098 | + | 156 | + |
| 041 | + | 099 | + | 157 | + |
| 042 | + | 100 | + | 158 | + |
| 043 | + | 101 | + | 159 | + |
| 044 | + | 102 | + | 160 | + |
| 045 | + | 103 | + | 161 | + |
| 046 | + | 104 | + | 162 | + |
| 047 | + | 105 | + | 163 | + |
| 048 | + | 106 | + | 164 | + |
| 049 | + | 107 | + | 165 | + |
| 050 | + | 108 | + | 166 | + |
| 051 | + | 109 | + | 167 | + |
| 052 | + | 110 | + | 168 | + |
| 053 | + | 111 | + | 169 | + |
| 054 | + | 112 | + | 170 | + |
| 055 | + | 113 | + | 171 | + |
| 056 | + | 114 | + | 172 | + |
| 057 | + | 115 | + | | |
| 058 | + | 116 | + | | |

| | | | | | |
|---|---|---|---|---|---|
| +: IC₅₀ < 10 nM; ++: 10 nM ≤IC₅₀ < 50 nM; +++: 50 nM ≤IC₅₀ < 100 nM. | | | | | |

As shown in Table 1, the compounds of the invention have good inhibitory activity against HPK1 kinase.

### Test Example 2. Activity of Degrading HPK1

### Steps:

1) Cell plating: Cells are resuscitated, Jurkat cell lines in good growth condition are selected, and cells in the logarithmic growth phase are collected and counted; cell suspensions are seeded into 12 or 24-well plates and incubated overnight at 37°C in a 5% CO₂ incubator.
2) Preparation of compounds to be tested: The compounds to be tested are serially diluted with DMSO according to the experimental requirements and added to the corresponding cell wells, to be incubated at 37°C in a 5% CO₂ incubator for 24 h.
3) Preparation of samples: After the reaction of the compound to be tested is completed, the cells are collected in 1.5 mL EP tubes, and centrifuged at 1500 rpm for 5 min; after the supernatant is discarded, the cells are washed once with PBS, and the BCA protein is quantified and the concentration is adjusted. The adjusted samples are added to 5*loading buffer, boiled at 100°C for 10 minutes, and then the samples are loaded after the temperature is returned to room temperature.
4) Detection of samples: Electrophoresis is performed using 10% SDS-PAGE with a sample loading volume of 8 µL/well. After the membrane transfer is completed, the samples are blocked with 5% BSA at room temperature for 1 h. After the residual blocking solution is washed away with TBST, HPK1 and GAPDH antibodies are added for incubation overnight at 4°C. Washing is performed 3 more times with TBST on a shaker, 10 minutes each time. After washing is completed, the secondary antibody is added for incubation at room temperature for 1 hour; then, washing is performed 3 more times with TBST on a shaker, 10 minutes each time; finally, ECL exposure solution is used to develop and image the bands to detect changes in HPK1 protein.

**Table 2 Ability of the compounds of the invention in degrading HPK1 protein**

| Compound No. | DC₅₀ (nM) | Compound No. | DC₅₀ (nM) | Compound No. | DC₅₀ (nM) |
|---|---|---|---|---|---|
| 003 | + | 111 | + | 154 | + |
| 008 | + | 112 | 17.28 | 155 | + |
| 013 | + | 113 | + | 156 | + |
| 014 | + | 117 | + | 157 | + |
| 015 | + | 119 | + | 158 | + |
| 016 | + | 129 | + | 159 | + |
| 018 | + | 132 | + | 160 | + |
| 024 | + | 135 | + | 161 | + |
| 028 | + | 139 | + | 162 | + |
| 049 | + | 140 | + | 163 | + |
| 052 | + | 141 | + | 164 | + |
| 079 | + | 142 | + | 165 | + |
| 081 | + | 144 | + | 166 | + |
| 085 | + | 148 | + | 167 | + |
| 099 | + | 149 | + | 168 | + |
| 105 | + | 150 | + | 169 | + |
| 108 | 22.28 | 151 | + | 170 | + |
| 109 | + | 152 | + | 171 | + |
| 110 | 12.40 | 153 | + | 172 | + |
| | | | | Compound a | 71.48 |

| | | | | | |
|---|---|---|---|---|---|
| +: DC₅₀ < 10 nM; ++: 10 nM ≤DC₅₀ < 50 nM; +++: 50 nM ≤DC₅₀ < 100 nM. | | | | | |

Compound a is C196 of WO2024/125631A1.

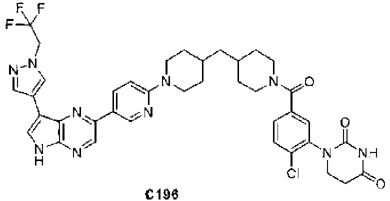

As shown in Table 2, the compounds of the invention have a strong ability to degrade HPK1 protein.

### Test Example 3. Selectivity of Degrading HPK1/GLK

### Steps:

1) Cell plating: Cells are resuscitated, Jurkat cell lines in good growth condition are selected, and cells in the logarithmic growth phase are collected and counted; cell suspensions are seeded into 12 or 24-well plates (20W/well) and incubated overnight at 37°C in a 5% CO₂ incubator.
2) Preparation of compounds to be tested: The compounds to be tested are serially diluted with DMSO according to the experimental requirements and added to the corresponding cell wells, to be incubated at 37°C in a 5% CO₂ incubator for 24 h.
3) Preparation of samples: After the reaction of the compound to be tested is completed, the cells are collected in 1.5 mL EP tubes, and centrifuged at 1500 rpm for 5 min; after the supernatant is discarded, the cells are washed once with PBS, and the BCA protein is quantified and the concentration is adjusted. The adjusted samples are added to 5*loading buffer, boiled at 100°C for 10 minutes, and then the samples are loaded after the temperature is returned to room temperature.
4) Detection of samples: Electrophoresis is performed using 10% SDS-PAGE with a sample loading volume of 10 µL/well. After the membrane transfer is completed, the samples are blocked with 5% skim milk at room temperature for 1 h. After the residual blocking solution is washed away with TBST, HPK1 and GLK antibodies are added for incubation overnight at 4°C. Washing is performed 3 more times with TBST on a shaker, 5 minutes each time. After washing is completed, the secondary antibody is added for incubation at room temperature in a shaker for 1 hour; then, washing is performed 3 more times with TBST on a shaker, 5 minutes each time; finally, the membrane is soaked in TBST and then subjected to chemiluminescence development.
5) Data Processing: After the PVDF membrane is developed, the data are exported and plotted using Imang J, and the gray values of the protein bands are analyzed using Image J software; the DC₅₀ curve is fitted using Graphpad Prism 9 software to obtain the results of the selectivity of the compound in degrading HPK1/GLK: HPK1/GLK Fold = DC₅₀ (HPK1) / DC₅₀ (GLK) .

**Table 3 Selectivity of the compounds of the invention in degrading HPK1/GLK**

| Compound No. | HPK1/GLK Fold | Compound No. | HPK1/GLK Fold | Compound No. | HPK1/GLK Fold |
|---|---|---|---|---|---|
| 011 | ++++ | 079 | ++++ | 130 | ++++ |
| 012 | ++++ | 081 | ++++ | 135 | ++++ |
| 014 | ++++ | 085 | ++++ | 144 | ++++ |
| 016 | ++++ | 086 | ++++ | 146 | ++++ |
| 018 | ++++ | 089 | ++++ | 150 | ++++ |
| 024 | ++++ | 099 | ++++ | 160 | ++++ |
| 042 | ++++ | 105 | ++++ | 162 | ++++ |
| 049 | ++++ | 107 | ++++ | 163 | ++++ |
| 052 | ++++ | 109 | ++++ | 165 | ++++ |
| 057 | ++++ | 112 | +++ | 167 | ++++ |
| 061 | ++++ | 113 | ++++ | 170 | ++++ |
| 062 | ++++ | 129 | ++++ | 172 | ++++ |

| | | | | | |
|---|---|---|---|---|---|
| +: Fold < 10; ++: 10≤Fold<100 ; +++: 100≤Fold<1000; ++++: 1000≤Fold | | | | | |

As shown in Table 3, the compounds of the invention show high selectivity in degrading HPK1 in an assay for the selectivity in degrading HPK1 and GLK protein.

### Test Example 4. Pharmacokinetic Properties

The test compound is administered to SD rats by gavage at a dose of 10 mg/kg. Blood samples are collected at 0 h (before administration) and at 0.083, 0.25, 1, 2, 4, 6, 8, and 24 h after oral gavage administration. The samples are placed in anticoagulant tubes containing heparin sodium, and the mixture is thoroughly vortexed and centrifuged at 6000 rpm for 3 minutes. Plasma concentrations are determined by LC-MS/MS, and relevant pharmacokinetic parameters are calculated using Phoenix WinNonlin 8.2.0 pharmacokinetic software.

**Table 4 Pharmacokinetic properties of the compounds of the invention**

| Compound No. | AUC (ng/ml*h) | Compound No. | AUC (ng/ml*h) | Compound No. | AUC (ng/ml*h) |
|---|---|---|---|---|---|
| 011 | 8987 | 057 | 11322 | 144 | 5752 |
| 014 | 11359 | 062 | 9925 | 150 | 13101 |
| 016 | 5002 | 085 | 8531 | 170 | 12703 |
| 024 | 12000 | 086 | 8503 | Compound a | 3852 |
| 049 | 9568 | 110 | 4069 | | |
| 052 | 6298 | 135 | 5485 | | |

As shown in Table 4, the compound of the invention has good exposure levels when administered orally.

## Claims

1. A compound of general formula (I) or stereoisomers, tautomers or pharmaceutically acceptable salts thereof, 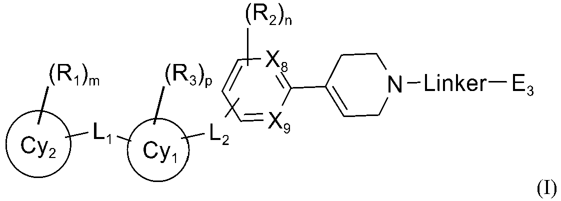 wherein
Cy₁ is selected from 5-12 membered heteroaryl or 8-10 membered fused bicyclic;
Cy₂ is selected from C₃-C₁₂ cycloalkyl, 3-12 membered heterocyclic, C₆-C₁₀ aryl, 5-12 membered heteroaryl or 8-10 fused bicyclic;
X₈ and X₉ are each independently selected from CRₐ or N;
L₁ and L₂ are each independently selected from chemical bonds, C₁-C₈ alkylene, C₃-C₁₂ cycloalkylene, 3-12 membered heterocyclic, -O-C₁-C₈ alkylene, -C₁-C₈ alkylene-O-, -NR_{b}-C₁-C₈ alkylene-, -C₁-C₈ alkylene-NR_{b}-, -O-, -NR_{b}C(O)-, -C(O)-NR_{b}-, -CO-, -SO-, -SO₂-, C₂-C₈ alkenyl or C₂-C₈ ynylene;
R₁ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, -POR_{b}R_{c} or -Linker-E3; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, C₁-C₈ haloalkyl, -C₁-C₈ alkyl-CONR_{b}R_{c}, -C₁-C₈ alkyl-NR_{b}COR_{c}, -C₁-C₈ alkyl-OR_{b}, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c;}
or two R₁ and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic, C₁-C₈ alkoxy, C₁-C₈ alkylamino, amino, hydroxyl, oxo, nitro, carboxyl or cyano;
R₂ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
or two R₂ and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
R₃ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, or -POR_{b}R_{c}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
Linker is selected from -(CH₂)ᵣ-; one or more CH₂ be optionally substituted by one or more selected from -COO-, -CONR_{b}-, -OCONR_{b}-, -NR_{b}CONR_{b}-, -O-, -NR_{b}-, -S-, -CO-, -CR_{b}=CR_{b}-, -C≡C-, -CR_{b}CR_{c}-, -CR_{b}=N-, -SO-, - SO₂-, -POR_{b}-, C₃-C₁₀ cycloalkylene, 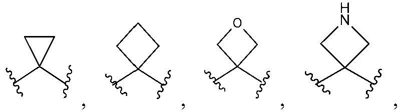 3-10 membered heterocycloalkylene, phenylene, 5-6 membered heteroarylene or -CR_{b}R_{c}-; the heterocycloalkylene group be further substituted by halogen or C₁-C₃ alkyl;
E₃ is selected from ligands for E₃ ligase;
Rₐ is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂R_{b}, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, or -POR_{b}R_{c}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, or -POR_{b}R_{c};
R_{b}, R_{c} and R_{d} are each independently selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, C₁-C₄ haloalkyl, -C₁-C₄ alkyl-CONRₑR_{f}, -C₁-C₃ alkyl-ORₑ, and -ORₑ or -NRₑR_{f}; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, C₁-C₈ haloalkyl, -C₁-C₈ alkyl-CONRₑR_{f}, -C₁-C₈ alkyl-ORₑ, oxo, -CN, -NO₂, -ORₑ, -SO₂Rₑ, -SO₂NRₑR_{f}, -CORₑ, -COORₑ, -CONRₑR_{f}, -C(=NRₑ)NR_{f}R_{g}, -NRₑR_{f}, -NRₑCOR_{f}, -NRₑCONR_{f}R_{g}, -NRₑCO₂R_{f}, -NRₑSONR_{f}R_{g}, -NRₑSO₂NR_{f}R_{g}, -NRₑSO₂R_{f}, -SO(=NRₑ)R_{f} or -PORₑR_{f};
R_{c} and R_{b}, or R_{d} and R_{c}, substituted on the same atom, be linked together to form a ring; the ring be further substituted by one or more substituents; the substituents are selected from halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, --CN, -NO₂, -ORₑ, -SO₂Rₑ, -SO₂NRₑR_{f}, -CORₑ, -COORₑ, -CONRₑR_{f}, -C(=NRₑ)NR_{f}R_{g}, -NRₑR_{f}, -NRₑCOR_{f}, -NRₑCONR_{f}R_{g}, -NRₑCO₂R_{f}, -NRₑSONR_{f}R_{g}, -NRₑSO₂NR_{f}R_{g}, -NRₑSO₂R_{f}, -SO(=NRₑ)R_{f} or -PORₑR_{f};
Rₑ, R_{f} and R_{g} are each independently selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, or 5-12 membered heteroaryl; the alkyl, the alkenyl, the alkynyl, the cycloalkyl, the heterocyclic, the aryl, and the heteroaryl optionally be further substituted by one or more substituents; the substituents are selected from halogen, C₁-C₈ alkyl, C₃-C₁₂ cycloalkyl, 3-12 membered heterocyclic, C₆-C₁₀ aryl, 5-12 membered heteroaryl, cyano, hydroxyl, amino, or nitro;
m, n, and p are each independently selected from 0, 1, 2, 3, 4, 5, and 6;
r is selected from an integer from 0 to 20.

2. The compound or stereoisomers, tautomers or pharmaceutically acceptable salts thereof according to claim 1, wherein Cy₁ is selected from 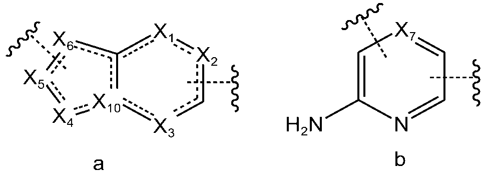 wherein
------- is a single bond or a double bond;
X₁, X₂, X₃, X₄, X₅, X₆ and X₁₀ are independently selected from O, S, C, CH, CH₂, N, NH, or CO, provided that the valence bond theory is satisfied;
X₇ is selected from CRₐ^{'} or N;
Rₐ^{'} is selected from H, halogen, C₁-C₈ alkyl, C₂-C₈ alkenyl, C₂-C₈ alkynyl, C₃-C₁₂ cycloalkyl, 3-12 membered heterocyclic, C₆-C₁₀ aryl, 5-12 membered heteroaryl, oxo, -CN, -NO₂, -OH, -NH₂, or -O- C₁-C₈ alkyl.

3. The compound or stereoisomers, tautomers or pharmaceutically acceptable salts thereof according to claim 2, a compound of general formula (IIa) or (IIb), or stereoisomers, tautomers, or pharmaceutically acceptable salts thereof:

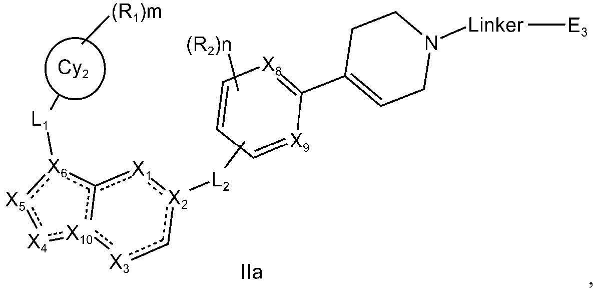

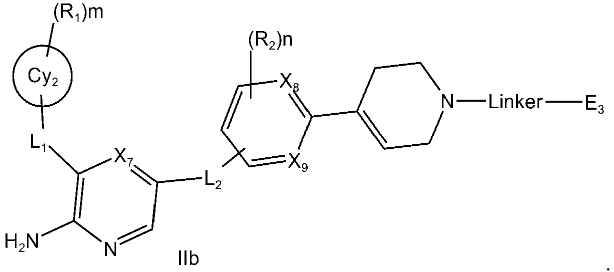

4. The compound or stereoisomers, tautomers or pharmaceutically acceptable salts thereof according to claim 3, a compound of general formula (IIIa) or (IIIb), or stereoisomers, tautomers, or pharmaceutically acceptable salts thereof: 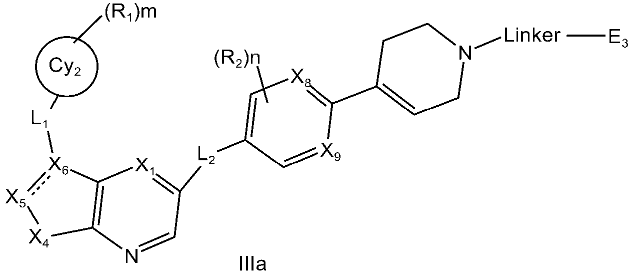 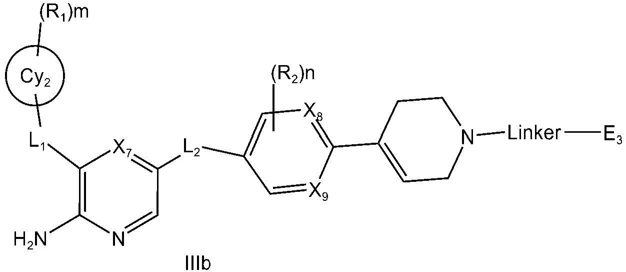 wherein
X₁ is selected from CH or N;
X₄ is selected from NH or S;
X₈ and X₉ are independently selected from CH, N or C-Me;
when -------- is selected from the double bond, X₆ is C, and X₅ be CH or N; when -------- is selected from the single bond, X₆ is N or CH, and X₅ is CO or CH2;
L₁ and L₂ are each independently selected from chemical bonds, -O-, -O-C₁-C₃ alkylene, or -C₁-C₃ alkylene-O-; Cy₂ is selected from 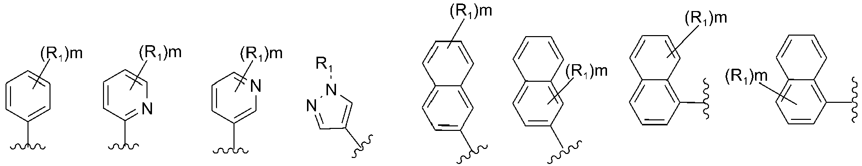 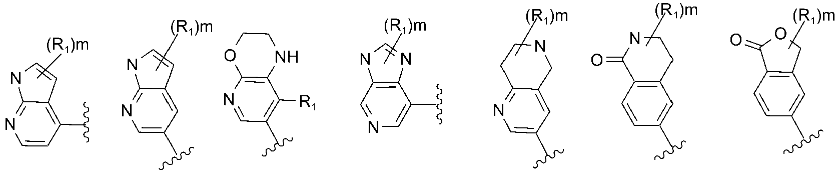
R₂ is selected from H, halogen, C₁-C₈ alkyl, C₂-C₈ alkenyl, C₂-C₈ alkynyl, C₃-C₁₂ cycloalkyl, 3-12 membered heterocyclic, -CN, -NO₂, -OR_{b}, -SO₂Rₐ, -SO₂NR_{b}R_{c}, -COR_{b}, -COOR_{b}, -CONR_{b}R_{c}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, or -POR_{b}R_{c}; the alkyl, the alkenyl, the alkynyl, the cycloalkyl, and the heterocyclic be further substituted by one or more substituents; the substituents are selected from halogen, oxo, -CN, -OR_{b} or -NR_{b}R_{c};
m is selected from 0, 1, 2, 3, 4, and 5;
n is selected from 0, 1, 2, and 3.

5. The compound or stereoisomers, tautomers or pharmaceutically acceptable salts thereof according to claim 4, a compound of general formula (IV), or stereoisomers, tautomers, or pharmaceutically acceptable salts thereof: 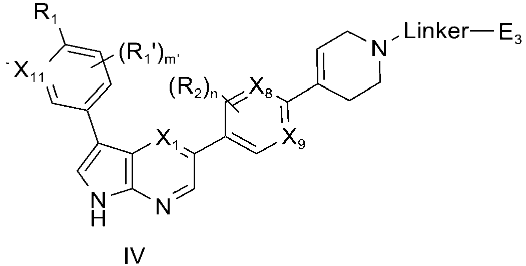 wherein
X₁ is selected from N or CH;
X₁₁ is selected from N or CH;
R₁ is selected from H, halogen, C₁-C₄ alkyl, C₃-C₈ cycloalkyl, 3-10 membered heterocyclic, C₆-C₁₀ aryl, 5-10 membered heteroaryl, -OR_{b}, -COR_{b}, -CONR_{b}R_{c}, -NR_{b}R_{c}, -NR_{b}COR_{c} or SO(=NR_{b})R_{c}, SO₂NR_{b}R_{c}, cyano or oxo; the alkyl, the cycloalkyl, the heterocyclic, the aryl, and the heteroaryl be further substituted by halogen, C₁-C₃ alkyl, C₃-C₅ cycloalkyl, C₁-C₃ haloalkyl, -C₁-C₃ alkyl-NR_{b}COR_{c}, -C₁-C₃ alkyl-CONR_{b}R_{c}, -C₁-C₃ alkyl-OR_{b}, hydroxyl, cyano, amino or nitro;
R₁' is selected from H, halogen, C₁-C₄ alkyl, hydroxyl, cyano, amino or nitro;
or R₁ and R₁', and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
or R₁ and R₁', and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
R_{b} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, C₁-C₃ alkoxy, hydroxyl, cyano, amino, or nitro;
R_{c} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, C₁-C₃ alkoxy, hydroxyl, cyano, amino, or nitro;
R₂ is selected from H or 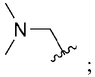
n or m' is selected from 0, 1, 2, or 3.

6. The compound or stereoisomers, tautomers or pharmaceutically acceptable salts thereof according to claim 3, a compound of general formula (IV), or stereoisomers, tautomers, or pharmaceutically acceptable salts thereof: 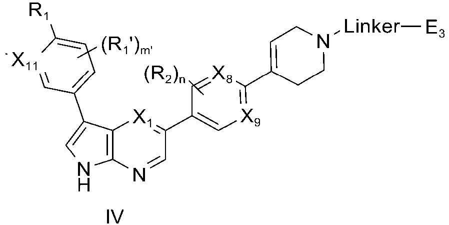
R₁ is selected from H, C₁-C₃ alkyl, halogen, C₁-C₃ haloalkyl, cyano, oxo, hydroxymethyl, hydroxyethyl, 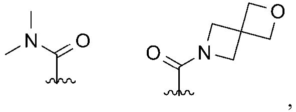 cyclopropyl, cyclobutyl, cyclopentyl, 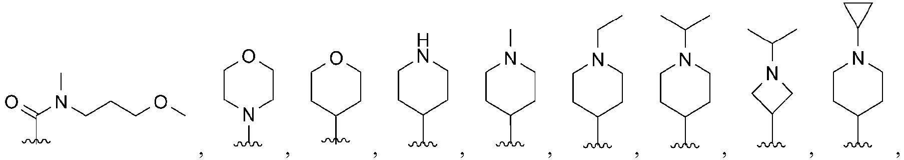 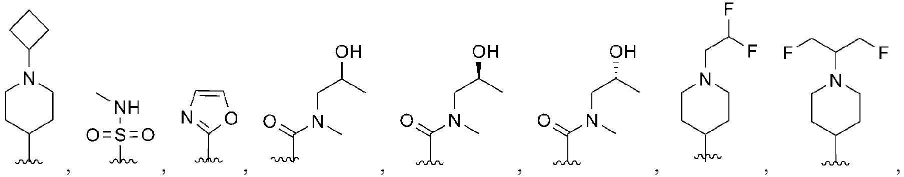 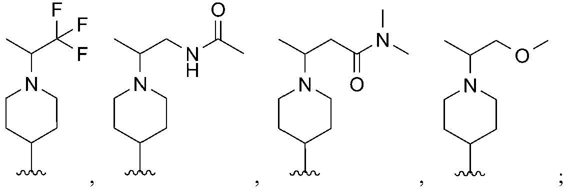
R₁' is selected from H, Cl, F, methyl, ethyl or cyano;
X₈ and X₉ are each independently selected from CRₐ or N;
Rₐ is selected from H, methyl, ethyl, isopropyl, halogen, methylamino, ethylamino, methoxy, ethoxy, 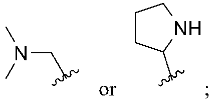
n or m' is selected from 0, 1, 2, or 3.

7. The compound or stereoisomers, tautomers or pharmaceutically acceptable salts thereof according to claim 1,
wherein 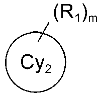 is selected from 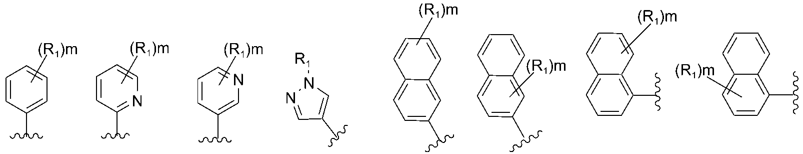 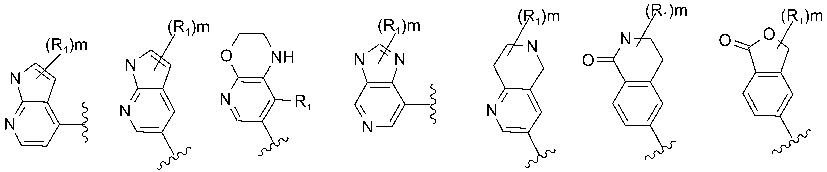 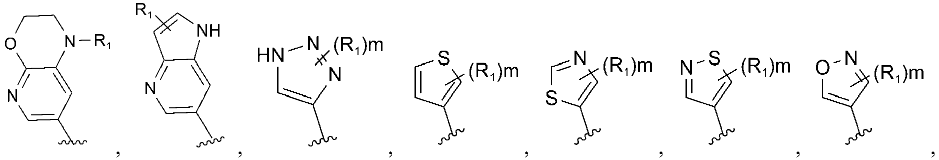 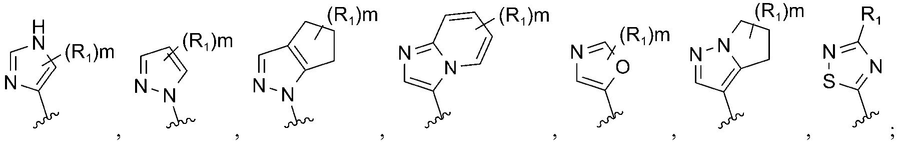
R₁ is selected from H, halogen, C₁-C₃ alkyl, C₃-C₆ cycloalkyl, 4-6 membered heterocyclic, C₆-C₁₀ aryl, 5-6 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c}, -POR_{b}R_{c} or -Linker-E3; the alkyl, the cycloalkyl, the heterocyclic, the aryl, and the heteroaryl optionally be further substituted by one or more substituents; the substituents are selected from halogen, C₁-C₃ alkyl, C₃-C₅ cycloalkyl, 3-6 membered heterocyclic, C₆-C₁₀ aryl, 5-7 membered heteroaryl, C₁-C₃ haloalkyl, -C₁-C₃ alkyl-CONR_{b}R_{c}, -C₁-C₃ alkyl-NR_{b}COR_{c}, -C₁-C₃ alkyl-OR_{b}, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR₃CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c};
or two R₁ and one or more atoms attached thereto (provided valence bond theory is satisfied) together form a 3-8 membered ring; the 3-8 membered ring contains 0, 1, or 2 heteroatoms selected from N, O, S, and P; the 3-8 membered ring optionally be further substituted by one or more substituents selected from halogen, C₁-C₈ alkyl, C₁-C₈ haloalkyl, C₃-C₁₂ cycloalkyl, 3-12 membered heterocyclic group, C₁-C₈ alkoxy, C₁-C₈ alkylamino, amino, hydroxyl, oxo, nitro, carboxyl or cyano;
m is selected from 0, 1, 2, 3, 4, and 5;
R_{b}, R_{c} and R_{d} are each independently selected from H, halogen, methyl, ethyl, propyl, isopropyl, C₃-C₆ cycloalkyl, 5-7 membered heterocyclic, C₆-C₁₀ aryl, 5-6 membered heteroaryl, C₁-C₄ haloalkyl, -C₁-C₄ alkyl-CON(CH₃)₂, -C₁-C₃ alkyl-OCH₃, -ORₑ or -NRₑR_{f}; the methyl, the ethyl, the propyl, the isopropyl, the cycloalkyl, the heterocyclic, the aryl, and the heteroaryl be optionally further substituted by one or more substituents; the substituents are selected from halogen, C₁-C₃ alkyl, C₃-C₆ cycloalkyl, 3-8 membered heterocyclic, C₆-C₁₀ aryl, 5-6 membered heteroaryl, C₁-C₄ haloalkyl, -C₁-C₄ alkyl-CON(CH₃)₂, -C₁-C₃ alkyl-OCH₃, oxo, -CN, -NO₂, -OH or -NH₂.

8. The compound or stereoisomers, tautomers or pharmaceutically acceptable salts thereof according to claim 7,
wherein 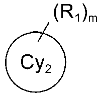 is selected from 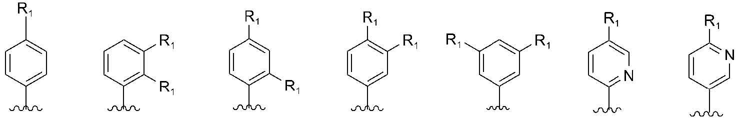 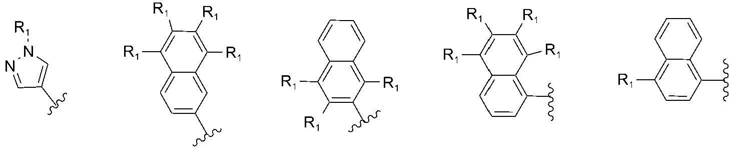 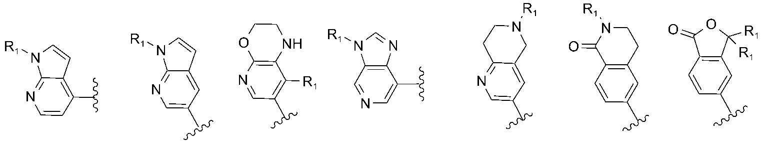 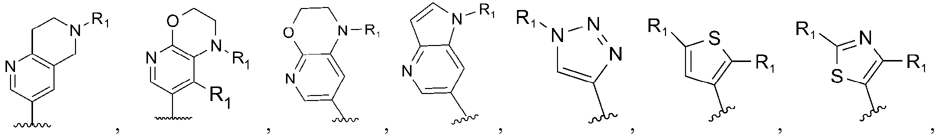 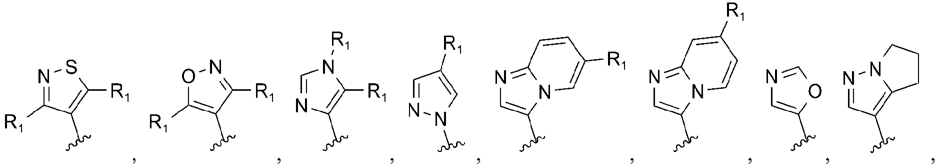 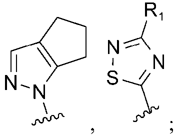 R₁ is selected from H, C₁-C₃ alkyl, halogen, C₁-C₃ haloalkyl, cyano, oxo, hydroxymethyl, hydroxyethyl, 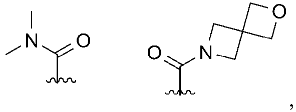 cyclopropyl, cyclobutyl, cyclopentyl,

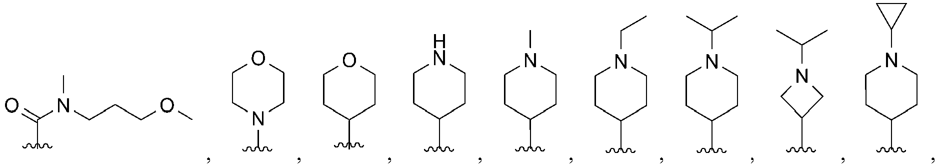

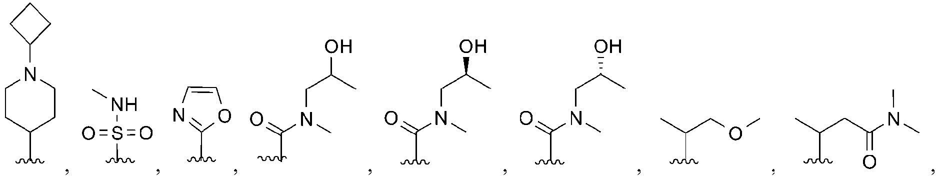

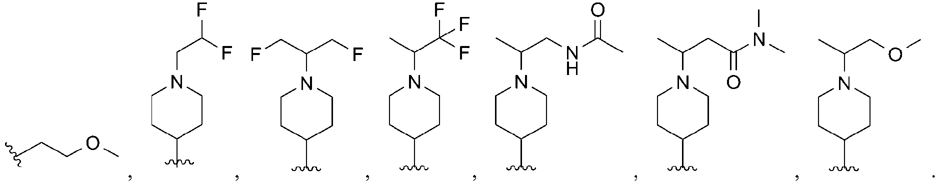

9. The compound or stereoisomers, tautomers or pharmaceutically acceptable salts thereof according to claim 8, 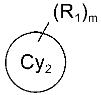 is selected from

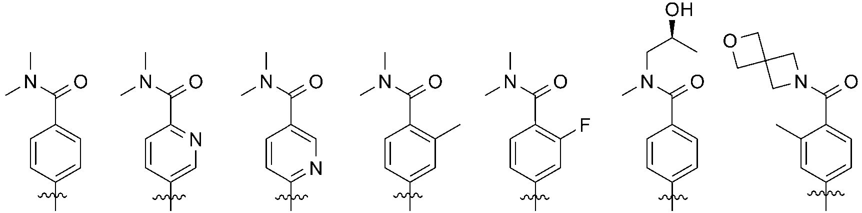

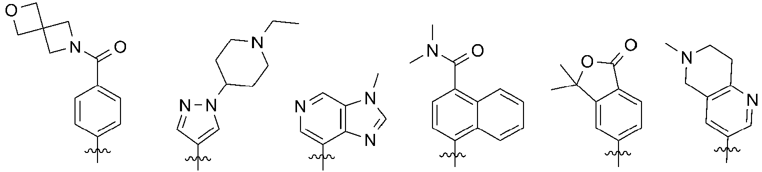

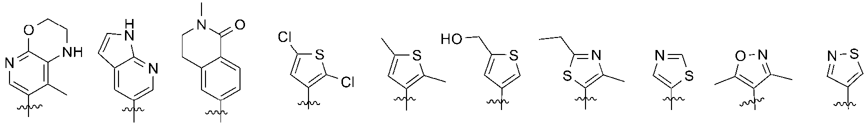

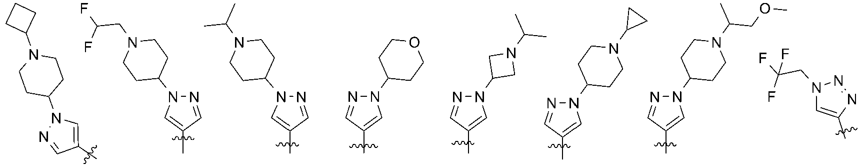

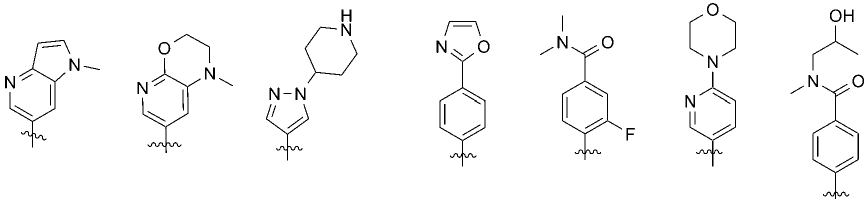

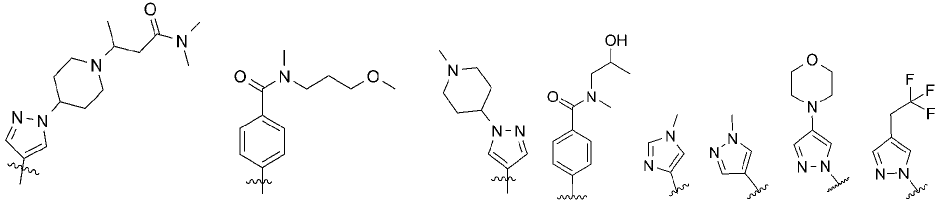

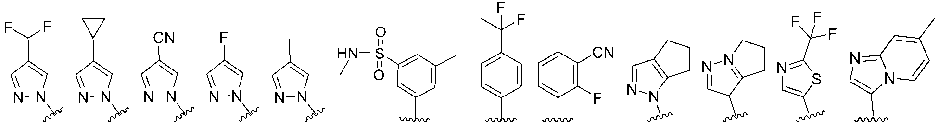

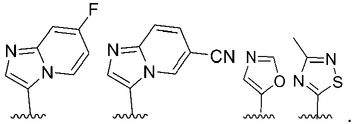

10. The compound or stereoisomers, tautomers or pharmaceutically acceptable salts thereof according to claim 1, a compound of general formula (VI), or stereoisomers, tautomers, or pharmaceutically acceptable salts thereof: 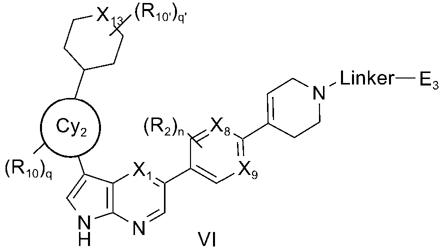
X₁₃ is selected from NH or O;
X₁ is selected from CH or N;
R₁₀ is selected from H, halogen, C₁-C₄ alkyl, C₁-C₄ haloalkyl, C₁-C₄ alkoxy, hydroxyl, cyano, amino or nitro;
R10' is selected from H, halogen, C₁-C₄ alkyl, C₃-C₅ cycloalkyl, 3-8 membered heterocyclic, C₁-C₄ haloalkyl, -C₁-C₄ alkyl-CONR_{b}R_{c}, -C₁-C₈ alkyl-OR_{b}, oxo or -CN; the alkyl, the cycloalkyl, or the heterocyclic optionally be further substituted by one or more substituents selected from halogen, C₁-C₈ alkyl, -CN, -CONR_{b}R_{c}, -NR_{b}COR_{c}, or --OR_{b}; preferably, R_{10'} is selected from H, halogen, cyano, oxo, methyl, ethyl, propyl, isopropyl, isobutyl, -CH₂F, -CHF₂, -CF₃, -CH₂CH₂F, -CH₂CHF₂, -CH₂CF₃, cyclopropyl, cyclobutyl, cyclopentyl, 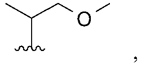 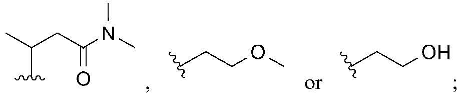
R₂ is selected from H, halogen, C₁-C₄ alkyl, C₃-C₆ cycloalkyl, 4-6 membered heterocyclic, -CN, -NO₂, -OR_{b} or -NR_{b}R_{c}, and the alkyl, the cycloalkyl, and the heterocyclic be further substituted by one or more substituents selected from halogen, oxo, -CN, -OR_{b}, and -NR_{b}R_{c};
R_{b} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by halogen, C₁-C₃ alkyl, hydroxyl, cyano, amino, or nitro;
R_{c} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by halogen, C₁-C₃ alkyl, hydroxyl, cyano, amino, or nitro;
q or q' is selected from 0, 1, 2, or 3;
Cy₂ is selected from a 5 membered heteroaromatic ring containing 1-3 heteroatoms; preferably, Cy₂ is selected from

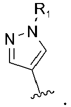

11. The compound or stereoisomers, tautomers or pharmaceutically acceptable salts thereof according to claim 1, a compound of general formula (VII), or stereoisomers, tautomers, or pharmaceutically acceptable salts thereof: 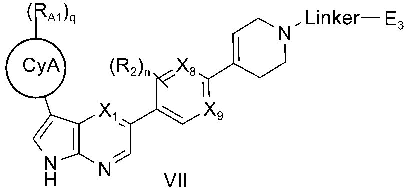 CyA is selected from phenyl, 5-10 membered heteroaryl or 8-10 membered fused bicyclic; the heteroaryl from which CyA is selected are preferably selected from 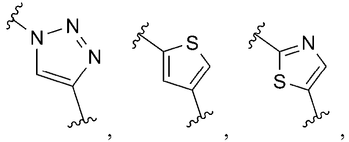 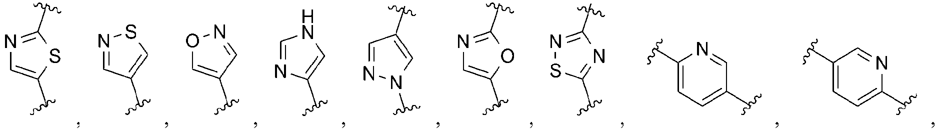 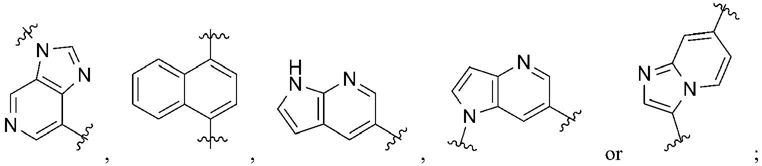 the fused bicyclic are selected from 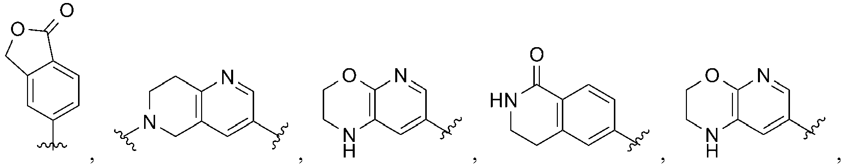 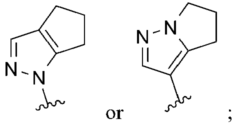
R_{A1} is selected from H, halogen, C₁-C₄ alkyl, C₃-C₈ cycloalkyl, 3-10 membered heterocyclic, C₆-C₁₀ aryl, 5-10 membered heteroaryl, oxo, -CN, -OR_{b} , -COR_{B}, -CONR_{B}R_{C}, -NR_{B}R_{C}, -NR_{B}COR_{C} or SO(=NR_{B})R_{C}; the alkyl, the cycloalkyl, the heterocyclic, the aryl, or the heteroaryl be further substituted by halogen, C₁-C₃ alkyl, hydroxyl, cyano, amino, or nitro;
R_{B} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, hydroxyl, cyano, amino, or nitro;
R_{C} is selected from H, C₁-C₃ alkyl, C₃-C₆ cycloalkyl, 5-7 membered heterocyclic, C₆-C₁₀ aryl or 5-10 membered heteroaryl; the alkyl, the cycloalkyl, the heterocyclic, the aryl, or the heteroaryl be further substituted by C₁-C₃ alkyl, hydroxyl, cyano, amino, or nitro;
q is selected from 0, 1, 2, or 3;
the definitions of X₁, X₈, X₉, R₂, n, Linker and E3 are as described in claim 1.

12. The compound or stereoisomers, tautomers or pharmaceutically acceptable salts thereof according to any one of claims 1 to 11,
E3 is selected from 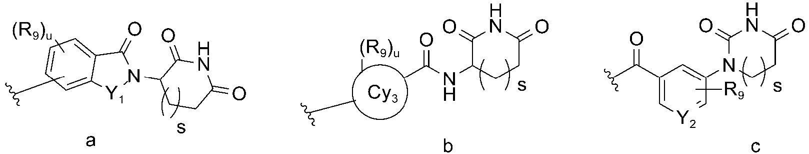 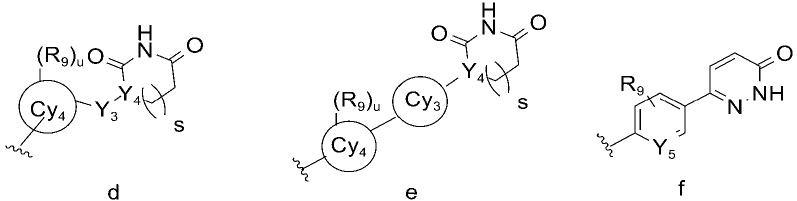 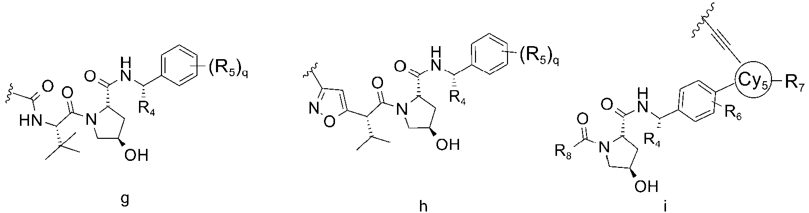 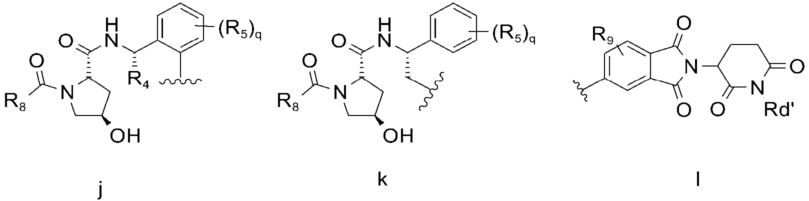
wherein
q, u, and s are each independently selected from 0, 1, and 2;
Y₁ is independently selected from CO, methylene, vinylene, or ethyl;
Y₂ is independently selected from CH or N;
Y₃ is independently selected from absent, CH₂, NH, NMe, or O;
Y₄ is independently selected from CH or N;
Y₅ is independently selected from CH or N;
R₄, R₅, R₆, R₇, R₈ and R₉ are each independently selected from H, halogen, C₁-C₈ alkyl, C₂-C₈ alkenyl, C₂-C₈ alkynyl, C₃-C₈ cycloalkyl, 5-12 membered heterocyclic, 6-10 membered aryl, 5-10 membered heteroaryl, oxo, -CN, -NO₂, -OR_{b}, -SO₂Rₐ, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR_{b}SONR_{c}R_{d}, -NR_{b}SO₂NR_{c}R_{d}, -NR_{b}SO₂R_{c}, -SO(=NR_{b})R_{c} or -POR_{b}R_{c}; the alkyl, the alkenyl, the alkynyl, the cycloalkyl, the heterocyclic, the aryl, and the heteroaryl be further substituted by one or more substituents; the substituents are selected from halogen, C₁-C₈ alkyl, C₂-C₈ alkenyl, C₂-C₈ alkynyl, C₃-C₈ cycloalkyl, 5-12 membered heterocyclic, 6-10 membered aryl, 6-10 membered heteroaryl, oxo, -CN, -NO₂, -OR_{b}, -SO₂R₃, -SO₂NR_{b}R_{c}, -COR_{b}, -COOR_{b}, -CONR_{b}R_{c}, -C(=NR_{b})NR_{c}R_{d}, -NR_{b}R_{c}, -NR_{b}COR_{c}, -NR_{b}CONR_{c}R_{d}, -NR_{b}CO₂R_{c}, -NR₃SONR_{c}R_{d}, -NR_{b}SO₂NR_{c}R_{d}, -NR_{b}SO₂R_{c}, -SO(=NR_{b})R_{c} or -POR_{b}R_{c};
Rd' is selected from H, -R_{f} OCOR_{g}, -R_{f}OCOOR_{g}, -R_{f}OCONR_{g}Rₕ, -COOR_{f} or -CONR_{f}R_{g};
R_{f}, R_{g} and Rₕ are each independently selected from H, C₁-C₈ alkyl, 3-8 membered cycloalkyl, 3-8 membered heterocyclic, C₁-C₆ alkyl 3-8 membered cycloalkyl or C₁-C₆ alkyl 3-8 membered heterocyclic;
Cy₃ is selected from 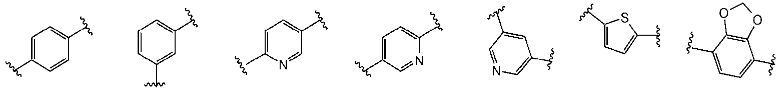 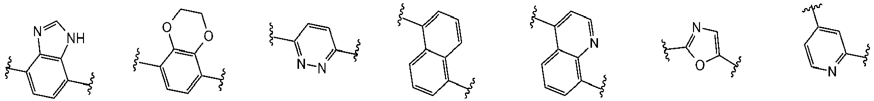 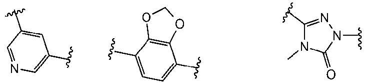
Cy₄ is selected from 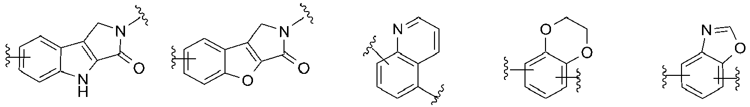 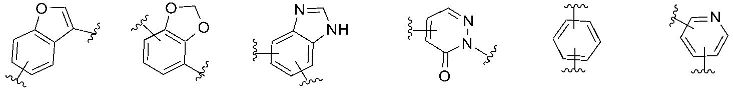 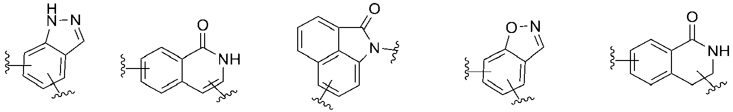 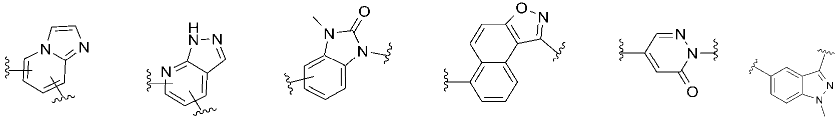 Cy₅ is selected from

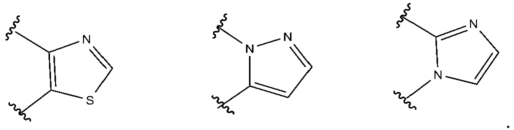

13. The compound or stereoisomers, tautomers or pharmaceutically acceptable salts thereof according to claim 1,
wherein Linker is selected from 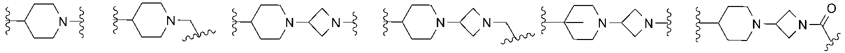 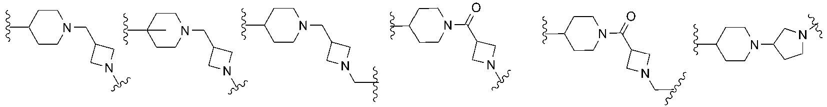 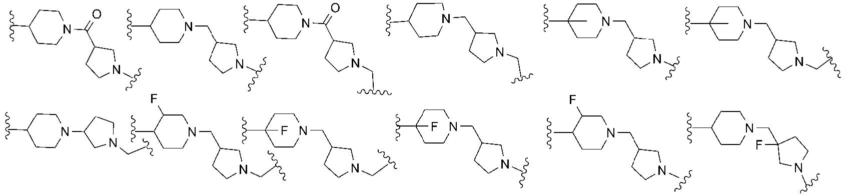 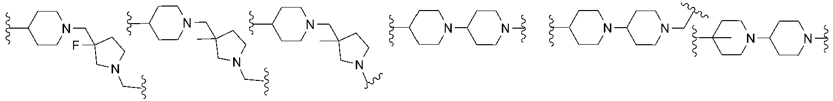 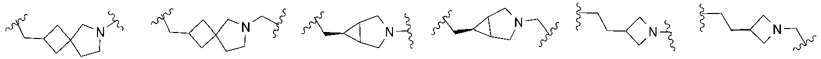 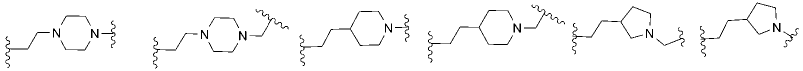 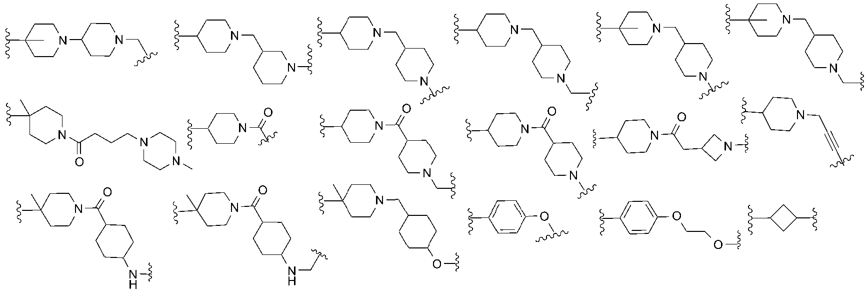 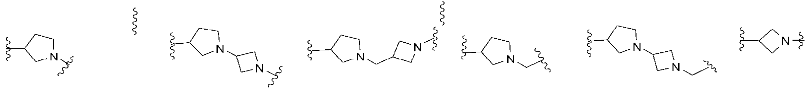 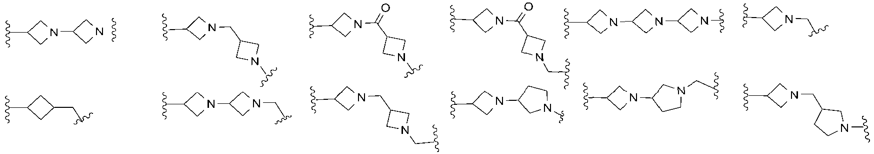 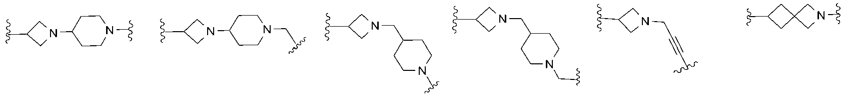 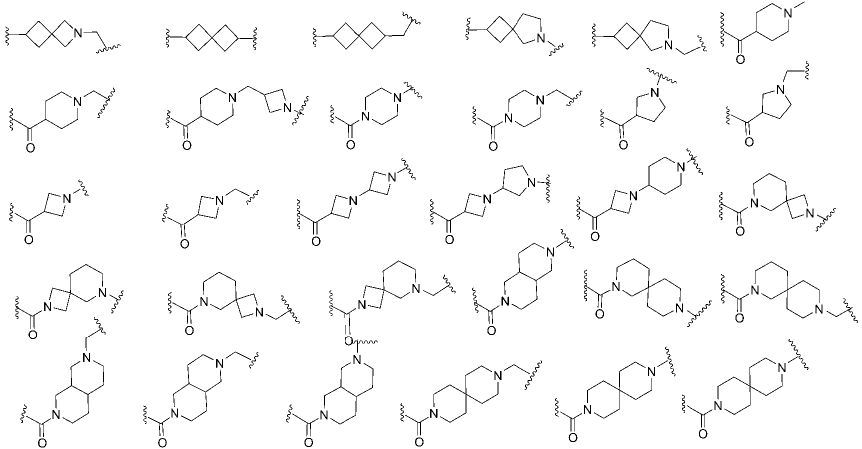 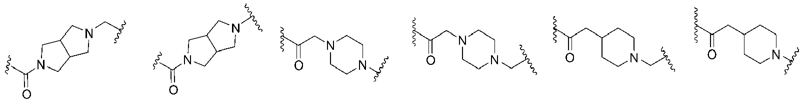 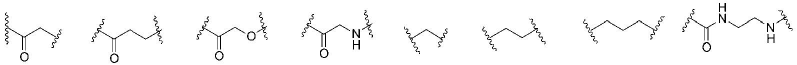 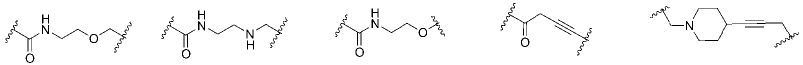 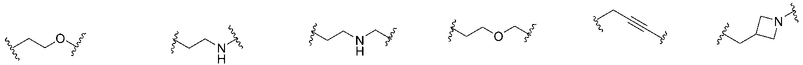 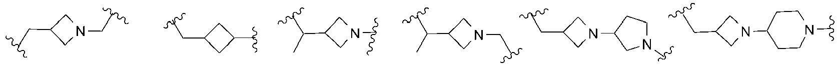 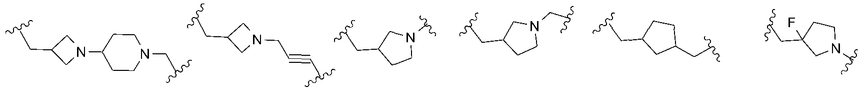 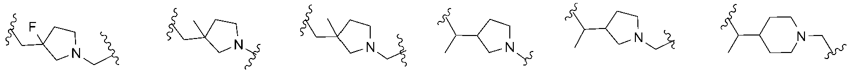 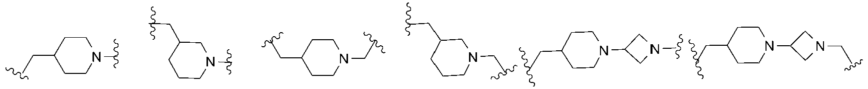 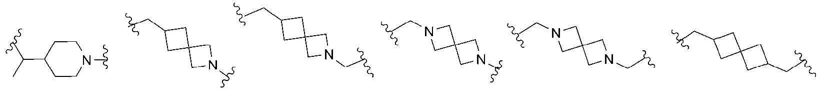 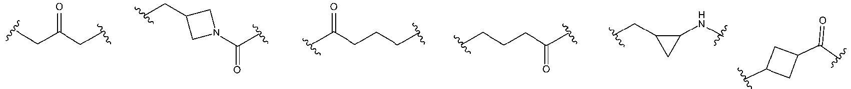 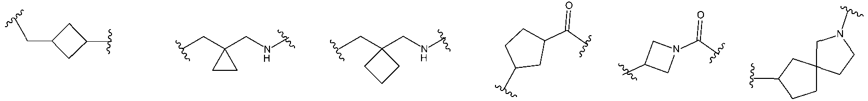 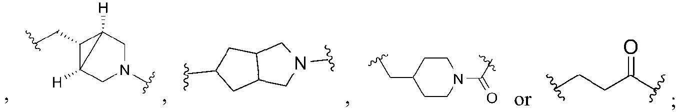 E3 is selected from

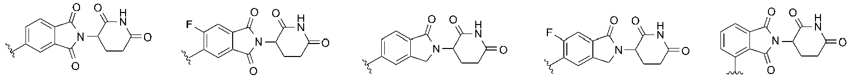

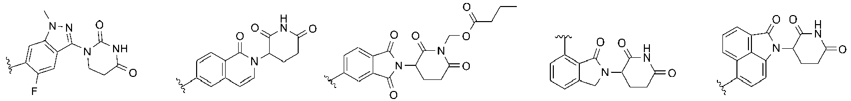

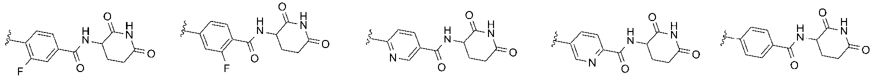

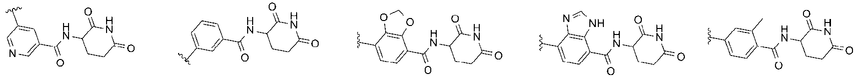

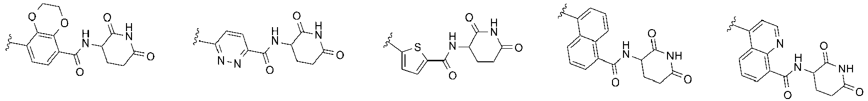

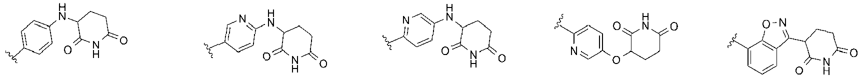

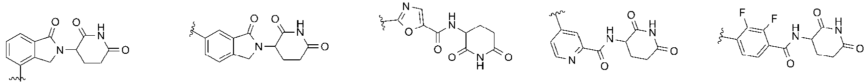

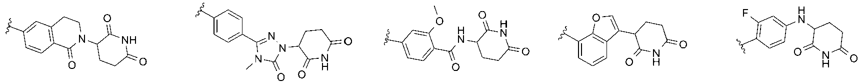

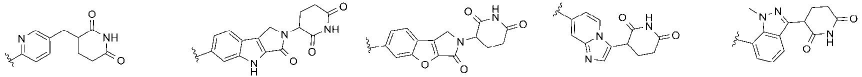

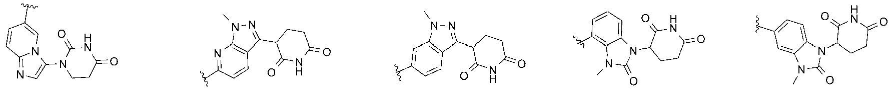

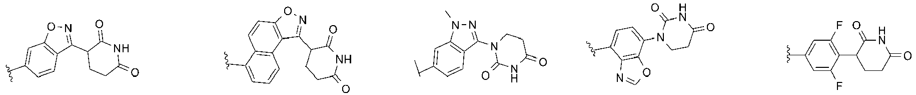

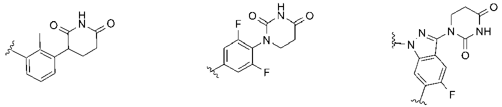

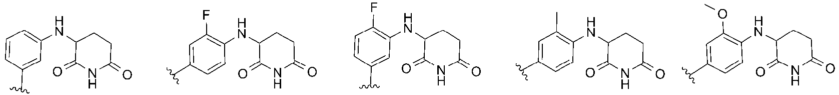

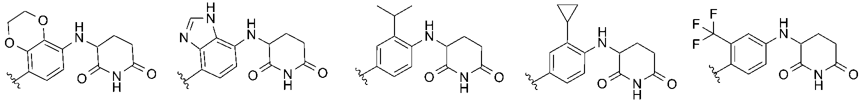

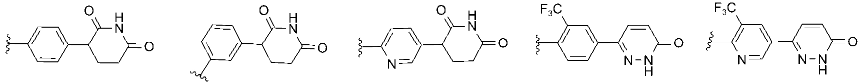

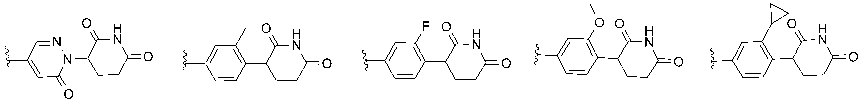

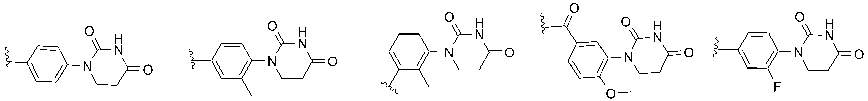

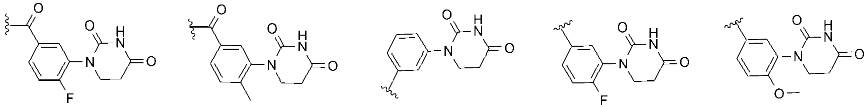

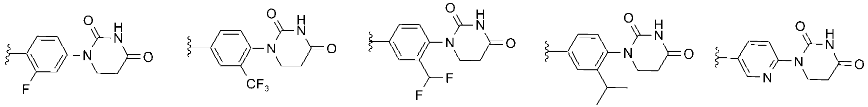

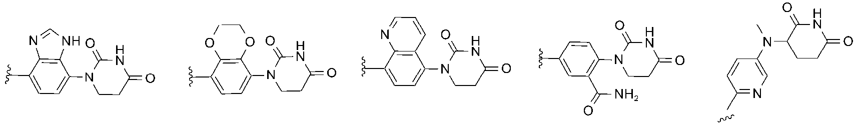

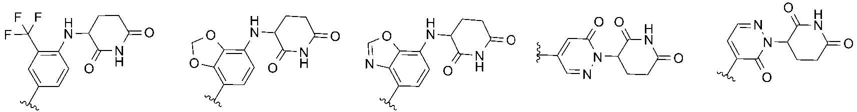

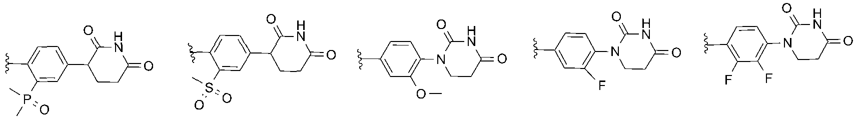

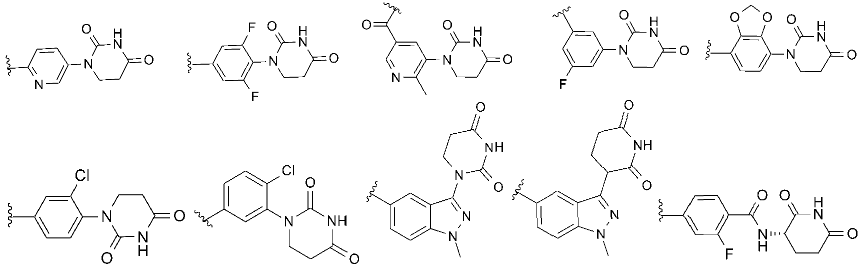

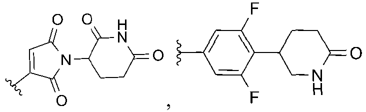

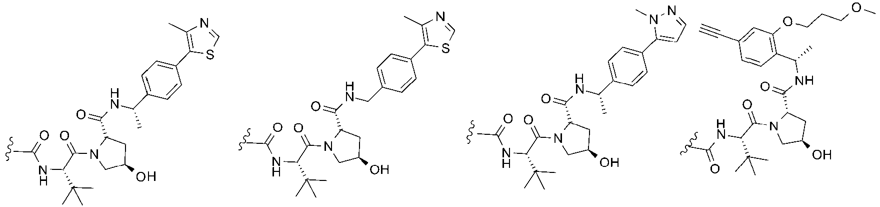

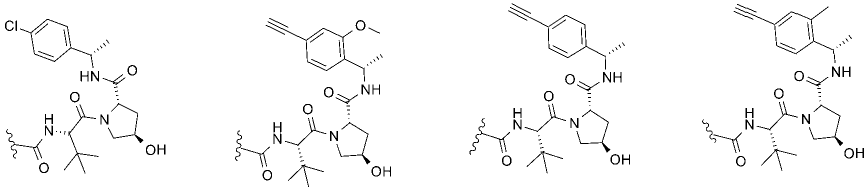

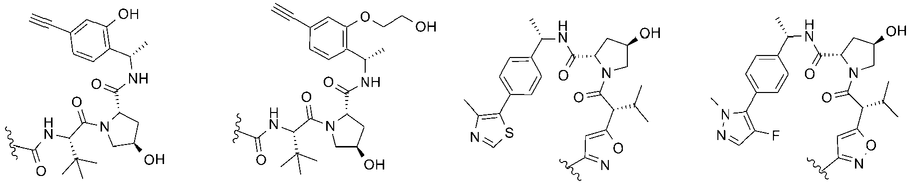

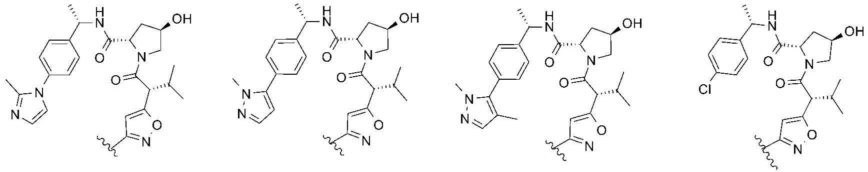

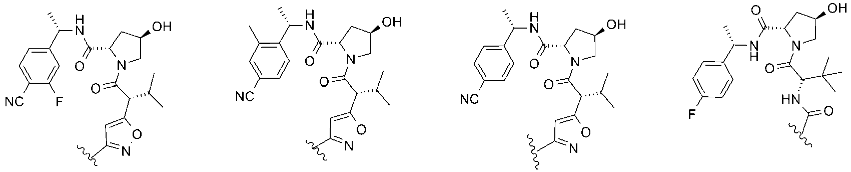

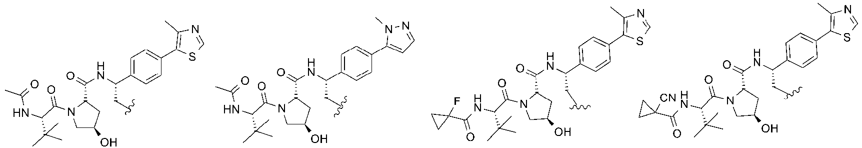

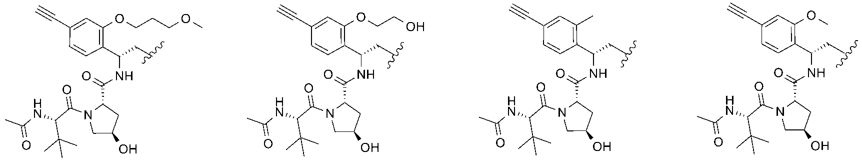

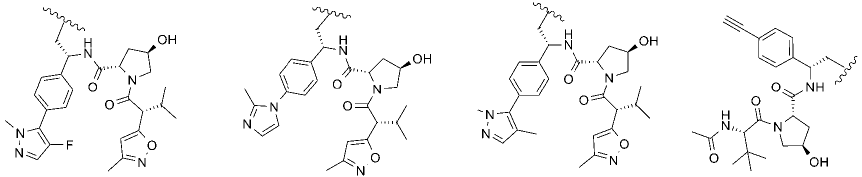

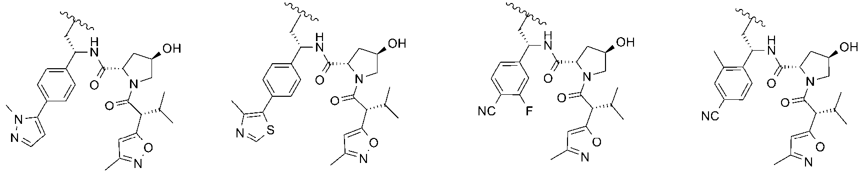

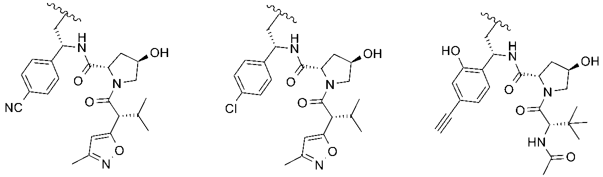

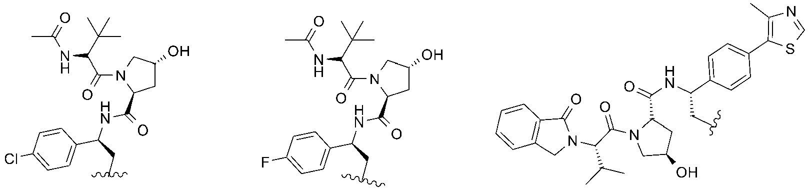

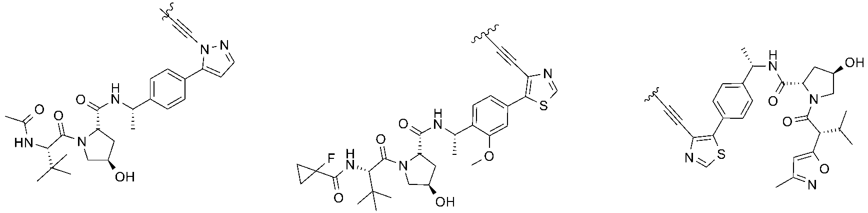

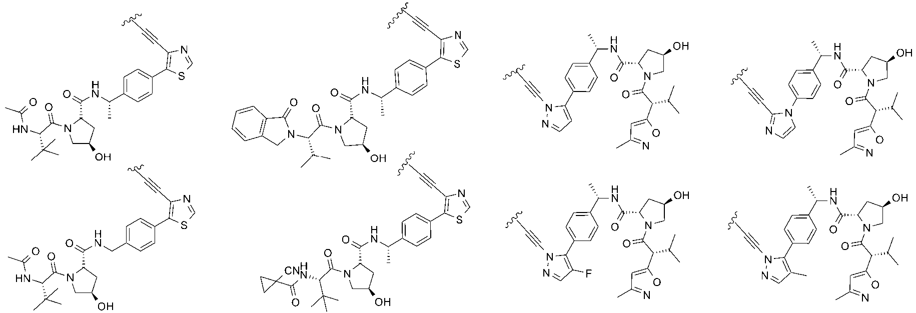

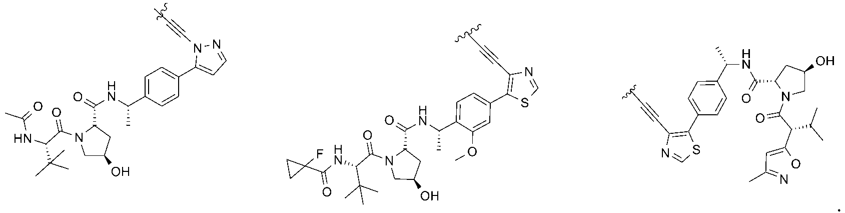

14. The compound or stereoisomers, tautomers or pharmaceutically acceptable salts thereof according to claim 1, wherein
X₈ and X₉ are each independently selected from CRₐ or N; Ra is selected from H, C₁-C₃ alkyl, -CH₂N(CH₃)₂, halogen, 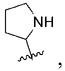 -NHCH₃, -O-C₁-C₃ alkyl; 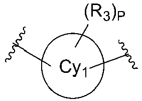 is selected from 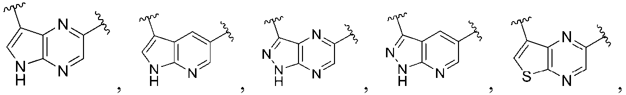 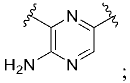
L₁ and L₂ are each independently selected from chemical bonds, O or -OCH₂-.

15. The compound or stereoisomers, tautomers or pharmaceutically acceptable salts thereof according to claim 1, wherein the compound is selected from

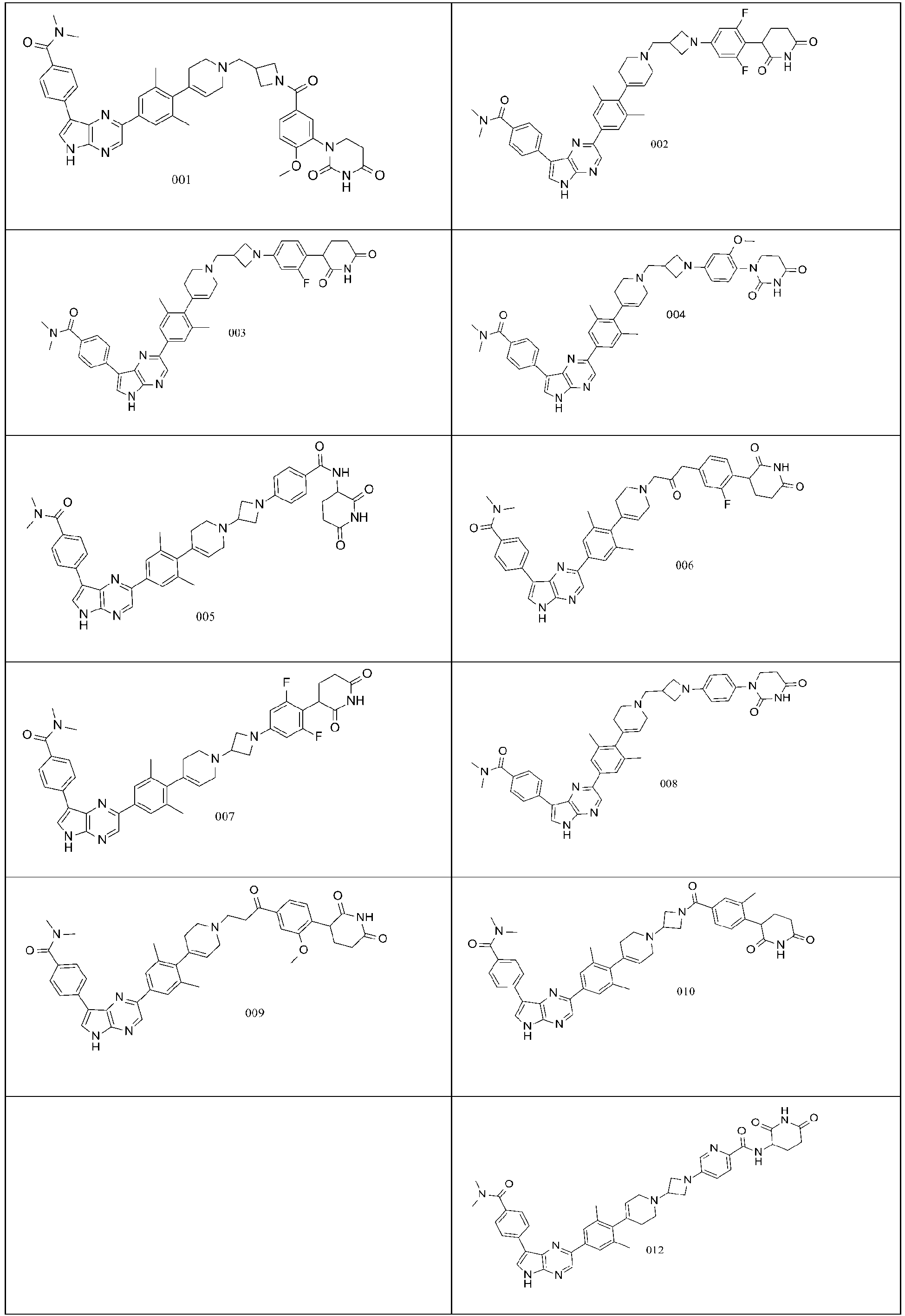

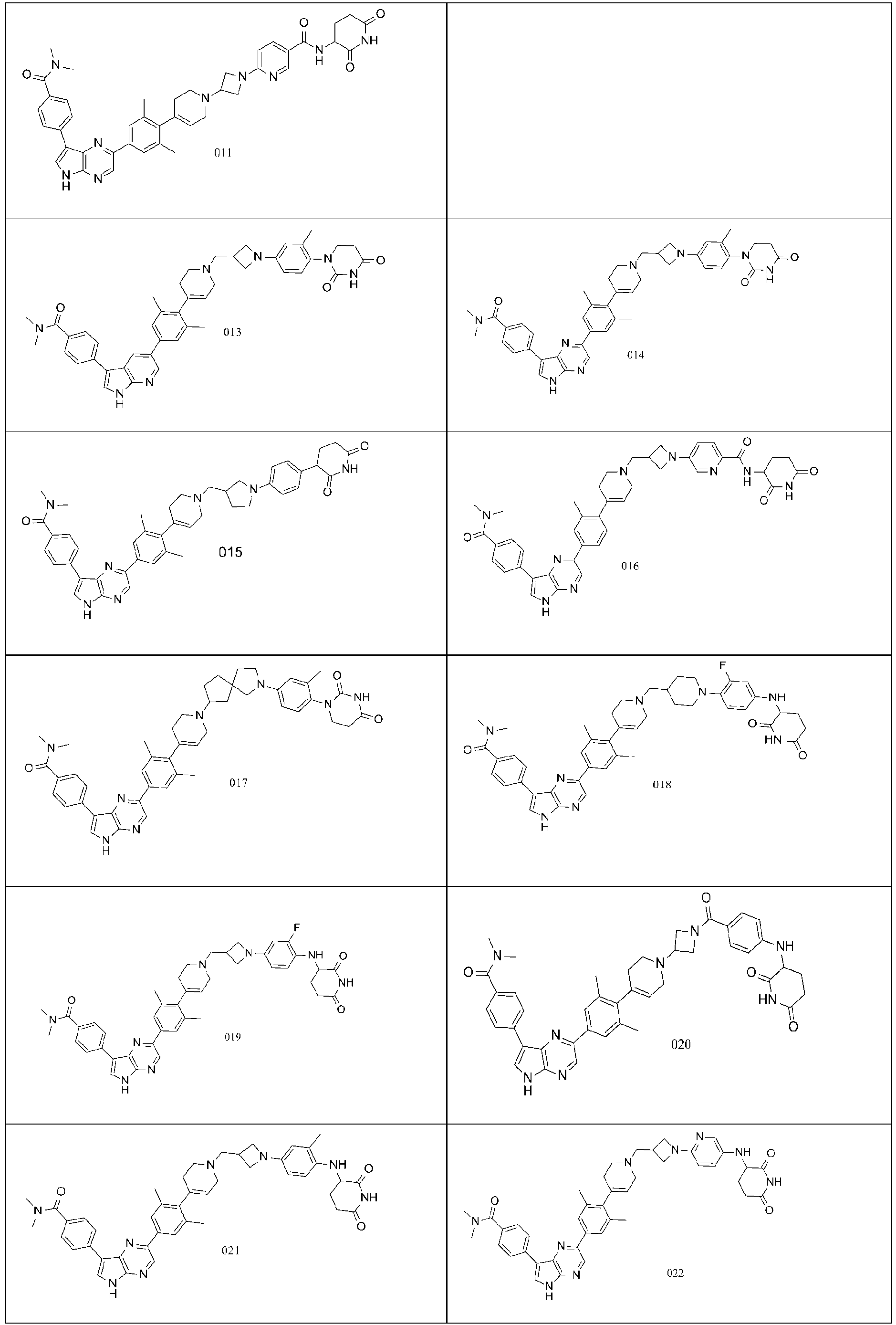

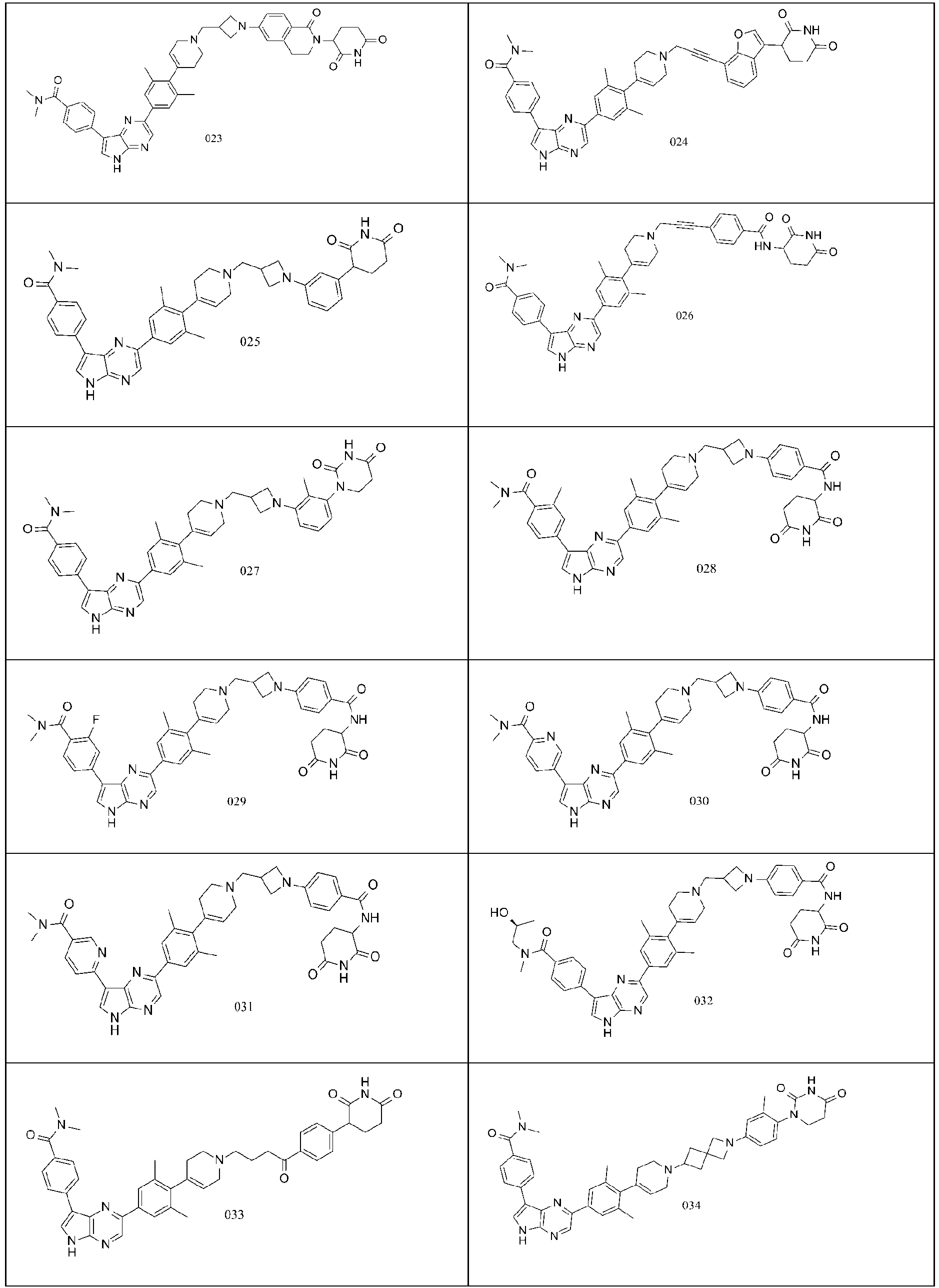

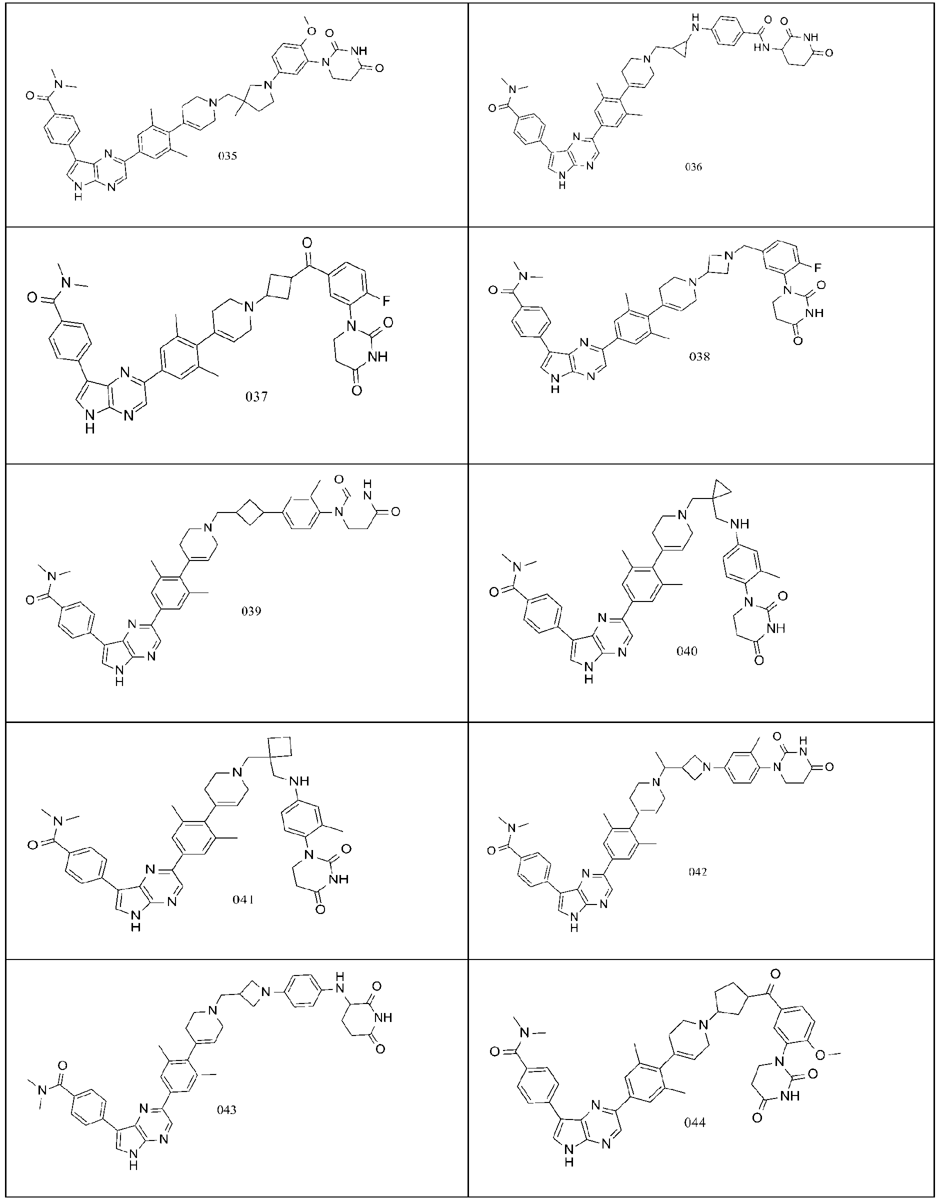

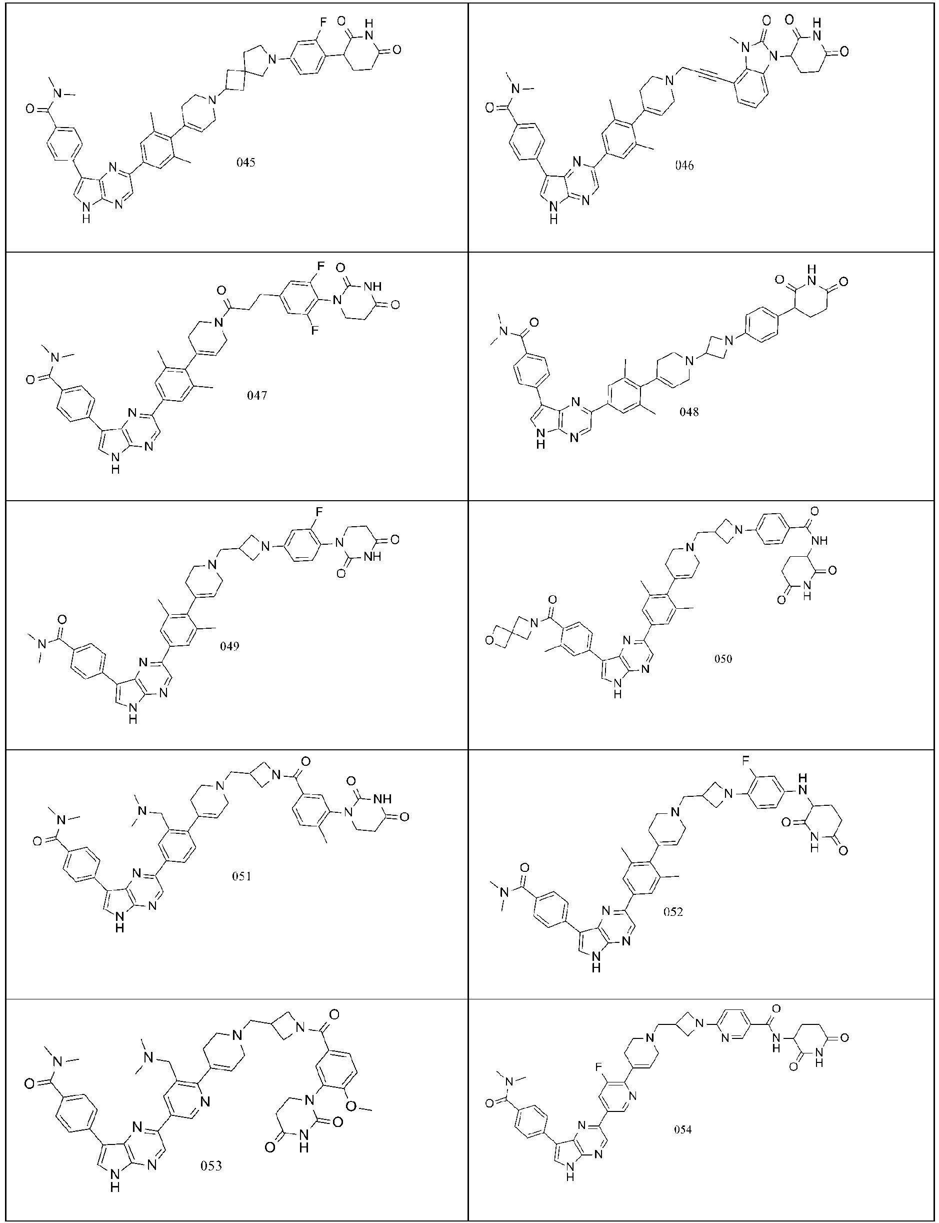

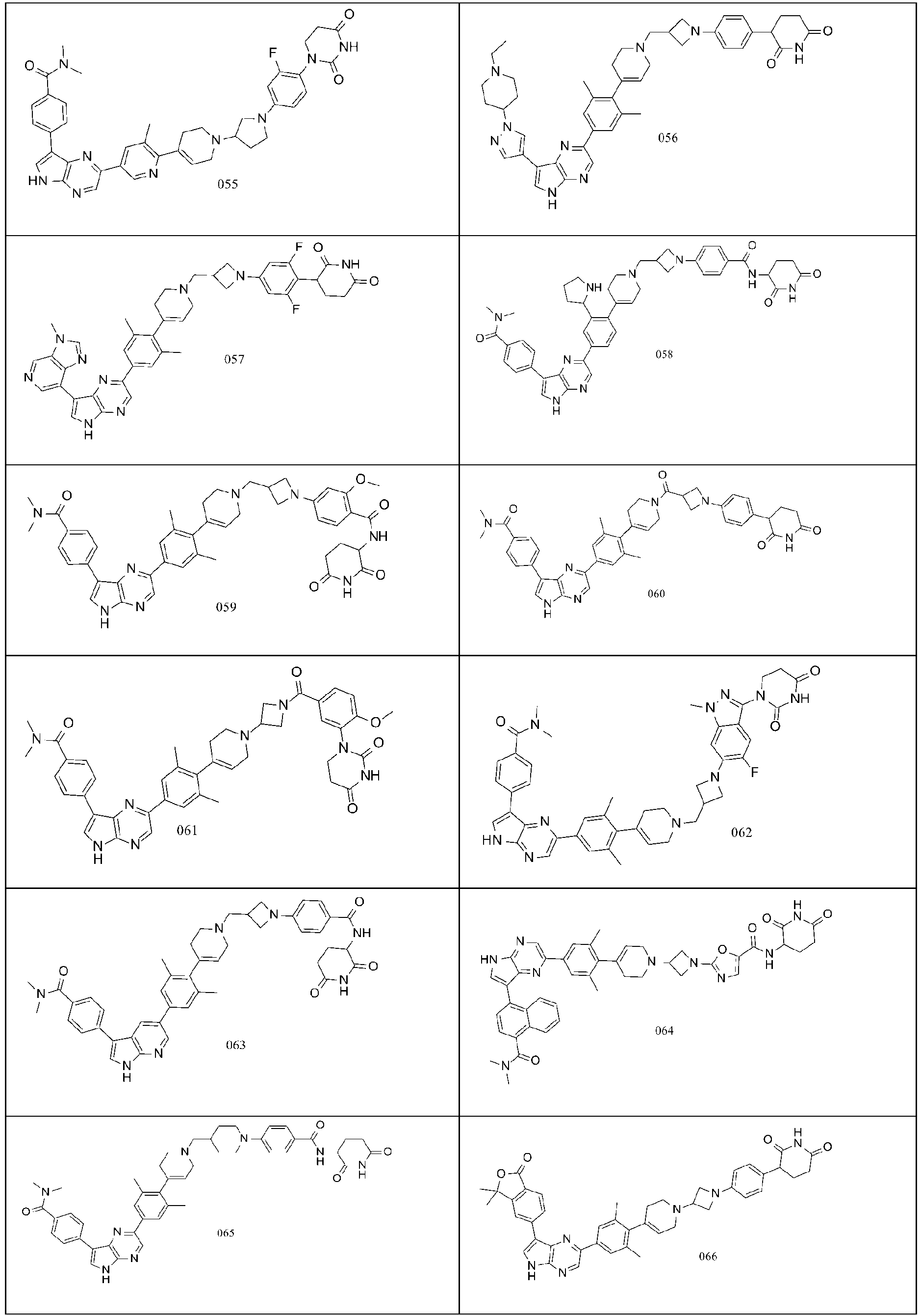

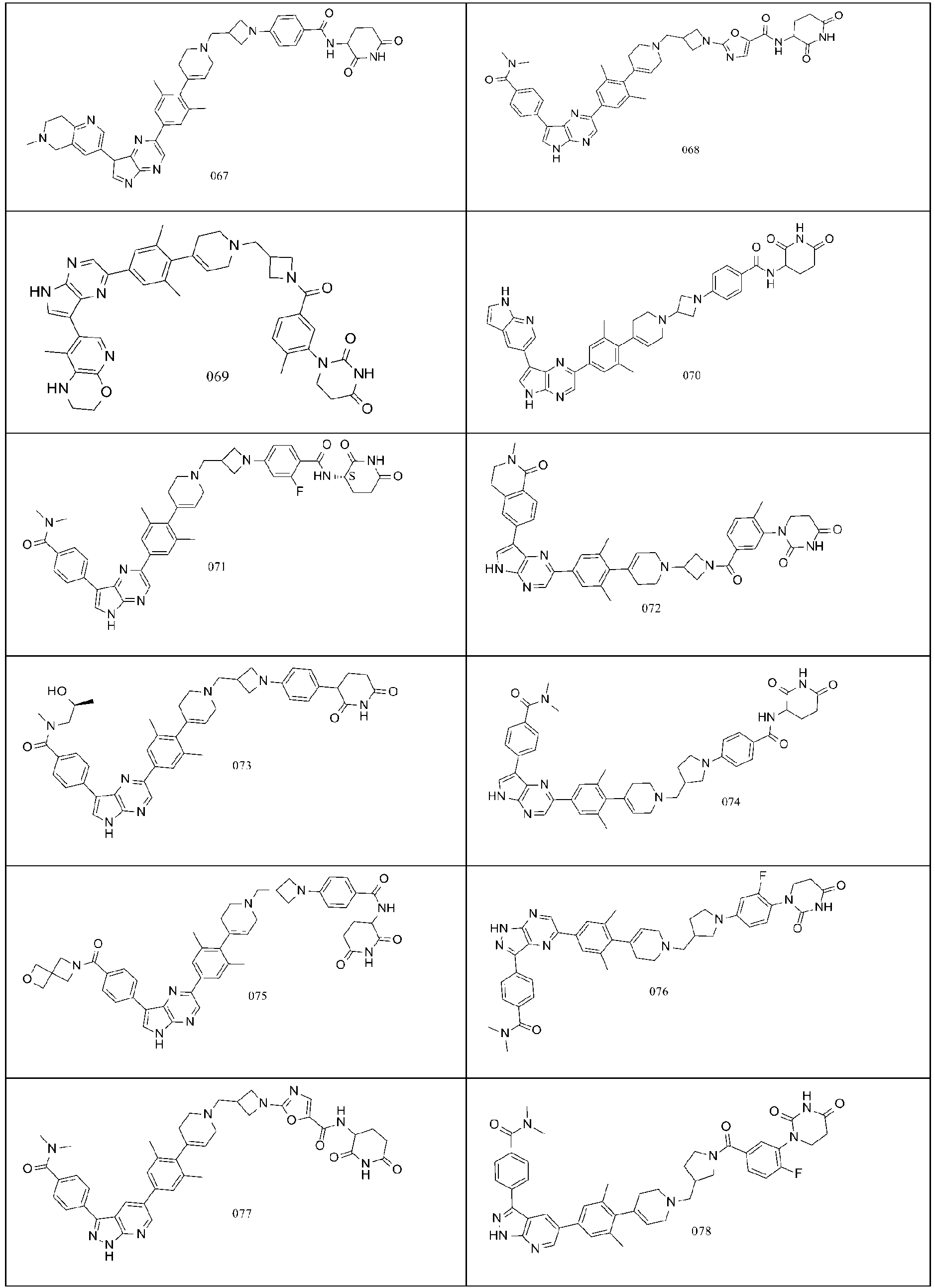

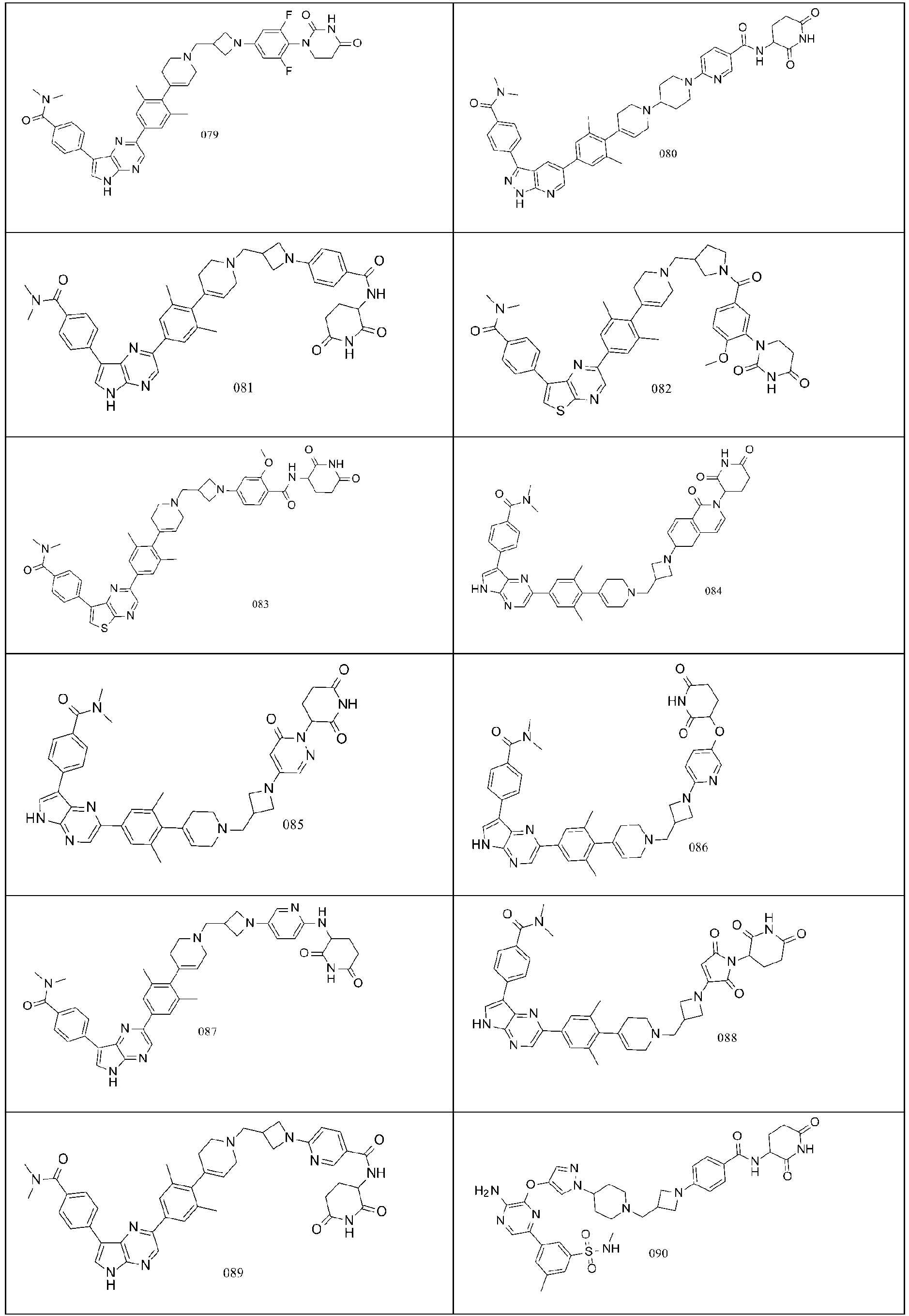

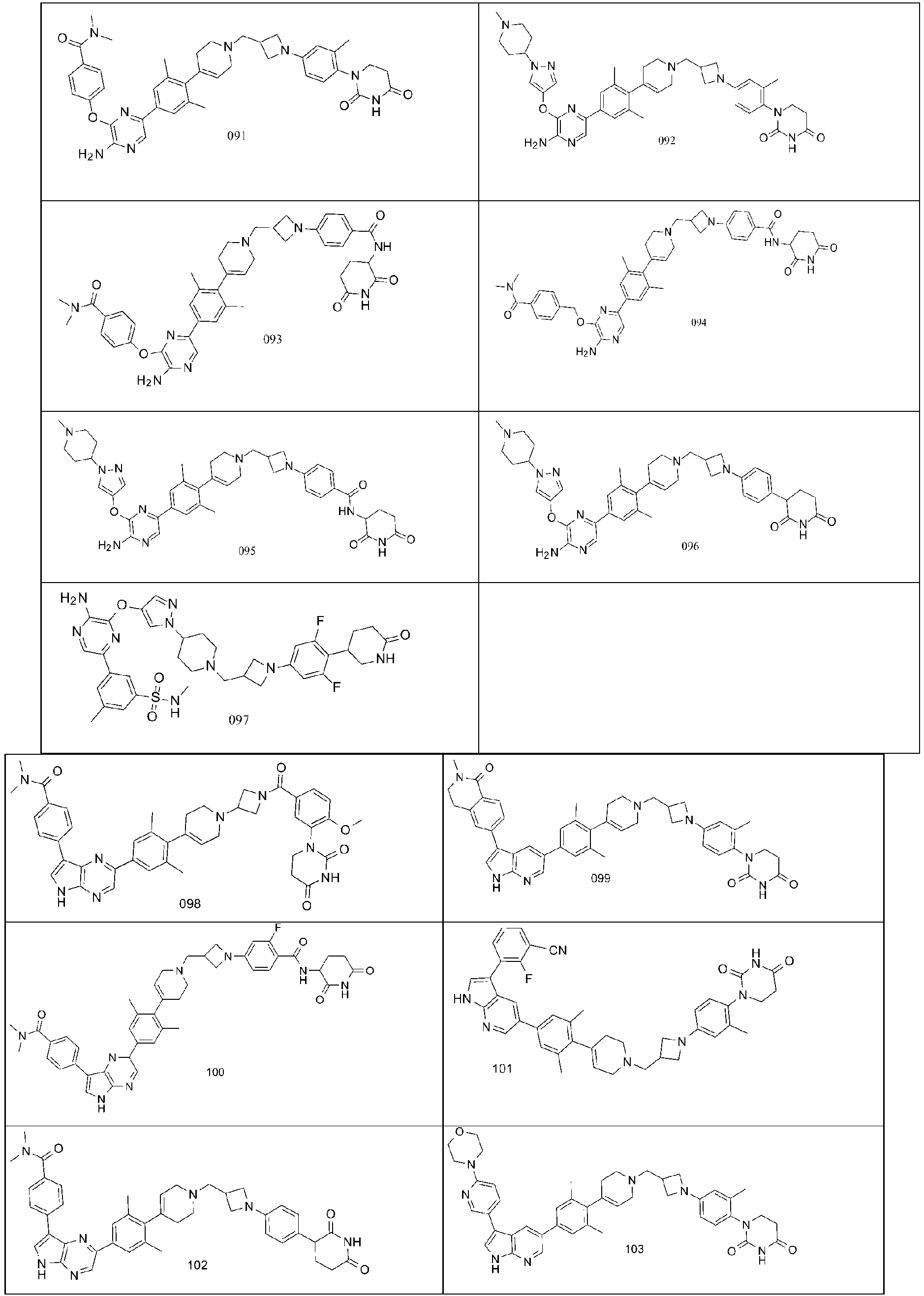

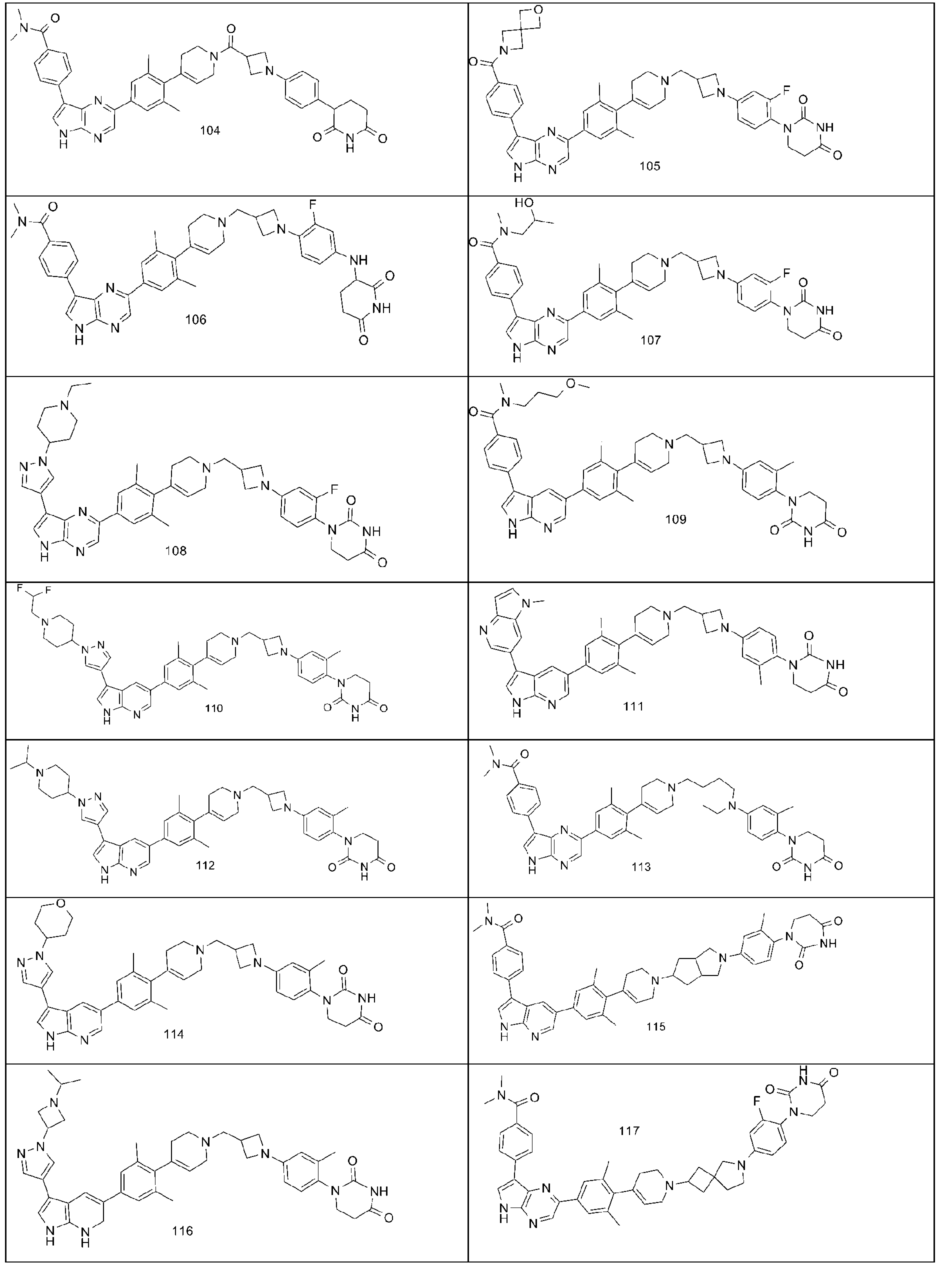

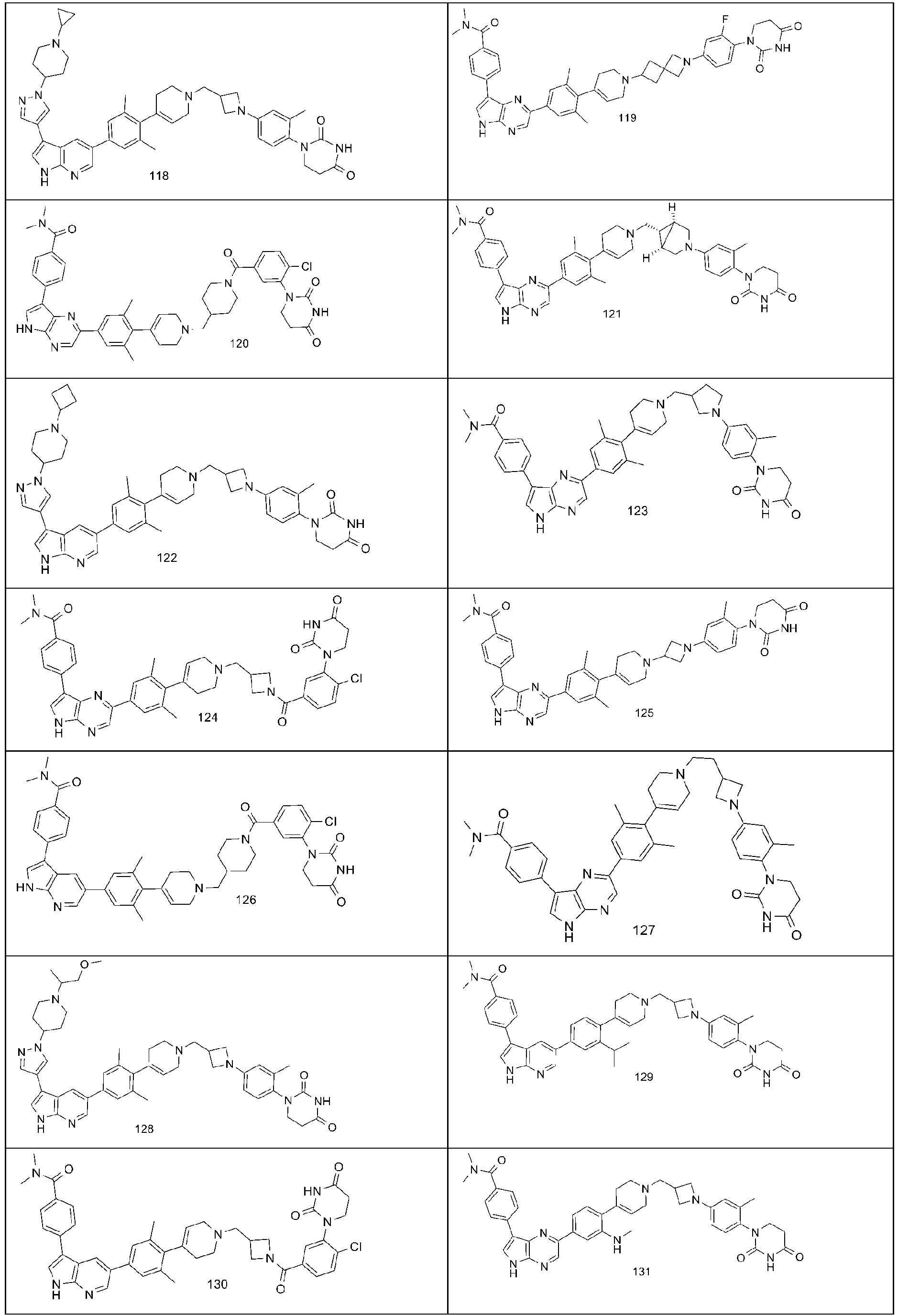

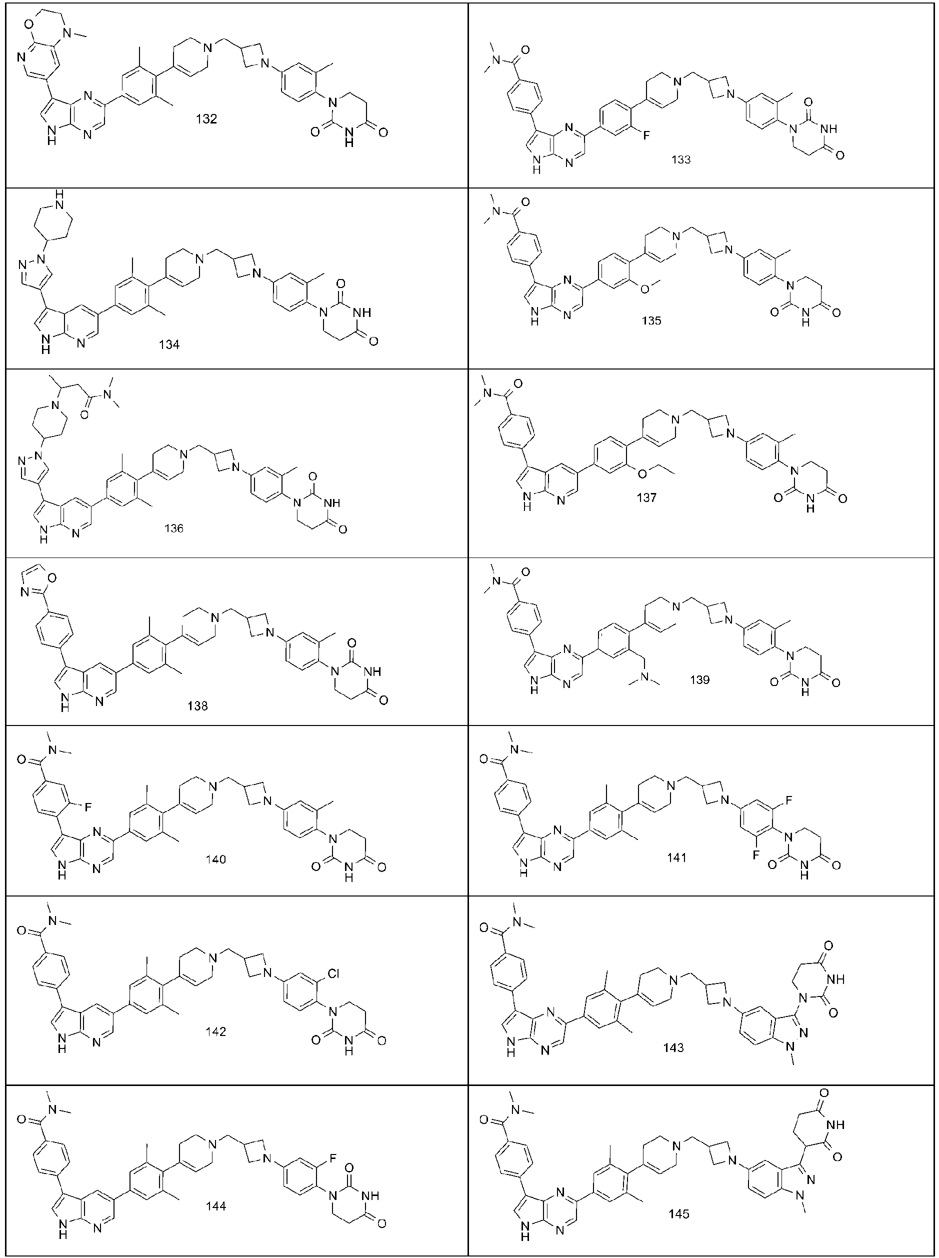

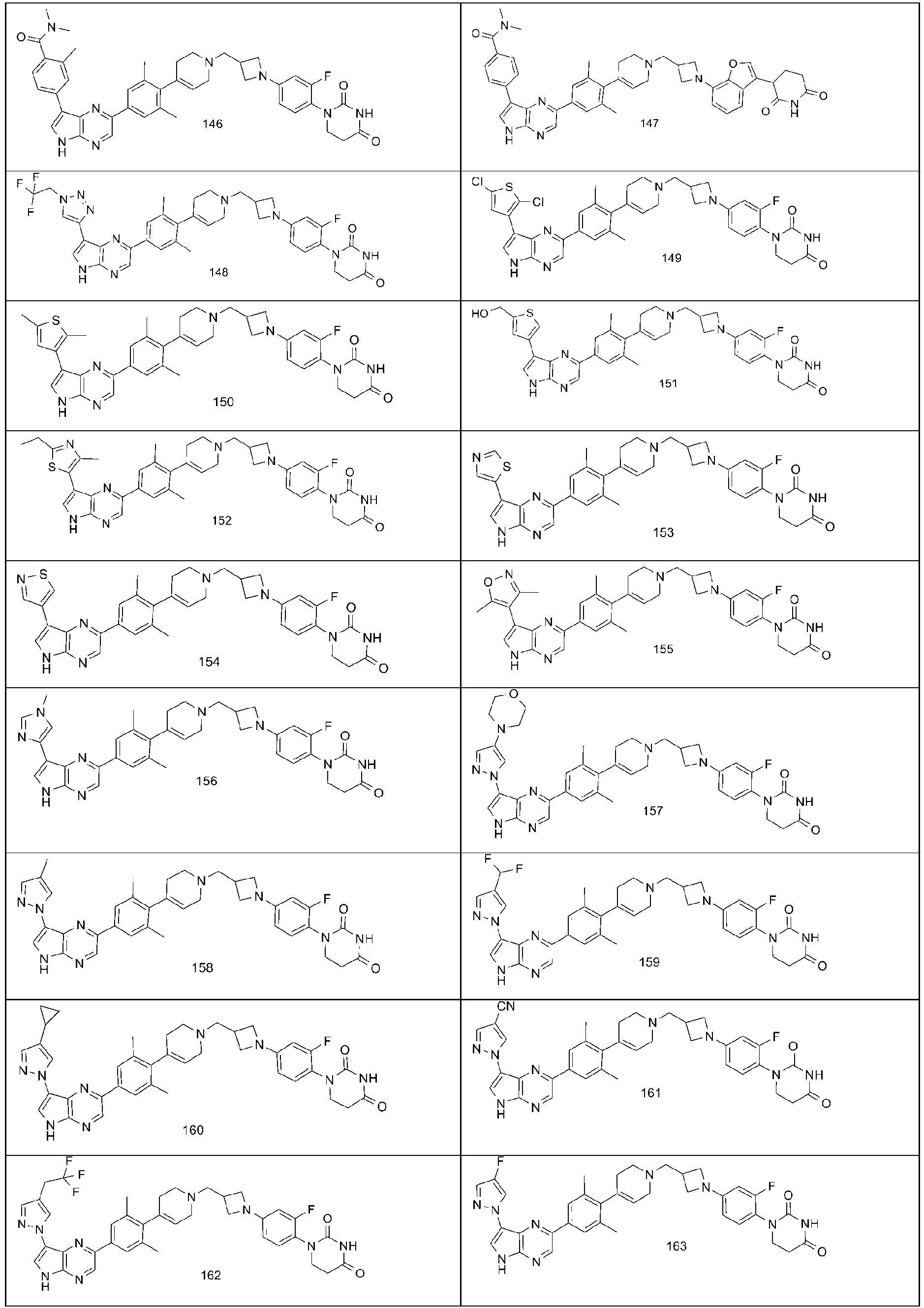

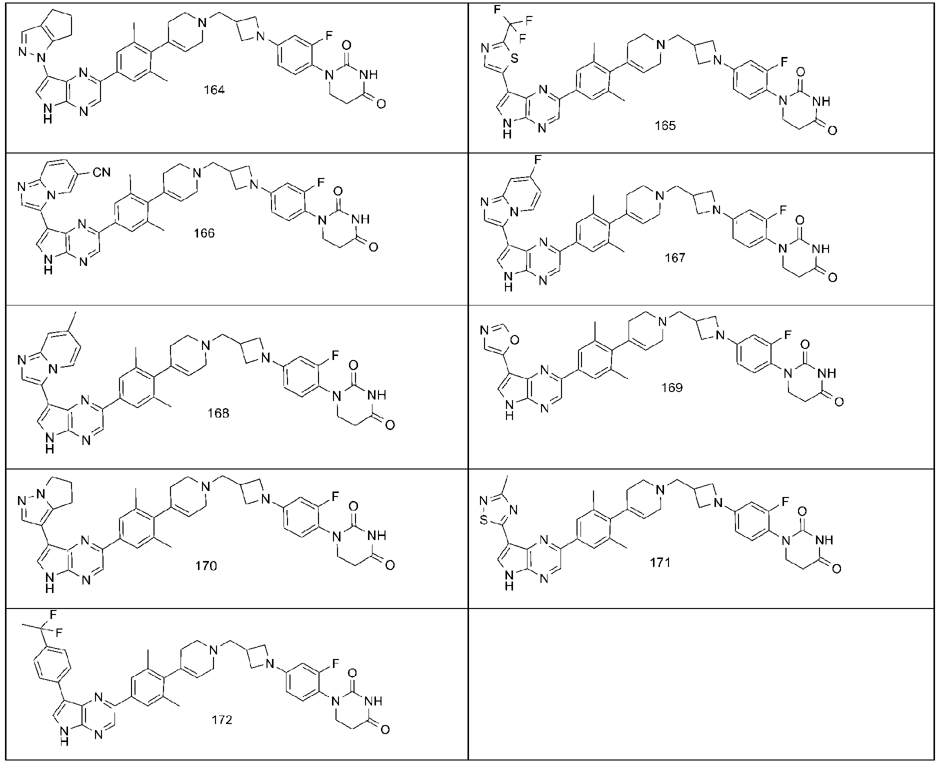

16. A pharmaceutical composition, comprising a therapeutically effective amount of the compound or stereoisomers, tautomers or pharmaceutically acceptable salts thereof according to any one of claims 1 to 15, and a pharmaceutically acceptable carrier or a combination thereof.

17. Use of the compound according to any one of claims 1 to 15, or a stereoisomer, tautomer or pharmaceutically acceptable salt thereof, or the pharmaceutical composition according to claim 16, in the manufacture of a medicament for degrading HPK1 protein.

18. The use according to claim 17, wherein, the medicament is for preventing and/or treating diseases associated with the activities or expression levels of HPK1 protein, wherein, the diseases comprise solid tumors, hematological disorders, or autoimmune diseases; preferably, the solid tumors are selected from one or more of lung cancer, squamous cell carcinoma, bladder cancer, gastric cancer, ovarian cancer, peritoneal cancer, breast cancer, mammary ductal carcinoma, head and neck cancer, endometrial cancer, uterine cancer, rectal cancer, liver cancer, kidney cancer, renal pelvis cancer, esophageal cancer, esophageal adenocarcinoma, glioma, prostate cancer, thyroid cancer, female reproductive system cancers, carcinoma in situ, lymphoma, neurofibromatosis, bone cancer, skin cancer, brain cancer, colon cancer, testicular cancer, gastrointestinal stromal tumor, oral cancer, pharyngeal cancer, colonic villonoma, melanoma, cell tumor, and sarcoma; preferably, the hematologic disorders comprise one or more of acute myeloid leukemia, acute lymphoblastic leukemia, chronic myeloid leukemia, myelofibrosis, myelodysplastic syndrome, diffuse large B-cell lymphoma, follicular lymphoma, chronic lymphocytic leukemia, small lymphocytic lymphoma, mantle cell lymphoma, Waldenström macroglobulinemia, multiple myeloma, and T-cell lymphoma; the autoimmune diseases are selected from one or more of systemic lupus erythematosus, rheumatoid arthritis, psoriasis, Graves' disease, Sjögren's syndrome, multiple sclerosis, and multiple sclerosis.
